# EUROPÄISCHE PATENTSCHRIFT

(11) **EP 1 177 194 B1**
(45) Veröffentlichungstag und Bekanntmachung des Hinweises auf die Patenterteilung: **17.09.2003**
(21) Anmeldenummer: 00927145.3
(22) Anmeldetag: 05.05.2000
(51) Int. Cl.: C07D 417/06, C07D 403/06, C07D 413/06, A01N 43/76, A01N 43/78, A01N 43/828, A01N 43/647

(54) **4-(3',4'-HETEROCYCLYLBENZOYL)PYRAZOLE ALS HERBIZIDE**
4-(3',4'-HETEROCYCLYL BENZOYL) PYRAZOLES AS HERBICIDAL AGENTS
4-(3',4'-HETEROCYCLYL BENZOYL) PYRAZOLES EN TANT QU'HERBICIDES

(30) Priorität: 07.05.1999 DE 19921240
(43) Veröffentlichungstag der Anmeldung: 06.02.2002
(73) Patentinhaber: BASF AKTIENGESELLSCHAFT, 67056 Ludwigshafen (DE)
(72) Erfinder: MAYER, Guido, D-67433 Neustadt (DE); BAUMANN, Ernst, D-67373 Dudenhofen (DE); DEYN, Wolfgang von, D-67435 Neustadt (DE); KUDIS, Steffen, D-68167 Mannheim (DE); LANGEMANN, Klaus, D-67551 Worms (DE); MISSLITZ, Ulf, D-67433 Neustadt (DE); NEIDLEIN, Ulf, D-68165 Mannheim (DE); WITSCHEL, Matthias, D-67061 Ludwigshafen (DE); RACK, Michael, D-69123 Heidelberg (DE); VOLK, Thorsten, D-68229 Mannheim (DE); OTTEN, Martina, D-67069 Ludwigshafen (DE); WESTPHALEN, Karl-Otto, D-67346 Speyer (DE); WALTER, Helmut, D-67283 Obrigheim (DE)
(74) Vertreter: Kinzebach, Werner, Dr.
(86) Internationale Anmeldenummer: EP0004040
(87) Internationale Veröffentlichungsnummer: WO00068228

(56) Entgegenhaltungen:
- EP-A- 0 822 187
- WO-A-96/05197
- WO-A-97/08164
- WO-A-97/09327

## Beschreibung

Die vorliegende Erfindung betrifft Pyrazolyl-Derivate benzokondensierter, ungesättigter 5-Ring-Stickstoffheterocyclen, Verfahren zur Herstellung derartiger Pyrazolyl-Derivate, Mittel, die derartige Verbindungen enthalten, sowie die Verwendung der Pyrazolyl-Derivate oder Mittel, die diese enthalten, zur Schadpflanzenbekämpfung.

Aus der WO 96/05197 sind Saccharin-Derivate mit herbizider Wirkung bekannt, die am Benzolkern des Saccharingerüstes mit einem (5-Hydroxy-pyrazol-4-yl)carbonyl-Rest substituiert sind. Die WO 97/30993 und die WO 97/09327 beschreiben Dioxothiochroman-Derivate und Dihydrobenzothiophen-Derivate mit herbizider Wirkung, die ebenfalls am Benzolkern der Schwefel-Heterocyclen einen (5-Hydroxypyrazol-4-yl)carbonyl-Rest aufweisen.

Aus der WO 97/08164 sind unter anderem benzkondensierte Derivate des γ-Butyrolactams mit herbizider Wirkung bekannt, die ebenfalls einen (5-Hydroxypyrazol-4-yl)carbonyl-Rest aufweisen.

Die herbiziden Eigenschaften der aus den genannten Druckschriften bekannten Verbindungen sowie deren Verträglichkeiten gegenüber Kulturpflanzen vermögen jedoch nur bedingt die Anforderungen an Herbizide zu befriedigen.

Die EP-A-822 187 beschreibt Herbizide auf der Basis arylsubstituierter Pyrazole der allgemeinen Formel 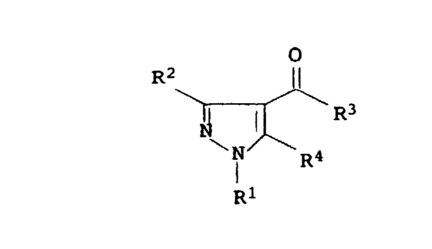 worin R¹ für Wasserstoff oder eine für ein Pestizid geeignete Schutzgruppe steht, R⁴ vorzugsweise Wasserstoff bedeutet und R² sowie R³ für Phenyl, Naphthyl oder heterocyclische Gruppen stehen, die gegebenenfalls substituiert sein können. R³ steht vorzugsweise für einen 5- oder 6-gliedrigen heterocyclischen Ring und insbesondere für eine Thiophengruppe. Die herbizide Wirkung der in dieser Schrift beschriebenen Verbindungen sowie ihre Verträglichkeit gegenüber Nutzpflanzen ist ebenfalls nicht zufriedenstellend.

Der vorliegenden Erfindung liegt somit die Aufgabe zugrunde neue Verbindungen mit herbizider Wirkung bereitzustellen, die vorzugsweise eine höhere Wirksamkeit als die herbiziden Substanzen des Standes der Technik und/oder eine bessere Selektivität gegenüber Schadpflanzen aufweisen.

Es wurde nun überraschenderweise gefunden, dass diese Aufgabe durch Pyrazolyl-Derivate benzokondensierter, ungesättigter 5-Ring-Stickstoffheterocyclen der nachstehend definierten, allgemeinen Formel I gelöst wird.

Demnach betrifft die vorliegende Erfindung Pyrazolyl-Derivate benzokondensierter, ungesättigter 5-Ring-Stickstoffheterocyclen der allgemeinen Formel I, 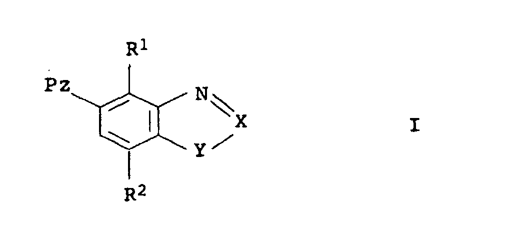 worin
- X: für N oder eine Gruppe C-R³ steht;
- Y: für O, S, SO, SO₂ oder NR⁴ steht;
oder
- X-Y: für S=N stehen, und X Schwefel bedeutet;
- R¹: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, C₁-C₆-Alkoxy-C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₁-C₆-Alkylamino-C₁-C₆-alkyl, oder Di-(C₁-C₆-alkyl)amino-C₁-C₆-alkyl;
- R²: Wasserstoff, Halogen oder C₁-C₆-Alkyl;
- R³: Wasserstoff, Halogen, Nitro, Cyano, Hydroxy, Amino, Mercapto, Rhodano, Hydrazid, C₁-C₆-Alkyl, C₁-C₆-Halogenalkyl, C₁-C₆-Hydroxyalkyl, C₁-C₆-Aminoalkyl, C₁-C₆-Alkoxy-C₁-C₆-alkyl, C₁-C₆-Alkylcarbonyl-C₁-C₆-alkyl, C₁-C₆-Alkoxy, C₁-C₆-Halogenalkoxy, C₁-C₆-Hydroxyalkoxy, C₁-C₆-Alkoxy-C₁-C₆-alkoxy, C₃-C₆-Cycloalkyl,
C₁-C₆-Alkylamino, Di-C₁-C₆-alkylamino, C₃-C₆-Cycloalkylamino, wobei die Alkyl- und Cycloalkylgruppen der drei letztgenannten Reste teilweise oder vollständig halogeniert und/oder ein, zwei oder drei Substituenten, ausgewählt unter C₁-C₄-Alkoxy oder Hydroxy tragen können,
C₁-C₆-Alkylthio, C₁-C₆-Halogenalkylthio, C₁-C₆-Hydroxyalkylthio, C₁-C₆-Alkoxy-C₁-C₆-alkylthio, C₁-C₆-Alkylsulfinyl, C₁-C₆-Alkylsulfonyl,
Phenyl, Naphthyl, Heterocyclyl, Phenylamino, Phenoxy, Diphenylamino, wobei die Phenyl- und Heterocyclylgruppen der sechs letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können,
C(O)OR⁵, oder C(O)N(R⁶)R⁷;
- R⁴: Wasserstoff, C₁-C₆-Alkyl, C₁-C₆-Halogenalkyl, C₁-C₆-Hydroxyalkyl, C₁-C₆-Alkoxy-C₁-C₆-alkyl,
Phenyl, Naphthyl, wobei die zwei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können; bedeuten, wobei
- R⁵: für Wasserstoff, C₁-C₆-Alkyl, C₁-C₆-Halogenalkyl, C₁-C₆-Hydroxyalkyl, C₁-C₆-Alkoxy-C₁-C₆-alkyl,
Phenyl, Naphthyl oder Heterocyclyl steht, wobei die drei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können; und
- R⁶, R⁷: unabhängig voneinander für Wasserstoff, C₁-C₆-Alkyl, C₁-C₆-Halogenalkyl, C₁-C₆-Hydroxyalkyl, C₁-C₆-Alkoxy-C₁-C₆-alkyl,
Phenyl oder Naphthyl stehen, wobei die zwei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können;
und Pz für einen Rest der Formel IIa oder IIb steht, 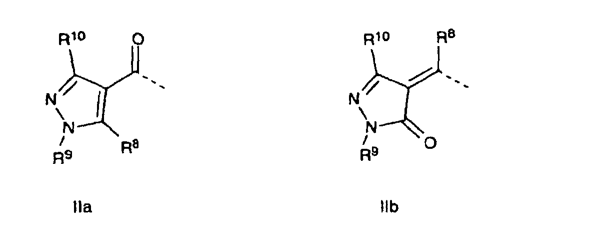 worin die Variablen R⁸, R⁹ und R¹⁰ folgende Bedeutung haben:
- R⁸: Hydroxy, Mercapto, Halogen, OR¹¹, SR¹¹, SOR¹², SO₂R¹², OSO₂R¹², P(O)R¹³R¹⁴, OP(O)R¹³R¹⁴, P(S)R¹³R¹⁴, OP(S)R¹³R¹⁴, NR¹⁵R¹⁶, ONR¹⁵R¹⁶ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁹: Wasserstoff, C₁-C₆-Alkyl, C₃-C₆-Cycloalkyl, C₁-C₆-Halogenalkyl, Hydroxy, C₁-C₆-Alkoxy oder C₁-C₆-Halogenalkoxy;
- R¹⁰: Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆-Halogenalkylthio;
- R¹¹: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl oder C₁-C₆-Alkoxyimino-C₁-C₆-alkyl, wobei die genannten Alkyl-, Cycloalkyl- und Alkoxyreste partiell oder vollständig halogeniert sein können und/oder eine, zwei oder drei der folgenden Gruppen tragen können: Cyano, C₁-C₄-Alkoxy, C₁-C₄-Alkylthio, Di- C₁-C₄-alkyl-amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Phenyl-C₁-C₆-alkyl, Phenylcarbonyl-C₁-C₆-alkyl, Phenylcarbonyl, Phenoxycarbonyl, Phenyloxythiocarbonyl, Phenylaminocarbonyl, N-(C₁-C₆-Alkyl)-N-(phenyl)-aminocarbonyl, Phenyl-C₂-C₆-alkenylcarbonyl, Heterocyclyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, Heterocyclylaminocarbonyl, N-(C₁-C₆-Alkyl)-N-(heterocyclyl)-aminocarbonyl, oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R¹²: C₁-C₆-Alkyl, C₃-C₆-Alkenyl, C₃-C₆-Alkinyl oder C₃-C₆-Cycloalkyl, wobei die vier genannten Reste partiell oder vollständig halogeniert sein können und/oder eine, zwei oder drei der folgenden Gruppen tragen können: Cyano, C₁-C₄-Alkoxy, C₁-C₄-Halogenalkoxy, C₁-C₄-Alkylthio, C₁-C₄-Halogenalkylthio, C₁-C₄-Alkylcarbonyl, C₁-C₄-Alkoxycarbonyl oder C₁-C₄-Halogenalkoxycarbonyl;
Phenyl, Phenyl-C₁-C₆-alkyl, Heterocyclyl oder Heterocyclyl-C₁-C₆-alkyl, wobei der Phenyl- und der Heterocyclyl-Rest der letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
- R¹³, R¹⁴: unabhängig voneinander Wasserstoff, Hydroxy, C₁-C₆-Alkyl, C₁-C₆-Alkoxy, C₁-C₆-Alkylthio, Phenyl, Phenyl-C₁-C₄-alkyl oder Phenoxy, wobei die drei letztgenannten Substituenten partiell oder vollständig halogeniert sein können und/oder einen, zwei oder drei der folgenden Reste tragen können: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
- R¹⁵: Wasserstoff, C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-alkyl)-amino oder C₁-C₆-Alkylcarbonylamino, wobei die genannten Alkyl-, Cycloalkyl- und Alkoxyreste partiell oder vollständig halogeniert sein können und/oder einen, zwei oder drei Reste der folgenden Gruppe tragen können: Cyano, C₁-C₄-Alkoxycarbonyl, C₁-C₄-Alkylaminocarbonyl, Di-(C₁-C₄-alkyl)-aminocarbonyl oder C₃-C₆-Cycloalkyl;
Phenyl, Phenyl-C₁-C₄-alkyl, Phenylcarbonyl, Heterocyclyl, Heterocyclyl-C₁-C₄-alkyl oder Heterocyclylcarbonyl, wobei der Phenyl- oder Heterocyclyl-Rest der sechs letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; und
- R¹⁶: Wasserstoff, C₁-C₆-Alkyl oder C₃-C₆-Alkenyl, C₃-C₆-Alkinyl; bedeutet,
sowie deren landwirtschaftlichen brauchbaren Salze.

Ferner wurden herbizide Mittel gefunden, die Pyrazolyl-Derivate der Formel I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Pyrazolylderivaten der Formel I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy- C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Im Falle von R⁸ = Hydroxy oder Mercapto {Z = O,S} steht IIa auch stellvertretend für die tautomeren Formen IIa' und IIa'' 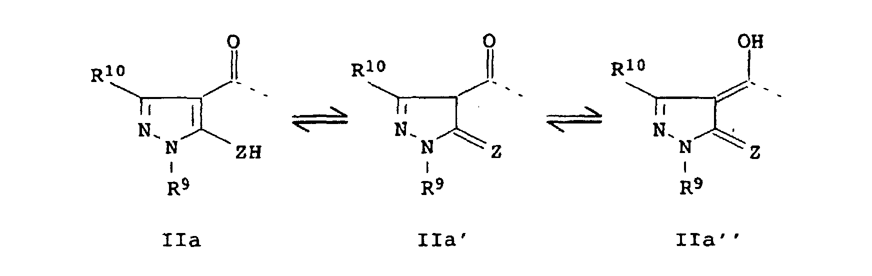 bzw. IIb auch stellvertretend für die tautomeren Formen IIb' und IIb''.

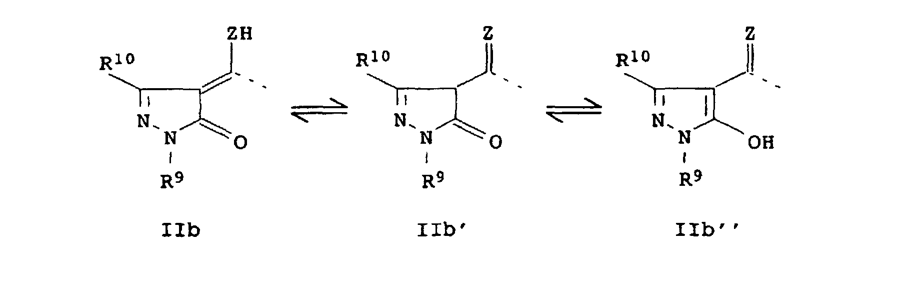

Die für die Substituenten R¹ bis R¹⁶ oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylamino-, N,N-Dialkylamino-, N-Halogenalkylamino-, N-Alkoxyamino-, N-Alkoxy-N-alkylamino-, N-Alkylcarbonylamino-, Alkylcarbonyl-, Halogenalkylcarbonyl, Alkoxycarbonyl-, Halogenalkoxycarbonyl, Alkylthiocarbonyl-, Alkylcarbonyloxy-, Alkylaminocarbonyl-, Dialkylaminocarbonyl-, Dialkylaminothiocarbonyl-, Alkoxyalkyl-, Alkoxyiminoalkyl-, Phenylalkylcarbonyl, Heterocyclylalkylcarbonyl, Phenylalkenylcarbonyl-, Heterocyclylalkenylcarbonyl-, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Alkoxyalkoxycarbonyl-, Alkenylcarbonyl-, Alkenyloxycarbonyl-, Alkenylaminocarbonyl-, N-Alkenyl-N-alkylaminocarbonyl-N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxy-, Alkinyloxy, Alkandiyl-, Alkendiyl-, Alkadiendiyl- oder Alkindiyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: z.B. Methyl, Ethyl, Propyl, 1-Methylethyl, Butyl, 1-Methylpropyl, 2-Methylpropyl oder 1,1-Dimethylethy
- C₁-C₆-Alkyl, sowie die Alkylteile von C₁-C₆-Alkylamino, Di-(C₁-C₆-alkyl)amino, N-(C₁-C₆-Alkoxy)-N-(C₁-C₆-alkyl)amino, N(C₁-C₆-Alkoxy)-N-(C₁-C₆-alkyl)-aminocarbonyl, N-(C₃-C₆ Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkyl)-N-phenylaminocarbonyl, N-(C₁-C₆-Alkyl)-N-heterocyclylaminocarbonyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 2,1-Dimethylpropyl, 1,2-Dimethylpropyl, 1-Methylpentyl, 2-Methylpentyl, 3-Methylpentyl, 4-Methylpentyl, 1, 1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl, sowie die Halogenalkylteile von N-C₁-C₆-Halogenalkylamino: C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-lodhexyl oder Dodecafluorhexyl;
- C₁-C₄-Alkoxy: z.B. Methoxy, Ethoxy, Propoxy, 1-Methylethoxy, Butoxy, 1-Methylpropoxy, 2-Methylpropoxy oder 1, 1-Dimethylethoxy;
- C₁-C₆-Alkoxy, sowie die Alkoxyteile von N-C₁-C₆-Alkoxyamino, N-(C₁-C₆-Alkoxy)-N-(C₁-C₆-alkyl)amino, C₁-C₆-Alkoxymino-C₁-C₆-alkyl, N-(C₁-C₆-Alkoxy)-N-(C₁-C₆-alkyl)-aminocarbonyl, N-(C₃-C₆-Alkenyl-N-(C₁-C₆-alkoxy)-aminocarbonyl und N-(C₃-C₆-Alkinyl)-N-(C₁-C₆-alkoxy)-aminocarbonyl : C₁-C₄-Alkoxy, wie voranstehend genannt, sowie z.B. Pentoxy, 1-Methylbutoxy, 2-Methylbutoxy, 3-Methylbutoxy, 1, 1-Dimethylpropoxy, 1, 2-Dixnethylpropoxy, 2, 2-Dimethylpropoxy, 1-Ethylpropoxy, Hexoxy, 1-Methylpentoxy, 2-Methylpentoxy, 3-Methylpentoxy, 4-Methylpentoxy, 1, 1-Dimethylbutoxy, 1, 2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Di-chlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy : C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy ;
- C₁-C₄-Alkylthio (C₁-C₄-Alkylsulfanyl: C₁-C₄-Alkyl-S-): z.B. Methylthio, Ethylthio, Propylthio, 1-Methylethylthio, Butylthio, 1-Methylpropylthio, 2-Methylpropylthio oder 1, 1-Dimethylethylthio;
- C₁-C₆-Alkylthio, sowie die Alkylthioteile von C₁-C₆ -Alkylthiocarbonyl: C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1, 1-Dimethylpropylthio, 1,2-Dimethylpropylthio, 1-Methylpentylthio, 2-Methylpentylthio, 3-Methylpentylthio, 4-Methylpentylthio, 1, 1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1, 2-Trimethylpropylthio, 1,2,2-Trimethylpropylthio, 1-Ethyl-1-methylpropylthio oder 1-Ethyl-2-methylpropylthio;
- C₁-C₄-Halogenalkylthio : einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2₋Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Tri-chlorpropylthio, 2,2,3,3,3-Pentafluorpropylthio, Heptafluorpropylthio, 1-( Fluormethyl )-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₆-Halogenalkylthio: C₁ -C₄-Halogenalkylthio, wie vorstehend genannt, sowie 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁- C₄ -Alkylsulfinyl (C₁-C₄-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₆-Alkylsulfinyl: C₁-C₄-Alkylsulfinyl, wie vorstehend genannt, sowie Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-nethylpropylsulfinyl;
- C₁-C₄-Halogenalkylsulfinyl: C₁-C₄-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, l-(Brommethyl)-2-bromethylsulfinyl, 4-Fluorbutylsulfinyl, 4-Chlorbutylsulfinyl, 4-Brombutylsulfinyl oder Nonafluorbutylsulfinyl;
- C₁-C₆-Halogenalkylsulfinyl: C₁-C₄-Halogenalkylsulfinyl, wie vorstehend genannt, sowie 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₄-Alkylsulfonyl (C₁-C₄-Alkyl-S(=O)₂-) z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl;
- C₁-C₆-Alkylsulfonyl: C₁-C₄-Alkylsulfonyl, wie vorstehend genannt, sowie Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₄-Halogenalkylsulfonyl einen C₁-C₄-Alkylsulfonylrest, wie voranstehend genannt, der partiell oder vollständig durch Fluor, Chlor, Brom und/oder Iod substituiert ist, also 2.B-Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl;
- C₁-C₆-Halogenalkysulfonyl: C₁-C₄-Halogenalkylsulfonyl, wie vorstehend genannt, sowie 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino: Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di-(C₁-C₄-alkyl)amino, also z.B. N,N-Dimethylamino, N,N-Di-ethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl) amino, N-Methyl-N-(2-methylpropyl) amino, N-(1,1-Di-methylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl) amino, N-Ethyl-N-(1,1-dimethylethyl)amino, N-(1-Methylethyl)-N-propylamino, N-Butyl-N-propylamino, N- (1-Methylpropyl)-N-propylamino, N-(2-Methylpropyl)-N-propylamino, N- (1,1-Dimethylethyl )-N-propylarnino, N-Butyl-N-(1-methylethyl)amino, N-(1-Methylethyl)-N-(1-methylpropyl) amino, N- (1-Methylethyl )-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl) amino;
- Di-(C₁-C₆-alkyl)amino: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonyl-C₁-C₆-alkyl, C₁-C₆-Alkylcarbonylamino: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₁-C₄-Halogenalkylcarbonyl: einen C₁-C₄-Alkylcarbonylrest, wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlor-fluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Di-fluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl )-2-chlorethylcarbonyl, 1-(Brommethyl)-2-bromethylcarbonyl, 4-Fluorbutylcarbonyl, 4-Chlorbutylcarbonyl, 4-Brombutylcarbonyl oder Nonafluorbutylcarbonyl;
- C₁-C₆-Halogenalkylcarbonyl: einen C₁-C₄-Halogenalkylcarbonylrest wie voranstehend genannt, sowie 5-Fluorpentylcarbonyl, 5-Chlorpentylcarbonyl, 5-Brompentylcarbonyl, Perfluorpentylcarbonyl, 6-Fluorhexylcarbonyl, 6-Chlorhexylcarbonyl, 6-Bromhexylcarbonyl oder Perfluorhexylcarbonyl;
- C₁-C₄-Alkoxycarbonyl also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₆-Alkoxycarbonyl: C₁-C₄-Alkoxycarbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-alogenalkoxycarbonyl: einen C₁-C₄-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-( Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl oder 4-Iodbutoxycarbonyl;
- C₁-C₆-Halogenoxycarbonyl; einen C₁-C₄-Halogenoxycarbonylrest wie voranstehend genannt, sowie 5-Fluorpentoxycarbonyl, 5-Chlorpentoxycarbonyl, 5-Brompentoxycarbonyl, 6-Fluorhexoxycarbonyl, 6-Chlorhexoxycarbonyl oder 6-Bromhexoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄ Alkylamino)carbonyl, wie vorstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di- (C₁-C₄-alkyl)-aminocarbonyl: z.B. N,N-Dimethylaminocarbonyl, N,N-Diethylaminocarbonyl, N,N-Di-(1-methylethyl) aminocarbonyl, N,N-Dipropylaminocarbonyl, N,N-Dibutylaminocarbonyl, N, N-Di- ( 1-methylpropyl )-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N- (1-Methylethyl ) -N- (2 -methylpropyl )-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N- (1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-dimetylpropyl)-N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-( 2-methylpentyl )-aminocarbonyl, N-Methyl-N-(3-methylpentyl)-aminocarbonyl, N-Methyl-H-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl )-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl )-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N, N-Di-(1-methylethyl) aminothiocarbonyl, N, N-Dipropylaminothiocarbonyl, N, N-Dibutylaminothiocarbonyl, N,N-Di-(1-methylpropyl)-aminothiocarbonyl, N,N-Di- (2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N- (1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2 methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N- (1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-ethyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aniinothiocarbonyl, N-Methyl-N-(2-methylpentyl)-aminothiocarbonyl, N-ethyl-N-(3-methylpentyl)-aminothiocarbonyl, N-Methyl-N-N-methylpentyl )-aminothiocarbonyl, N-ethyl-N-(1,1-dimethylbutyl)-aminothiocarbonyl1 N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-(2,3-dimethylbutyl)-aminothiocarbonyl, N-Methyl-N-(3,3-dimethylbutyl )-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₆-Hydroxyalkyl: durch ein bis drei OH-Gruppen substituiertes C₁-C₆-Alkyl, z.B Hydroxymethyl, 1-Hydroxyethyl, 2-Hydroxyethyl, 1,2-Bishydroxyethyl, 1-Hydroxypropyl, 2-Hydroxypropyl, 3-Hydroxypropyl, 4-Hydroxybutyl, 2,2-Dimethyl-3-hydroxypropyl;
- Phenyl-C₁-C₆-alkyl: durch einen Phenylrest substituiertes C₁-C₆-Alkyl, z.B. Benzyl, 1-Phenylethyl und 2-Phenylethyl, wobei der Phenylrest in der angegebenen Weise teilweise oder vollständig halogeniert sein kann oder einen bis drei der für Phenyl oben angegebenen Substituenten aufweisen kann; Heterocyclyl-C₁-C₆-alkyl steht dementsprechend für ein durch einen Heterocyclylrest substituiertes C₁-C₆-Alkyl;
- C₁-C₆-Alkoxy-C₁-C₆-alkyl: durch C₁-C₆-Alkoxy, wie vorstehend genannt, substituiertes C₁-C₆-Alkyl, also z.B. Methoxymethyl, Ethoxymethyl, Propoxymethyl, (1-Methylethoxy)methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Prop-oxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₆-Alkoxy-C₁-C₆-alkoxy, sowie die Alkoxyalkoxyteile von C₁-C₆-Alkoxy-C₁-C₆-alkoxycarbonyl durch C₁-C₆-Alkoxy, wie vorstehend genannt, substituiertes C₁-C₆-Alkoxy, also z.B. für Methoxymethoxy, Ethoxymethoxy, Propoxymethoxy, (1-Methylethoxy)methoxy, Butoxymethoxy, (1-Methylpropoxylmethoxy, (2-Methylpropoxy)methoxy, (1, 1-Dimethylethoxy)methoxy, 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy) butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₁-C₆-Alkylcarbonyl-C₁-C₆-alkyl: Durch eine C₁-C₆-Alkylcarbonylgruppe substituiertes C₁-C₆-Alkyl, worin beide der C₁-C₆-Alkylgruppen ein oder mehrere Substituenten, ausgewählt unter C₁-C₄-Alkoxy und/oder Hydroxy aufweisen können: z.B. Acetylmethyl (=2-Oxopropyl), 2-(Acetyl)ethyl (=3-Oxo-n-butyl), 3-Oxo-n-pentyl, 1,1-Dimethyl-2-oxopropyl, 3-Hydroxy-2-oxopropyl oder 3-Hydroxy-2-oxobutyl.
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆) alkylaminocarbonyl, N-(C₃-C₆-Alkenyl)-N-(C₁-C₆-alkoxy)aminocarbonyl: z.B. Prop-2-en-1-yl, But-1-en-4-yl, 1-Methyl-prop-2-en-1-yl, 2-Methyl-prop-2-en-1-yl, 2-Buten-1-yl, 1-Penten-3-yl, 1-Penten-4-yl, 2-Penten-4-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, Hex-3-en-1-yl, Hex-4-en-1-yl, Hex-5-en-1-yl, 1-Methyl-pent-3-en-1-yl, 2-Methyl-pent-3-en-1-yl, 3-Methyl-pent-3-en-1-y1, 4-Methyl-pent-3-en-1-y1, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-2-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1, 3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methyl-prop-2-en-1-yl;
- C₂-C₆-Alkenyl, sowie die Alkenylteile von C₂-C₆-Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Tri-chlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆ -Alkinyl, sowie die Alkinylteile von C₃-C₆-Alkinylcarbonyl, C₃-C₆-Alkinyloxy, C₃-C₆-Alkinyloxycarbony, C₃-C6-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl : z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z.B. 1,1-Difluor-prop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iod-but-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₁-C₆-Alkandiyl: Methandiyl, Ethan-1,1-diyl, Ethan-1,2-diyl, Propan-1,1-diyl, Propan-1,2-diyl, Propan-1,3-diyl, Propan-2,2-diyl, Butan-1,1-diyl, Butan-1,2-diyl, Butan-1,3-diyl, Butan-1,4-diyl, 2-Methyl-propan-1,3-diyl, 2-Methyl-propan-1,2-diyl, 2-Methyl-propan-1,1-diyl, 1-Methyl-propan-1,2-diyl, 1-Methyl-propan-2,2-diyl, 1-Methyl-propan-1,1-diyl, Pentan-1,1-diyl, Pentan-1,2-diyl, Pentan-1,3-diyl, Pentan-1,5-diyl, Pentan-2,3-diyl, Pentan-2,2-diyl, 1-Methyl-butan-1,1-diyl, 1-Methyl-butan-1,2-diyl, 1-Methyl-butan-1,3-diyl, 1-Methyl-butan-1,4-diyl, 2-Methyl-butan-1, 1-diyl, 2-Methyl-butan-1,2-diyl, 2-Methyl-butan-1,3-diyl, 2-Methyl-butan-1,4-diyl, 2,2-Dimethyl-propan-1,1-diyl, 2,2-Dimethyl-propan-1,3-diyl, 1,1-Dimethyl-propan-1,3-diyl, 1,1-Dimethyl-propan-1,2-diyl, 2,3-Dimethyl-propan-1,3-diyl, 2,3-Dimethyl-propan-1,2-diyl, 1,3-Dimethyl-propan-1,3-diyl, Hexan-1,1-diyl, Hexan-1,2-diyl, Hexan-1,3-diyl, Hexan-1,4-diyl-, Hexan-1,5-diyl, Hexan-1,6-diyl, Hexan-2,5-diyl, 2-Methyl-pentan-1,1-diyl, 1-Methyl-pentan-1,2-diyl, 1-Methyl-pentan-1,3-diyl, 1-Methyl-pentan-1,4-diyl, 1-Methyl-pentan-1,5-diyl, 2-Methyl-pentan-1,1-diyl, 2-Methyl-pentan-1,2-diyl, 2-Methyl-pentan-1,3-diyl, 2-Methyl-pentan-1,4-diyl, 2-Methyl-pentan-1,5-diyl, 3-Methyl-pentan-1,1-diyl, 3-Methyl-pentan-1,2-diyl, 3-Methyl-pentan-1,3-diyl, 3-Methyl-pentan-1,4-diyl, 3-Methyl-pentan-1,5-diyl, 1,1-Dimethyl-butan-1,2-diyl, 1,1-Dimethyl-butan-1,3-diyl, 1,1-Dimethyl-butan-1,4-diyl, 1,2-Dimethyl-butan-1,1-diyl, 1,2-Dimethyl-butan-1,2-diyl, 1,2-Dimethyl-butan-1,3-diyl, 1,2-Dimethyl-butan-1,4-diyl, 1,3-Dimethyl-butan-1, 1-diyl, 1,3-Dimethyl-butan-1,2-diyl, 1,3-Dimethyl-butan-1,3-diyl, 1,3-Dimethyl-butan-1,4-diyl, 1-Ethyl-butan-1,1-diyl, 1-Ethyl-butan-1,2-diyl, 1-Ethyl-butan-1,3-diyl, 1-Ethyl-butan-1,4-diyl, 2-Ethyl-butan-1,1-diyl, 2-Ethyl-butan-1,2-diyl, 2-Ethyl-butan-1,3-diyl, 2-Ethyl-butan-1,4-diyl, 2-Ethyl-butan-2,3-diyl, 2,2-Dimethyl-butan-1,1-diyl, 2,2-Dimethyl-butan-1,3-diyl, 2,2-Dimethyl-butan-1,4-diyl, 1-Isopropyl-propan-1,1-diyl, 1-Isopropyl-propan-1,2-diyl, 1-Isopropyl-propan-1,3-diyl, 2-Isopropyl-propan-1,1-diyl, 2-Isopropyl-propan-1,2-diyl, 2-Isopropyl-propan-1,3-diyl, 1,2,3-Trimethyl-propan-1,1-diyl, 1,2,3-Trimethyl-propan-1,2-diyl oder 1,2,3-Trimethyl-propan-1,3-diyl;
- C₂-C₆-Alkendiyl: Ethen-1,1-diyl, Ethen-1,2-diyl, 1-Propen-1,1-diyl, 1-Propen-1,2-diyl, 1-Propen-1,3-diyl, 2-Propen-1,1-diyl, 2-Propen-1,2-diyl, 2-Propen-1,3-diyl, 1-Buten-1,1-diy 1-Buten-1,2-diyl, 1-Buten-1,3-diyl, 1-Buten-1,4-diyl, 2-Buten-1,1-diyl, 2-Buten-1,2-diyl, 2-Buten-1,3-diyl, 2-Buten-1,4-diyl, 3-Buten-1, 1-diyl, 3-Buten-1,2-diyl, 3-Buten-1,3-diyl, 3-Buten-1,4-diyl, 1-Methyl-1-propen-1,2-diyl, 1-Methyl-1-propen-1,3-diyl, 1-Methyl-2-propen-1,1-diyl, 1-Methyl-2-propen-1,2-diyl, 1-Methyl-2-propen-1,3-diyl, 2-Methyl-1, 1-propen-1, 1-diyl, 2-Methyl-1-propen-1,3-diyl, 3-Buten-1,1-diyl, 3-Buten-1,2-diyl, 3-Buten-1,3-diyl, 3-Buten-1,4-diyl, 1-Penten-1,1-diyl, 1-Penten-1,2-diyl, 1-Penten-1,3-diyl, 1-Penten-1,4-diyl, 1-Penten-1,5-diyl, 1-Hexen-1,1-diyl, 1-Hexen-1,2-diyl, 1-Hexen-1,3-diyl, 1-Hexen-1,4-diyl, 1-Hexen-1,5-diyl oder 1-Hexen-1,6-diyl;
- C₂-C₆-Alkadiendiyl: 1,3-Butadien-1,1-diyl, 1,3-Butadien-1,2-diyl, 1,3-Butadien-1,3-diyl, 1,3-Butadien-1,4-diyl, 1,3-Pen-tadien-1, 1-diyl, 1,3-Pentadien-1,2-diyl, 1,3-Pentadien-1,3-diyl, 1,3-Pentadien-1,4-diyl, 1,3-Pentadien-1,5-diyl, 2,4-Pentadien-1,1-diyl, 2,4-Pentadien-1,2-diyl, 2,4-Pentadien-1,3-diyl, 2,4-Pentadien-1,4-diyl, 2,4-Pentadien-1,5-diyl, 1-Methyl-1,3-butadien-1,4-diyl, 1,3-Hexadien-1, 1-diyl, 1,3-Hexadien-1,2-diyl, 1,3-Hexadien-1,3-diyl, 1,3-Hexadien-1,4-diyl, 1,3-Hexadien-1,5-diyl, 1,3-Hexadien-1,6-diyl, 1-Methyl-1,3-pentadien-1,2-diyl, 1-Methyl-1,3-pentadien-1,3-diyl, 1-Methyl-1,3-pentadien-1,4-diyl oder 1-Methyl-1,3-pentadien-1,5-diyl;
- C₂-C₆-Alkindiyl: Ethin-1,2-diyl, 1-Propin-1,3-diyl, 2-Propin-1,1-diyl, 2-Propin-1,3-diyl, 1-Butin-1,3-diyl, 1-Butin-1,4-diyl, 2-butin-1,1-diyl, 2-Butin-1,4-diyl, 1-Methyl-2-propin-1,1-diyl, 1-Methyl-2-propin-1,3-diyl, 1-Pentin-1,3-diyl, 1-Pentin-1,4-diyl, 1-Pentin-1,5-diyl, 2-Pentin-1,1-diyl, 2-Pentin-1,4-diyl, 2-Pentin-1,5-diyl, 3-Pentin-1,1-diyl, 3-Pentin-1,2-diyl, 3-Pentin-1,5-diyl, 4-Pentin-1,1-diyl, 4-Pentin-1,2-diyl, 4-Pentin-1,3-diyl, 4-Pentin-1,5-diyl, 1-Hexin-1,3-diyl, 1-Hexin-1,4-diyl, 1-Hexin-1,5-diyl, 1-Hexin-1,6-diyl, 2-Hexin-1,1-diyl, 2-Hexin-1,4-diyl, 2-Hexin-1,5-diyl, 2-Hexin-1,6-diyl, 3-Hexin-1,1-diyl, 3-Hexin-1,2-diyl, 3-Hexin-1,5-diyl, 3-Hexin-1,6-diyl, 4-Hexin-1,1-diyl, 4-Hexin-1,2-diyl, 4-Hexin-1,3-diyl, 4-Hexin-1,6-diyl, 5-Hexin-1,1-diyl, 5-Hexin-1,2-diyl, 5-Hexin-1,3-diyl, 5-Hexin-1,4-diyl oder 5-Hexin-1,6-diyl;
- C₃-C₆-Cycloalkyl, sowie die Cycloalkylteile von C₃-C₆-Cycloalkylamino und C₃-C₆-Cycloalkylcarbonyl: z.B. Cyclopropyl, Cyclobutyl, Cyclopentyl oder Cyclohexyl;
- Heterocyclyl, sowie Heterocyclylteile von Heterocyclyloxy, Heterocyclylcarbonyl, Heterocyclyl-C₁-C₄-alkyl, Heterocyclyl-C₁-C₆-alkyl, Heterocyclylsulfonyl oder Heterocyclyloxysulfonyl, Heterocyclyloxycarbonyl, Heterocyclyloxythiocarbonyl, Heterocyclyl-C₂-C₆-alkenylcarbonyl, Heterocyclylcarbonyl-C₁-C₆-alkyl, N-(C₁-C₆-Alkyl)-N-(heterocyclyl)-aminocarbonyl, Heterocyclylaminocarbonyl: ein gesättigter, partiell gesättigter oder ungesättigter 5- oder 6-gliedriger, heterocyclischer Ring, der ein, zwei, drei oder vier gleiche oder verschiedene Heteroatome, ausgewählt aus folgender Gruppe: Sauerstoff, Schwefel oder Stickstoff, enthält, also z.B. C-gebundene 5-gliedrige Ringe wie:
   Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4, 5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3, 4-Dihydro-5H-pyrrol-3-yl, 2-Furyl, 3-Furyl, 2-Thienyl, 3-Thienyl, Pyrrol-2-yl, Pyrrol-3-yl, Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydro-isothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1, 3-Dioxolan-4-yl, 1,3-0xathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1, 2-Dithiol-3-yl, Δ³-1, 2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4 ,5-Dihydrooxazol-4-yl, 4, 5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2, 3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2, 5-Dihydrothiazol-4-yl, 2,5-Dihydrothiazol-5-yl, 2,3-Dihydrothiazol-2-yl, 2,3-Dihydrothiazol-4-y1, 2,3-Dihydrothiazol-5-yl, 1,3-Dioxol-2-yl, 1,3-Dioxol-4-yl, 1,3-Dithiol-2-yl, 1,3-Dithiol-4-yl, 1, 3-0xathiol-2-yl, 1,3-Oxathiol-4-yl, 1, 3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Δ²-Oxadiazolin-4-yl, 1,2,3-Δ²-Oxadiazolin-5-yl, 1,2, 4-Δ⁴-Oxadiazolin-3-yl, 1,2, 4-Δ⁴-Oxadiazolin-5-yl, 1,2,4-Δ²-Oxadiazolin-3-yl, 1,2, 4-Δ²-Oxadiazolin-5-yl, 1,2,4-Δ³-Oxadiazolin-3-yl, 1,2,4-Δ³-Oxadiazolin-5-yl, 1,3, 4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3, 4-Δ²-Thiadiazolin-2-yl, 1,3,4-Δ²-Thiadiazolin-5-yl, 1,3, 4-Δ³-Thiadiazolin-2-yl, 1,3,4-Thiadiazolin-2-yl, 1,3,2-Dioxathiolan-4-yl, 1,2,3-Δ²-Triazolin-4-yl, 1, 2, 3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2, 4-Δ³-Triazolin-5-yl, 1,2,4-Δ¹-Triazolin-2-yl, 1,2,4-Triazolin-3-yl, 3H-1, 2, 4-Dithiazol-5-yl, 2H-1,3,4-Dithiazol-5-yl, 2H-1,3,4-Oxathiazol-5-yl, 1,2,3-Oxadiazol-4-yl, 1,2,3-Oxadiazol-5-yl, 1,2,4-Oxadiazol-3-yl, 1,2,4-Oxadiazol-S-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   C-gebundene 6-gliedrige Ringe wie: Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3, 4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6Dihydropyran-6-yl, 2H-5,6-Dihydrothiopyran-2-yl, 2H-5,6-Dihydrothiopyran-3-yl, 2H-5, 6-Dihydrothiopyran-4-yl, 2H-5,6Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran₋3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, Pyridin-2-yl, Pyridin-3-yl, Pyridin-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1, 3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5, 6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5, 6-Dihydro-1, 2oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3, 6-Dihydro-1,2-oxazin-3-yl, 2H-3,6-Dihydro-1,2-oxazin-4-yl, 2H-3, 6-Dihydro-1, 2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4 H-5, 6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4, 5, 6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-0xazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1, 3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl, 3,4-Dihydropyrimidin-6-yl, Pyridazin-3-yl, Pyridazin-4-yl, Pyrimidin-2-yl, Pyrimidin-4-yl, Pyrimidin-5-yl, Pyrazin-2₋yl, 1,3,5-Triazin-2-yl, 1,2,4-Triazin-3-yl, 1,2,4-Triazin-5-yl, l,2,4-Triazin-6-yl oder 1,2,4,5-Tetra-zin-3-yl;
   N-gebundene 5-gliedrige Ringe wie:
      Tetrahydropyrrol-1-yl, 2,3-Dihydro-1H-pyrrol-1-yl, 2,5-Dihydro-1H-pyrrol-1-yl, pyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl, 4,5-Dihydro-1H-pyrazol-1-yl, 2,5-Dihydro-1H-pyrazol-1-yl, 2, 3-Dihydro-1H-pyrazol-1-yl, 2,5-Dihydroisoxazol-2-yl, 2,3-Dihydroisoxazol-2-yl, 2,5-Dihydroisothiazol-2-yl, 2, 3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, Pyrazol-1-yl, Imidazol-1-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2, 4-Δ³-Triazolin-1-yl, 1,2,4-Δ¹-Triazolin-4-yl, 1,2, 3-Triazol-1-yl, 1,2,4-Triazol-1-yl, Tetrazol-1-yl;
      N-gebundene 6-gliedrige Ringe wie:
         Piperidin-1-yl, 1,2,3,4-Tetrahydropyridin-1-yl, 1,2,5,6-Tetrahydropyridin-1-yl, 1,4-Dihydropyridin-1-yl, 1, 2-Dihydropyridin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl (Morpholinyl), Tetrahydro-1,2-oxazin-2-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihydro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
      sowie N-gebundene cyclische Imide wie:
         Phthalsäureimid, Tetrahydrophthalsäureimid, Succinimid, Maleinimid, Glutarimid, 5-Oxo-triazolin-1-yl, 5-Oxo-1,3,4-oxadiazolin-4-yl oder 2,4-Dioxo-(1H,3H)-pyrimidin-3-yl;
   wobei mit einem ankondensierten Phenylring oder mit einem C₃-C₆-Carbocyclus oder einem weiteren 5- bis 6-gliedrigen Heterocyclus ein bicyclisches Ringsystem ausgebildet werden kann,
   wobei gegebenenfalls der Schwefel der genannten Heterocyclen zu S=0 oder S(=0)₂ oxidiert sein kann
   und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.

Alle Phenylringe bzw. Heterocyclylreste sowie alle Phenylkomponenten in Phenoxy, Phenylalkyl, Phenylcarbonylalkyl, Phenylcarbonyl, Phenylalkenylcarbonyl, Phenoxycarbonyl, Phenyloxythiocarbonyl, Phenylaminocarbonyl und N-Alkyl-N-phenylaminocarbonyl, Phenylsulfonyl oder Phenoxysulfonyl bzw. Heterocyclylkomponenten in Heterocyclyloxy, Heterocyclylalkyl, Heterocyclylcarbonylalkyl, Heterocyclylcarbonyl, Heterocyclyloxythiocarbonyl, Heterocyclylalkenylcarbonyl, Heterocyclyloxycarbonyl, Heterocyclylaminocarbonyl, N-Alkyl-N-heterocyclylaminocarbonyl, Heterocyclylsulfonyl oder Heterocyclyloxysulfonyl sind, soweit nicht anders angegeben, vorzugsweise unsubstituiert oder tragen ein, zwei oder drei Halogenatome und/oder eine Nitrogruppe, einen Cyanorest und/oder einen oder zwei Methyl-, Trifluormethyl-, Methoxy- oder Trifluormethoxysubstituenten. Im Hinblick auf die Verwendung der erfindungsgemäßen Verbindungen der Formel I als Herbizide haben die Variablen X, Y, R¹ bis R¹⁶ vorzugsweise folgende Bedeutungen, und zwar jeweils für sich alleine oder in Kombination:
- R¹: Wasserstoff, Halogen, C₁-C₆-Alkyl, C₁-C₆-Alkyloxy, C₁-C₆-Alkylthio, C₁-C₆-Alkylsulfinyl, C₁-C₆-Alkylsulfonyl, C₁-C₆-Halogenalkyl, C₁-C₆-Alkoxyalkyl, C₁-C₆-Alkylsulfonyl-C₁-C₆-alkyl, besonders bevorzugt Methyl, Chlor, Methoxy, Methylthio, Methylsulfinyl, Methylsulfonyl, Brommethyl, Methoxymethyl, Methylsulfonylmethyl;
- R²: Wasserstoff, Halogen, z.B. Chlor oder Brom, C₁-C₆-Alkyl, z.B. Methyl;
- X: C-R³ mit den für R³ zuvor genannten Bedeutungen oder N;
- Y: S, SO₂ oder NR⁴ mit den für R⁴ zuvor genannten Bedeutungen;
- Pz: Rest der allgemeinen Formel IIa, worin R⁸, R⁹ und R¹⁰ die zuvor genannten Bedeutungen haben.

Bevorzugt sind insbesondere Verbindungen der Formel I, worin Y für O, S, SO₂ oder N-R⁴ und X für C-R³ stehen. Bevorzugt sind auch Verbindungen der Formel I, worin X für N und Y für S oder N-R⁴ stehen.

Bevorzugt haben R⁸, R⁹ und R¹⁰ unabhängig voneinander die folgenden Bedeutungen:
- R⁸: Hydroxy, Halogen, OR¹¹, SR¹¹, SO₂R¹², OSO₂R¹², NR¹⁵R¹⁶, ONR¹⁵R¹⁶ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; insbesondere Hydroxy, OR¹¹ und OSO₂R¹², speziell Hydroxy, C₁-C₄-Alkyloxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
- R⁹: C₁-C₆-Alkyl, insbesondere C₁-C₄-Alkyl, und
- R¹⁰: Wasserstoff oder C₁-C₄-Alkyl, insbesondere Wasserstoff oder C₁-C₄-Alkyl.

Bevorzugt sind auch Verbindungen, in denen R⁹ für C₃-C₆-Cycloalkyl, insbesondere Cyclopropyl steht.

Besonders bevorzugt sind Verbindungen der allgemeinen Formel I,
worin X für C-R³ steht und
- R³: für Wasserstoff, Halogen, Cyano, Rhodano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkoxy-C₁-C₆-alkyl,
Phenyl oder Pyridyl, wobei die zwei letztgenannten Reste teilweise oder vollständig halogeniert sein können und/oder einen, zwei oder drei, insbesondere einen der folgenden Reste: Halogen, C₁-C₄-Alkoxy, C₁-C₄-Halogenalkyl, und C₁-C₄-Halogenalkoxy, tragen können;
oder
COOR⁵ mit den für R⁵ zuvor genannten Bedeutungen steht. Hierin steht R⁵ insbesondere für Wasserstoff oder C₁-C₆-Alkyl und besonders bevorzugt für Wasserstoff, Methyl, Ethyl, n-Propyl, iso-Propyl, n-Butyl, 2-Butyl, iso-Butyl und tert.-Butyl.

Bevorzugt sind auch Verbindungen I, in denen R³ C₃-C₆-Cycloalkyl oder Phenoxy, das wie für Phenyl angegeben substituiert sein kann, bedeutet.

Beispiele für bevorzugte Reste R³ sind Wasserstoff, Fluor, Chlor, Brom, Cyano, Rhodano, Methyl, Ethyl, n-Propyl, Isopropyl, n-Butyl, 2-Butyl, Isobutyl, tert-Butyl, Chlormethyl, Dichlormethyl, Trichlormethyl, Fluormethyl, Difluormethyl, Trifluormethyl, 2,2,2-Trifluorethyl, Methoxymethyl, Ethoxymethyl, Methoxy, Ethoxy, 1-Propoxy, 2-Propoxy, 1-Butoxy, 2-Butoxy, 2-Methylprop-1-oxy, tert-Butyloxy, Difluormethyloxy, Trifluormethyloxy, 2,2,2-Trifluorethyl-1-oxy, (Methoxy)methyloxy, Methylsulfanyl, Ethylsulfanyl, n-Propylsulfanyl, Isopropylsulfanyl, 1-Butylsulfanyl, 2-Butylsulfanyl, 2-Methylprop-1-ylsulfanyl, tert-Butylsulfanyl, Fluormethylsulfanyl, Trifluormethylsulfanyl, 2,2,2-Trifluorethyl-1-sulfanyl, 2-(Methylcarbonyl)ethyl, Phenyl, 2-, 3- oder 4-Fluorphenyl, 2-, 3- oder 4-Chlorphenyl, 2-, 3- oder 4-Hydroxyphenyl, 2-, 3- oder 4-Methoxyphenyl, 2-, 3- oder 4-(Trifluormethoxy)phenyl, 2-, 3- oder 4-(Difluormethoxy)phenyl, 2-, 3- oder 4-(Trifluormethyl)phenyl, 2-, 3- oder 4-Tolyl, 2-, 3- oder 4-Pyridinyl, 2-, 3- oder 4-Fluorphenoxy, 2-, 3- oder 4-Methoxyphenoxy, 2-, 3- oder 4-Trifluormethylphenoxy, 2-, 3- oder 4-Chlorphenoxy, Methoxycarbonyl, Ethoxycarbonyl, Trifluormethoxycarbonyl, Isopropoxycarbonyl, tert-Butoxycarbonyl und Phenoxycarbonyl.

Ganz besonders bevorzugte Verbindungen der allgemeinen Formel I mit X = C-R³ sind solche Verbindungen, worin R³ für Wasserstoff, Halogen, insbesondere Fluor oder Chlor, C₁-C₄-Alkyl, C₁-C₄-Halogenalkyl, insbesondere Fluormethyl, Difluormethyl, Trifluormethyl und 2,2,2-Trifluorethyl, oder für Phenyl oder Phenoxy steht, wobei Phenyl oder Phenoxy einen, zwei oder drei und insbesondere einen Substituenten, ausgewählt unter C₁-C₄-Alkyl, insbesondere Methyl, Halogen, insbesondere Fluor oder Chlor, C₁-C₄-Alkoxy, insbesondere Methoxy, oder Halogenalkoxy, insbesondere Trifluormethoxy, tragen kann.

Unter den vorstehend genannten Pyrazolderivaten der allgemeinen Formel I sind solche Verbindungen besonders bevorzugt, die sich von der Benzothiazol-5-carbonsäure ableiten, also Verbindungen der allgemeinen Formel I, worin X für einen Rest C-R³ steht und Y ausgewählt ist unter S, SO, und SO₂. Unter den Pyrazol-Derivaten des Benzothiazols sind wiederum solche bevorzugt, worin R³ eine der zuvor als bevorzugt genannten Bedeutungen aufweist. Insbesondere steht Y für S oder SO₂.

Erfindungsgemäß bevorzugt sind auch solche Pyrazolylderivate, die sich von der Benzooxazol-5-carbonsäure ableiten, d.h. Verbindungen der allgemeinen Formel I, worin X für eine Gruppe C-R³ mit den zuvor für R³ angegebenen Bedeutungen und Y für ein Sauerstoffatom stehen. Hierunter sind wiederum solche Verbindungen bevorzugt,
worin R³ die zuvor als bevorzugt angegebenen Bedeutungen aufweist. Ebenfalls bevorzugt sind Pyrazolderivate der allgemeinen Formel I, die sich von der Benzimidazol-5-carbonsäure ableiten, also Verbindungen der allgemeinen Formel I, worin X für C-R³ mit den für R³ zuvor genannten Bedeutungen und Y für eine Gruppe N-R⁴ mit den zuvor für R⁴ genannten Bedeutungen stehen. Hierunter sind solche Benzimidazolderivate der allgemeinen Formel I bevorzugt, worin R³ die zuvor als für R³ bevorzugt genannten Bedeutungen aufweist. Ferner sind Benzimidazol-Derivate der allgemeinen Formel I bevorzugt, worin R⁴ für Wasserstoff, C₁-C₆-Alkyl oder C₁-C₆-Halo-genalkyl, insbesondere für Wasserstoff, Methyl, Ethyl, n-Propyl und iso-Propyl.

Erfindungsgemäß bevorzugt sind auch Pyrazolylderivate der Benzotriazol-5-carbonsäure, also Verbindungen der allgemeinen Formel I, worin X für Stickstoff und Y für eine Gruppe N-R⁴ mit den zuvor für R⁴ angegebenen Bedeutungen steht. Hierunter sind wiederum solche Verbindungen bevorzugt, worin R⁴ die zuvor als bevorzugt angegebenen Bedeutungen aufweist.

Erfindungsgemäß bevorzugt sind auch Pyrazolylderivate der Benzothiadiazol-5-carbonsäure, also Verbindungen der allgemeinen Formel I, worin X für N und Y für S steht. Ebenfalls bevorzugt sind Pyrazolderivate der Benzoisothiadiazolcarbonsäure, also Verbindungen der allgemeinen Formel I, worin X-Y für S=N steht und X für S steht.

Unter den als bevorzugt angegebenen Pyrazolyl-Derivaten der allgemeinen Formel I sind wiederum solche Verbindungen bevorzugt, worin Pz in Formel I für eine Gruppe der allgemeinen Formel IIa steht. Hierunter sind wiederum Verbindungen der allgemeinen Formel I besonders bevorzugt, worin die Variablen R⁸, R⁹ und R¹⁰ in Formel IIa für sich alleine, und besonders bevorzugt in Kombination miteinander, die folgenden Bedeutungen aufweisen:
- R⁸: Hydroxy, Halogen, OR¹¹, SR¹¹, SO₂R¹², OSO₂R¹², NR¹⁵R¹⁶ ONR¹⁵R¹⁶ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy, insbesondere Hydroxy, OR¹¹ und OSO₂R¹²;
- R⁹: C₁-C₄-Alkyl oder Cyclopropyl;
- R¹⁰: Wasserstoff oder C₁-C₄-Alkyl.

Unter den Verbindungen der allgemeinen Formel I, worin Pz für einen Pyrazolylrest der allgemeinen Formel IIa steht, sind solche Verbindungen ganz besonders bevorzugt, worin die Variablen R⁸, R⁹ und R¹⁰ gemeinsam die folgenden Bedeutungen aufweisen:
- R⁸: für Hydroxy, C₁-C₄-Alkyloxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
- R⁹: für C₁-C₄-Alkyl oder Cyclopropyl und
- R¹⁰: für Wasserstoff oder C₁-C₄-Alkyl stehen.

Ganz besonders bevorzugte Reste der allgemeinen Formel IIa sind die in Tabelle 1 angegebenen Reste IIaI bis IIa90.

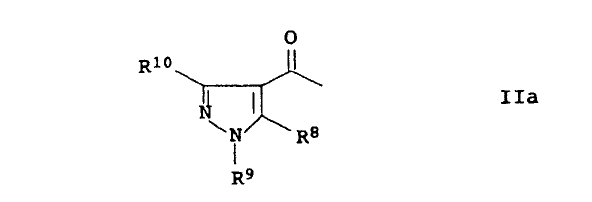

**Tabelle 1**

| IIa | R⁸ | R⁹ | R¹⁰ |
|---|---|---|---|
| IIa1 IIa1 | OH | CH₃ | H |
| IIa2 | OCH₃ | CH₃ | H |
| IIa3 | OCH₂-C₆H₅ | CH₃ | H |
| IIa4 | OC(O)CH₃ | CH₃ | H |
| IIa5 | OC(O)C₆H₅ | CH₃ | H |
| IIa6 | OC(O)-(3-C₆H₄F) | CH₃ | H |
| IIa7 | OS(O)₂CH₃ | CH₃ | H |
| IIa8 | OS(O)₂-(4-C₆H₄CH₃) | CH₃ | H |
| IIa9 | OH | C₂H₅ | H |
| IIa10 | OCH₃ | C₂H₅ | H |
| IIa11 | OCH₂-C₆H₅ | C₂H₅ | H |
| IIa12 | OC(O)CH₃ | C₂H₅ | H |
| IIa13 | OC(O)C₆H₅ | C₂H₅ | H |
| IIa14 | OC(O)-(3-C₆H₄F) | C₂H₅ | H |
| IIa15 | OS(O)₂CH₃ | C₂H₅ | H |
| IIa16 | OS(O)₂-(4-C₆H₄CH₃) | C₂H₅ | H |
| IIa17 | OH | i-C₃H₇ | H |
| IIa18 | OCH₃ | i-C₃H₇ | H |
| IIa | R⁸ | R⁹ | R¹⁰ |
| IIa19 | OCH₂-C₆H₅ | i-C₃H₇ | H |
| IIa20 | OC(O)CH₃ | i-C₃H₇ | H |
| IIa21 | OC(O)C₆H₅ | i-C₃H₇ | H |
| IIa22 | OC(O)-(3-C₆H₄F) | i-C₃H₇ | H |
| IIa23 | OS(O)₂CH₃ | i-C₃H₇ | H |
| IIa24 | OS(O)₂-(4-C₆H₄CH₃) | i-C₃H₇ | H |
| IIa25 | OH | t-C₄H₉ | H |
| IIa26 | OCH₃ | t-C₄H₉ | H |
| IIa27 | OCH₂-C₆H₅ | t-C₄H₉ | H |
| IIa28 8 | OC(O)CH₃ | t-C₄H₉ | H |
| IIa29 | OC(O)C₆H₅ | t-C₄H₉ | H |
| IIa30 | OC(O)-(3-C₆H₄F) | t-C₄H₉ | H |
| IIa31 | OS(O)₂CH₃ | t-C₄H9 | H |
| IIa32 | OS(O)₂-(4-C₆H₄CH₃) | t-C₄H₉ | H |
| IIa33 | OH | CH₃ | CH₃ |
| IIa34 | OCH₃ | CH₃ | CH₃ |
| IIa35 | OCH₂-C₆H₅ | CH₃ | CH₃ |
| IIa36 | OC(O)CH₃ | CH₃ | CH3 |
| Ila37 | OC(0)C₆H₅ | CH₃ | CH₃ |
| IIa38 | OC(O)-(3-C₆H₄F) | CH₃ | CH₃ |
| IIa39 | OS(O)₂CH₃ | CH₃ | CH₃ |
| IIa40 | OS(O)₂-(4-C₆H₄CH₃) | CH₃ | CH₃ |
| IIa41 | OH | C₂H₅ | CH₃ |
| IIa42 | OCH₃ | C₂H₅ | CH₃ |
| IIa43 | OCH₂-C₆H₅ | C₂H₅ | CH₃ |
| IIa44 | OC(O)CH₃ | C₂H₅ | CH₃ |
| IIa45 | OC(O)C₆H₅ | C₂H₅ | CH₃ |
| IIa46 | OC(O)-(3-C₆H₄F) | C₂H₅ | CH₃ |
| IIa47 | OS(0)₂CH₃ | C₂H₅ | CH₃ |
| Ila48 | OS(0)₂-(4-C₆H₄CH₃) | C₂H₅ | CH₃ |
| IIa49 | OH | i-C₃H₇ | CH₃ |
| IIa50 | OCH₃ | i-C₃H₇ | CH₃ |
| IIa51 | OCH₂-C₆H₅ | i-C₃H₇ | CH₃ |
| IIa52 | OC(O)CH₃ | i-C₃H₇ | CH₃ |
| Ila53 | OC(0)C₆Hₛ | i-C₃H₇ | CH₃ |
| IIa54 | OC(0)-(3-C₆H₄F) | i-C₃H₇ | CH₃ |
| IIa55 | OS(O)₂CH₃ | i-C₃H₇ | CH₃ |
| IIa56 | OS(O)₂-(4-C₆H₄CH₃) | i-C₃H₇ | CH₃ |
| IIa57 | OH | t-C₄H₉ | CH₃ |
| IIa | R⁸ | R⁹ | R¹⁰ |
| IIa58 | OCH₃ | t-C₄H₉ | CH₃ |
| IIa59 | OCH₂-C₆H₅ | t-C₄H₉ | CH₃ |
| IIa60 | OC(O)CH₃ | t-C₄H₉ | CH₃ |
| IIa61 | OC(O)C₆H₅ | t-C₄H₉ | CH₃ |
| IIa62 | OC(O)-(3-C₆H₄F) | t-C₄H₉ | CH₃ |
| IIa63 | OS(O)₂CH₃ | t-C₄H₉ | CH₃ |
| IIa64 | OS(O)₂-(4-C₆H₄CH₃) | t-C₄H₉ | CH₃ |
| IIa65 | OH | c-C₃H₅ | CH₃ |
| IIa66 | OCH₃ | c-C₃H₅ | CH₃ |
| IIa67 | OCH₂-C₆H₅ | c-C₃H₅ | CH₃ |
| IIa68 | OC(O)CH₃ | c-C₃H₅ | CH₃ |
| IIa69 | OC(O)C₆H₅ | c-C₃H₅ | CH₃ |
| IIa70 | OC(O)-(3-C₆H₄F) | c-C₃H₅ | CH₃ |
| IIa71 | OS(O)₂CH₃ | c-C₃H₅ | CH₃ |
| IIa72 | OS(O)₂-(4-C₆H₄CH₃) | c-C₃H₅ | CH₃ |
| IIa73 | OH | c-C₃H₅ | H |
| IIa74 | OCH₃ | c-C₃H₅ | H |
| IIa75 | OCH₂-C₆H₅ | c-C₃H₅ | H |
| IIa76 | OC(O)CH₃ | C-C₃H₅ | H |
| IIa77 | OC(O)C₆H₅ | c-C₃H₅ | H |
| IIa78 | OC(O)-(3-C₆H₄F) | c-C₃H₅ | H |
| IIa79 | OS(O)₂CH₃ | c-C₃H₅ | H |
| IIa80 | OS(O)₂-(4-C₆H₄CH₃) | C-C₃H₅ | H |
| IIa81 | OC(O)C-C₃H₅ | CH₃ | H |
| IIa82 | OC(O)-C-C₃H₅ | C₂H₅ | H |
| IIa83 | OC(O)-c-C₃H₅ | i-C₃H₇ | H |
| IIa84 | OC(O)-c-C₃H₅ | t-C₄H₉ | H |
| IIa85 | OC(O)-C-C₃H₅ | c-C₃H₅ | H |
| IIa86 | OC(O)-c-C₃H₅ | CH₃ | CH₃ |
| IIa87 | OC(O)-c-C₃H₅ | C₂H₅ | CH₃ |
| IIa88 | OC(O)-C-C₃H₅ | i-C₃H₇ | CH₃ |
| IIa89 | OC(O)-c-C₃H₅ | t-C₄H₉ | CH₃ |
| IIa90 | OC(O)-c-C₃H₅ | C-C₃H₅ | CH₃ |
| i-C₃H₇: Isopropyl | | | |
| c-C₃H₅: Cyclopropyl | | | |
| t-C₄H₉: tertiär-Butyl | | | |
| C₆H₅: Phenyl | | | |
| 3-C₆H₄F: 3-Fluorphenyl | | | |
| 4-C₆H₄CH₃: 4-Methylphenyl | | | |

**Tabelle A:**

| Besonders bevorzugte Kombinationen von R¹, R² und R³ | | | |
|---|---|---|---|
| | R³ | R¹ | R² |
| 1 | H | CH₃ | CH₃ |
| 2 | F | CH₃ | CH₃ |
| 3 | C1 | CH₃ | CH₃ |
| 4 | Br | CH₃ | CH₃ |
| 5 | OH | CH₃ | CH₃ |
| 6 | SH | CH₃ | CH₃ |
| 7 | NH₂ | CH₃ | CH₃ |
| 8 | CN | CH₃ | CH₃ |
| 9 | NO₂ | CH₃ | CH₃ |
| 10 | SCN | CH₃ | CH₃ |
| 11 | NH-NH₂ | CH₃ | CH₃ |
| 12 | CH₃ | CH₃ | CH₃ |
| 13 | C₂H₅ | CH₃ | CH₃ |
| 14 | n-C₃H₇ | CH₃ | CH₃ |
| 15 | i-C₃H₇ | CH₃ | CH₃ |
| 16 | n-C₄H₉ | CH₃ | CH₃ |
| 17 | S-C₄H₉ | CH₃ | CH₃ |
| 18 | i-C₄H₉ | CH₃ | CH₃ |
| 19 | t-C₄H₉ | CH₃ | CH₃ |
| 20 | CH₂Cl | CH₃ | CH₃ |
| 21 | CHCl₂ | CH₃ | CH₃ |
| 22 | CCl₃ | CH₃ | CH₃ |
| 23 | CH₂F | CH₃ | CH₃ |
| 24 | CHF₂ | CH₃ | CH₃ |
| 25 | CF₃ | CH₃ | CH₃ |
| 62 | CH₂CF₃ | CH₃ | CH₃ |
| 27 | CH₂OCH₃ | CH₃ | CH₃ |
| 28 | CH₂OCH₂CH₃ | CH₃ | CH₃ |
| 29 | CH₂NH₂ | CH₃ | CH₃ |
| 30 | OCH₃ | CH₃ | CH₃ |
| 31 | OC₂H₅ | CH₃ | CH₃ |
| 32 | O-n-C₃H₇ | CH₃ | CH₃ |
| 33 | O-i-C₃H₇ | CH₃ | CH₃ |
| 34 | O-n-C₄H₉ | CH₃ | CH₃ |
| 35 | O-S-C₄H₉ | CH₃ | CH₃ |
| 36 | O-i-C₄H₉ | CH₃ | CH₃ |
| 37 | O-t-C₄H₉ | CH₃ | CH₃ |
| 38 | OCHF₂ | CH₃ | CH₃ |
| 39 | OCF₃ | CH₃ | CH₃ |
| 40 | OCH₂CF₃ | CH₃ | CH₃ |
| 41 | OCH₂OCH₃ | CH₃ | CH₃ |
| 42 | SCH₃ | CH₃ | CH₃ |
| 43 | SC₂H₅ | CH₃ | CH₃ |
| 44 | S-n-C₃H₇ | CH₃ | CH₃ |
| 45 | S-i-C₃H₇ | CH₃ | CH₃ |
| 46 | S-n-C₄H₉ | CH₃ | CH₃ |
| 47 | S-s-C₄H₉ | CH₃ | CH₃ |
| 48 | S-i-C₄H₉ | CH₃ | CH₃ |
| 49 | S-t-C₄H₉ | CH₃ | CH₃ |
| 50 | SCHF₂ | CH₃ | CH₃ |
| 51 | SCF₃ | CH₃ | CH₃ |
| 52 | SCH₂CF₃ | CH₃ | CH₃ |
| 53 | SCH₂OCH₃ | CH₃ | CH₃ |
| 54 | NHCH₃ | CH₃ | CH₃ |
| 55 | NHC₂H₅ | CH₃ | CH₃ |
| 56 | NH-Phenyl | CH₃ | CH₃ |
| 57 | N(CH₃)₂ | CH₃ | CH₃ |
| 58 | N(CH₂CH₃)₂ | CH₃ | CH₃ |
| 59 | N(Phenyl)₂ | CH₃ | CH₃ |
| 60 | (CH₂)₂COCH₃ | CH₃ | CH₃ |
| 61 | Phenyl | CH₃ | CH₃ |
| 62 | 2-F-Phenyl | CH₃ | CH₃ |
| 63 | 3-F-Phenyl | CH₃ | CH₃ |
| 64 | 4-F-Phenyl | CH₃ | CH₃ |
| 65 | 2-Cl-Phenyl | CH₃ | CH₃ |
| 66 | 3-Cl-Phenyl | CH₃ | CH₃ |
| 67 | 4-Cl-Phenyl | CH₃ | CH₃ |
| 68 | 2-OH-Phenyl | CH₃ | CH₃ |
| 69 | 3-OH-Phenyl | CH₃ | CH₃ |
| 70 | 4-OH-Phenyl | CH₃ | CH₃ |
| 71 | 2-OCH₃-Phenyl | CH₃ | CH₃ |
| 72 | 3-OCH₃-Phenyl | CH₃ | CH₃ |
| 73 | 4-OCH₃-Phenyl | CH₃ | CH₃ |
| 74 | 2-OCF₃-Phenyl | CH₃ | CH₃ |
| 75 | 3-OCF₃-Phenyl | CH₃ | CH₃ |
| 76 | 4-OCF₃-Phenyl | CH₃ | CH₃ |
| 77 | 2-OCHF₂-Phenyl | CH₃ | CH₃ |
| 78 | 3- OCHF₂-Phenyl | CH₃ | CH₃ |
| 79 | 4-OCHF₂-Phenyl | CH₃ | CH₃ |
| 80 | 2-CF₃-Phenyl | CH₃ | CH₃ |
| 81 | 3-CF₃-Phenyl | CH₃ | CH₃ |
| 82 | 4-CF₃-Phenyl | CH₃ | CH₃ |
| 83 | 2-CH₃-Phenyl | CH₃ | CH3 |
| 84 | 3-CH₃-Phenyl | CH₃ | CH₃ |
| 85 | 4-CH₃-Phenyl | CH₃ | CH₃ |
| 86 | 2-NO₂-Phenyl | CH₃ | CH₃ |
| 87 | 3-NO₂-Phenyl | CH₃ | CH₃ |
| 88 | 4-NO₂-Phenyl | CH₃ | CH₃ |
| 89 | 2-Pyridyl | CH₃ | CH₃ |
| 90 | 3-Pyridyl | CH₃ | CH₃ |
| 91 | 4-Pyridyl | CH₃ | CH₃ |
| 92 | 3'-CH₃-2-pyridyl | CH₃ | CH₃ |
| 93 | 4'-CH₃-2-pyridyl | CH₃ | CH₃ |
| 94 | 5'-CH₃-2-pyridyl | CH₃ | CH₃ |
| 95 | 6'-CH₃-2-pyridyl | CH₃ | CH₃ |
| 96 | 2'-CH₃-3-pyridyl | CH₃ | CH₃ |
| 97 | 4'-CH₃-3-pyridyl | CH₃ | CH₃ |
| 98 | 5'-CH₃-3-pyridyl | CH₃ | CH₃ |
| 99 | 6'-CH₃-3-pyridyl | CH₃ | CH₃ |
| 100 | 2'-CH₃-4-pyridyl | CH₃ | CH₃ |
| 101 | 3'-CH₃-4-pyridyl | CH₃ | CH₃ |
| 102 | 3'-Cl-2-pyridyl | CH₃ | CH₃ |
| 103 | 4'-Cl-2-pyridyl | CH₃ | CH₃ |
| 104 | 5'-Cl-2-pyridyl | C_{H3} | CH₃ |
| 105 | 6'-Cl-2-pyridyl | CH₃ | CH₃ |
| 106 | 2'-Cl-3-pyridyl | CH₃ | CH₃ |
| 107 | 4'-Cl-3-pyridyl | CH₃ | CH₃ |
| 108 | 5'-Cl-3-pyridyl | CH₃ | CH₃ |
| 109 | 6'-Cl-3-pyridyl | CH₃ | CH₃ |
| 110 | 2'-Cl-4-pyridyl | CH₃ | CH₃ |
| 111 | 3'-Cl-4-pyridyl | CH₃ | CH₃ |
| 112 | Cyclohexylamino | CH₃ | CH₃ |
| 113 | Cyclopentylamino | CH₃ | CH₃ |
| 114 | Morpholino | CH₃ | CH₃ |
| 115 | CO₂H | CH₃ | CH₃ |
| 116 | CO₂CH₃ | CH₃ | CH₃ |
| 117 | CO₂C₂H₅ | CH₃ | CH₃ |
| 118 | CO₂-n-C₃H₇ | CH₃ | CH₃ |
| 119 | CO₂-i-C₃H₇ | CH₃ | CH₃ |
| 120 | CO₂-n-C₄H₉ | CH₃ | CH₃ |
| 121 | CO₂-s-C₄H₉ | CH₃ | CH₃ |
| 122 | CO₂-i-C₄H₉ | CH₃ | CH₃ |
| 123 | CO₂-t-C₄H₉ | CH₃ | CH₃ |
| 124 | CO₂-Ph | CH₃ | CH₃ |
| 125 | CO₂-3-Pyridyl | CH₃ | CH₃ |
| 126 | CONHCH₃ | CH₃ | CH₃ |
| 127 | CONHC₂H₅ | CH₃ | CH₃ |
| 128 | CONHPh | CH₃ | CH₃ |
| 129 | CON(CH₃)₂ | CH₃ | CH₃ |
| 130 | CON(CH₂CH₃)₂ | CH₃ | CH₃ |
| 131 | CON(Phenyl)₂ | CH₃ | CH₃ |
| 132 | H | OCH₃ | CH₃ |
| 133 | F | OCH₃ | CH₃ |
| 134 | C1 | OCH₃ | CH₃ |
| 135 | Br | OCH₃ | CH₃ |
| 136 | OH | OCH₃ | CH₃ |
| 137 | SH | OCH₃ | CH₃ |
| 138 | NH₂ | OCH₃ | CH₃ |
| 139 | CN | OCH₃ | CH₃ |
| 140 | NO₂ | OCH₃ | CH₃ |
| 141 | SCN | OCH₃ | CH₃ |
| 142 | NH-NH₂ | OCH₃ | CH₃ |
| 143 | CH₃ | OCH₃ | CH₃ |
| 144 | C₂H₅ | OCH₃ | CH₃ |
| 145 | n-C₃H₇ | OCH₃ | CH₃ |
| 146 | i-C₃H₇ | OCH₃ | CH₃ |
| 147 | n-C₄H₉ | OCH₃ | CH₃ |
| 148 | s-C₄H₉ | OCH₃ | CH₃ |
| 149 | i-C₄H₉ | OCH₃ | CH₃ |
| 150 | t-C₄H₉ | OCH₃ | CH₃ |
| 151 | CH₂Cl | OCH₃ | CH₃ |
| 152 | CHCl₂ | OCH₃ | CH₃ |
| 153 | CCl₃ | OCH₃ | CH₃ |
| 154 | CH₂F | OCH₃ | CH₃ |
| 155 | CHF₂ | OCH₃ | CH₃ |
| 156 | CF₃ | OCH₃ | CH₃ |
| 157 | CH₂CF₃ | OCH₃ | CH₃ |
| 158 | CH₂OCH₃ | OCH₃ | CH₃ |
| 159 | CH₂OCH₂CH₃ | OCH₃ | CH₃ |
| 160 | CH₂NH₂ | OCH₃ | CH₃ |
| 161 | OCH₃ | OCH₃ | CH₃ |
| 162 | OC₂H₅ | OCH₃ | CH₃ |
| 163 | O-n-C₃H₇ | OCH₃ | CH₃ |
| 164 | O-i-C₃H₇ | OCH₃ | CH₃ |
| 165 | O-n-C₄H₉ | OCH₃ | CH₃ |
| 166 | O-s-C₄H₉ | OCH₃ | CH₃ |
| 167 | O-i-C₄H₉ | OCH₃ | CH₃ |
| 168 | O-t-C₄H₉ | OCH₃ | CH₃ |
| 169 | OCHF₂ | OCH₃ | CH₃ |
| 170 | OCF₃ | OCH₃ | CH₃ |
| 171 | OCH₂CF₃ | OCH₃ | CH₃ |
| 172 | OCH₂OCH₃ | OCH₃ | CH₃ |
| 173 | SCH₃ | OCH₃ | CH₃ |
| 174 | SC₂H₅ | OCH₃ | CH₃ |
| 175 | S-n-C₃H₇ | OCH₃ | CH₃ |
| 176 | S-i-C₃H₇ | OCH₃ | CH₃ |
| 177 | S-n-C₄H₉ | OCH₃ | CH₃ |
| 178 | S-s-C₄H₉ | OCH₃ | CH₃ |
| 179 | S-i-C₄H₉ | OCH₃ | CH₃ |
| 180 | S-t-C₄H₉ | OCH₃ | CH₃ |
| 181 | SCHF₂ | OCH₃ | CH₃ |
| 182 | SCF₃ | OCH₃ | CH₃ |
| 183 | SCH₂CF₃ | OCH₃ | CH₃ |
| 184 | SCH₂OCH₃ | OCH₃ | CH₃ |
| 185 | NHCH₃ | OCH₃ | CH₃ |
| 186 | NHC₂H₅ | OCH₃ | CH₃ |
| 187 | NHPhenyl | OCH₃ | CH₃ |
| 188 | N(CH₃)₂ | OCH₃ | CH₃ |
| 189 | N(CH₂CH₃)₂ | OCH₃ | CH₃ |
| 190 | N(Phenyl)₂ | OCH₃ | CH₃ |
| 191 | (CH₂)₂COCH₃ | OCH₃ | CH₃ |
| 192 | Phenyl | OCH₃ | CH₃ |
| 193 | 2-F-Phenyl | OCH₃ | CH₃ |
| 194 | 3-F-Phenyl | OCH₃ | CH₃ |
| 195 | 4-F-Phenyl | OCH₃ | CH₃ |
| 196 | 2-Cl-Phenyl | OCH₃ | CH₃ |
| 197 | 3-C1-Phenyl | OCH₃ | CH₃ |
| 198 | 4-Cl-Phenyl | OCH₃ | CH₃ |
| 199 | 2-OH-Phenyl | OCH₃ | CH₃ |
| 200 | 3-OH-Phenyl | OCH₃ | CH₃ |
| 201 | 4-OH-Phenyl | OCH₃ | CH₃ |
| 202 | 2-OCH₃-Phenyl | OCH₃ | CH₃ |
| 203 | 3-OCH₃-Phenyl | OCH₃ | CH₃ |
| 204 | 4-OCH₃-Phenyl | OCH₃ | CH₃ |
| 205 | 2-OCF₃-Phenyl | OCH₃ | CH₃ |
| 206 | 3-OCF₃-Phenyl | OCH₃ | CH₃ |
| 207 | 4-OCF₃-Phenyl | OCH₃ | CH₃ |
| 208 | 2-OCHF₂-Phenyl | OCH₃ | CH₃ |
| 209 | 3-OCHF₂-Phenyl | OCH₃ | CH₃ |
| 210 | 4-OCHF₂-Phenyl | OCH₃ | CH₃ |
| 211 | 2-CF₃-Phenyl | OCH₃ | CH₃ |
| 212 | 3-CF₃-Phenyl | OCH₃ | CH₃ |
| 213 | 4-CF₃-Phenyl | OCH₃ | CH₃ |
| 214 | 2-CH₃-Phenyl | OCH₃ | CH₃ |
| 215 | 3-CH₃-Phenyl | OCH₃ | CH₃ |
| 216 | 4-CH₃-Phenyl | OCH₃ | CH₃ |
| 217 | 2-NO₂-Phenyl | OCH₃ | CH₃ |
| 218 | 3-NO₂-Phenyl | OCH₃ | CH₃ |
| 219 | 4-NO₂-Phenyl | OCH₃ | CH₃ |
| 220 | 2-Pyridyl | OCH₃ | CH₃ |
| 221 | 3-Pyridyl | OCH₃ | CH₃ |
| 222 | 4-Pyridyl | OCH₃ | CH₃ |
| 223 | 3'-CH₃-2-pyridyl | OCH₃ | CH₃ |
| 224 | 4'-CH₃-2-pyridyl | OCH₃ | CH₃ |
| 225 | 5'-CH₃-2-pyridyl | OCH₃ | CH₃ |
| 226 | 6'-CH₃-2-pyridyl | OCH₃ | CH₃ |
| 227 | 2'-CH₃-3-pyridyl | OCH₃ | CH₃ |
| 228 | 4'-CH₃-3-pyridyl | OCH₃ | CH₃ |
| 229 | 5'-CH₃-3-pyridyl | OCH₃ | CH₃ |
| 230 | 6'-CH₃-3-pyridyl | OCH₃ | CH₃ |
| 231 | 2'-CH₃-4-pyridyl | OCH₃ | CH₃ |
| 232 | 3'-CH₃-4-pyridyl | OCH₃ | CH₃ |
| 233 | 3'-Cl-2-pyridyl | OCH₃ | CH₃ |
| 234 | 4'-Cl-2-pyridyl | OCH₃ | CH₃ |
| 235 | 5'-Cl-2-pyridyl | OCH₃ | CH₃ |
| 236 | 6'-Cl-2-pyridyl | OCH₃ | CH₃ |
| 237 | 2'-Cl-3-pyridyl | OCH₃ | CH₃ |
| 238 | 4'-Cl-3-pyridyl | OCH₃ | CH3 |
| 239 | 5'-Cl-3-pyridyl | OCH₃ | CH₃ |
| 240 | 6'-Cl-3-pyridyl | OCH₃ | CH₃ |
| 241 | 2'-Cl-4-pyridyl | OCH₃ | CH₃ |
| 242 | 3'-Cl-4-pyridyl | OCH₃ | CH₃ |
| 243 | Cyclohexylamino | OCH₃ | CH₃ |
| 244 | Cyclopentylamino | OCH₃ | CH₃ |
| 245 | Morpholino | OCH₃ | CH₃ |
| 246 | CO₂H | OCH₃ | CH₃ |
| 247 | CO₂CH₃ | OCH₃ | CH₃ |
| 248 | CO₂C₂H₅ | OCH₃ | CH₃ |
| 249 | CO₂-n-C₃H₇ | OCH₃ | CH₃ |
| 250 | CO₂-i-C₃H₇ | OCH₃ | CH₃ |
| 251 | CO₂-n-C₄H₉ | OCH₃ | CH₃ |
| 252 | CO₂-s-C₄H₉ | OCH₃ | CH₃ |
| 253 | CO₂-i-C₄H₉ | OCH₃ | CH₃ |
| 254 | CO₂-t-C₄H₉ | OCH₃ | CH₃ |
| 255 | CO₂-Ph | OCH₃ | CH₃ |
| 256 | CO₂-3-Pyridyl | OCH₃ | CH₃ |
| 257 | CONHCH₃ | OCH₃ | CH₃ |
| 258 | CONHC₂H₅ | OCH₃ | CH₃ |
| 259 | CONHPhenyl | OCH₃ | CH₃ |
| 260 | CON(CH₃)₂ | OCH₃ | CH₃ |
| 261 | CON(CH₂CH₃)₂ | OCH₃ | CH₃ |
| 262 | CON(Phenyl)₂ | OCH₃ | CH₃ |
| 263 | H | Cl | CH₃ |
| 264 | F | Cl | CH₃ |
| 265 | C1 | Cl | CH₃ |
| 266 | Br | Cl | CH₃ |
| 267 | OH | Cl | CH₃ |
| 268 | SH | Cl | CH₃ |
| 269 | NH₂ | Cl | CH₃ |
| 270 | CN | Cl | CH₃ |
| 271 | NO₂ | Cl | CH₃ |
| 272 | SCN | Cl | CH₃ |
| 273 | NH-NH₂ | Cl | CH₃ |
| 274 | CH₃ | Cl | CH₃ |
| 275 | C₂H₅ | Cl | CH₃ |
| 276 | n-C₃H₇ | Cl | CH₃ |
| 277 | i-C₃H₇ | Cl | CH₃ |
| 278 | n-C₄H₉ | Cl | CH₃ |
| 279 | S-C₄H₉ | Cl | CH₃ |
| 280 | i-C₄H₉ | Cl | CH₃ |
| 281 | t-C₄H₉ | Cl | CH₃ |
| 282 | CH₂Cl | Cl | CH₃ |
| 283 | CHCl₂ | Cl | CH₃ |
| 284 | CCl₃ | Cl | CH₃ |
| 285 | CH₂F | Cl | CH₃ |
| 286 | CHF₂ | Cl | CH₃ |
| 287 | CF₃ | Cl | CH₃ |
| 288 | CH₂CF₃ | Cl | CH₃ |
| 289 | CH₂OCH₃ | Cl | CH₃ |
| 290 | CH₂OCH₂CH₃ | Cl | CH₃ |
| 291 | CH₂NH₂ | Cl | CH₃ |
| 292 | OCH₃ | Cl | CH₃ |
| 293 | OC₂H₅ | Cl | CH₃ |
| 294 | O-n-C₃H₇ | Cl | CH₃ |
| 295 | O-i-C₃H₇ | Cl | CH₃ |
| 296 | O-n-C₄H₉ | Cl | CH₃ |
| 297 | O-s-C4H9 | Cl | CH₃ |
| 298 | O-i-C₄H₉ | Cl | CH₃ |
| 299 | O-t-C₄H₉ | Cl | CH₃ |
| 300 | OCHF₂ | Cl | CH₃ |
| 301 | OCF₃ | Cl | CH₃ |
| 302 | OCH₂CF₃ | Cl | CH₃ |
| 303 | OCH₂OCH₃ | Cl | CH₃ |
| 304 | SCH₃ | Cl | CH₃ |
| 305 | SC₂H₅ | Cl | CH₃ |
| 306 | S-n-C₃H₇ | Cl | CH₃ |
| 307 | S-i-C₃H₇ | Cl | CH₃ |
| 308 | S-n-C₄H₉ | Cl | CH₃ |
| 309 | S-S-C₄H₉ | Cl | CH₃ |
| 310 | S-i-C₄H₉ | Cl | CH₃ |
| 311 | S-t-C₄H₉ | Cl | CH₃ |
| 312 | SCHF₂ | Cl | CH₃ |
| 313 | SCF₃ | Cl | CH₃ |
| 314 | SCH₂CF₃ | Cl | CH₃ |
| 315 | SCH₂OCH₃ | Cl | CH₃ |
| 316 | NHCH₃ | Cl | CH₃ |
| 317 | NHC₂H₅ | Cl | CH₃ |
| 318 | NH-Phenyl | Cl | CH₃ |
| 319 | N(CH₃)₂ | Cl | CH₃ |
| 320 | N(CH₂CH₃)₂ | Cl | CH₃ |
| 321 | N(Phenyl)₂ | Cl | CH₃ |
| 322 | (CH₂)₂COCH₃ | Cl | CH₃ |
| 323 | Phenyl | Cl | CH₃ |
| 324 | 2-F-Phenyl | Cl | CH₃ |
| 325 | 3-F-Phenyl | Cl | CH₃ |
| 362 | 4-F-Phenyl | Cl | CH₃ |
| 327 | 2-Cl-Phenyl | Cl | CH₃ |
| 328 | 3-Cl-Phenyl | Cl | CH₃ |
| 329 | 4-Cl-Phenyl | Cl | CH₃ |
| 330 | 2-OH-Phenyl | Cl | CH₃ |
| 331 | 3-OH-Phenyl | Cl | CH₃ |
| 332 | 4-OH-Phenyl | Cl | CH₃ |
| 333 | 2-OCH₃-Phenyl | Cl | CH₃ |
| 334 | 3-OCH₃-Phenyl | Cl | CH₃ |
| 335 | 4-OCH₃-Phenyl | Cl | CH₃ |
| 336 | 2-OCF₃-Phenyl | Cl | CH₃ |
| 337 | 3-OCF₃-Phenyl | Cl | CH₃ |
| 338 | 4-OCF₃-Phenyl | Cl | CH₃ |
| 339 | 2-OCHF₂-Phenyl | Cl | CH₃ |
| 340 | 3- OCHF₂-Phenyl | Cl | CH₃ |
| 341 | 4-OCHF₂-Phenyl | Cl | CH₃ |
| 342 | 2-CF₃-Phenyl | Cl | CH₃ |
| 343 | 3-CF₃-Phenyl | Cl | CH₃ |
| 344 | 4-CF₃-Phenyl | Cl | CH₃ |
| 345 | 2-CH₃-Phenyl | Cl | CH₃ |
| 346 | 3-CH₃-Phenyl | Cl | CH₃ |
| 347 | 4-CH₃-Phenyl | Cl | CH₃ |
| 348 | 2-NO₂-Phenyl | Cl | CH₃ |
| 349 | 3-NO₂-Phenyl | Cl | CH₃ |
| 350 | 4-NO₂-Phenyl | Cl | CH₃ |
| 351 | 2-Pyridyl | Cl | CH₃ |
| 352 | 3-Pyridyl | Cl | CH₃ |
| 353 | 4-Pyridyl | Cl | CH₃ |
| 354 | 3'-CH₃-2-pyridyl | Cl | CH₃ |
| 355 | 4'-CH₃-2-pyridyl | Cl | CH₃ |
| 356 | 5'-CH₃-2-pyridyl | Cl | CH₃ |
| 357 | 6'-CH₃-2-pyridyl | Cl | CH₃ |
| 358 | 2'-CH₃-3-pyridyl | Cl | CH₃ |
| 359 | 4'-CH₃-3-pyridyl | Cl | CH₃ |
| 360 | 5'-CH₃-3-pyridyl | Cl | CH₃ |
| 361 | 6'-CH₃-3-pyridyl | Cl | CH₃ |
| 362 | 2'-CH₃-4-pyridyl | Cl | CH₃ |
| 363 | 3'-CH₃-4-pyridyl | Cl | CH₃ |
| 364 | 3'-Cl-2-pyridyl | Cl | CH₃ |
| 365 | 4'-Cl-2-pyridyl | Cl | CH₃ |
| 366 | 5'-Cl-2-pyridyl | Cl | CH₃ |
| 367 | 6'-Cl-2-pyridyl | Cl | CH₃ |
| 368 | 2'-Cl-3-pyridyl | Cl | CH₃ |
| 369 | 4'-Cl-3-pyridyl | Cl | CH₃ |
| 370 | 5'-Cl-3-pyridyl | Cl | CH₃ |
| 371 | 6'-Cl-3-pyridyl | Cl | CH₃ |
| 372 | 2'-Cl-4-pyridyl | Cl | CH₃ |
| 373 | 3'-Cl-4-pyridyl | Cl | CH₃ |
| 374 | Cyclohexylamino | Cl | CH₃ |
| 375 | Cyclopentylamino | Cl | CH₃ |
| 376 | Morpholino | Cl | CH₃ |
| 377 | CO₂H | Cl | CH₃ |
| 378 | CO₂CH₃ | Cl | CH₃ |
| 379 | CO₂C₂H₅ | Cl | CH₃ |
| 380 | CO₂-n-C₃H₇ | Cl | CH₃ |
| 381 | CO₂-i-C₃H₇ | Cl | CH₃ |
| 382 | CO₂-n-C₄H₉ | Cl | CH₃ |
| 383 | CO₂-S-C₄H₉ | Cl | CH₃ |
| 384 | CO₂-i-C₄H₉ | Cl | CH₃ |
| 385 | CO₂-t-C₄H₉ | Cl | CH₃ |
| 386 | CO₂-Phenyl | Cl | CH₃ |
| 387 | CO₂-3-Pyridyl | Cl | CH₃ |
| 388 | CONHCH₃ | Cl | CH₃ |
| 389 | CONHC₂H₅ | Cl | CH₃ |
| 390 | CONH-Phenyl | Cl | CH₃ |
| 391 | CON(CH₃)₂ | Cl | CH₃ |
| 392 | CON(CH₂CH₃)₂ | Cl | CH₃ |
| 393 | CON(Phenyl)₂ | Cl | CH₃ |
| 394 | H | CH₃ | H |
| 394 | F | CH₃ | H |
| 396 | Cl | CH₃ | H |
| 397 | Br | CH₃ | H |
| 398 | OH | CH₃ | H |
| 399 | SH | CH₃ | H |
| 400 | NH₂ | CH₃ | H |
| 401 | CN | CH₃ | H |
| 402 | NO₂ | CH₃ | H |
| 403 | SCN | CH₃ | H |
| 404 | NH-NH2 | CH₃ | H |
| 405 | CH₃ | CH₃ | H |
| 406 | C₂H₅ | CH₃ | H |
| 407 | n-C₃H₇ | CH₃ | H |
| 408 | i-C₃H₇ | CH₃ | H |
| 049 | n-C₄H₉ | CH₃ | H |
| 410 | s-C₄H₉ | CH₃ | H |
| 411 | 1-C₄H₉ | CH₃ | H |
| 412 | t-C₄H₉ | CH₃ | H |
| 413 | CH₂Cl | CH₃ | H |
| 414 | CHCl₂ | CH₃ | H |
| 415 | CCl₃ | CH₃ | H |
| 416 | CH₂F | CH₃ | H |
| 417 | CHF₂ | CH₃ | H |
| 418 | CF₃ | CH₃ | H |
| 419 | CH₂CF₃ | CH₃ | H |
| 420 | CH₂OCH₃ | CH₃ | H |
| 421 | CH₂OCH₂CH₃ | CH₃ | H |
| 422 | CH₂NH₂ | CH₃ | H |
| 423 | OCH₃ | CH₃ | H |
| 424 | OC₂H₅ | CH₃ | H |
| 425 | O-n-C₃H₇ | CH₃ | H |
| 462 | O-i-C₃H₇ | CH₃ | H |
| 427 | O-n-C₄H₉ | CH₃ | H |
| 428 | O-S-C₄H₉ | CH₃ | H |
| 429 | O-i-C₄H₉ | CH₃ | H |
| 430 | O-t-C₄H₉ | CH₃ | H |
| 431 | OCHF₂ | CH₃ | H |
| 432 2 | OCF₃ | CH₃ | H |
| 433 | OCH₂CF₃ | CH₃ | H |
| 434 | OCH₂OCH₃ | CH₃ | H |
| 435 | SCH₃ | CH₃ | H |
| 436 | SC₂H₅ | CH₃ | H |
| 437 | S-n-C₃H₇ | CH₃ | H |
| 438 | S-i-C₃H₇ | CH₃ | H |
| 439 | S-n-C₄H₉ | CH₃ | H |
| 440 | S-S-C₄H₉ | CH₃ | H |
| 441 | S-i-C₄H₉ | CH₃ | H |
| 442 | S-t-C₄H₉ | CH₃ | H |
| 443 | SCHF₂ | CH₃ | H |
| 444 | SCF₃ | CH₃ | H |
| 445 | SCH₂CF₃ | CH₃ | H |
| 446 | SCH₂OCH₃ | CH₃ | H |
| 447 | NHCH₃ | CH₃ | H |
| 448 | NHC₂H₅ | CH₃ | H |
| 449 | NH-Phenyl | CH₃ | H |
| 450 | N(CH₃)₂ | CH₃ | H |
| 451 | N(CH₂CH₃)₂ | CH₃ | H |
| 452 | N(Phenyl)₂ | CH₃ | H |
| 453 | (CH₂)₂COCH₃ | CH₃ | H |
| 454 | Phenyl | CH₃ | H |
| 455 | 2-F-Phenyl | CH₃ | H |
| 456 | 3-F-Phenyl | CH₃ | H |
| 457 | 4-F-Phenyl | CH₃ | H |
| 458 | 2-Cl-Phenyl | CH₃ | H |
| 459 | 3-Cl-Phenyl | CH₃ | H |
| 460 | 4-Cl-Phenyl | CH₃ | H |
| 461 | 2-OH-Phenyl | CH₃ | H |
| 462 | 3-OH-Phenyl | CH₃ | H |
| 463 | 4-OH-Phenyl | CH₃ | H |
| 464 | 2-OCH₃-Phenyl | CH₃ | H |
| 465 | 3-OCH₃-Phenyl | CH₃ | H |
| 466 | 4-OCH₃-Phenyl | CH₃ | H |
| 467 | 2-OCF₃-Phenyl | CH₃ | H |
| 468 | 3-OCF₃-Phenyl | CH₃ | H |
| 469 | 4-OCF₃-Phenyl | CH₃ | H |
| 470 | 2-OCHF₂-Phenyl | CH₃ | H |
| 471 | 3- OCHF₂-Phenyl | CH₃ | H |
| 472 | 4-OCHF₂-Phenyl | CH₃ | H |
| 473 | 2-CF₃-Phenyl | CH₃ | H |
| 474 | 3-CF₃-Phenyl | CH₃ | H |
| 475 | 4-CF₃-Phenyl | CH₃ | H |
| 476 | 2-CH₃-Phenyl | CH₃ | H |
| 477 | 3-CH₃-Phenyl | CH₃ | H |
| 478 | 4-CH₃-Phenyl | CH₃ | H |
| 479 | 2-NO₂-Phenyl | CH₃ | H |
| 480 | 3-NO₂-Phenyl | CH₃ | H |
| 481 | 4-NO₂-Phenyl | CH₃ | H |
| 482 | 2-Pyridyl | CH₃ | H |
| 483 | 3-Pyridyl | CH₃ | H |
| 484 | 4-Pyridyl | CH₃ | H |
| 485 | 3'-CH₃-2-pyridyl | CH₃ | H |
| 486 | 4'-CH₃-2-pyridyl | CH₃ | H |
| 487 | 5'-CH₃-2-pyridyl | CH₃ | H |
| 488 | 6'-CH₃-2-pyridyl | CH₃ | H |
| 489 | 2'-CH₃-3-pyridyl | CH₃ | H |
| 490 | 4'-CH₃-3-pyridyl | CH₃ | H |
| 491 | 5'-CH₃-3-pyridyl | CH₃ | H |
| 492 | 6'-CH₃-3-pyridyl | CH₃ | H |
| 493 | 2'-CH₃-4-pyridyl | CH₃ | H |
| 494 | 3'-CH₃-4-pyridyl | CH₃ | H |
| 495 | 3'-Cl-2-pyridyl | CH₃ | H |
| 496 | 4'-Cl-2-pyridyl | CH₃ | H |
| 497 | 5'-Cl-2-pyridyl | CH₃ | H |
| 498 | 6'-Cl-2-pyridyl | CH₃ | H |
| 499 | 2'-Cl-3-pyridyl | CH₃ | H |
| 500 | 4'-Cl-3-pyridyl | CH₃ | H |
| 501 | 5'-Cl-3-pyridyl | CH₃ | H |
| 502 | 6'-Cl-3-pyridyl | CH₃ | H |
| 503 | 2'-Cl-4-pyridyl | CH₃ | H |
| 504 | 3'-Cl-4-pyridyl | CH₃ | H |
| 505 | Cyclohexylamino | CH₃ | H |
| 506 | Cyclopentylamino | CH₃ | H |
| 507 | Morpholino | CH₃ | H |
| 508 | CO₂H | CH₃ | H |
| 509 | CO₂CH₃ | CH₃ | H |
| 510 | CO₂C₂H₅ | CH₃ | H |
| 511 | CO₂-n-C₃H₇ | CH₃ | H |
| 512 | CO₂-i-C₃H₇ | CH₃ | H |
| 513 | CO₂-n-C₄H₉ | CH₃ | H |
| 514 | CO₂-S-C₄H₉ | CH₃ | H |
| 515 | CO₂-i-C₄H₉ | CH₃ | H |
| 516 | CO₂-t-C₄H₉ | CH₃ | H |
| 517 | CO₂-Ph | CH₃ | H |
| 518 | CO₂-3-Pyridyl | CH₃ | H |
| 519 | CONHCH₃ | CH₃ | H |
| 520 | CONHC₂H₅ | CH₃ | H |
| 521 | CONH-Phenyl | CH₃ | H |
| 522 | CON(CH₃)₂ | CH₃ | H |
| 523 | CON(CH₂CH₃)₂ | CH₃ | H |
| 524 | CON(Phenyl)₂ | CH₃ | H |
| 525 | H | OCH₃ | H |
| 526 | F | OCH₃ | H |
| 527 | Cl | OCH₃ | H |
| 528 | Br | OCH₃ | H |
| 529 | OH | OCH₃ | H |
| 530 | SH | OCH₃ | H |
| 531 | NH₂ | OCH₃ | H |
| 532 | CN | OCH₃ | H |
| 533 | NO₂ | OCH₃ | H |
| 534 | SCN | OCH₃ | H |
| 535 | NH-NH₂ | OCH₃ | H |
| 536 | CH₃ | OCH₃ | H |
| 537 | C₂H₅ | OCH₃ | H |
| 538 | n-C₃H₇ | OCH₃ | H |
| 539 | i-C₃H₇ | OCH₃ | H |
| 540 | n-C₄H₉ | OCH₃ | H |
| 541 | s-C₄H₉ | OCH₃ | H |
| 542 | i-C₄H₉ | OCH₃ | H |
| 543 | t-C₄H₉ | OCH₃ | H |
| 544 | CH₂Cl | OCH₃ | H |
| 545 | CHCl₂ | OCH₃ | H |
| 546 | CCl₃ | OCH₃ | H |
| 547 | CH₂F | OCH₃ | H |
| 548 | CHF₂ | OCH₃ | H |
| 549 | CF₃ | OCH₃ | H |
| 550 | CH₂CF₃ | OCH₃ | H |
| 551 | CH₂OCH₃ | OCH₃ | H |
| 552 | CH₂OCH₂CH₃ | OCH₃ | H |
| 553 | CH₂NH₂ | OCH₃ | H |
| 554 | OCH₃ | OCH₃ | H |
| 555 | OC₂H₅ | OCH₃ | H |
| 556 | O-n-C₃H₇ | OCH₃ | H |
| 557 | O-i-C₃H₇ | OCH₃ | H |
| 558 | O-n-C₄H₉ | OCH₃ | H |
| 559 | O-S-C₄H₉ | OCH₃ | H |
| 560 | O-i-C₄H₉ | OCH₃ | H |
| 561 | O-t-C₄H₉ | OCH₃ | H |
| 562 | OCHF₂ | OCH₃ | H |
| 563 | OCF₃ | OCH₃ | H |
| 564 | OCH₂CF₃ | OCH₃ | H |
| 565 | OCH₂OCH₃ | OCH₃ | H |
| 566 | SCH₃ | OCH₃ | H |
| 567 | SC₂H₅ | OCH₃ | H |
| 568 | S-n-C₃H₇ | OCH₃ | H |
| 569 | S-i-C₃H₇ | OCH₃ | H |
| 570 | S-n-C₄H₉ | OCH₃ | H |
| 571 | S-S-C₄H9 | OCH₃ | H |
| 572 | S-i-C₄H₉ | OCH₃ | H |
| 573 | S-t-C₄H₉ | OCH₃ | H |
| 574 | SCHF₂ | OCH₃ | H |
| 575 | SCF₃ | OCH₃ | H |
| 576 | SCH₂CF₃ | OCH₃ | H |
| 577 | SCH₂OCH₃ | OCH₃ | H |
| 578 | NHCH₃ | OCH₃ | H |
| 579 | NHC₂H₅ | OCH₃ | H |
| 580 | NHPh | OCH₃ | H |
| 581 | N(CH₃)₂ | OCH₃ | H |
| 582 | N(CH₂CH₃)₂ | OCH₃ | H |
| 583 | N(Phenyl)₂ | OCH₃ | H |
| 584 | (CH₂)₂COCH₃ | OCH₃ | H |
| 585 | Phenyl | OCH₃ | H |
| 586 | 2-F-Phenyl | OCH₃ | H |
| 587 | 3-F-Phenyl | OCH₃ | H |
| 588 | 4-F-Phenyl | OCH₃ | H |
| 589 | 2-Cl-Phenyl | OCH₃ | H |
| 590 | 3-Cl-Phenyl | OCH₃ | H |
| 591 | 4-Cl-Phenyl | OCH₃ | H |
| 592 | 2-OH-Phenyl | OCH₃ | H |
| 593 | 3-OH-Phenyl | OCH₃ | H |
| 594 | 4-OH-Phenyl | OCH₃ | H |
| 595 | 2-OCH₃-Phenyl | OCH₃ | H |
| 596 | 3-OCH₃-Phenyl | OCH₃ | H |
| 597 | 4-OCH₃-Phenyl | OCH₃ | H |
| 598 | 2-OCF₃-Phenyl | OCH₃ | H |
| 599 | 3-OCF₃-Phenyl | OCH₃ | H |
| 600 | 4-OCF₃-Phenyl | OCH₃ | H |
| 601 | 2-OCHF₂-Phenyl | OCH₃ | H |
| 602 | 3-OCHF₂-Phenyl | OCH₃ | H |
| 603 | 4-OCHF₂-Phenyl | OCH₃ | H |
| 604 | 2-CF₃-Phenyl | OCH₃ | H |
| 605 | 3-CF₃-Phenyl | OCH₃ | H |
| 606 | 4-CF₃-Phenyl | OCH₃ | H |
| 607 | 2-CH₃-Phenyl | OCH₃ | H |
| 608 | 3-CH₃-Phenyl | OCH₃ | H |
| 609 | 4-CH₃-Phenyl | OCH₃ | H |
| 610 | 2-NO₂-Phenyl | OCH₃ | H |
| 611 | 3-NO₂-Phenyl | OCH₃ | H |
| 612 | 4-NO₂-Phenyl | OCH₃ | H |
| 613 | 2-Pyridyl | OCH₃ | H |
| 614 | 3-Pyridyl | OCH₃ | H |
| 615 | 4-Pyridyl | OCH₃ | H |
| 616 | 3'-CH₃-2-pyridyl | OCH₃ | H |
| 617 | 4'-CH₃-2-pyridyl | OCH₃ | H |
| 618 | 5'-CH₃-2-pyridyl | OCH₃ | H |
| 619 | 6'-CH₃-2-pyridyl | OCH₃ | H |
| 620 | 2'-CH₃-3-pyridyl | OCH₃ | H |
| 621 | 4'-CH₃-3-pyridyl | OCH₃ | H |
| 622 | 5'-CH₃-3-pyridyl | OCH₃ | H |
| 623 | 6'-CH₃-3-pyridyl | OCH₃ | H |
| 624 | 2'-CH₃-4-pyridyl | OCH₃ | H |
| 625 | 3'-CH₃-4-pyridyl | OCH₃ | H |
| 626 | 3'-Cl-2-pyridyl | OCH₃ | H |
| 627 | 4'-Cl-2-pyridyl | OCH₃ | H |
| 628 | 5'-Cl-2-pyridyl | OCH₃ | H |
| 629 | 6'-Cl-2-pyridyl | OCH₃ | H |
| 630 | 2'-Cl-3-pyridyl | OCH₃ | H |
| 631 | 4'-Cl-3-pyridyl | OCH₃ | H |
| 632 | 5'-Cl-3-pyridyl | OCH₃ | H |
| 633 | 6'-Cl-3-pyridyl | OCH₃ | H |
| 634 | 2'-Cl-4-pyridyl | OCH₃ | H |
| 635 | 3'-Cl-4-pyridyl | OCH₃ | H |
| 636 | Cyclohexylamino | OCH₃ | H |
| 637 | Cyclopentylamino | OCH₃ | H |
| 638 | Morpholino | OCH₃ | H |
| 639 | CO₂H | OCH₃ | H |
| 640 | CO₂CH3 | OCH₃ | H |
| 641 | CO₂C₂H₅ | OCH₃ | H |
| 642 | CO₂-n-C₃H₇ | OCH₃ | H |
| 643 | CO₂-i-C₃H₇ | OCH₃ | H |
| 644 | CO₂-n-C4H9 | OCH₃ | H |
| 645 | CO₂-S-C₄H₉ | OCH₃ | H |
| 646 | CO₂-i-C₄H₉ | OCH₃ | H |
| 647 | CO₂-t-C₄H₉ | OCH₃ | H |
| 648 | CO₂-Ph | OCH₃ | H |
| 649 | CO₂-3-Pyridyl | OcH₃ | H |
| 650 | CONHCH₃ | OCH₃ | H |
| 651 | CONHC₂H₅ | OCH₃ | H |
| 652 | CONH-Phenyl | OCH₃ | H |
| 653 | CON(CH₃)₂ | OCH₃ | H |
| 654 | CON(CH₂CH₃)₂ 2 | OCH₃ | H |
| 655 | CON(Phenyl)₂ | OCH₃ | H |
| 656 | H | Cl | H |
| 657 | F | Cl | H |
| 658 | Cl | Cl | H |
| 659 | Br | Cl | H |
| 660 | OH | Cl | H |
| 661 | SH | Cl | H |
| 662 | NH₂ | Cl | H |
| 663 | CN | Cl | H |
| 664 | NO₂ | Cl | H |
| 665 | SCN | Cl | H |
| 666 | NH-NH₂ | Cl | H |
| 667 | CH₃ | Cl | H |
| 668 | C₂H₅ | Cl | H |
| 669 | n-C₃H₇ | Cl | H |
| 670 | i-C₃H₇ | Cl | H |
| 671 | n-C₄H₉ | Cl | H |
| 672 | s-C₄H₉ | Cl | H |
| 673 | i-C₄H₉ | Cl | H |
| 674 | t-C₄H₉ | Cl | H |
| 675 | CH₂Cl | Cl | H |
| 676 | CHCl₂ | Cl | H |
| 677 | CC1₃ | Cl | H |
| 678 | CH₂F | Cl | H |
| 679 | CHF₂ | Cl | H |
| 680 | CF₃ | Cl | H |
| 681 | CH₂CF₃ | Cl | H |
| 682 | CH₂OCH₃ | Cl | H |
| 683 | CH₂OCH₂CH₃ | Cl | H |
| 684 | CH₂NH₂ | Cl | H |
| 685 | OCH₃ | Cl | H |
| 686 | OC₂H₅ | Cl | H |
| 687 | O-n-C₃H₇ | Cl | H |
| 688 | O-i-C₃H₇ | Cl | H |
| 689 | O-n-C₄H₉ | Cl | H |
| 690 | O-s-C₄H₉ | Cl | H |
| 691 | O-i-C₄H₉ | Cl | H |
| 692 | O-t-C₄H₉ | Cl | H |
| 693 | OCHF₂ | Cl | H |
| 694 | OCF₃ | Cl | H |
| 695 | OCH₂CF₃ | Cl | H |
| 696 | OCH₂OCH₃ | Cl | H |
| 697 | SCH₃ | Cl | H |
| 698 | SC₂H₅ | Cl | H |
| 699 | S-n-C₃H₇ | Cl | H |
| 700 | S-i-C₃H₇ | Cl | H |
| 701 | S-n-C₄H₉ | Cl | H |
| 702 | S-S-C₄H₉ | Cl | H |
| 703 | S-i-C₄H₉ | Cl | H |
| 704 | S-t-C₄H₉ | Cl | H |
| 705 | SCHF₂ | Cl | H |
| 706 | SCF₃ | Cl | H |
| 707 | SCH₂CF₃ | Cl | H |
| 708 | SCH₂OCH₂ | Cl | H |
| 709 | NHCH₃ | Cl | H |
| 710 | NHC₂H₅ | Cl | H |
| 711 | NHPh | Cl | H |
| 712 | N(CH₃)₂ | Cl | H |
| 713 | N(CH₂CH₃)₂ | Cl | H |
| 714 | N(Phenyl)₂ | Cl | H |
| 715 | (CH₂)₂COCH₃ | Cl | H |
| 716 | Phenyl | Cl | H |
| 717 | 2-F-Phenyl | Cl | H |
| 718 | 3-F-Phenyl | Cl | H |
| 719 | 4-F-Phenyl | Cl | H |
| 720 | 2-Cl-Phenyl | Cl | H |
| 721 | 3-Cl-Phenyl | Cl | H |
| 722 | 4-Cl-Phenyl | Cl | H |
| 723 | 2-OH-Phenyl | Cl | H |
| 724 | 3-OH-Phenyl | Cl | H |
| 725 | 4-OH-Phenyl | Cl | H |
| 726 | 2-OCH₃-Phenyl | Cl | H |
| 727 | 3-OCH₃-Phenyl | Cl | H |
| 728 | 4-OCH₃-Phenyl | Cl | H |
| 729 | 2-OCF₃-Phenyl | Cl | H |
| 730 | 3-OCF₃-Phenyl | Cl | H |
| 731 | 4-OCF₃-Phenyl | Cl | H |
| 732 | 2-OCHF₂-Phenyl | Cl | H |
| 733 | 3- OCHF₂-Phenyl | Cl | H |
| 734 | 4-OCHF₂-Phenyl | Cl | H |
| 735 | 2-CF₃-Phenyl | Cl | H |
| 736 | 3-CF₃-Phenyl | Cl | H |
| 737 | 4-CF₃-Phenyl | Cl | H |
| 738 | 2-CH₃-Phenyl | Cl | H |
| 739 | 3-CH₃-Phenyl | Cl | H |
| 740 | 4-CH₃-Phenyl | Cl | H |
| 741 | 2-NO₂-Phenyl | Cl | H |
| 742 | 3-NO₂-Phenyl | Cl | H |
| 743 | 4-NO₂-Phenyl | Cl | H |
| 744 | 2-Pyridyl | Cl | H |
| 745 | 3-Pyridyl | Cl | H |
| 746 | 4-Pyridyl | Cl | H |
| 747 | 3'-CH₃-2-pyridyl | Cl | H |
| 748 | 4'-CH₃-2-pyridyl | Cl | H |
| 749 | 5'-CH₃-2-pyridyl | Cl | H |
| 750 | 6'-CH₃-2-pyridyl | Cl | H |
| 751 | 2'-CH₃-3-pyridyl | Cl | H |
| 752 | 4'-CH₃-3-pyridyl | Cl | H |
| 753 | 5'-CH₃-3-pyridyl | Cl | H |
| 754 | 6'-CH₃-3-pyridyl | Cl | H |
| 755 | 2'-CH₃-4-pyridyl | Cl | H |
| 756 | 3'-CH₃-4-pyridyl | Cl | H |
| 757 | 3'-Cl-2-pyridyl | Cl | H |
| 758 | 4'-Cl-2-pyridyl | Cl | H |
| 759 | 5'-Cl-2-pyridyl | Cl | H |
| 760 | 6'-Cl-2-pyridyl | Cl | H |
| 761 | 2'-Cl-3-pyridyl | Cl | H |
| 762 | 4'-Cl-3-pyridyl | Cl | H |
| 763 | 5'-Cl-3-pyridyl | Cl | H |
| 764 | 6'-Cl-3-pyridyl | Cl | H |
| 765 | 2'-Cl-4-pyridyl | Cl | H |
| 766 | 3'-Cl-4-pyridyl | Cl | H |
| 767 | Cyclohexylamino | Cl | H |
| 768 | Cyclopentylamino | Cl | H |
| 769 | Morpholino | Cl | H |
| 770 | CO₂H | Cl | H |
| 771 | CO₂CH₃ | Cl | H |
| 772 | CO₂C₂H₅ | Cl | H |
| 773 | CO₂-n-C₃H₇ | Cl | H |
| 774 | CO₂-i-C₃H₇ | Cl | H |
| 775 | CO₂-n-C₄H₉ | Cl | H |
| 776 | CO₂-s-C₄H₉ | Cl | H |
| 777 | CO₂-i-C₄H₉ | Cl | H |
| 778 | CO₂-t-C₄H₉ | Cl | H |
| 779 | CO₂-Phenyl | Cl | H |
| 780 | CO₂-3-Pyridyl | Cl | H |
| 781 | CONHCH₃ | Cl | H |
| 782 | CONHC₂H₅ | Cl | H |
| 783 | CONH-Phenyl | Cl | H |
| 784 | CON(CH₃)₂ | Cl | H |
| 785 | CON(CH₂CH₃)₂ | Cl | H |
| 786 | CON(Phenyl)₂ | Cl | H |
| 787 | 2-Fluorphenoxy | CH₃ | CH₃ |
| 788 | 2-Fluorphenoxy | OCH₃ | CH₃ |
| 789 | 2-Fluorphenoxy | Cl | CH₃ |
| 790 | 2-Fluorphenoxy | CH₃ | H |
| 791 | 2-Fluorphenoxy | OCH₃ | H |
| 792 | 2-Fluorphenoxy | Cl | H |
| 793 | Phenoxy | CH₃ | CH₃ |
| 794 | Phenoxy | OCH₃ | CH₃ |
| 795 | Phenoxy | Cl | CH₃ |
| 796 | Phenoxy | CH₃ | H |
| 797 | Phenoxy | OCH₃ | H |
| 798 | Phenoxy | Cl | H |
| 799 | 2-Methoxyphenoxy | CH₃ | CH₃ |
| 800 | 2-Methoxyphenoxy | OCH₃ | CH₃ |
| 801 | 2-Methoxyphenoxy | Cl | CH₃ |
| 802 | 2-Methoxyphenoxy | CH₃ | H |
| 803 | 2-Methoxyphenoxy | OCH₃ | H |
| 804 | 2-Methoxyphenoxy | Cl | H |
| 805 | Cyclopropyl | CH₃ | CH₃ |
| 806 | Cyclopropyl | OCH₃ | CH₃ |
| 807 | Cyclopropyl | Cl | CH₃ |
| 808 | Cyclopropyl | CH₃ | H |
| 809 | Cyclopropyl | OCH₃ | H |
| 810 | Cyclopropyl | Cl | H |
| Hier und im folgenden bedeuten beispielsweise: | | | |
| 2-F-Phenyl = 2-Fluorphenyl | | | |
| 2-Cl-Phenyl = 2-Chlorphenyl | | | |
| 2-OH-Phenyl = 2-Hydroxyphenyl | | | |
| 2-OCH₃-Phenyl = 2-Methoxyphenyl | | | |
| 2-OCF₃-Phenyl = 2-Trifluormethoxyphenyl | | | |
| 2-OCHF₂-Phenyl = 2-Difluormethoxyphenyl | | | |
| 2-NO₂-Phenyl = 2-Nitrophenyl | | | |
| 3'-CH₃-2-pyridyl = 3'-Methylpyridin-2-yl | | | |

Beispiele für erfindungsgemäße besonders bevorzugte Benzothiazol-5-ylcarbonyl-Derivate von Pyrazolen (Verbindungen I-1 = Verbindungen I mit X = C-R³ und Y = S) sind die in den Tabellen 2 bis 21 genannten Verbindungen.
- Tabelle 2: Verbindungen I-1a.1 bis I-1a.810 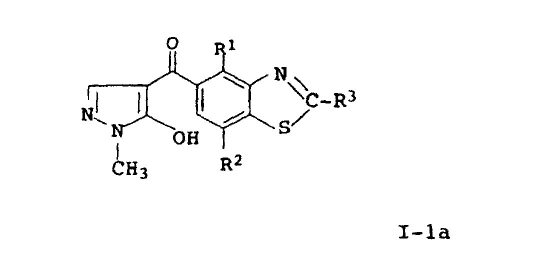 Verbindungen der allgemeinen Formel I-1a, in der die Substituenten R1, R2 und R3 für jede einzelne Verbindung jeweils einer Zeile der Tabelle A entsprechen.
- Tabelle 3: Verbindungen I-1b.1 bis I-1b.810 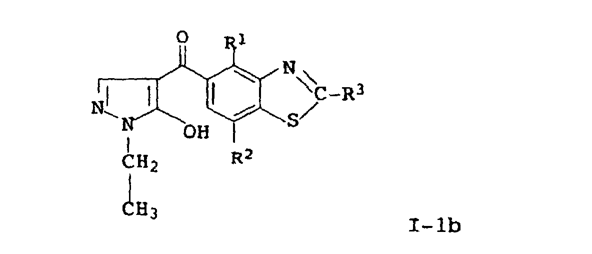 Verbindungen der allgemeinen Formel I-1b, in der die Substituenten R1, R2 und R3 für jede einzelne Verbindung jeweils einer Zeile der Tabelle A entsprechen.
- Tabelle 4: Verbindungen I-1c.1 bis I-1c.810 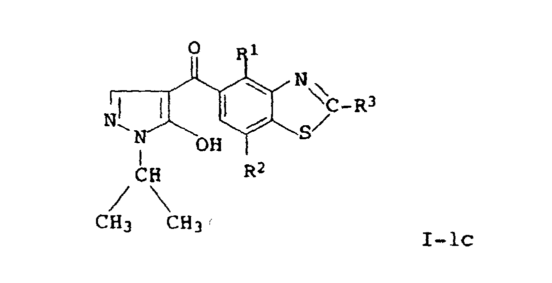 Verbindungen der allgemeinen Formel I-1c in der die Substituenten R1, R2 und R3 für jede einzelne Verbindung jeweils einer Zeile der Tabelle A entsprechen.
- Tabelle 5: Verbindungen I-1d.1 bis I-1d.810 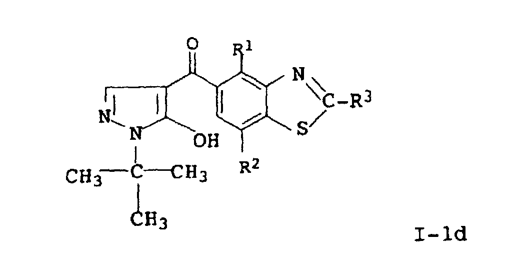 Verbindungen der allgemeinen Formel I-1b, in der die Substituenten R1, R2 und R3 für jede einzelne Verbindung jeweils einer Zeile der Tabelle A entsprechen.
- Tabelle 6: Verbindungen I-1e.1 bis I-1e.810 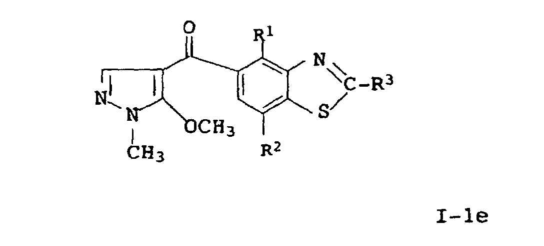 Verbindungen der allgemeinen Formel I-1e, in der die Substituenten R1 R2 und R3 für jede einzelne Verbindung jeweils einer Zeile der Tabelle A entsprechen.
- Tabelle 7: Verbindungen I-1f.1 bis I-1f.810 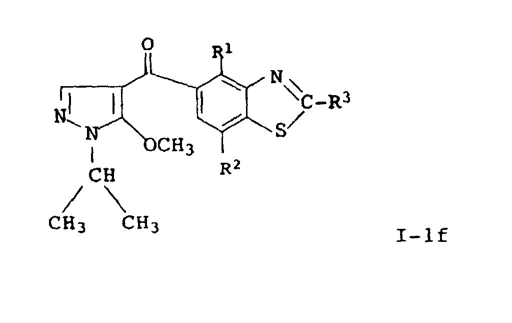 Verbindungen der allgemeinen Formel I-1f, in der die Substituenten R1, R2 und R3 für jede einzelne Verbindung jeweils einer Zeile der Tabelle A entsprechen.
- Tabelle 8: Verbindungen I-1g.1 bis I-1g.810 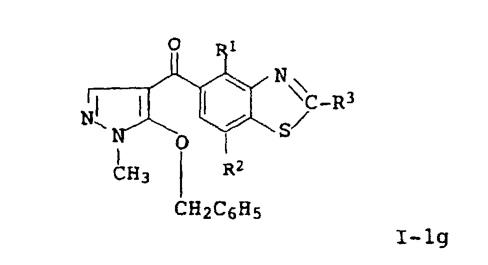 Verbindungen der allgemeinen Formel I-1g, in der die Substituenten R1, R2 und R3 für jede einzelne Verbindung jeweils einer Zeile der Tabelle A entsprechen.
- Tabelle 9: Verbindungen I-1h.1 bis I-1h.810 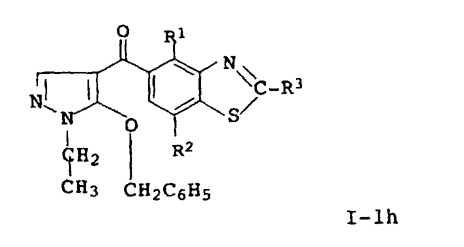 Verbindungen der allgemeinen Formel I-1h, in der die Substituenten R1, R2 und R3 für jede einzelne Verbindung jeweils einer Zeile der Tabelle A entsprechen.
- Tabelle 10: Verbindungen I-1i.1 bis I-1i.810 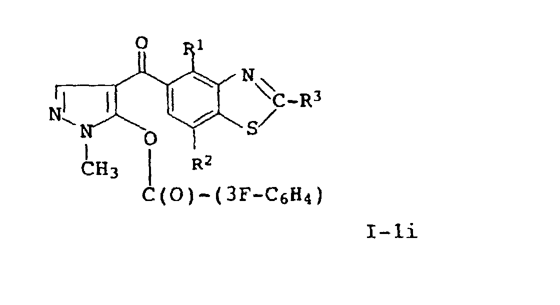 Verbindungen der allgemeinen Formel I-1i, in der die Substituenten R1, R2 und R3 für jede einzelne Verbindung jeweils einer Zeile der Tabelle A entsprechen.
- Tabelle l1: Verbindungen I-1k.1 bis I-lk.810 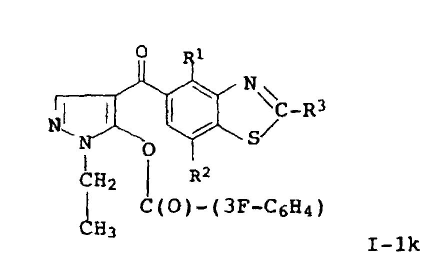 Verbindungen der allgemeinen Formel I-1k, in der die Substituenten R1, R2 und R3 für jede einzelne Verbindung jeweils einer Zeile der Tabelle A entsprechen.
- Tabelle 12: Verbindungen 1-1l.1 bis I-1l.810 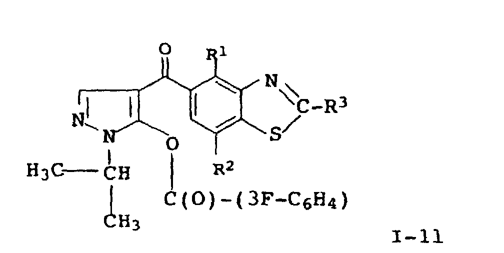 Verbindungen der allgemeinen Formel I-1l, in der die Substituenten R1, R2 und R3 für jede einzelne Verbindung jeweils einer Zeile der Tabelle A entsprechen.
- Tabelle 13: Verbindungen I-1m.1 bis I-1m.810 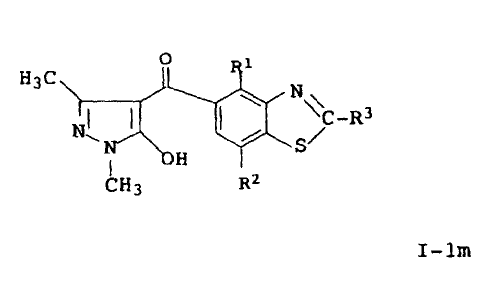 Verbindungen der allgemeinen Formel I-1m, in der die Substituenten R1, R2 und R3 für jede einzelne Verbindung jeweils einer Zeile der Tabelle A entsprechen.
- Tabelle 14: Verbindungen I-1n.1 bis I-1n.810 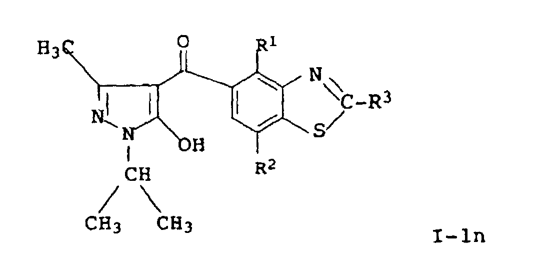 Verbindungen der allgemeinen Formel I-1n, in der die Substituenten R1, R2 und R3 für jede einzelne Verbindung jeweils einer Zeile der Tabelle A entsprechen.
- Tabelle 15: Verbindungen I-lo.1 bis I-1o.810 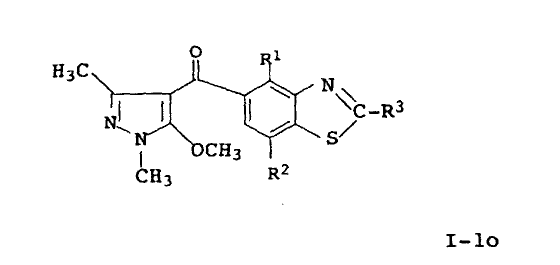 Verbindungen der allgemeinen Formel I-1p, in der die Substituenten R1, R2 und R3 für jede einzelne Verbindung jeweils einer Zeile der Tabelle A entsprechen.
- Tabelle 16: Verbindungen I-1p.1 bis I-1p.810 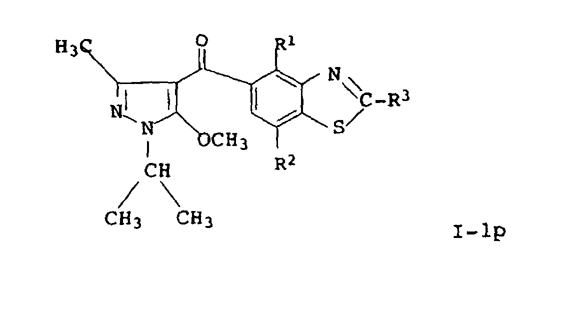 Verbindungen der allgemeinen Formel I-1p, in der die Substituenten R1, R2 und R3 für jede einzelne Verbindung jeweils einer Zeile der Tabelle A entsprechen.
- Tabelle 17: Verbindungen I-1q.1 bis I-1q.810 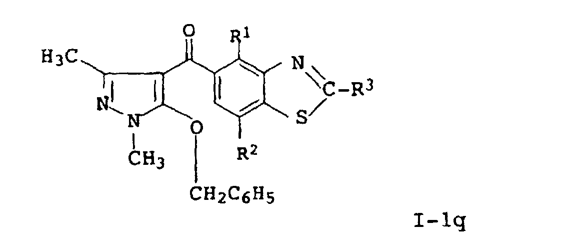 Verbindungen der allgemeinen Formel I-1g, in der die Substituenten R1, R2 und R3 für jede einzelne Verbindung jeweils einer Zeile der Tabelle A entsprechen.
- Tabelle 18: Verbindungen I-1r.1 bis I-1r.810 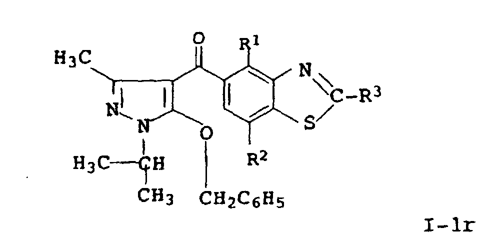 Verbindungen der allgemeinen Formel I-1r, in der die Substituenten R1, R2 und R3 für jede einzelne Verbindung jeweils einer Zeile der Tabelle A entsprechen.
- Tabelle 19: Verbindungen I-1s.1 bis I-1s.810 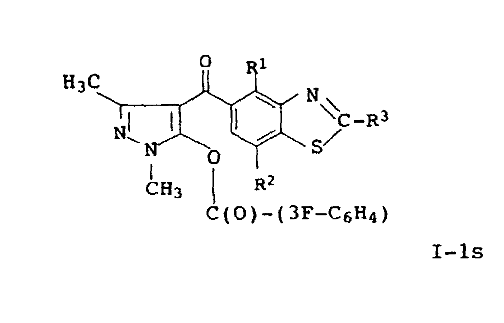 Verbindungen der allgemeinen Formel I-1s, in der die Substituenten R1, R2 und R3 für jede einzelne Verbindung jeweils einer Zeile der Tabelle A entsprechen.
- Tabelle 20: Verbindungen I-1t.1 bis I-1t.810 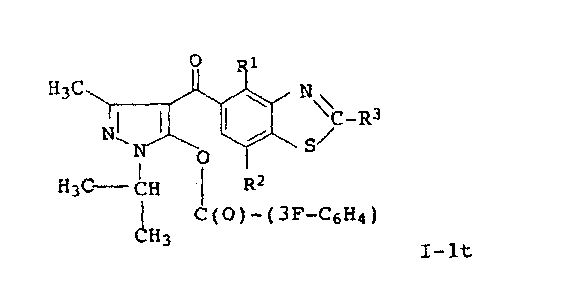 Verbindungen der allgemeinen Formel I-1t, in der die Substituenten R1, R2 und R3 für jede einzelne Verbindung jeweils einer Zeile der Tabelle A entsprechen.
- Tabelle 20a: Verbindungen I-1u.1 bis I-1u.810 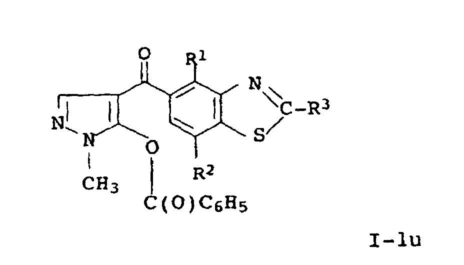 Verbindungen der allgemeinen Formel I-1u, in der die Substituenten R1, R2 und R3 für jede einzelne Verbindung jeweils einer Zeile der Tabelle A entsprechen.
- Tabelle 20b: Verbindungen I-1v.1 bis I-1v.810 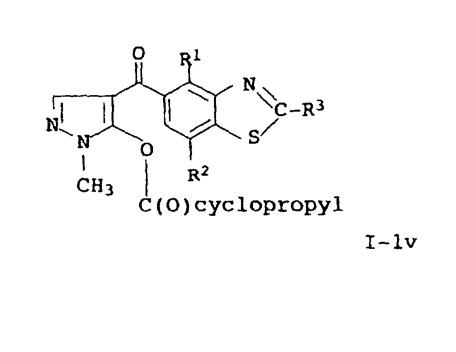 Verbindungen der allgemeinen Formel I-1v in der die Substituenten R1, R2 und R3 für jede einzelne Verbindung jeweils einer Zeile der Tabelle A entsprechen.
- Tabelle 20c: Verbindungen I-1w.1 bis I-1w.810 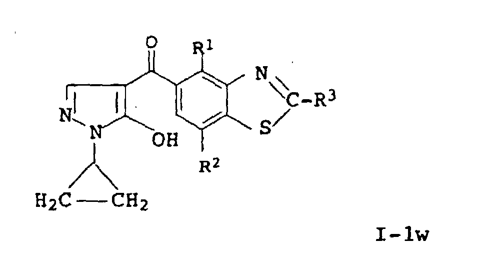 Verbindungen der allgemeinen Formel I-1w, in der die Substituenten R1, R2 und R3 für jede einzelne Verbindung jeweils einer Zeile der Tabelle A entsprechen.
   Beispiele für erfindungsgemäß besonders bevorzugte Pyrazolylderivate sind sind die Benzothiazol-S-dioxidverbindungen 1-1'a.1 bis 1-1'a.810, 1-1'b.1 bis 1-1'b.810, ................. 1-1'w.1 bis 1-1'w.810 (Verbindungen I-1' = Verbindungen I mit X = C-R₃ und Y = SO₂). Sie unterscheiden sich von den in den Tabellen 1 bis 21 aufgeführten Benzothiazolverbindungen 1-1a.1 bis 1-1a.810, 1-1b.1 bis 1-1b.810, ................. 1-1w.1 bis 1-1w.810 darin, daß das heterocyclische Schwefelatom als SO₂-Gruppe vorliegt.
   Beispiele für erfindungsgemäße besonders bevorzugte Pyrazolylderivate von Benzoxazol-5-carbonylverbindungen (Verbindungen 1-2 = Verbindungen I mit X = C-R³ und Y = O) sind die in den Tabellen 21 bis 40 genannten Verbindungen.
- Tabelle 21: Verbindungen I-2a.1 bis I-2a.810 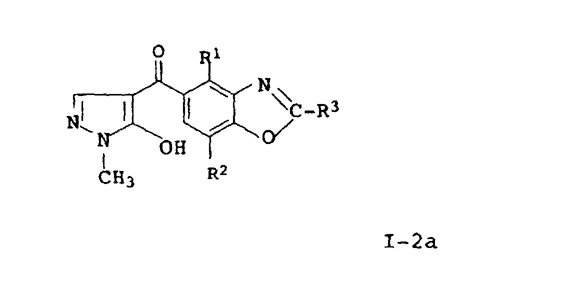 Verbindungen der allgemeinen Formel I-2a, in der die Substituenten R1, R2 und R3 für jede einzelne Verbindung jeweils einer Zeile der Tabelle A entsprechen.
- Tabelle 22: Verbindungen I-2b.1 bis I-2b.810 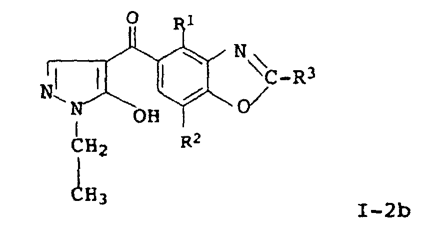 Verbindungen der allgemeinen Formel I-2b, in der die Substituenten R1, R2 und R3 für jede einzelne Verbindung jeweils einer Zeile der Tabelle A entsprechen.
- Tabelle 23: Verbindungen I-2c.1 bis I-2c.810 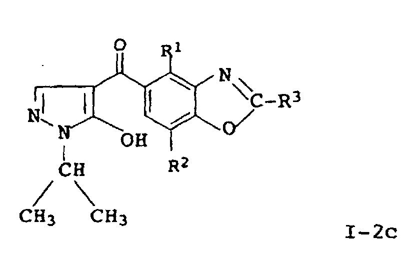 Verbindungen der allgemeinen Formel I-2c, in der die Substituenten R1, R2 und R3 für jede einzelne Verbindung jeweils einer Zeile der Tabelle A entsprechen.
- Tabelle 24: Verbindungen I-2d.1 bis I-2d.810 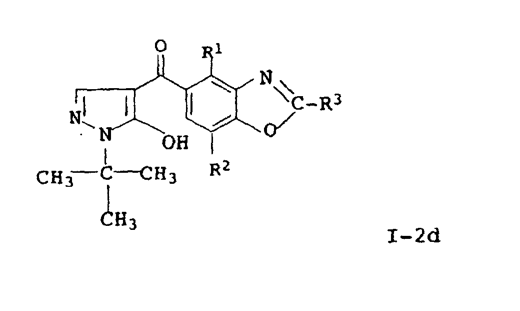 Verbindungen der allgemeinen Formel I-2b, in der die Substituenten R1, R2 und R3 für jede einzelne Verbindung jeweils einer Zeile der Tabelle A entsprechen.
- Tabelle 25: Verbindungen I-2e.1 bis I-2e.810 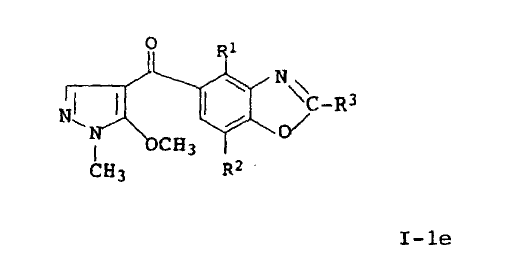 Verbindungen der allgemeinen Formel I-le, in der die Substituenten R1, R2 und R3 für jede einzelne Verbindung jeweils einer Zeile der Tabelle A entsprechen.
- Tabelle 26: Verbindungen I-2f.1 bis I-2f.810 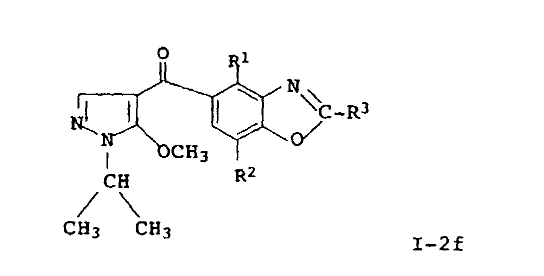 Verbindungen der allgemeinen Formel I-2f, in der die Substituenten R1, R2 und R3 für jede einzelne Verbindung jeweils einer Zeile der Tabelle A entsprechen.
- Tabelle 27: Verbindungen I-2g.1 bis I-2g.810 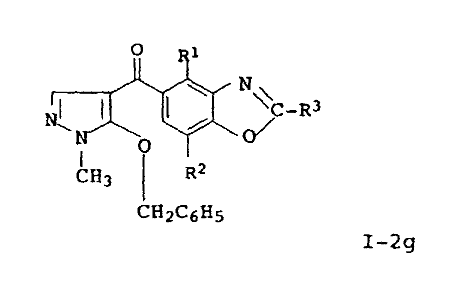 Verbindungen der allgemeinen Formel I-22g, in der die Substituenten R1, R2 und R3 für jede einzelne Verbindung jeweils einer Zeile der Tabelle A entsprechen.
- Tabelle 28: Verbindungen I-2h.1 bis I-2h.810 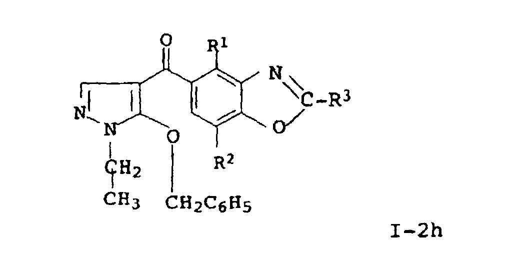 Verbindungen der allgemeinen Formel I-2h, in der die Substituenten R1 R2 und R3 für jede einzelne Verbindung jeweils einer Zeile der Tabelle A entsprechen.
- Tabelle 29: Verbindungen I-2i.1 bis I-2i.810 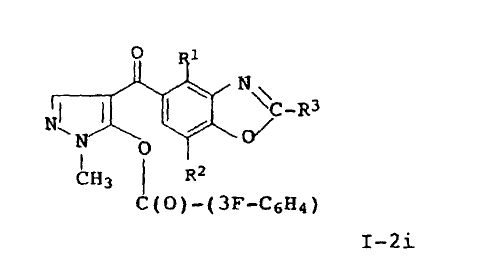 Verbindungen der allgemeinen Formel I-2i, in der die Substituenten R1 R2 und R3 für jede einzelne Verbindung jeweils einer Zeile der Tabelle A entsprechen.
- Tabelle 30: Verbindungen I-2k.1 bis I-2k.810 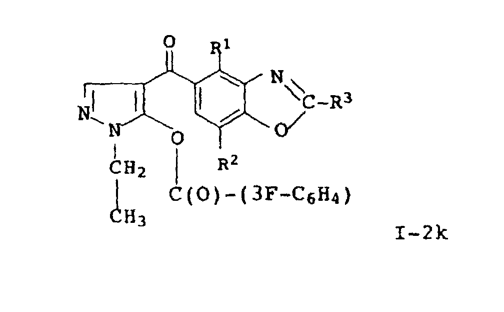 Verbindungen der allgemeinen Formel I-2k, in der die Substituenten R1, R2 und R3 für jede einzelne Verbindung jeweils einer Zeile der Tabelle A entsprechen.
- Tabelle 31: Verbindungen I-21.1 bis 1-21.810 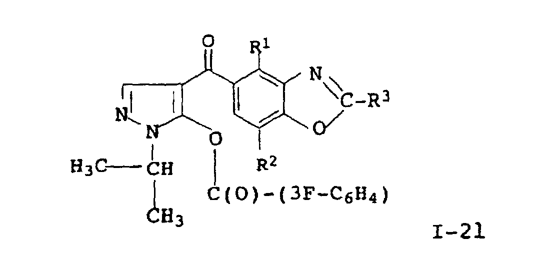 Verbindungen der allgemeinen Formel 1-21, in der die Substituenten R1, R2 und R3 für jede einzelne Verbindung jeweils einer Zeile der Tabelle A entsprechen.
- Tabelle 32: Verbindungen I-2m.1 bis I-2m.810 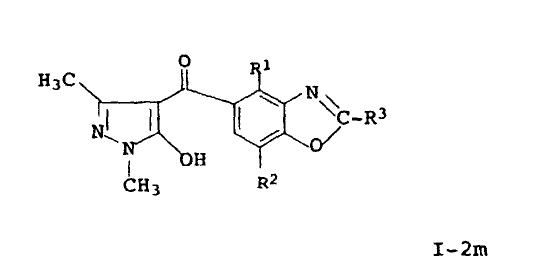 Verbindungen der allgemeinen Formel I-2m, in der die Substituenten R1, R2 und R3 für jede einzelne Verbindung jeweils einer Zeile der Tabelle A entsprechen.
- Tabelle 33: Verbindungen I-2n.1 bis I-2n.810 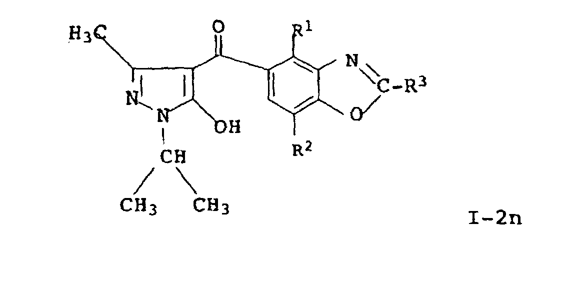 Verbindungen der allgemeinen Formel I-2n, in der die Substituenten R1, R2 und R3 für jede einzelne Verbindung jeweils einer Zeile der Tabelle A entsprechen.
- Tabelle 34: Verbindungen I-20.1 bis I-2o.810 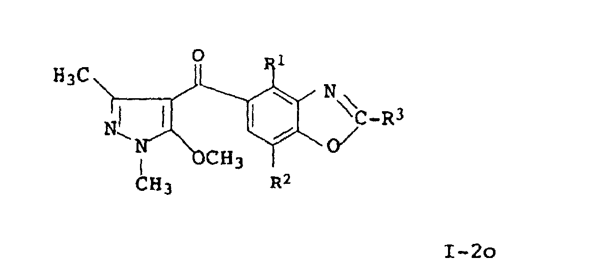 Verbindungen der allgemeinen Formel I-2o, in der die Substituenten R1, R2 und R3 für jede einzelne Verbindung jeweils einer Zeile der Tabelle A entsprechen.
- Tabelle 35: Verbindungen I-2p.1 bis I-2p.810 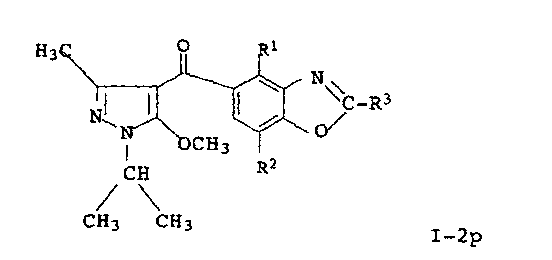 Verbindungen der allgemeinen Formel I-2p, in der die Substituenten R1, R2 und R3 für jede einzelne Verbindung jeweils einer Zeile der Tabelle A entsprechen.
- Tabelle 36: Verbindungen I-2q.1 bis I-2q.810 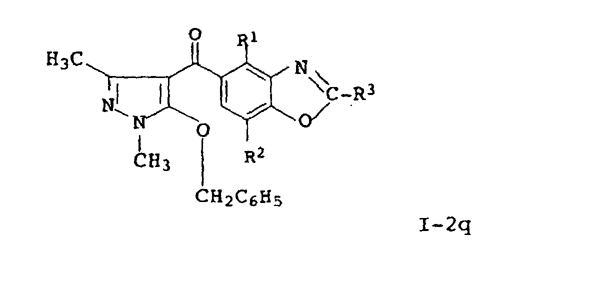 Verbindungen der allgemeinen Formel I-2q, in der die Substituenten R1 R2 und R3 für jede einzelne Verbindung jeweils einer Zeile der Tabelle A entsprechen.
- Tabelle 37: Verbindungen I-2r.1 bis I-2r.810 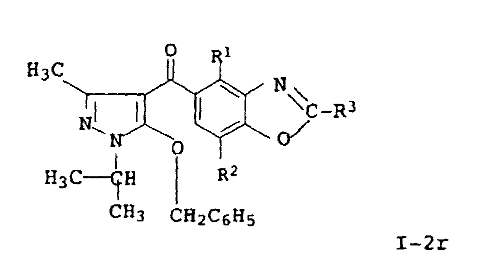 Verbindungen der allgemeinen Formel I-2r, in der die Substituenten R1 R2 und R3 für jede einzelne Verbindung jeweils einer Zeile der Tabelle A entsprechen.
- Tabelle 38: Verbindungen I-2s.1 bis I-2s.810 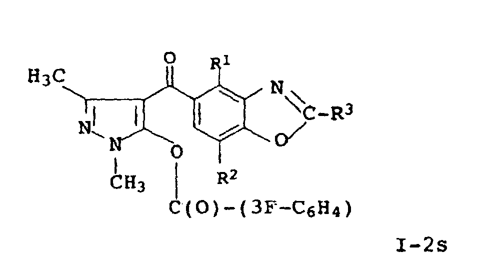 Verbindungen der allgemeinen Formel I-2s, in der die Substituenten R1 R2 und R3 für jede einzelne Verbindung jeweils einer Zeile der Tabelle A entsprechen.
- Tabelle 39: Verbindungen I-2t.1 bis I-2t.810 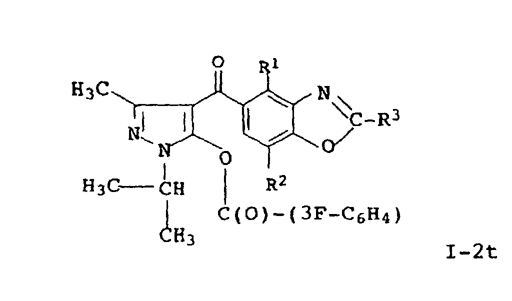 Verbindungen der allgemeinen Formel I-2t, in der die Substituenten R1, R2 und R3 für jede einzelne Verbindung jeweils einer Zeile der Tabelle A entsprechen.
- Tabelle 39a: Verbindungen I-2u.1 bis I-2u.810 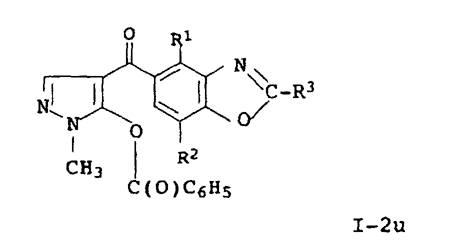 Verbindungen der allgemeinen Formel I-2u, in der die Substituenten R1, R2 und R3 für jede einzelne Verbindung jeweils einer Zeile der Tabelle A entsprechen.
- Tabelle 39b: Verbindungen I-2v.1 bis I-2v.810 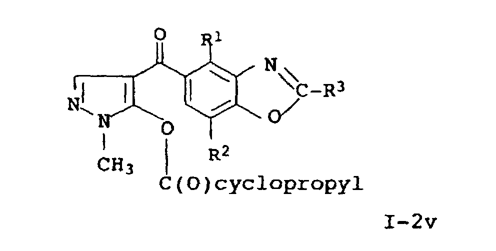 Verbindungen der allgemeinen Formel I-2v, in der die Substituenten R1, R2 und R3 für jede einzelne Verbindung jeweils einer Zeile der Tabelle A entsprechen.
- Tabelle 39c: Verbindungen I-2w.1 bis I-2w.810 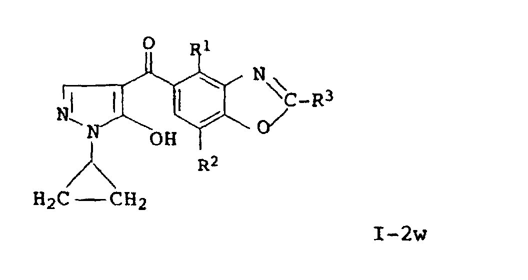 Verbindungen der allgemeinen Formel I-2w, in der die Substituenten R1, R2 und R3 für jede einzelne Verbindung jeweils einer Zeile der Tabelle A entsprechen.

In der folgenden Tabelle B sind besonders bevorzugte Kombinationen R¹, R², R³ und R⁴ für erfindungsgemäße Pyrazolderivate der allgemeinen Formel I angegeben, die sich von Benzimidazol-5-carbonsäuren ableiten.

**Tabelle B**

| | R³ | R¹ | R² | R⁴ |
|---|---|---|---|---|
| 1 | CH₃ | CH₃ | CH₃ | H |
| 2 | C₂H₅ | CH₃ | CH₃ | H |
| 3 | n-C₃H₇ | CH₃ | CH₃ | H |
| 4 | i-C₃H₇ | CH₃ | CH₃ | H |
| 5 | n-C₄H₉ | CH₃ | CH₃ | H |
| 6 | S-C₄H₉ | CH₃ | CH₃ | H |
| 7 | i-C₄H₉ | CH₃ | CH₃ | H |
| 8 | t-C₄H₉ | CH₃ | CH₃ | H |
| 9 | CH₂Cl | CH₃ | CH₃ | H |
| 10 | CHCl₂ | CH₃ | CH₃ | H |
| 11 | CCl₃ | CH₃ | CH₃ | H |
| 12 | CH₂F | CH₃ | CH₃ | H |
| 13 | CHF₂ | CH₃ | CH₃ | H |
| 14 | CF₃ | CH₃ | CH₃ | H |
| 15 | CH₂CF₃ | CH₃ | CH₃ | H |
| 16 | CH₂OCH₃ | CH₃ | CH₃ | H |
| 17 | CH₂OCH₂CH₃ | CH₃ | CH₃ | H |
| 18 | CH₂NH₂ | CH₃ | CH₃ | H |
| 19 | (CH₂)₂COCH₃ | CH₃ | CH₃ | H |
| 20 | Phenyl | CH₃ | CH₃ | H |
| 21 | 2-F-Phenyl | CH₃ | CH₃ | H |
| 22 | 3-F-Phenyl | CH₃ | CH₃ | H |
| 23 | 4-F-Phenyl | CH₃ | CH₃ | H |
| 24 | 2-Cl-Phenyl | CH₃ | CH₃ | H |
| 25 | 3-Cl-Phenyl | CH₃ | CH₃ | H |
| 26 | 4-Cl-Phenyl | CH₃ | CH₃ | H |
| 27 | 2-OH-Phenyl | CH₃ | CH₃ | H |
| 28 | 3-OH-Phenyl | CH₃ | CH₃ | H |
| 29 | 4-OH-Phenyl | CH₃ | CH₃ | H |
| 30 | 2-OCH₃-Phenyl | CH₃ | CH₃ | H |
| 31 | 3-OCH₃-Phenyl | CH₃ | CH₃ | H |
| 32 | 4-OCH₃-Phenyl | CH₃ | CH₃ | H |
| 33 | 2-OCF₃-Phenyl | CH₃ | CH₃ | H |
| 34 | 3-OCF₃-Phenyl | CH₃ | CH₃ | H |
| 35 | 4-OCF₃-Phenyl | CH₃ | CH₃ | H |
| 36 | 2-OCHF₂-Phenyl | CH₃ | CH₃ | H |
| 37 | 3- OCHF₂-Phenyl | CH₃ | CH₃ | H |
| 38 | 4-OCHF₂-Phenyl | CH₃ | CH₃ | H |
| 39 | 2-CF₃-Phenyl | CH₃ | CH₃ | H |
| 40 | 3-CF₃-Phenyl | CH₃ | CH₃ | H |
| 41 | 4-CF₃-Phenyl | CH₃ | CH₃ | H |
| 42 | 2-CH₃-Phenyl | CH₃ | CH₃ | H |
| 43 | 3-CH₃-Phenyl | CH₃ | CH₃ | H |
| 44 | 4-CH₃-Phenyl | CH₃ | CH₃ | H |
| 45 | 2-NO₂-Phenyl | CH₃ | CH₃ | H |
| 46 | 3-NO₂-Phenyl | CH₃ | CH₃ | H |
| 47 | 4-NO₂-Phenyl | CH₃ | CH₃ | H |
| 48 | 2-Pyridyl | CH₃ | CH₃ | H |
| 49 | 3-Pyridyl | CH₃ | CH₃ | H |
| 50 | 4-Pyridyl | CH₃ | CH₃ | H |
| 51 | Cyclohexylamino | CH₃ | CH₃ | H |
| 52 | Cyclopentylamino | CH₃ | CH₃ | H |
| 53 | H | OCH₃ | CH₃ | H |
| 54 | CH₃ | OCH₃ | CH₃ | H |
| 55 | C₂H₅ | OCH₃ | CH₃ | H |
| 56 | n-C₃H₇ | OCH₃ | CH₃ | H |
| 57 | i-C₃H₇ | OCH₃ | CH₃ | H |
| 58 | n-C₄H₉ | OCH₃ | CH₃ | H |
| 59 | s-C₄H₉ | OCH₃ | CH₃ | H |
| 60 | i-C₄H₉ | OCH₃ | CH₃ | H |
| 61 | t-C₄H₉ | OCH₃ | CH₃ | H |
| 62 | CH₂Cl | OCH₃ | CH₃ | H |
| 63 | CHCl₂ | OCH₃ | CH₃ | H |
| 64 | CCl₃ | OCH₃ | CH₃ | H |
| 65 | CH₂F | OCH₃ | CH₃ | H |
| 66 | CHF₂ | OCH₃ | CH₃ | H |
| 67 | CF₃ | OCH₃ | CH₃ | H |
| 68 CH₂CF₃ | | OCH₃ | CH₃ | H |
| 69 | CH₂OCH₃ | OCH₃ | CH₃ | H |
| 70 | CH₂OCH₂CH₃ | OCH₃ | CH₃ | H |
| 71 | CH₂NH₂ | OCH₃ | CH₃ | H |
| 72 | (CH₂)₂COCH₃ | OCH₃ | CH₃ | H |
| 73 | Phenyl | OCH₃ | CH₃ | H |
| 74 | 2-F-Phenyl | OCH₃ | CH₃ | H |
| 75 | 3-F-Phenyl | OCH₃ | CH₃ | H |
| 76 | 4-F-Phenyl | OCH₃ | CH₃ | H |
| 77 | 2-Cl-Phenyl | OCH₃ | CH₃ | H |
| 78 | 3-Cl-Phenyl | OCH₃ | CH₃ | H |
| 79 | 4-Cl-Phenyl | OCH₃ | CH₃ | H |
| 80 | 2-OH-Phenyl | OCH₃ | CH₃ | H |
| 81 | 3-OH-Phenyl | OCH₃ | CH₃ | H |
| 82 | 4-OH-Phenyl | OCH₃ | CH₃ | H |
| 83 | 2-OCH₃-Phenyl | OCH₃ | CH₃ | H |
| 84 | 3-OCH₃-Phenyl | OCH₃ | CH₃ | H |
| 85 | 4-OCH₃-Phenyl | OCH₃ | CH₃ | H |
| 86 | 2-OCF₃-Phenyl | OCH₃ | CH₃ | H |
| 87 | 3-OCF₃-Phenyl | OCH₃ | CH₃ | H |
| 88 | 4-OCF₃-Phenyl | OCH₃ | CH₃ | H |
| 89 | 2-OCHF₂-Phenyl | OCH₃ | CH₃ | H |
| 90 | 3-OCHF₂-Phenyl | OCH₃ | CH₃ | H |
| 91 | 4-OCHF₂-Phenyl | OCH₃ | CH₃ | H |
| 92 | 2-CF₃-Phenyl | OCH₃ | CH₃ | H |
| 93 | 3-CF₃-Phenyl | OCH₃ | CH₃ | H |
| 94 | 4-CF₃-Phenyl | OCH₃ | CH₃ | H |
| 95 | 2-CH₃-Phenyl | OCH₃ | CH₃ | H |
| 96 | 3-CH₃-Phenyl | OCH₃ | CH₃ | H |
| 97 | 4-CH₃-Phenyl | OCH₃ | CH₃ | H |
| 98 | 2-NO₂-Phenyl | OCH₃ | CH₃ | H |
| 99 | 3-NO₂-Phenyl | OCH₃ | CH₃ | H |
| 00 | 4-NO₂-Phenyl | OCH₃ | CH₃ | H |
| 101 | 2-Pyridyl | OCH₃ | CH₃ | H |
| 102 | 3-Pyridyl | OCH₃ | CH₃ | H |
| 103 | 4-Pyridyl | OCH₃ | CH₃ | H |
| 104 | Cyclohexylamino | OCH₃ | CH₃ | H |
| 105 | Cyclopentylamino | OCH₃ | CH₃ | H |
| 106 | H | Cl | CH₃ | H |
| 107 | CH₃ | Cl | CH₃ | H |
| 108 | C₂H₅ | Cl | CH₃ | H |
| 109 | n-C₃H₇ | Cl | CH₃ | H |
| 110 | i-C₃H₇ | Cl | CH₃ | H |
| 111 | n-C₄H₉ | Cl | CH₃ | H |
| 112 | s-C₄H₉ | Cl | CH₃ | H |
| 113 | i-C₄H₉ | Cl | CH₃ | H |
| 114 | t-C₄H₉ | Cl | CH₃ | H |
| 115 | CH₂C1 | Cl | CH₃ | H |
| 116 | CHCl₂ | Cl | CH₃ | H |
| 117 | CCl₃ | Cl | CH₃ | H |
| 118 | CH₂F | Cl | CH₃ | H |
| 119 | CHF₂ | Cl | CH₃ | H |
| 120 | CF₃ | Cl | CH₃ | H |
| 121 | CH₂CF₃ | Cl | CH₃ | H |
| 122 | CH₂OCH₃ | Cl | CH₃ | H |
| 123 | CH₂OCH₂CH₃ | Cl | CH₃ | H |
| 124 | CH₂NH₂ | Cl | CH₃ | H |
| 125 | (CH₂)₂COCH₃ | Cl | CH₃ | H |
| 126 | Phenyl | Cl | CH₃ | H |
| 127 | 2-F-Phenyl | Cl | CH₃ | H |
| 128 | 3-F-Phenyl | Cl | CH₃ | H |
| 129 | 4-F-Phenyl | Cl | CH₃ | H |
| 130 | 2-Cl-Phenyl | Cl | CH₃ | H |
| 131 | 3-Cl-Phenyl | Cl | CH₃ | H |
| 132 | 4-Cl-Phenyl | Cl | CH₃ | H |
| 133 | 2-OH-Phenyl | Cl | CH₃ | H |
| 134 | 3-OH-Phenyl | Cl | CH₃ | H |
| 135 | 4-OH-Phenyl | Cl | CH₃ | H |
| 136 | 2-OCH₃-Phenyl | Cl | CH₃ | H |
| 137 | 3-OCH₃-Phenyl | Cl | CH₃ | H |
| 138 | 4-OCH₃-Phenyl | Cl | CH₃ | H |
| 139 | 2-OCF₃-Phenyl | Cl | CH₃ | H |
| 140 | 3-OCF₃-Phenyl | Cl | CH₃ | H |
| 141 | 4-OCF₃-Phenyl | Cl | CH₃ | H |
| 142 | 2-OCHF₂-Phenyl | Cl | CH₃ | H |
| 143 | 3-OCHF₂-Phenyl | Cl | CH₃ | H |
| 144 | 4-OCHF₂-Phenyl | Cl | CH₃ | H |
| 145 | 2-CF₃-Phenyl | Cl | CH₃ | H |
| 146 | 3-CF₃-Phenyl | Cl | CH₃ | H |
| 147 | 4-CF₃-Phenyl | Cl | CH₃ | H |
| 148 | 2-CH₃-Phenyl | Cl | CH₃ | H |
| 149 | 3-CH₃-Phenyl | Cl | CH₃ | H |
| 150 | 4-CH₃-Phenyl | Cl | CH₃ | H |
| 151 | 2-NO₂-Phenyl | Cl | CH₃ | H |
| 152 | 3-NO₂-Phenyl | Cl | CH₃ | H |
| 153 | 4 -NO2-Phenyl | Cl | CH₃ | H |
| 154 | 2-Pyridyl | Cl | CH₃ | H |
| 155 | 3-Pyridyl | Cl | CH₃ | H |
| 156 | 4-Pyridyl | Cl | CH₃ | H |
| 157 | Cyclohexylamino | Cl | CH₃ | H |
| 158 | Cyclopentylamino | Cl | CH₃ | H |
| 159 | CH₃ | CH₃ | H | H |
| 160 | C₂H₅ | CH₃ | H | H |
| 161 | n-C₃H₇ | CH₃ | H | H |
| 162 | i-C₃H₇ | CH₃ | H | |
| 163 | n-C₄H₉ | CH₃ | H | H |
| 164 | S-C₄H₉ | CH₃ | H | H |
| 165 | i-C₄H_{g} | CH₃ | H | H |
| 166 | t-C₄H₉ | CH₃ | H | H |
| 167 | CH₂Cl | C_{H3} | H | H |
| 168 | CHCl₂ | CH₃ | H | H |
| 169 | CCl₃ | CH₃ | H | H |
| 170 | CH₂F | CH₃ | H | H |
| 171 | CHF₂ | CH₃ | H | H |
| 172 | CF₃ | CH₃ | H | H |
| 173 | CH₂CF₃ | CH₃ | H | H |
| 174 | CH₂OCH₃ | CH₃ | H | H |
| 175 | CH₂OCH₂CH₃ | CH₃ | H | H |
| 176 | CH₂NH₂ | CH₃ | H | H |
| 177 | (CH₂)₂COCH₃ | CH₃ | H | H |
| 178 | Phenyl | CH₃ | H | H |
| 179 | 2-F-Phenyl | CH₃ | H | H |
| 180 | 3-F-Phenyl | CH₃ | H | H |
| 181 | 4-F-Phenyl | CH₃ | H | H |
| 182 | 2-C1-Phenyl | CH₃ | H | H |
| 183 | 3-C1-Phenyl | CH₃ | H | H |
| 184 | 4-C1-Phenyl | CH₃ | H | H |
| 185 | 2-OH-Phenyl | CH₃ | H | H |
| 186 | 3-OH-Phenyl | CH₃ | H | H |
| 187 | 4-OH-Phenyl | CH₃ | H | H |
| 188 | 2-OCH₃-Phenyl | CH₃ | H | H |
| 189 | 3-OCH₃-Phenyl | CH₃ | H | H |
| 190 | 4-OCH₃-Phenyl | CH₃ | H | H |
| 191 | 2-OCF₃-Phenyl | CH₃ | H | H |
| 192 | 3-OCF₃-Phenyl | CH₃ | H | H |
| 193 | 4-OCF₃-Phenyl | CH₃ | H | H |
| 194 | 2-OCHF₂-Phenyl | CH₃ | H | H |
| 195 | 3- OCHF₂-Phenyl | CH₃ | H | H |
| 196 | 4-OCHF₂-Phenyl | CH₃ | H | H |
| 197 | 2-CF₃-Phenyl | CH₃ | H | H |
| 198 | 3-CF₃-Phenyl | CH₃ | H | H |
| 199 | 4-CF₃-Phenyl | CH₃ | H | H |
| 200 | 2-CH₃-Phenyl | CH₃ | H | H |
| 201 | 3-CH₃-Phenyl | CH₃ | H | H |
| 202 | 4-CH₃-Phenyl | CH₃ | H | H |
| 203 | 2-NO₂-Phenyl | CH₃ | H | H |
| 204 | 3-NO₂-Phenyl | CH₃ | H | H |
| 205 | 4-NO₂-Phenyl | CH₃ | H | H |
| 206 | 2-Pyridyl | CH₃ | H | H |
| 207 | 3-Pyridyl | CH₃ | H | H |
| 208 | 4-Pyridyl | CH₃ | H | H |
| 209 | Cyclohexylamino | CH₃ | H | H |
| 210 | Cyclopentylamino | CH₃ | H | H |
| 211 | H | OCH₃ | H | H |
| 212 | CH₃ | OCH₃ | H | H |
| 213 | C₂H₅ | OCH₃ | H | H |
| 214 | n-C₃H₇ | OCH₃ | H | H |
| 215 | i-C₃H₇ | OCH₃ | H | H |
| 216 | n-C₄H₉ | OCH₃ | H | H |
| 217 | s-C₄H₉ | OCH₃ | H | H |
| 218 | i-C₄H₉ | OCH₃ | H | H |
| 219 | t-C₄H₉ | OCH₃ | H | H |
| 220 | CH₂Cl | OCH₃ | H | H |
| 221 | CHCl₂ | OCH₃ | H | H |
| 222 | CCl₃ | OCH₃ | H | H |
| 223 | CH₂F | OCH₃ | H | H |
| 224 | CHF₂ | OCH₃ | H | H |
| 225 | CF₃ | OCH₃ | H | H |
| 226 | CH₂CF₃ | OCH₃ | H | H |
| 227 | CH₂OCH₃ | OCH₃ | H | H |
| 228 | CH₂OCH₂CH₃ | OCH₃ | H | H |
| 229 | CH₂NH₂ | OCH₃ | H | H |
| 230 | (CH₂)₂COCH₃ | OCH₃ | H | H |
| 231 | Phenyl | OCH₃ | H | H |
| 232 | 2-F-Phenyl | OCH₃ | H | H |
| 233 | 3-F-Phenyl | OCH₃ | H | H |
| 234 | 4-F-Phenyl | OCH₃ | H | H |
| 235 | 2-Cl-Phenyl | OCH₃ | H | H |
| 236 | 3-Cl-Phenyl | OCH₃ | H | H |
| 237 | 4-Cl-Phenyl | OCH₃ | H | H |
| 238 | 2-OH-Phenyl | OCH₃ | H | H |
| 239 | 3-OH-Phenyl | OCH₃ | H | H |
| 240 | 4-OH-Phenyl | OCH₃ | H | H |
| 241 | 2-OCH₃-Phenyl | OCH₃ | H | H |
| 242 | 3-OCH₃-Phenyl | OCH₃ | H | H |
| 243 | 4-OCH₃-Phenyl | OCH₃ | H | H |
| 244 | 2-OCF₃-Phenyl | OCH₃ | H | H |
| 245 | 3-OCF₃-Phenyl | OCH₃ | H | H |
| 246 | 4-OCF₃-Phenyl | OCH₃ | H | H |
| 247 | 2-OCHF₂-Phenyl | OCH₃ | H | H |
| 248 | 3-OCHF₂-Phenyl | OCH₃ | H | H |
| 249 | 4-OCHF₂-Phenyl | OCH₃ | H | H |
| 250 | 2-CF₃-Phenyl | OCH₃ | H | H |
| 251 | 3-CF₃-Phenyl | OCH₃ | H | H |
| 252 | 4-CF₃-Phenyl | OCH₃ | H | H |
| 253 | 2-CH₃-Phenyl | OCH₃ | H | H |
| 254 | 3-CH₃-Phenyl | OCH₃ | H | H |
| 255 | 4-CH₃-Phenyl | OCH₃ | H | H |
| 256 | 2-NO₂-Phenyl | OCH₃ | H | H |
| 257 | 3-NO₂-Phenyl | OCH₃ | H | H |
| 258 | 4-NO₂-Phenyl | OCH₃ | H | H |
| 259 | 2-Pyridyl | OCH₃ | H | H |
| 260 | 3-Pyridyl | OCH₃ | H | H |
| 261 | 4-Pyridyl | OCH₃ | H | H |
| 262 | Cyclohexylamino | OCH₃ | H | H |
| 263 | Cyclopentylamino | OCH₃ | H | H |
| 264 | H | Cl | H | H |
| 265 | CH₃ | Cl | H | H |
| 266 | C₂H₅ | Cl | H | H |
| 267 | n-C₃H₇ | Cl | H | H |
| 268 | i-C₃H₇ | Cl | H | H |
| 269 | n-C₄H₉ | Cl | H | H |
| 270 | s-C₄H₉ | Cl | H | H |
| 271 | i-C₄H₉ | Cl | H | H |
| 272 | t-C₄H₉ | Cl | H | H |
| 273 | CH₂Cl | Cl | H | H |
| 274 | CHCl₂ | Cl | H | H |
| 275 | CCl₃ | Cl | H | H |
| 276 | CH₂F | Cl | H | H |
| 277 | CHF₂ | Cl | H | H |
| 278 | CF₃ | Cl | H | H |
| 279 | CH₂CF₃ | Cl | H | H |
| 280 | CH₂OCH₃ | Cl | H | H |
| 281 | CH₂OCH₂CH₃ | Cl | H | H |
| 282 | CH₂NH₂ | Cl | H | H |
| 283 | (CH₂)₂COCH₃ | Cl | H | H |
| 284 | Phenyl | Cl | H | H |
| 285 | 2-F-Phenyl | Cl | H | H |
| 286 | 3-F-Phenyl | Cl | H | H |
| 287 | 4-F-Phenyl | Cl | H | H |
| 288 | 2-Cl-Phenyl | Cl | H | H |
| 289 | 3-Cl-Phenyl | Cl | H | H |
| 290 | 4-Cl-Phenyl | Cl | H | H |
| 291 | 2-OH-Phenyl | Cl | H | H |
| 292 | 3-OH-Phenyl | Cl | H | H |
| 293 | 4-OH-Phenyl | Cl | H | H |
| 294 | 2-OCH₃-Phenyl | Cl | H | H |
| 295 | 3-OCH₃-Phenyl | Cl | H | H |
| 296 | 4-OCH₃-Phenyl | Cl | H | H |
| 297 | 2-OCF₃-Phenyl | Cl | H | H |
| 298 | 3-OCF₃-Phenyl | Cl | H | H |
| 299 | 4-OCF₃-Phenyl | Cl | H | H |
| 300 | 2-OCHF₂-Phenyl | Cl | H | H |
| 301 | 3-OCHF₂-Phenyl | Cl | H | H |
| 302 | 4-OCHF₂-Phenyl | Cl | H | H |
| 303 3 | 2-CF₃-Phenyl | Cl | H | H |
| 304 | 3-CF₃-Phenyl | Cl | H | H |
| 305 | 4-CF₃-Phenyl | Cl | H | H |
| 306 | 2-CH₃-Phenyl | Cl | H | H |
| 307 | 3-CH₃-Phenyl | Cl | H | H |
| 308 | 4-CH₃-Phenyl | Cl | H | H |
| 309 | 2-NO₂-Phenyl | Cl | H | H |
| 310 | 3-NO₂-Phenyl | Cl | H | H |
| 311 | 4-NO₂-Phenyl | Cl | H | H |
| 312 | 2-Pyridyl | Cl | H | H |
| 313 | 3-Pyridyl | Cl | H | H |
| 314 | 4-Pyridyl | Cl | H | H |
| 315 | Cyclohexylamino | Cl | H | H |
| 316 | Cyclopentylamino | Cl | H | H |
| 317 | CH₃ | CH₃ | CH₃ | CH₃ |
| 318 | C₂H₅ | CH₃ | CH₃ | CH₃ |
| 319 | n-C₃H₇ | CH₃ | CH₃ | CH₃ |
| 320 | i-C₃H₇ | CH₃ | CH₃ | CH₃ |
| 321 | n-C₄H₉ | CH₃ | CH₃ | CH₃ |
| 322 | S-C₄H₉ | CH₃ | CH₃ | CH₃ |
| 323 | i-C₄H₉ | CH₃ | CH₃ | CH₃ |
| 324 | t-C₄H₉ | CH₃ | CH₃ | CH₃ |
| 325 | CH₂Cl | CH₃ | CH₃ | CH₃ |
| 326 | CHCl₂ | CH₃ | CH₃ | CH₃ |
| 327 | CCl₃ | CH₃ | CH₃ | CH₃ |
| 328 | CH₂F | CH₃ | CH₃ | CH₃ |
| 329 | CHF₂ | CH₃ | CH₃ | CH₃ |
| 330 | CF₃ | CH₃ | CH₃ | CH₃ |
| 331 | CH₂CF₃ | CH₃ | CH₃ | CH₃ |
| 332 | CH₂OCH₃ | CH₃ | CH₃ | CH₃ |
| 333 | CH₂OCH₂CH₃ | CH₃ | CH₃ | CH₃ |
| 334 | CH₂NH₂ | CH₃ | CH₃ | CH₃ |
| 335 | (CH₂)₂COCH₃ | CH₃ | CH₃ | CH₃ |
| 336 | Phenyl | CH₃ | CH₃ | CH₃ |
| 337 | 2-F-Phenyl | CH₃ | CH₃ | CH₃ |
| 338 | 3-F-Phenyl | CH₃ | CH₃ | CH₃ |
| 339 | 4-F-Phenyl | CH₃ | CH₃ | CH₃ |
| 340 | 2-Cl-Phenyl | CH₃ | CH₃ | CH₃ |
| 341 | 3-C1-Phenyl | CH₃ | CH₃ | CH₃ |
| 342 | 4-C1-Phenyl | CH₃ | CH₃ | CH₃ |
| 343 | 2-OH-Phenyl | CH₃ | CH₃ | CH₃ |
| 344 | 3-OH-Phenyl | CH₃ | CH₃ | CH₃ |
| 345 | 4-OH-Phenyl | CH₃ | CH₃ | CH₃ |
| 346 | 2-OCH₃-Phenyl | CH₃ | CH₃ | CH₃ |
| 347 | 3-OCH₃-Phenyl | CH₃ | CH₃ | CH₃ |
| 348 | 4-OCH₃-Phenyl | CH₃ | CH₃ | CH₃ |
| 349 | 2-OCF₃-Phenyl | CH₃ | CH₃ | CH₃ |
| 350 | 3-OCF₃-Phenyl | CH₃ | CH₃ | CH₃ |
| 351 | 4-OCF₃-Phenyl | CH₃ | CH₃ | CH₃ |
| 352 | 2-OCHF₂-Phenyl | CH₃ | CH₃ | CH₃ |
| 353 | 3- OCHF₂-Phenyl | CH₃ | CH₃ | CH₃ |
| 354 | 4-OCHF₂-Phenyl | CH₃ | CH₃ | CH₃ |
| 355 | 2-CF₃-Phenyl | CH₃ | CH₃ | CH₃ |
| 356 | 3-CF₃-Phenyl | CH₃ | CH₃ | CH₃ |
| 357 | 4-CF₃-Phenyl | CH₃ | CH₃ | CH₃ |
| 358 | 2-CH₃-Phenyl | CH₃ | CH₃ | CH₃ |
| 359 | 3-CH₃-Phenyl | CH₃ | CH₃ | CH₃ |
| 360 | 4-CH₃-Phenyl | CH₃ | CH₃ | CH₃ |
| 361 | 2-NO₂-Phenyl | CH₃ | CH₃ | CH₃ |
| 362 | 3-NO₂-Phenyl | CH₃ | CH₃ | CH₃ |
| 363 | 4-NO₂-Phenyl | CH₃ | CH3 | CH₃ |
| 364 | 2-Pyridyl | CH₃ | CH₃ | CH₃ |
| 365 | 3-Pyridyl | CH₃ | CH₃ | CH₃ |
| 366 | 4-Pyridyl | CH₃ | CH₃ | CH₃ |
| 367 | Cyclohexylamino | CH₃ | CH₃ | CH₃ |
| 368 | Cyclopentylamino | CH₃ | CH₃ | CH₃ |
| 369 | H | OCH₃ | CH₃ | CH₃ |
| 370 | CH₃ | OCH₃ | CH₃ | CH₃ |
| 371 | C₂H₅ | OCH₃ | CH₃ | CH₃ |
| 372 | n-C₃H₇ | OCH₃ | CH₃ | CH₃ |
| 373 | i-C₃H₇ | OCH₃ | CH₃ | CH₃ |
| 374 | n-C₄H₉ | OCH₃ | CH₃ | CH₃ |
| 375 | s-C₄H₉ | OCH₃ | C_{H}3 | CH₃ |
| 376 | i-C₄H₉ | OCH₃ | CH₃ | CH₃ |
| 377 | t-C₄H₉ | OCH₃ | CH₃ | CH₃ |
| 378 | CH₂Cl | OCH₃ | CH₃ | CH₃ |
| 379 | CHCl₂ | OCH₃ | CH₃ | CH₃ |
| 380 | CCl₃ | OCH₃ | CH₃ | CH₃ |
| 381 | CH₂F | OCH₃ | CH₃ | CH₃ |
| 382 | CHF₂ | OCH₃ | CH₃ | CH₃ |
| 383 | CF₃ | OCH₃ | CH₃ | CH₃ |
| 384 | CH₂CF₃ | OCH₃ | CH₃ | CH₃ |
| 385 | CH₂OCH₃ | OCH₃ | CH₃ | CH₃ |
| 386 | CH₂OCH₂CH₃ | OCH₃ | CH₃ | CH₃ |
| 387 | CH₂NH₂ | OCH₃ | CH₃ | CH₃ |
| 388 | (CH₂)₂COCH₃ | OCH₃ | CH₃ | CH₃ |
| 389 | Phenyl | OCH₃ | CH₃ | CH₃ |
| 390 | 2-F-Phenyl | OCH₃ | CH₃ | CH₃ |
| 391 | 3-F-Phenyl | OCH₃ | CH₃ | CH₃ |
| 392 | 4-F-Phenyl | OCH₃ | CH₃ | CH₃ |
| 393 | 2-Cl-Phenyl | OCH₃ | CH₃ | CH₃ |
| 394 | 3-Cl-Phenyl | OCH₃ | CH₃ | CH₃ |
| 395 | 4-Cl-Phenyl | OCH₃ | CH₃ | CH₃ |
| 396 | 2-OH-Phenyl | OCH₃ | CH₃ | CH₃ |
| 397 | 3-OH-Phenyl | OCH₃ | CH₃ | CH₃ |
| 398 | 4-OH-Phenyl | OCH₃ | CH₃ | CH₃ |
| 399 | 2-OCH₃-Phenyl | OCH₃ | CH₃ | CH₃ |
| 400 | 3-OCH₃-Phenyl | OCH₃ | CH₃ | CH₃ |
| 401 | 4-OCH₃-Phenyl | OCH₃ | CH₃ | CH₃ |
| 402 | 2-OCF₃-Phenyl | OCH₃ | CH₃ | CH₃ |
| 403 | 3-OCF₃-Phenyl | OCH₃ | CH₃ | CH₃ |
| 404 | 4-OCF₃-Phenyl | OCH₃ | CH₃ | CH₃ |
| 405 | 2-OCHF₂-Phenyl | OCH₃ | CH₃ | CH₃ |
| 406 | 3-OCHF₂-Phenyl | OCH₃ | CH₃ | CH₃ |
| 407 | 4-OCHF₂-Phenyl | OCH₃ | CH₃ | CH₃ |
| 408 | 2-CF₃-Phenyl | OCH₃ | CH₃ | CH₃ |
| 409 | 3-CF₃-Phenyl | OCH₃ | CH₃ | CH₃ |
| 410 | 4-CF₃-Phenyl | OCH₃ | CH₃ | CH₃ |
| 411 | 2-CH₃-Phenyl | OCH₃ | CH₃ | CH3 |
| 412 | 3-CH₃-Phenyl | OCH₃ | CH₃ | CH₃ |
| 413 | 4-CH₃-Phenyl | OCH₃ | CH₃ | CH₃ |
| 414 | 2-NO₂-Phenyl | OCH₃ | CH₃ | CH₃ |
| 415 | 3-NO₂-Phenyl | OCH₃ | CH₃ | CH₃ |
| 416 | 4-NO₂-Phenyl | OCH₃ | CH₃ | CH₃ |
| 417 | 2-Pyridyl | OCH₃ | CH₃ | CH₃ |
| 418 | 3-Pyridyl | OCH₃ | CH₃ | CH₃ |
| 419 | 4-Pyridyl | OCH₃ | CH₃ | CH₃ |
| 420 | Cyclohexylamino | OCH₃ | CH₃ | CH₃ |
| 421 | Cyclopentylamino | OCH₃ | CH₃ | CH₃ |
| 422 | H | Cl | CH₃ | CH₃ |
| 423 | CH₃ | Cl | CH₃ | CH₃ |
| 424 | C₂H₅ | Cl | CH₃ | CH₃ |
| 445 | n-C₃H₇ | Cl | CH₃ | CH₃ |
| 426 | i-C₃H₇ | Cl | CH₃ | CH₃ |
| 427 | n-C₄H₉ | Cl | CH₃ | CH₃ |
| 428 | s-C₄H₉ | Cl | CH₃ | CH₃ |
| 429 | i-C₄H₉ | Cl | CH₃ | CH₃ |
| 430 | t-C₄H₉ | Cl | CH₃ | CH₃ |
| 431 | CH₂Cl | Cl | CH₃ | CH₃ |
| 432 | CHCl₂ | Cl | CH₃ | CH₃ |
| 433 | CCl₃ | Cl | CH₃ | CH₃ |
| 434 | CH₂F | Cl | CH₃ | CH₃ |
| 435 | CHF₂ | Cl | CH₃ | CH₃ |
| 436 | CF₃ | Cl | CH₃ | CH₃ |
| 437 | CH₂CF₃ | Cl | CH₃ | CH₃ |
| 438 | CH₂OCH₃ | Cl | CH₃ | CH₃ |
| 439 | CH₂OCH₂CH₃ | Cl | CH₃ | CH₃ |
| 440 | CH₂MH₂ | Cl | CH₃ | CH₃ |
| 441 | (CH₂)₂COCH₃ | Cl | CH₃ | CH₃ |
| 442 | Phenyl | Cl | CH₃ | CH₃ |
| 443 | 2-F-Phenyl | Cl | CH₃ | CH₃ |
| 444 | 3-F-Phenyl | Cl | CH₃ | CH₃ |
| 445 | 4-F-Phenyl | Cl | CH₃ | CH₃ |
| 446 | 2-Cl-Phenyl | Cl | CH₃ | CH₃ |
| 447 | 3-Cl-Phenyl | Cl | CH₃ | CH₃ |
| 448 | 4-Cl-Phenyl | Cl | CH₃ | CH₃ |
| 449 | 2-OH-Phenyl | Cl | CH₃ | CH₃ |
| 450 | 3-OH-Phenyl | Cl | CH₃ | CH₃ |
| 451 | 4-OH-Phenyl | Cl | CH₃ | CH₃ |
| 452 | 2-OCH₃-Phenyl | Cl | CH₃ | CH₃ |
| 453 | 3-OCH₃-Phenyl | Cl | CH₃ | CH₃ |
| 454 | 4-OCH₃-Phenyl | Cl | CH₃ | CH₃ |
| 455 | 2-OCF₃-Phenyl | Cl | CH₃ | CH₃ |
| 456 | 3-OCF₃-Phenyl | Cl | CH₃ | CH₃ |
| 457 | 4-OCF₃-Phenyl | Cl | CH₃ | CH₃ |
| 458 | 2-OCHF₂-Phenyl | Cl | CH₃ | CH₃ |
| 459 | 3-OCHF₂-Phenyl | Cl | CH₃ | CH₃ |
| 460 | 4-OCHF₂-Phenyl | Cl | CH₃ | CH₃ |
| 461 | 2-CF₃-Phenyl | Cl | CH₃ | CH₃ |
| 462 | 3-CF₃-Phenyl | Cl | CH₃ | CH₃ |
| 463 | 4-CF₃-Phenyl | Cl | CH₃ | CH₃ |
| 464 | 2-CH₃-Phenyl | Cl | CH₃ | CH₃ |
| 465 | 3-CH₃-Phenyl | Cl | CH₃ | CH₃ |
| 466 | 4-CH₃-Phenyl | Cl | CH₃ | CH₃ |
| 467 | 2-NO₂-Phenyl | Cl | CH₃ | CH₃ |
| 468 | 3-NO₂-Phenyl | Cl | CH₃ | CH₃ |
| 469 | 4-NO₂-Phenyl | Cl | CH₃ | CH₃ |
| 470 | 2-Pyridyl | Cl | CH₃ | CH₃ |
| 471 | 3-Pyridyl | Cl | CH₃ | CH₃ |
| 472 | 4-Pyridyl | Cl | CH₃ | CH₃ |
| 473 | Cyclohexylamino | Cl | CH₃ | CH₃ |
| 474 | Cyclopentylamino | Cl | CH₃ | CH₃ |
| 475 | CH₃ | CH₃ | H | CH₃ |
| 476 | C₂H₅ | CH₃ | H | CH₃ |
| 477 | n-C₃H₇ | CH₃ | H | CH₃ |
| 478 | i-C₃H₇ | CH₃ | H | CH₃ |
| 479 | n-C₄H₉ | CH₃ | H | CH₃ |
| 480 | S-C₄H₉ | CH₃ | H | CH₃ |
| 481 | i-C₄H₉ | CH₃ | H | CH₃ |
| 482 | t-C₄H₉ | CH₃ | H | CH₃ |
| 483 | CH₂Cl | CH₃ | H | CH₃ |
| 484 | CHCl₂ | CH₃ | H | CH₃ |
| 485 | CCl₃ | CH₃ | H | CH₃ |
| 486 | CH₂F | CH₃ | H | CH₃ |
| 487 | CHF₂ | CH₃ | H | CH₃ |
| 488 | CF₃ | CH₃ | H | CH₃ |
| 489 | CH₂CF₃ | CH₃ | H | CH₃ |
| 490 | CH₂OCH₃ | CH₃ | H | CH₃ |
| 491 | CH₂OCH₂CH₃ | CH₃ | H | CH₃ |
| 492 | CH₂NH₂ | CH₃ | H | CH₃ |
| 493 | (CH₂)₂COCH₃ | CH₃ | H | CH₃ |
| 494 | Phenyl | CH₃ | H | CH₃ |
| 495 | 2-F-Phenyl | CH₃ | H | CH₃ |
| 496 | 3-F-Phenyl | CH₃ | H | CH₃ |
| 497 | 4-F-Phenyl | CH₃ | H | CH₃ |
| 498 | 2-Cl-Pheny | CH₃ | H | CH₃ |
| 499 | 3-Cl-Phenyl | CH₃ | H | CH₃ |
| 500 | 4-Cl-Phenyl | CH₃ | H | CH₃ |
| 501 | 2-OH-Phenyl | CH₃ | H | CH₃ |
| 502 | 3-OH-Phenyl | CH₃ | H | CH₃ |
| 503 | 4-OH-Phenyl | CH₃ | H | CH₃ |
| 504 | 2-OCH₃-Phenyl | CH₃ | H | CH₃ |
| 505 | 3-OCH₃-Phenyl | CH₃ | H | CH₃ |
| 506 | 4-OCH₃-Phenyl | CH₃ | H | CH₃ |
| 507 | 2-OCF₃-Phenyl | CH₃ | H | CH₃ |
| 508 | 3-OCF₃-Phenyl | CH₃ | H | CH₃ |
| 509 | 4-OCF₃-Phenyl | CH₃ | H | CH₃ |
| 510 | 2-OCHF₂-Phenyl | CH₃ | H | CH₃ |
| 511 | 3- OCHF₂-Phenyl | CH₃ | H | CH₃ |
| 512 | 4-OCHF₂-Phenyl | CH₃ | H | CH₃ |
| 513 | 2-CF₃-Phenyl | CH₃ | H | CH₃ |
| 514 | 3-CF₃-Phenyl | CH₃ | H | CH₃ |
| 515 | 4-CF₃-Phenyl | CH₃ | H | CH₃ |
| 516 | 2-CH₃-Phenyl | CH₃ | H | CH₃ |
| 517 | 3-CH₃-Phenyl | CH₃ | H | CH₃ |
| 518 | 4-CH₃-Phenyl | CH₃ | H | CH₃ |
| 519 | 2-NO₂-Phenyl | CH₃ | H | CH₃ |
| 520 | 3-NO₂-Phenyl | CH₃ | H | CH₃ |
| 521 | 4-NO₂-Phenyl | CH₃ | H | CH₃ |
| 522 | 2-Pyridyl | CH₃ | H | CH₃ |
| 523 | 3-Pyridyl | CH₃ | H | CH₃ |
| 524 | 4-Pyridyl | CH₃ | H | CH₃ |
| 525 | Cyclohexylamino | CH₃ | H | CH₃ |
| 526 | Cyclopentylamino | CH₃ | H | CH₃ |
| 527 | H | OCH₃ | H | CH₃ |
| 528 | CH₃ | OCH₃ | H | CH₃ |
| 529 | C₂H₅ | OCH₃ | H | CH₃ |
| 530 | n-C₃H₇ | OCH₃ | H | CH₃ |
| 531 | i-C₃H₇ | OCH₃ | H | CH₃ |
| 532 | n-C₄H₉ | OCH₃ | H | CH₃ |
| 533 | S-C₄H₉ | OCH₃ | H | CH₃ |
| 534 | i-C₄H₉ | OCH₃ | H | CH₃ |
| 535 | t-C₄H₉ | OCH₃ | H | CH₃ |
| 536 | CH₂Cl | OCH₃ | H | CH₃ |
| 537 | CHCl₂ | OCH₃ | H | CH₃ |
| 538 | CCl₃ | OCH₃ | H | CH₃ |
| 539 | CH₂F | OCH₃ | H | CH₃ |
| 540 | CHF₂ | OCH₃ | H | CH₃ |
| 541 | CF₃ | OCH₃ | H | CH₃ |
| 542 | CH₂CF₃ | OCH₃ | H | CH₃ |
| 543 | CH₂OCH₃ | OCH₃ | H | CH₃ |
| 544 | CH₂OCH₂CH₃ | OCH₃ | H | CH₃ |
| 545 | CH₂NH₂ | OCH₃ | H | CH₃ |
| 546 | (CH₂)₂COCH₃ | OCH₃ | H | CH₃ |
| 547 | Phenyl | OCH₃ | H | CH₃ |
| 548 | 2-F-Phenyl | OCH₃ | H | CH₃ |
| 549 | 3-F-Phenyl | OCH₃ | H | CH₃ |
| 550 | 4-F-Phenyl | OCH₃ | H | CH₃ |
| 551 | 2-Cl-Phenyl | OCH₃ | H | CH₃ |
| 552 | 3-Cl-Phenyl | OCH₃ | H | CH₃ |
| 553 | 4-Cl-Phenyl | OCH₃ | H | CH₃ |
| 554 | 2-OH-Phenyl | OCH₃ | H | CH₃ |
| 555 | 3-OH-Phenyl | OCH₃ | H | CH₃ |
| 556 | 4-OH-Phenyl | OCH₃ | H | CH₃ |
| 557 | 2-OCH₃-Phenyl | OCH₃ | H | CH₃ |
| 558 | 3-OCH₃-Phenyl | OCH₃ | H | CH₃ |
| 559 | 4-OCH₃-Phenyl | OCH₃ | H | CH₃ |
| 560 | 2-OCF₃-Phenyl | OCH₃ | H | CH₃ |
| 561 | 3-OCF₃-Phenyl | OCH₃ | H | CH₃ |
| 562 | 4-OCF₃-Phenyl | OCH₃ | H | CH₃ |
| 563 | 2-OCHF₂-Phenyl | OCH₃ | H | C_{H3} |
| 564 | 3-OCHF₂-Phenyl | OCH₃ | H | CH₃ |
| 565 | 4-OCHF₂-Phenyl | OCH₃ | H | CH₃ |
| 566 | 2-CF₃-Phenyl | OCH₃ | H | CH₃ |
| 567 | 3-CF₃-Phenyl | OCH₃ | H | CH₃ |
| 568 | 4-CF₃-Phenyl | OCH₃ | H | CH₃ |
| 569 | 2-CH₃-Phenyl | OCH₃ | H | CH₃ |
| 570 | 3-CH₃-Phenyl | OCH₃ | H | CH₃ |
| 571 | 4-CH₃-Phenyl | OCH₃ | H | CH₃ |
| 572 | 2-NO₂-Phenyl | OCH₃ | H | CH₃ |
| 573 | 3-NO₂-Phenyl | OCH₃ | H | CH₃ |
| 574 | 4-NO₂-Phenyl | OCH₃ | H | CH₃ |
| 575 | 2-Pyridyl | OCH₃ | H | CH₃ |
| 576 | 3-Pyridyl | OCH₃ | H | CH₃ |
| 577 | 4-Pyridyl | OCH₃ | H | CH₃ |
| 578 | Cyclohexylamino | OCH₃ | H | CH₃ |
| 579 | Cyclopentylamino | OCH₃ | H | CH₃ |
| 580 | H | Cl | H | CH₃ |
| 581 | CH₃ | Cl | H | CH₃ |
| 582 | C₂H₅ | Cl | H | CH₃ |
| 583 | n-C₃H₇ | Cl | H | CH₃ |
| 584 | i-C₃H₇ | Cl | H | CH₃ |
| 585 | n-C₄H₉ | Cl | H | CH₃ |
| 586 | s-C₄H₉ | Cl | H | CH₃ |
| 587 | i-C₄H₉ | Cl | H | CH₃ |
| 588 | t-C₄H₉ | Cl | H | CH₃ |
| 589 | CH₂Cl | Cl | H | CH₃ |
| 590 | CHCl₂ | Cl | H | CH₃ |
| 591 | CCl₃ | Cl | H | CH₃ |
| 592 | CH₂F | Cl | H | CH₃ |
| 593 | CHF₂ | Cl | H | CH₃ |
| 594 | CF₃ | Cl | H | CH₃ |
| 595 | CH₂CF₃ | Cl | H | CH₃ |
| 596 | CH₂OCH₃ | Cl | H | CH₃ |
| 597 | CH₂OCH₂CH₃ | Cl | H | CH₃ |
| 598 | CH₂NH₂ | Cl | H | CH₃ |
| 599 | (CH₂)₂COCH₃ | Cl | H | CH₃ |
| 600 | Phenyl | Cl | H | CH₃ |
| 701 | 2-F-Phenyl | Cl | H | CH₃ |
| 702 | 3-F-Phenyl | Cl | H | CH₃ |
| 703 | 4-F-Phenyl | Cl | H | CH₃ |
| 704 | 2-Cl-Phenyl | Cl | H | CH₃ |
| 705 | 3-Cl-Phenyl | Cl | H | CH₃ |
| 706 | 4-Cl-Phenyl | Cl | H | CH₃ |
| 707 | 2-OH-Phenyl | Cl | H | CH₃ |
| 708 | 3-OH-Phenyl | Cl | H | CH₃ |
| 709 | 4-OH-Phenyl | Cl | H | CH₃ |
| 710 | 2-OCH₃-Phenyl | Cl | H | CH₃ |
| 711 | 3-OCH₃-Phenyl | Cl | H | CH₃ |
| 712 | 4-OCH₃-Phenyl | Cl | H | CH₃ |
| 713 | 2-OCF₃-Phenyl | Cl | H | CH₃ |
| 714 | 3-OCF₃-Phenyl | Cl | H | CH₃ |
| 715 | 4-OCF₃-Phenyl | Cl | H | CH₃ |
| 716 | 2-OCHF₂-Phenyl | Cl | H | CH₃ |
| 717 | 3-OCHF₂-Phenyl | Cl | H | CH₃ |
| 718 | 4-OCHF₂-Phenyl | Cl | H | CH₃ |
| 719 | 2-CF₃-Phenyl | Cl | H | CH₃ |
| 720 | 3-CF₃-Phenyl | Cl | H | CH₃ |
| 721 | 4-CF₃-Phenyl | Cl | H | CH₃ |
| 722 | 2-CH₃-Phenyl | Cl | H | CH₃ |
| 723 | 3-CH₃-Phenyl | Cl | H | CH₃ |
| 724 | 4-CH₃-Phenyl | Cl | H | CH₃ |
| 725 | 2-NO₂-Phenyl | Cl | H | CH₃ |
| 726 | 3-NO₂-Phenyl | Cl | H | CH₃ |
| 727 | 4-NO₂-Phenyl | Cl | H | CH₃ |
| 728 | 2-Pyridyl | Cl | H | CH₃ |
| 729 | 3-Pyridyl | Cl | H | CH₃ |
| 730 | 4-Pyridyl | Cl | H | CH₃ |
| 731 | Cyclohexylamino | Cl | H | CH₃ |
| 732 | Cyclopentylamino | Cl | H | CH₃ |
| 733 | CH₃ | CH₃ | CH₃ | C₂H₅ |
| 734 | C₂H₅ | CH₃ | CH₃ | C₂H₅ |
| 735 | n-C₃H₇ | CH₃ | CH₃ | C₂H₅ |
| 736 | i-C₃H₇ | CH₃ | CH₃ | C₂H₅ |
| 737 | H-C₄H₉ | CH₃ | CH₃ | C₂H₅ |
| 738 | S-C₄H₉ | CH₃ | CH₃ | C₂H₅ |
| 739 | i-C₄H₉ | CH₃ | CH₃ | C₂H₅ |
| 740 | t-C₄H₉ | CH₃ | CH₃ | C_{z}Hₛ |
| 741 | CH₂Cl | CH₃ | CH₃ | C₂H₅ |
| 742 | CHCl₂ | CH₃ | CH₃ | C₂H₅ |
| 743 | CCl₃ | CH₃ | CH₃ | C₂H₅ |
| 744 | CH₂F | CH₃ | CH₃ | C₂H₅ |
| 745 | CHF₂ | CH₃ | CH₃ | C₂H₅ |
| 746 | CF₃ | CH₃ | CH₃ | C₂H₅ |
| 747 | CH₂CF₃ | CH₃ | CH₃ | C₂H₅ |
| 748 | CH₂OCH₃ | CH₃ | CH₃ | C₂H₅ |
| 749 | CH₂OCH₂CH₃ | CH₃ | CH₃ | C₂H₅ |
| 750 | CH₂NH₂ | CH₃ | CH₃ | C₂H₅ |
| 751 | (CH₂)₂COCH₃ | CH₃ | CH₃ | C₂H₅ |
| 752 | Phenyl | CH₃ | CH₃ | C₂H₅ |
| 753 | 2-F-Phenyl | CH₃ | CH₃ | C₂H₅ |
| 754 | 3-F-Phenyl | CH₃ | CH₃ | C₂H₅ |
| 755 | 4-F-Phenyl | CH₃ | CH₃ | C₂H₅ |
| 756 | 2-Cl-Phenyl | CH₃ | CH₃ | C₂H₅ |
| 757 | 3-Cl-Phenyl | CH₃ | CH₃ | C₂H₅ |
| 758 | 4-Cl-Phenyl | CH₃ | CH₃ | C₂H₅ |
| 759 | 2-OH-Phenyl | CH₃ | CH₃ | C₂H₅ |
| 760 | 3-OH-Phenyl | CH₃ | CH₃ | C₂H₅ |
| 761 | 4-OH-Phenyl | CH₃ | CH₃ | C₂H₅ |
| 762 | 2-OCH₃-Phenyl | CH₃ | CH₃ | C₂H₅ |
| 763 | 3-OCH₃-Phenyl | CH₃ | CH₃ | C₂H₅ |
| 764 | 4-OCH₃-Phenyl | CH₃ | CH₃ | C₂H₅ |
| 765 | 2-OCF₃-Phenyl | CH₃ | CH₃ | C₂H₅ |
| 766 | 3-OCF₃-Phenyl | CH₃ | CH₃ | C₂H₅ |
| 767 | 4-OCF₃-Phenyl | CH₃ | CH₃ | C₂H₅ |
| 768 | 2-OCHF₂-Phenyl | CH₃ | CH₃ | C₂H₅ |
| 769 | 3- OCHF₂-Phenyl | CH₃ | CH₃ | C₂H₅ |
| 770 | 4-OCHF₂-Phenyl | CH₃ | CH₃ | C₂H₅ |
| 771 | 2-CF₃-Phenyl | CH₃ | CH₃ | C₂H₅ |
| 772 | 3-CF₃-Phenyl | CH₃ | CH₃ | C₂H₅ |
| 773 | 4-CF₃-Phenyl | CH₃ | CH₃ | C₂H₅ |
| 774 | 2-CH₃-Phenyl | CH₃ | CH₃ | C₂H₅ |
| 775 | 3-CH₃-Phenyl | CH₃ | CH₃ | C₂H₅ |
| 776 | 4-CH₃-Phenyl | CH₃ | CH₃ | C₂H₅ |
| 777 | 2-NO₂-Phenyl | CH₃ | CH₃ | C₂H₅ |
| 778 | 3-NO₂-Phenyl | CH₃ | CH₃ | C₂H₅ |
| 779 | 4-NO₂-Phenyl | CH₃ | CH₃ | C₂H₅ |
| 780 | 2-Pyridyl | CH₃ | CH₃ | C₂H₅ |
| 781 | 3-Pyridyl | CH₃ | CH₃ | C₂H₅ |
| 782 | 4-Pyridyl | CH₃ | CH₃ | C₂H₅ |
| 783 | Cyclohexylamino | CH₃ | CH₃ | C₂H₅ |
| 784 | Cyclopentylamino | CH₃ | CH₃ | C₂H₅ |
| 785 | H | OCH₃ | CH₃ | C₂H₅ |
| 786 | CH₃ | OCH₃ | CH₃ | C₂H₅ |
| 787 | C₂H₅ | OCH₃ | CH₃ | C₂H₅ |
| 788 | n-C₃H₇ | OCH₃ | CH₃ | C₂H₅ |
| 789 | i-C₃H₇ | OCH₃ | CH₃ | C₂H₅ |
| 790 | n-C₄H₉ | OCH₃ | CH₃ | C₂H₅ |
| 791 | s-C₄H₉ | OCH₃ | CH₃ | C₂H₅ |
| 792 | i-C₄H₉ | OCH₃ | CH₃ | C₂H₅ |
| 793 | t-C₄H₉ | OCH₃ | CH₃ | C₂H₅ |
| 794 | CH₂Cl | OCH₃ | CH₃ | C₂H₅ |
| 795 | CHCl₂ | OCH₃ | CH₃ | C₂H₅ |
| 796 | CCl₃ | OCH₃ | CH₃ | C₂H₅ |
| 797 | CH₂F | OCH₃ | CH₃ | C₂H₅ |
| 798 | CHF₂ | OCH₃ | CH₃ | C₂H₅ |
| 799 | CF₃ | OCH₃ | CH₃ | C₂H₅ |
| 800 | CH₂CF₃ | OCH₃ | CH₃ | C₂H₅ |
| 801 | CH₂OCH₃ | OCH₃ | CH₃ | C₂H₅ |
| 802 | CH₂OCH₂CH₃ | OCH₃ | CH₃ | C₂H₅ |
| 803 | CH₂NH₂ | OCH₃ | CH₃ | C₂H₅ |
| 804 | (CH₂)₂COCH₃ | OCH₃ | CH₃ | C₂H₅ |
| 805 | Phenyl | OCH₃ | CH₃ | C₂H₅ |
| 806 | 2-F-Phenyl | OCH₃ | CH₃ | C₂H₅ |
| 807 | 3-F-Phenyl | OCH₃ | CH₃ | C₂H₅ |
| 808 | 4-F-Phenyl | OCH₃ | CH₃ | C₂H₅ |
| 809 | 2-Cl-Phenyl | OCH₃ | CH₃ | C₂H₅ |
| 810 | 3-Cl-Phenyl | OCH₃ | CH₃ | C₂H₅ |
| 811 | 4-Cl-Phenyl | OCH₃ | CH₃ | C₂H₅ |
| 812 | 2-OH-Phenyl | OCH₃ | CH₃ | C₂H₅ |
| 813 | 3-OH-Phenyl | OCH₃ | CH₃ | C₂H₅ |
| 814 | 4-OH-Phenyl | OCH₃ | CH₃ | C₂H₅ |
| 815 | 2-OCH₃-Phenyl | OCH₃ | CH₃ | C₂H₅ |
| 816 | 3-OCH₃-Phenyl | OCH₃ | CH₃ | C₂H₅ |
| 817 | 4-OCH₃-Phenyl | OCH₃ | CH₃ | C₂H₅ |
| 818 | 2-OCF₃-Phenyl | OCH₃ | CH₃ | C₂H₅ |
| 819 | 3-OCF₃-Phenyl | OCH₃ | CH₃ | C₂H₅ |
| 820 | 4-OCF₃-Phenyl | OCH₃ | CH₃ | C₂H₅ |
| 821 | 2-OCHF₂-Phenyl | OCH₃ | CH₃ | C₂H₅ |
| 822 | 3-OCHF₂-Phenyl | OCH₃ | CH₃ | C₂H₅ |
| 823 | 4-OCHF₂-Phenyl | OCH₃ | CH₃ | C₂H₅ |
| 824 | 2-CF₃-Phenyl | OCH₃ | CH₃ | C₂H₅ |
| 825 | 3-CF₃-Phenyl | OCH₃ | CH₃ | C₂H₅ |
| 826 | 4-CF₃-Phenyl | OCH₃ | CH₃ | C₂H₅ |
| 827 | 2-CH₃-Phenyl | OCH₃ | CH₃ | C₂H₅ |
| 828 | 3-CH₃-Phenyl | OCH₃ | CH₃ | C₂H₅ |
| 829 | 4-CH₃-Phenyl | OCH₃ | CH₃ | C₂H₅ |
| 830 | 2-NO₂-Phenyl | OCH₃ | CH₃ | C₂H₅ |
| 831 | 3-NO₂-Phenyl | OCH₃ | CH₃ | C₂H₅ |
| 832 | 4-NO₂-Phenyl | OCH₃ | CH₃ | C₂H₅ |
| 833 | 2-Pyridyl | OCH₃ | CH₃ | C₂H₅ |
| 834 | 3-Pyridyl | OCH₃ | CH₃ | C₂H₅ |
| 835 | 4-Pyridyl | OCH₃ | CH₃ | C₂H₅ |
| 836 | Cyclohexylamino | OCH₃ | CH₃ | C₂H₅ |
| 837 | Cyclopentylamino | OCH₃ | CH₃ | C₂H₅ |
| 838 | H | Cl | CH₃ | C₂H₅ |
| 839 | CH₃ | Cl | CH₃ | C₂H₅ |
| 840 | C₂H₅ | Cl | CH₃ | C₂H₅ |
| 841 | n-C₃H₇ | Cl | CH₃ | C₂H₅ |
| 842 | i-C₃H₇ | Cl | CH₃ | C₂H₅ |
| 843 | n-C₄H₉ | Cl | CH₃ | C₂H₅ |
| 844 | s-C₄H₉ | Cl | CH₃ | C₂H₅ |
| 845 | i-C₄H₉ | Cl | CH₃ | C₂H₅ |
| 846 | t-C₄H₉ | Cl | CH₃ | C₂H₅ |
| 847 | CH₂Cl | Cl | CH₃ | C₂H₅ |
| 848 | CHCl₂ | Cl | CH₃ | C₂H₅ |
| 849 | CCl₃ | Cl | CH₃ | C₂H₅ |
| 850 | CH₂F | Cl | CH₃ | C₂H₅ |
| 851 | CHF₂ | Cl | CH₃ | C₂H₅ |
| 852 | CF₃ | Cl | CH₃ | C₂H₅ |
| 853 | CH₂CF₃ | Cl | CH₃ | C₂H₅ |
| 854 | CH₂OCH₃ | Cl | CH₃ | C₂H₅ |
| 855 | CH₂OCH₂CH₃ | Cl | CH₃ | C₂H₅ |
| 856 | CH₂NH₂ | Cl | CH₃ | C₂H₅ |
| 857 | (CH₂)₂COCH₃ | Cl | CH₃ | C₂H₅ |
| 858 | Phenyl | Cl | CH₃ | C₂H₅ |
| 859 | 2-F-Phenyl | Cl | CH₃ | C₂H₅ |
| 860 | 3-F-Phenyl | Cl | CH₃ | C₂H₅ |
| 861 | 4-F-Phenyl | Cl | CH₃ | C₂H₅ |
| 862 | 2-Cl-Phenyl | Cl | CH₃ | C₂H₅ |
| 863 | 3-Cl-Phenyl | Cl | CH₃ | C₂H₅ |
| 864 | 4-Cl-Phenyl | Cl | CH₃ | C₂H₅ |
| 865 | 2-OH-Phenyl | Cl | CH₃ | C₂H₅ |
| 866 | 3-OH-Phenyl | Cl | CH₃ | C₂H₅ |
| 867 | 4-OH-Phenyl | Cl | CH₃ | C₂H₅ |
| 868 | 2-OCH₃-Phenyl | Cl | CH₃ | C₂H₅ |
| 869 | 3-OCH₃-Phenyl | Cl | CH₃ | C₂H₅ |
| 870 | 4-OCH₃-Phenyl | Cl | CH₃ | C₂H₅ |
| 871 | 2-OCF₃-Phenyl | Cl | CH₃ | C₂H₅ |
| 872 | 3-OCF₃-Phenyl | Cl | CH₃ | C₂H₅ |
| 873 | 4-OCF₃-Phenyl | Cl | CH₃ | C₂H₅ |
| 874 | 2-OCHF₂-Phenyl | Cl | CH₃ | C₂H₅ |
| 875 | 3-OCHF₂-Phenyl | Cl | CH₃ | C₂H₅ |
| 876 | 4-OCHF₂-Phenyl | Cl | CH₃ | C₂H₅ |
| 877 | 2-CF₃-Phenyl | Cl | CH₃ | C₂H₅ |
| 878 | 3-CF₃-Phenyl | Cl | CH₃ | C₂H₅ |
| 879 | 4-CF₃-Phenyl | Cl | CH₃ | C₂H₅ |
| 880 | 2-CH₃-Phenyl | Cl | CH₃ | C₂H₅ |
| 881 | 3-CH₃-Phenyl | Cl | CH₃ | C₂H₅ |
| 882 | 4-CH₃-Phenyl | Cl | CH₃ | C₂H₅ |
| 883 | 2-NO₂-Phenyl | Cl | CH₃ | C₂H₅ |
| 884 | 3-NO₂-Phenyl | Cl | CH₃ | C₂H₅ |
| 885 | 4-NO₂-Phenyl | Cl | CH₃ | C₂H₅ |
| 886 | 2-Pyridyl | Cl | CH₃ | C₂H₅ |
| 887 | 3-Pyridyl | Cl | CH₃ | C₂H₅ |
| 888 | 4-Pyridyl | Cl | CH₃ | C₂H₅ |
| 889 | Cyclohexylamino | Cl | CH₃ | C₂H₅ |
| 890 | Cyclopentylamino | Cl | CH₃ | C₂H₅ |
| 891 | CH₃ | CH₃ | H | C₂H₅ |
| 892 | C₂H₅ | CH₃ | H | C₂H₅ |
| 893 | n-C₃H₇ | CH₃ | H | C₂H₅ |
| 894 | i-C₃H₇ | CH₃ | H | C₂H₅ |
| 895 | n-C₄H₉ | CH₃ | H | C₂H₅ |
| 896 | s-C₄H₉ | CH₃ | H | C₂H₅ |
| 897 | i-C₄H₉ | CH₃ | H | C₂H₅ |
| 898 | t-C₄H₉ | CH₃ | H | C₂H₅ |
| 899 | CH₂Cl | CH₃ | H | C₂H₅ |
| 900 | CHCl₂ | CH₃ | H | C₂H₅ |
| 901 | CCl₃ | CH₃ | H | C₂H₅ |
| 902 | CH₂F | CH₃ | H | C₂H₅ |
| 903 | CHF₂ | CH₃ | H | C₂H₅ |
| 904 | CF₃ | CH₃ | H | C₂H₅ |
| 905 | CH₂CF₃ | CH₃ | H | C₂H₅ |
| 906 | CH₂OCH₃ | CH₃ | H | C₂H₅ |
| 907 | CH₂OCH₂CH₃ | CH₃ | H | C₂H₅ |
| 908 | CH₂NH₂ | CH₃ | H | C₂H₅ |
| 909 | (CH₂)₂COCH₃ | CH₃ | H | C₂H₅ |
| 910 | Phenyl | CH₃ | H | C₂H₅ |
| 911 | 2-F-Phenyl | CH₃ | H | C₂H₅ |
| 912 | 3-F-Phenyl | CH₃ | H | C₂H₅ |
| 913 | 4-F-Phenyl | CH₃ | H | C₂H₅ |
| 914 | 2-Cl-Phenyl | CH₃ | H | C₂H₅ |
| 915 | 3-Cl-Phenyl | CH₃ | H | C₂H₅ |
| 916 | 4-Cl-Phenyl | CH₃ | H | C₂H₅ |
| 917 | 2-OH-Phenyl | CH₃ | H | C₂H₅ |
| 918 | 3-OH-Phenyl | CH₃ | H | C₂H₅ |
| 919 | 4-OH-Phenyl | CH₃ | H | C₂H₅ |
| 920 | 2-OCH₃-Phenyl | CH₃ | H | C₂H₅ |
| 921 | 3-OCH₃-Phenyi | CH₃ | H | C₂H₅ |
| 922 | 4-OCH₃-Phenyl | CH₃ | H | C₂H₅ |
| 923 | 2-OCF₃-Phenyl | CH₃ | H | C₂H₅ |
| 924 | 3-OCF₃-Phenyl | CH₃ | H | C₂H₅ |
| 925 | 4-OCF₃-Phenyl | CH₃ | H | C₂H₅ |
| 926 | 2-OCHF₂-Phenyl | CH₃ | H | C₂H₅ |
| 927 | 3- OCHF₂-Phenyl | CH₃ | H | C₂H₅ |
| 928 | 4-OCHF₂-Phenyl | CH₃ | H | C₂H₅ |
| 929 | 2-CF₃-Phenyl | CH₃ | H | C₂H₅ |
| 930 | 3-CF₃-Phenyl | CH₃ | H | C₂H₅ |
| 931 | 4-CF₃-Phenyl | CH₃ | H | C₂H₅ |
| 932 | 2-CH₃-Phenyl | CH₃ | H | C₂H₅ |
| 933 | 3-CH₃-Phenyl | CH₃ | H | C₂H₅ |
| 934 | 4-CH₃-Phenyl | CH₃ | H | C₂H₅ |
| 935 | 2-NO₂-Phenyl | CH₃ | H | C₂H₅ |
| 936 | 3-NO₂-Phenyl | CH₃ | H | C₂H₅ |
| 937 | 4-NO₂-Phenyl | CH₃ | H | C₂H₅ |
| 938 | 2-Pyridyl | CH₃ | H | C₂H₅ |
| 939 | 3-Pyridyl | CH₃ | H | C₂H₅ |
| 940 | 4-Pyridyl | CH₃ | H | C₂H₅ |
| 941 | Cyclohexylamino | CH₃ | H | C₂H₅ |
| 942 | Cyclopentylamino | CH₃ | H | C₂H₅ |
| 943 | H | OCH₃ | H | C₂H₅ |
| 944 | CH₃ | OCH₃ | H | C₂H₅ |
| 945 | C₂H₅ | OCH₃ | H | C₂H₅ |
| 946 | n-C₃H₇ | OCH₃ | H | C₂H₅ |
| 947 | i-C₃H₇ | OCH₃ | H | C₂H₅ |
| 948 | n-C₄H₉ | OCH₃ | H | C₂H₅ |
| 949 | S-C₄H₉ | OCH₃ | H | C₂H₅ |
| 950 | i-C₄H₉ | OCH₃ | H | C₂H₅ |
| 951 | t-C₄H₉ | OCH₃ | H | C₂H₅ |
| 952 | CH₂Cl | OCH₃ | H | C₂H₅ |
| 953 | CHCl₂ | OCH₃ | H | C₂H₅ |
| 954 | CCl₃ | OCH₃ | H | C₂H₅ |
| 955 | CH₂F | OCH₃ | H | C₂H₅ |
| 956 | CHF₂ | OCH₃ | H | C₂H₅ |
| 957 | CF₃ | OCH₃ | H | C₂H₅ |
| 958 | CH₂CF₃ | OCH₃ | H | C₂H₅ |
| 959 | CH₂OCH₃ | OCH₃ | H | C₂H₅ |
| 960 | CH₂OCH₂CH₃ | OCH₃ | H | C₂H₅ |
| 961 | CH₂NH₂ | OCH₃ | H | C₂H₅ |
| 962 | (CH₂)₂COCH₃ | OCH₃ | H | C₂H₅ |
| 963 | Phenyl | OCH₃ | H | C₂H₅ |
| 964 | 2-F-Phenyl | OCH₃ | H | C₂H₅ |
| 965 | 3-F-Phenyl | OCH₃ | H | C₂H₅ |
| 966 | 4-F-Phenyl | OCH₃ | H | C₂H₅ |
| 967 | 2-Cl-Phenyl | OCH₃ | H | C₂H₅ |
| 968 | 3-Cl-Phenyl | OCH₃ | H | C₂H₅ |
| 969 | 4-Cl-Phenyl | OCH₃ | H | C₂H₅ |
| 970 | 2-OH-Phenyl | OCH₃ | H | C₂H₅ |
| 971 | 3-OH-Phenyl | OCH₃ | H | C₂H₅ |
| 972 | 4-OH-Phenyl | OCH₃ | H | C₂H₅ |
| 973 | 2-OCH₃-Phenyl | OCH₃ | H | C₂H₅ |
| 974 | 3-OCH₃-Phenyl | OCH₃ | H | C₂H₅ |
| 975 | 4-OCH₃-Phenyl | OCH₃ | H | C₂H₅ |
| 976 | 2-OCF₃-Phenyl | OCH₃ | H | C₂H₅ |
| 977 | 3-OCF₃-Phenyl | OCH₃ | H | C₂H₅ |
| 978 | 4-OCF₃-Phenyl | OCH₃ | H | C₂H₅ |
| 979 | 2-OCHP₂-Phenyl | OCH₃ | H | C₂H₅ |
| 980 | 3-OCHF₂-Phenyl | OCH₃ | H | C₂H₅ |
| 981 | 4-OCHF₂-Phenyl | OCH₃ | H | C₂H₅ |
| 982 | 2-CF₃-Phenyl | OCH₃ | H | C₂H₅ |
| 983 | 3-CF₃-Phenyl | OCH₃ | H | C₂H₅ |
| 984 | 4-CF₃-Phenyl | OCH₃ | H | C₂H₅ |
| 985 | 2-CH₃-Phenyl | OCH₃ | H | C₂H₅ |
| 986 | 3-CH₃-Phenyl | OCH₃ | H | C₂H₅ |
| 987 | 4-CH₃-Phenyl | OCH₃ | H | C₂H₅ |
| 988 | 2-NO₂-Phenyl | OCH₃ | H | C₂H₅ |
| 989 | 3-NO₂-Phenyl | OCH₃ | H | C₂H₅ |
| 990 | 4-NO₂-Phenyl | OCH₃ | H | C₂H₅ |
| 991 | 2-Pyridyl | OCH₃ | H | C₂H₅ |
| 992 | 3-Pyridyl | OCH₃ | H | C₂H₅ |
| 993 | 4-Pyridyl | OCH₃ | H | C₂H₅ |
| 994 | Cyclohexylamino | OCH₃ | H | C₂H₅ |
| 995 | Cyclopentylamino | OCH₃ | H | C₂H₅ |
| 996 | H | Cl | H | C₂H₅ |
| 997 | CH₃ | Cl | H | C₂H₅ |
| 998 | C₂H₅ | Cl | H | C₂H₅ |
| 999 | n-C₃H₇ | Cl | H | C₂H₅ |
| 999 | i-C₃H₇ | Cl | H | C₂H₅ |
| 1000 | n-C₄H₉ | Cl | H | C₂H₅ |
| 1001 | S-C₄H₉ | Cl | H | C₂H₅ |
| 1002 | i-C₄H₉ | Cl | H | C₂H₅ |
| 1003 | t-C₄H₉ | Cl | H | C₂H₅ |
| 1004 | CH₂Cl | Cl | H | C₂H₅ |
| 1005 | CHCl₂ | Cl | H | C₂H₅ |
| 1006 | CCl₃ | Cl | H | C₂H₅ |
| 1007 | CH₂F | Cl | H | C₂H₅ |
| 1008 | CHF₂ | Cl | H | C₂H₅ |
| 1009 | CF₃ | Cl | H | C₂H₅ |
| 1010 | CH₂CF₃ | Cl | H | C₂H₅ |
| 1011 | CH₂OCH₃ | Cl | H | C₂H₅ |
| 1012 | CH₂OCH₂CH₃ | Cl | H | C₂H₅ |
| 1013 | CH₂NH₂ | Cl | H | C₂H₅ |
| 1014 | (CH₂)₂COCH₃ | Cl | H | C₂H₅ |
| 1015 | Phenyl | Cl | H | C₂H₅ |
| 1016 | 2-F-Phenyl | Cl | H | C₂H₅ |
| 1017 | 3-F-Phenyl | Cl | H | C₂H₅ |
| 1018 | 4-F-Phenyl | Cl | H | C₂H₅ |
| 1019 | 2-Cl-Phenyl | Cl | H | C₂H₅ |
| 1020 | 3-Cl-Phenyl | Cl | H | C₂H₅ |
| 1021 | 4-Cl-Phenyl | Cl | H | C₂H₅ |
| 1022 | 2-OH-Phenyl | Cl | H | C₂H₅ |
| 1023 | 3-OH-Phenyl | Cl | H | C₂H₅ |
| 1024 | 4-OH-Phenyl | Cl | H | C₂H₅ |
| 1025 | 2-OCH₃-Phenyl | Cl | H | C₂H₅ |
| 1026 | 3-OCH₃-Phenyl | Cl | H | C₂H₅ |
| 1027 | 4-OCH₃-Phenyl | Cl | H | C₂H₅ |
| 1028 | 2-OCF₃-Phenyl | Cl | H | C₂H₅ |
| 1029 | 3-OCF₃-Phenyl | Cl | H | C₂H₅ |
| 1030 | 4-OCF₃-Phenyl | Cl | H | C₂H₅ |
| 1031 | 2-OCHF₂-Phenyl | Cl | H | C₂H₅ |
| 1032 | 3-OCHF₂-Phenyl | Cl | H | C₂H₅ |
| 1033 | 4-OCHF₂-Phenyl | Cl | H | C₂H₅ |
| 1034 | 2-CF₃-Phenyl | Cl | H | C₂H₅ |
| 1035 | 3-CF₃-Phenyl | Cl | H | C₂H₅ |
| 1036 | 4-CF₃-Phenyl | Cl | H | C₂H₅ |
| 1037 | 2-CH₃-Phenyl | Cl | H | C₂H₅ |
| 1038 | 3-CH₃-Phenyl | Cl | H | C₂H₅ |
| 1039 | 4-CH₃-Phenyl | Cl | H | C₂H₅ |
| 1040 | 2-NO₂-Phenyl | Cl | H | C₂H₅ |
| 1041 | 3-NO₂-Phenyl | Cl | H | C₂H₅ |
| 1042 | 4-NO₂-Phenyl | Cl | H | C₂H₅ |
| 1043 | 2-Pyridyl | Cl | H | C₂H₅ |
| 1044 | 3-Pyridyl | Cl | H | C₂H₅ |
| 1045 | 4-Pyridyl | Cl | H | C₂H₅ |
| 1046 | Cyclohexylamino | Cl | H | C₂H₅ |
| 1047 | Cyclopentylamino | Cl | H | C₂H₅ |
| 1048 | CH₃ | CH₃ | CH₃ | i-C₃H₇ |
| 1049 | C₂H₅ | CH₃ | CH₃ | i-C₃H₇ |
| 1050 | n-C₃H₇ | CH₃ | CH₃ | i-C₃H₇ |
| 1051 | i-C₃H₇ | CH₃ | CH₃ | i-C₃H₇ |
| 1052 | n-C₄H₉ | CH₃ | CH₃ | i-C₃H₇ |
| 1053 | S-C₄H₉ | CH₃ | CH₃ | i-C₃H₇ |
| 1054 | i-C₄H₉ | CH₃ | CH₃ | i-C₃H₇ |
| 1055 | t-C₄H₉ | CH₃ | CH_{3.} | i-C₃H₇ |
| 1056 | CH₂Cl | CH₃ | CH₃ | i-C₃H₇ |
| 1057 | CHCl₂ | CH₃ | CH₃ | i-C₃H₇ |
| 1058 | CCl₃ | CH₃ | CH₃ | i-C₃H₇ |
| 1059 | CH₂F | CH₃ | CH₃ | i-C₃H₇ |
| 1060 | CHF₂ | CH₃ | CH₃ | i-C₃H₇ |
| 1061 | CF₃ | CH₃ | CH₃ | i-C₃H₇ |
| 1062 | CH₂CF₃ | CH₃ | CH₃ | i-C₃H₇ |
| 1063 | CH₂OCH₃ | CH₃ | CH₃ | i-C₃H₇ |
| 1064 | CH₂OCH₂CH₃ | CH₃ | CH₃ | i-C₃H₇ |
| 1065 | CH₂NH₂ | CH₃ | CH₃ | i-C₃H₇ |
| 1066 | (CH₂)₂COCH₃ | CH₃ | CH₃ | i-C₃H₇ |
| 1067 | Phenyl | CH₃ | CH₃ | i-C₃H₇ |
| 1068 | 2-F-Phenyl | CH₃ | CH₃ | i-C₃H₇ |
| 1069 | 3-F-Phenyl | CH₃ | CH₃ | i-C₃H₇ |
| 1070 | 4-F-Phenyl | CH₃ | CH₃ | i-C₃H₇ |
| 1071 | 2-Cl-Phenyl | CH₃ | CH₃ | i-C₃H₇ |
| 1072 | 3-Cl-Phenyl | CH₃ | CH₃ | i-C₃H₇ |
| 1073 | 4-Cl-Phenyl | CH₃ | CH₃ | i-C₃H₇ |
| 1074 | 2-OH-Phenyl | CH₃ | CH₃ | i-C₃H₇ |
| 1075 | 3-OH-Phenyl | CH₃ | CH₃ | i-C₃H₇ |
| 1076 | 4-OH-Phenyl | CH3 | CH₃ | i-C₃H₇ |
| 1077 | 2-OCH₃-Phenyl | CH₃ | CH₃ | i-C₃H₇ |
| 1078 | 3-OCH₃-Phenyl | CH₃ | CH₃ | i-C₃H₇ |
| 1079 | 4-OCH₃-Phenyl | CH₃ | CH₃ | i-C₃H₇ |
| 1080 | 2-OCF₃-Phenyl | CH₃ | CH₃ | i-C₃H₇ |
| 1081 | 3-OCF₃-Phenyl | CH₃ | CH₃ | i-C₃H₇ |
| 1082 | 4-OCF₃-Phenyl | CH₃ | CH₃ | i-C₃H₇ |
| 1083 | 2-OCHF₂-Phenyl | CH₃ | CH₃ | i-C₃H₇ |
| 1084 | 3-OCHF₂-Phenyl | CH₃ | CH₃ | i-C₃H₇ |
| 1085 | 4-OCHF₂-Phenyl | CH₃ | CH₃ | i-C₃H₇ |
| 1086 | 2-CF₃-Phenyl | CH₃ | CH₃ | i-C₃H₇ |
| 1087 | 3-CF₃-Phenyl | CH₃ | CH₃ | i-C₃H₇ |
| 1088 | 4-CF₃-Phenyl | CH₃ | CH₃ | i-C₃H₇ |
| 1089 | 2-CH₃-Phenyl | CH₃ | CH₃ | i-C₃H₇ |
| 1090 | 3-CH₃-Phenyl | CH₃ | CH₃ | i-C₃H₇ |
| 1091 | 4-CH₃-Phenyl | CH₃ | CH₃ | i-C₃H₇ |
| 1092 | 2-NO₂-Phenyl | CH₃ | CH₃ | i-C₃H₇ |
| 1093 | 3-NO₂-Phenyl | CH₃ | CH₃ | i-C₃H₇ |
| 1094 | 4-NO₂-Phenyl | CH₃ | CH₃ | i-C₃H₇ |
| 1095 | 2-Pyridyl | CH₃ | CH₃ | i-C₃H₇ |
| 1096 | 3-Pyridyl | CH₃ | CH₃ | i-C₃H₇ |
| 1097 | 4-Pyridyl | CH₃ | CH₃ | i-C₃H₇ |
| 1098 | Cyclohexylamino | CH₃ | CH₃ | i-C₃H₇ |
| 1099 | Cyclopentylamino | CH₃ | CH₃ | i-C3H₇ |
| 1100 | H | OCH₃ | CH₃ | i-C₃H₇ |
| 1101 | CH₃ | OCH₃ | CH₃ | i-C₃H₇ |
| 1102 | C₂H₅ | OCH₃ | CH₃ | i-C₃H₇ |
| 1103 | n-C₃H₇ | OCH₃ | CH₃ | i-C₃H₇ |
| 1104 | i-C₃H₇ | OCH₃ | CH₃ | i-C₃H₇ |
| 1105 | n-C₄H₉ | OCH₃ | CH₃ | i-C₃H₇ |
| 1106 | S-C₄H₉ | OCH₃ | CH₃ | i-C₃H₇ |
| 1107 | i-C₄H₉ | OCH₃ | CH₃ | i-C₃H₇ |
| 1108 | t-C₄H₉ | OCH₃ | CH₃ | i-C₃H₇ |
| 1109 | CH₂Cl | OCH₃ | CH₃ | i-C₃H₇ |
| 1110 | CHCl₂ | OCH₃ | CH₃ | i-C₃H₇ |
| 1111 | CCl₃ | OCH₃ | CH₃ | i-C₃H₇ |
| 1112 | CH₂F | OCH₃ | CH₃ | i-C₃H₇ |
| 1113 | CHF₂ | OCH₃ | CH₃ | i-C₃H₇ |
| 1114 | CF₃ | OCH₃ | CH₃ | i-C₃H₇ |
| 1115 | CH₂CF₃ | OCH₃ | CH₃ | i-C₃H₇ |
| 1116 | CH₂OCH₃ | OCH₃ | CH₃ | i-C₃H₇ |
| 1117 | CH₂OCH₂CH₃ | OCH₃ | CH₃ | i-C₃H₇ |
| 1118 | CH₂NH₂ | OCH₃ | CH₃ | i-C₃H₇ |
| 1119 | (CH₂)₂COCH₃ | OCH₃ | CH₃ | i-C₃H₇ |
| 1120 | Phenyl | OCH₃ | CH₃ | i-C₃H₇ |
| 1121 | 2-F-Phenyl | OCH₃ | CH₃ | i-C₃H₇ |
| 1122 | 3-F-Phenyl | OCH₃ | CH₃ | i-C₃H₇ |
| 1123 | 4-F-Phenyl | OCH₃ | CH₃ | i-C₃H₇ |
| 1124 | 2-Cl-Phenyl | OCH₃ | CH₃ | i-C₃H₇ |
| 1125 | 3-Cl-Phenyl | OCH₃ | CH₃ | i-C₃H₇ |
| 1126 | 4-Cl-Phenyl | OCH₃ | CH₃ | i-C₃H₇ |
| 1127 | 2-OH-Phenyl | OCH₃ | CH₃ | i-C₃H₇ |
| 1128 | 3-OH-Phenyl | OCH₃ | CH₃ | i-C₃H₇ |
| 1129 | 4-OH-Phenyl | OCH₃ | CH₃ | i-C₃H₇ |
| 1130 | 2-OCH₃-Phenyl | OCH₃ | CH₃ | i-C₃H₇ |
| 1131 | 3-OCH₃-Phenyl | OCH₃ | CH₃ | i-C₃H₇ |
| 1132 | 4-OCH₃-Phenyl | OCH₃ | CH₃ | i-C₃H₇ |
| 1133 | 2-OCF₃-Phenyl | OCH₃ | CH₃ | i-C₃H₇ |
| 1134 | 3-OCF₃-Phenyl | OCH₃ | CH₃ | i-C₃H₇ |
| 1135 | 4-OCF₃-Phenyl | OCH₃ | CH₃ | i-C₃H₇ |
| 1136 | 2-OCHF₂-Phenyl | OCH₃ | CH₃ | i-C₃H₇ |
| 1137 | 3-OCHF₂-Phenyl | OCH₃ | CH₃ | i-C₃H₇ |
| 1138 | 4-OCHF₂-Phenyl | OCH₃ | CH₃ | i-C₃H₇ |
| 1139 | 2-CF₃-Phenyl | OCH₃ | CH₃ | i-C₃H₇ |
| 1140 | 3-CF₃-Phenyl | OCH₃ | CH₃ | i-C₃H₇ |
| 1141 | 4-CF₃-Phenyl | OCH₃ | CH₃ | i-C₃H₇ |
| 1142 | 2-CH₃-Phenyl | OCH₃ | CH₃ | i-C₃H₇ |
| 1143 | 3-CH₃-Phenyl | OCH₃ | CH₃ | i-C₃H₇ |
| 1144 | 4-CH₃-Phenyl | OCH₃ | CH₃ | i-C₃H₇ |
| 1145 | 2-NO₂-Phenyl | OCH₃ | CH₃ | i-C₃H₇ |
| 1146 | 3-NO₂-Phenyl | OCH₃ | CH₃ | i-C₃H₇ |
| 1147 | 4-NO₂-Phenyl | OCH₃ | CH₃ | i-C₃H₇ |
| 1148 | 2-Pyridyl | OCH₃ | CH₃ | i-C₃H₇ |
| 1149 | 3-Pyridyl | OCH₃ | CH₃ | i-C₃H₇ |
| 1150 | 4-Pyridyl | OCH₃ | CH₃ | i-C₃H₇ |
| 1151 | Cyclohexylamino | OCH₃ | CH₃ | i-C₃H₇ |
| 1152 | Cyclopentylamino | OCH₃ | CH₃ | i-C₃H₇ |
| 1153 | H | Cl | CH₃ | i-C₃H₇ |
| 1154 | CH₃ | Cl | CH₃ | i-C₃H₇ |
| 1155 | C₂H₅ | Cl | CH₃ | i-C₃H₇ |
| 1156 | n-C₃H₇ | Cl | CH₃ | i-C₃H₇ |
| 1157 | i-C₃H₇ | Cl | CH₃ | i-C₃H₇ |
| 1158 | n-C₄H₉ | Cl | CH₃ | i-C₃H₇ |
| 1159 | s-C₄H₉ | Cl | CH₃ | i-C₃H₇ |
| 1160 | i-C₄H₉ | Cl | CH₃ | i-C₃H₇ |
| 1161 | t-C₄H₉ | Cl | CH₃ | i-C₃H₇ |
| 1162 | CH₂Cl | Cl | CH₃ | i-C₃H₇ |
| 1163 | CHCl₂ | Cl | CH₃ | i-C₃H₇ |
| 1164 | CCl₃ | Cl | CH₃ | i-C₃H₇ |
| 1165 | CH₂F | Cl | CH₃ | i-C₃H₇ |
| 1166 | CHF₂ | Cl | CH₃ | i-C₃H₇ |
| 1167 | CF₃ | Cl | CH₃ | i-C₃H₇ |
| 1168 | CH₂CF₃ | Cl | CH₃ | i-C₃H₇ |
| 1169 | CH₂OCH₃ | Cl | CH₃ | i-C₃H₇ |
| 1170 | CH₂OCH₂CH₃ | Cl | CH₃ | i-C₃H₇ |
| 1171 | CH₂NH₂ | Cl | CH₃ | i-C₃H₇ |
| 1172 | (CH₂)₂COCH₃ | Cl | CH₃ | i-C₃H₇ |
| 1173 | Phenyl | Cl | CH₃ | i-C₃H₇ |
| 1174 | 2-F-Phenyl | Cl | CH₃ | i-C₃H₇ |
| 1175 | 3-F-Phenyl | Cl | CH₃ | i-C₃H₇ |
| 1176 | 4-F-Phenyl | Cl | CH₃ | i-C₃H₇ |
| 1177 | 2-Cl-Phenyl | Cl | CH₃ | i-C₃H₇ |
| 1178 | 3-Cl-Phenyl | Cl | CH₃ | i-C₃H₇ |
| 1179 | 4-Cl-Phenyl | Cl | CH₃ | i-C₃H₇ |
| 1180 | 2-OH-Phenyl | Cl | CH₃ | i-C₃H₇ |
| 1181 | 3-OH-Phenyl | Cl | CH₃ | i-C₃H₇ |
| 1182 | 4-OH-Phenyl | Cl | CH₃ | i-C₃H₇ |
| 1183 | 2-OCH₃-Phenyl | Cl | CH₃ | i-C₃H₇ |
| 1184 | 3-OCH₃-Phenyl | Cl | CH₃ | i-C₃H₇ |
| 1185 | 4-OCH₃-Phenyl | Cl | CH₃ | i-C₃H₇ |
| 1186 | 2-OCF₃-Phenyl | Cl | CH₃ | i-C₃H₇ |
| 1187 | 3-OCF₃-Phenyl | Cl | CH₃ | i-C₃H₇ |
| 1188 | 4-OCF₃-Phenyl | Cl | CH₃ | i-C₃H₇ |
| 1189 | 2-OCHF₂-Phenyl | Cl | CH₃ | i-C₃H₇ |
| 1190 | 3-OCHF₂-Phenyl | Cl | CH₃ | i-C₃H₇ |
| 1191 | 4-OCHF₂-Phenyl | Cl | CH₃ | i-C₃H₇ |
| 1192 | 2-CF₃-Phenyl | Cl | CH₃ | i-C₃H₇ |
| 1193 | 3-CF₃-Phenyl | Cl | CH₃ | i-C₃H₇ |
| 1194 | 4-CF₃-Phenyl | Cl | CH₃ | i-C₃H₇ |
| 1195 | 2-CH₃-Phenyl | Cl | CH₃ | i-C₃H₇ |
| 1196 | 3-CH₃-Phenyl | Cl | CH₃ | i-C₃H₇ |
| 1197 | 4-CH₃-Phenyl | Cl | CH₃ | i-C₃H₇ |
| 1198 | 2-NO₂-Phenyl | Cl | CH₃ | i-C₃H₇ |
| 1199 | 3-NO₂-Phenyl | Cl | CH₃ | i-C₃H₇ |
| 1200 | 4-NO₂-Phenyl | Cl | CH₃ | i-C₃H₇ |
| 1201 | 2-Pyridyl | Cl | CH₃ | i-C₃H₇ |
| 1202 | 3-Pyridyl | Cl | CH₃ | i-C₃H₇ |
| 1203 | 4-Pyridyl | Cl | CH₃ | i-C₃H₇ |
| 1204 | Cyclohexylamino | Cl | CH₃ | i-C₃H₇ |
| 1205 | Cyclopentylamino | Cl | CH₃ | i-C₃H₇ |
| 1206 | CH₃ | CH₃ | H | i-C₃H₇ |
| 1207 | C₂H₅ | CH₃ | H | i-C₃H₇ |
| 1208 | n-C₃H₇ | CH₃ | H | i-C₃H₇ |
| 1209 | i-C₃H₇ | CH₃ | H | i-C₃H₇ |
| 1210 | n-C₄H₉ | CH₃ | H | i-C₃H₇ |
| 1211 | S-C₄H₉ | CH₃ | H | i-C₃H₇ |
| 1212 | i-C₄H₉ | CH₃ | H | i-C₃H₇ |
| 1213 | t-C₄H₉ | CH₃ | H | i-C₃H₇ |
| 1214 | CH₂Cl | CH₃ | H | i-C₃H₇ |
| 1215 | CHCl₂ | CH₃ | H | i-C₃H₇ |
| 1116 | CCl₃ | CH₃ | H | i-C₃H₇ |
| 1217 | CH₂F | CH₃ | H | i-C₃H₇ |
| 1218 | CHF₂ | CH₃ | H | i-C₃H₇ |
| 1219 | CF₃ | CH₃ | H | i-C₃H₇ |
| 1220 | CH₂CF₃ | CH₃ | H | i-C₃H₇ |
| 1221 | CH₂OCH₃ | CH₃ | H | i-C₃H₇ |
| 1222 | CH₂OCH₂CH₃ | CH₃ | H | i-C₃H₇ |
| 1223 | CH₂MH₂ | CH₃ | H | i-C₃H₇ |
| 1224 | (CH₂)₂COCH₃ | CH₃ | H | i-C₃H₇ |
| 1225 | Phenyl | CH₃ | H | i-C₃H₇ |
| 1226 | 2-F-Phenyl | CH₃ | H | i-C₃H₇ |
| 1227 | 3-F-Phenyl | CH₃ | H | i-C₃H₇ |
| 1228 | 4-F-Phenyl | CH₃ | H | i-C₃H₇ |
| 1229 | 2-Cl-Phenyl | CH₃ | H | i-C₃H₇ |
| 1230 | 3-Cl-Phenyl | CH₃ | H | i-C₃H₇ |
| 1231 | 4-Cl-Phenyl | CH₃ | H | i-C₃H₇ |
| 1232 | 2-OH-Phenyl | CH₃ | H | i-C₃H₇ |
| 1233 | 3-OH-Phenyl | CH₃ | H | i-C₃H₇ |
| 1234 | 4-OH-Phenyl | CH₃ | H | i-C₃H₇ |
| 1235 | 2-OCH₃-Phenyl | CH₃ | H | i-C₃H₇ |
| 1236 | 3-OCH₃-Phenyl | CH₃ | H | i-C₃H₇ |
| 1237 | 4-OCH₃-Phenyl | CH₃ | H | i-C₃H7 |
| 1238 | 2-OCF₃-Phenyl | CH₃ | H | i-C₃H₇ |
| 1239 | 3-OCF₃-Phenyl | CH₃ | H | i-C₃H₇ |
| 1240 | 4-OCF₃-Phenyl | CH₃ | H | i-C₃H₇ |
| 1241 | 2-OCHF₂-Phenyl | CH₃ | H | i-C₃H₇ |
| 1242 | 3- OCHF₂-Phenyl | CH₃ | H | i-C₃H₇ |
| 1243 | 4-OCHF₂-Phenyl | CH₃ | H | i-C₃H₇ |
| 1244 | 2-CF₃-Phenyl | CH₃ | H | i-C₃H₇ |
| 1245 | 3-CF₃-Phenyl | CH₃ | H | i-C₃H₇ |
| 1246 | 4-CF₃-Phenyl | CH₃ | H | i-C₃H₇ |
| 1247 | 2-CH₃-Phenyl | CH₃ | H | i-C₃H₇ |
| 1248 | 3-CH₃-Phenyl | CH₃ | H | i-C₃H₇ |
| 1249 | 4-CH₃-Phenyl | CH₃ | H | i-C₃H₇ |
| 1250 | 2-NO₂-Phenyl | CH₃ | H | i-C₃H₇ |
| 1251 | 3-NO₂-Phenyl | CH₃ | H | i-C₃H₇ |
| 1252 | 4-NO₂-Phenyl | CH₃ | H | i-C₃H₇ |
| 1253 | 2-Pyridyl | CH₃ | H | i-C₃H₇ |
| 1254 | 3-Pyridyl | CH₃ | H | i-C₃H₇ |
| 1255 | 4-Pyridyl | CH₃ | H | i-C₃H₇ |
| 1256 | Cyclohexylamino | CH₃ | H | i-C₃H₇ |
| 1257 | Cyclopentylamino | CH₃ | H | i-C₃H₇ |
| 1258 | H | OCH₃ | H | i-C₃H₇ |
| 1259 | CH₃ | OCH₃ | H | i-C₃H₇ |
| 1260 | C₂H₅ | OCH₃ | H | i-C₃H₇ |
| 1261 | n-C₃H₇ | OCH₃ | H | i-C₃H₇ |
| 1262 | i-C₃H₇ | OCH₃ | H | i-C₃H₇ |
| 1263 | n-C₄H₉ | OCH₃ | H | i-C₃H₇ |
| 1264 | S-C₄H₉ | OCH₃ | H | i-C₃H₇ |
| 1265 | i-C₄H₉ | OCH₃ | H | i-C₃H₇ |
| 1266 | t-C₄H₉ | OCH₃ | H | i-C₃H₇ |
| 1267 | CH₂Cl | OCH₃ | H | i-C₃H₇ |
| 1268 | CHCl₂ | OCH₃ | H | i-C₃H₇ |
| 1269 | CCl₃ | OCH₃ | H | i-C₃H₇ |
| 1270 | CH₂F | OCH₃ | H | i-C₃H₇ |
| 1271 | CHF₂ | OCH₃ | H | i-C₃H₇ |
| 1272 | CF₃ | OCH₃ | H | i-C₃H₇ |
| 1273 | CH₂CF₃ | OCH₃ | H | i-C₃H₇ |
| 1274 | CH₂OCH₃ | OCH₃ | H | i-C₃H₇ |
| 1275 | CH₂OCH₂CH₃ | OCH₃ | H | i-C₃H₇ |
| 1276 | CH₂NH₂ | OCH₃ | H | i-C₃H₇ |
| 1277 | (CH₂)₂COCH₃ | OCH₃ | H | i-C₃H₇ |
| 1278 | Phenyl | OCH₃ | H | i-C₃H₇ |
| 1279 | 2-F-Phenyl | OCH₃ | H | i-C₃H₇ |
| 1280 | 3-F-Phenyl | OCH₃ | H | i-C₃H₇ |
| 1281 | 4-F-Phenyl | OCH₃ | H | i-C₃H₇ |
| 1282 | 2-Cl-Phenyl | OCH₃ | H | i-C₃H₇ |
| 1283 | 3-Cl-Phenyl | OCH₃ | H | i-C₃H₇ |
| 1284 | 4-Cl-Phenyl | OCH₃ | H | i-C₃H₇ |
| 1285 | 2-OH-Phenyl | OCH₃ | H | i-C₃H₇ |
| 1286 | 3-OH-Phenyl | OCH₃ | H | i-C₃H₇ |
| 1287 | 4-OH-Phenyl | OCH₃ | H | i-C₃H₇ |
| 1288 | 2-OCH₃-Phenyl | OCH₃ | H | i-C₃H₇ |
| 1289 | 3-OCH₃-Phenyl | OCH₃ | H | i-C₃H₇ |
| 1290 | 4-OCH₃-Phenyl | OCH₃ | H | i-C₃H₇ |
| 1291 | 2-OCF₃-Phenyl | OCH₃ | H | i-C₃H₇ |
| 1292 | 3-OCF₃-Phenyl | OCH₃ | H | i-C₃H₇ |
| 1293 | 4-OCF₃-Phenyl | OCH₃ | H | i-C₃H₇ |
| 1294 | 2-OCHF₂-Phenyl | OCH₃ | H | i-C₃H₇ |
| 1295 | 3-OCHF₂-Phenyl | OCH₃ | H | i-C₃H₇ |
| 1296 | 4-OCHF₂-Phenyl | OCH₃ | H | i-C₃H₇ |
| 1297 | 2-CF₃-Phenyl | OCH₃ | H | i-C₃H₇ |
| 1298 | 3-CF₃-Phenyl | OCH₃ | H | i-C₃H₇ |
| 1299 | 4-CF₃-Phenyl | OCH₃ | H | i-C₃H₇ |
| 1300 | 2-CH₃-Phenyl | OCH₃ | H | i-C₃H₇ |
| 1301 | 3-CH₃-Phenyl | OCH₃ | H | i-C₃H₇ |
| 1302 | 4-CH₃-Phenyl | OCH₃ | H | i-C₃H₇ |
| 1303 | 2-NO₂-Phenyl | OCH₃ | H | i-C₃H₇ |
| 1304 | 3-NO₂-Phenyl | OCH₃ | H | i-C₃H₇ |
| 1305 | 4-NO₂-Phenyl | OCH₃ | H | i-C₃H₇ |
| 1306 | 2-Pyridyl | OCH₃ | H | i-C₃H₇ |
| 1307 | 3-Pyridyl | OCH₃ | H | i-C₃H₇ |
| 1308 | 4-Pyridyl | OCH₃ | H | i-C₃H₇ |
| 1309 | Cyclohexylamino | OCH₃ | H | i-C₃H₇ |
| 1310 | Cyclopentylamino | OCH₃ | H | i-C₃H₇ |
| 1311 | H | Cl | H | i-C₃H₇ |
| 1312 | CH₃ | Cl | H | i-C₃H₇ |
| 1313 | C₂H₅ | Cl | H | i-C₃H₇ |
| 1314 | n-C₃H₇ | Cl | H | i-C₃H₇ |
| 1315 | i-C₃H₇ | Cl | H | i-C₃H₇ |
| 1316 | n-C₄H₉ | Cl | H | i-C₃H₇ |
| 1317 | S-C₄H₉ | Cl | H | i-C₃H₇ |
| 1318 | i-C₄H₉ | Cl | H | i-C₃H₇ |
| 1319 | t-C₄H₉ | Cl | H | i-C₃H₇ |
| 1320 | CH₂Cl | Cl | H | i-C₃H₇ |
| 1321 | CHCl₂ | Cl | H | i-C₃H₇ |
| 1322 | CH₃ | Cl | H | i-C₃H₇ |
| 1323 | CH₂F | Cl | H | i-C₃H₇ |
| 1324 | CHF₂ | Cl | H | i-C₃H₇ |
| 1325 | CF₃ | Cl | H | i-C₃H₇ |
| 1326 | CH₂CF₃ | Cl | H | i-C₃H₇ |
| 1327 | CH₂OCH₃ | Cl | H | i-C₃H₇ |
| 1328 | CH₂OCH₂CH₃ | Cl | H | i-C₃H₇ |
| 1329 | CH₂NH₂ | Cl | H | i-C₃H₇ |
| 1330 | (CH₂)₂COCH₃ | Cl | H | i-C₃H₇ |
| 1331 | Phenyl | Cl | H | i-C₃H₇ |
| 1332 | 2-F-Phenyl | Cl | H | i-C₃H₇ |
| 1333 | 3-F-Phenyl | Cl | H | i-C₃H₇ |
| 1334 | 4-F-Phenyl | Cl | H | i-C₃H₇ |
| 1335 | 2-Cl-Phenyl | Cl | H | i-C₃H₇ |
| 1336 | 3-Cl-Phenyl | Cl | H | i-C₃H₇ |
| 1337 | 4-Cl-Phenyl | Cl | H | i-C₃H₇ |
| 1338 | 2-OH-Phenyl | Cl | H | i-C₃H₇ |
| 1339 | 3-OH-Phenyl | Cl | H | i-C3H7 |
| 1340 | 4-OH-Phenyl | Cl | H | i-C₃H₇ |
| 1341 | 2-OCH₃-Phenyl | Cl | H | i-C₃H₇ |
| 1342 | 3-OCH₃-Phenyl | Cl | H | i-C₃H₇ |
| 1343 | 4-OCH₃-Phenyl | Cl | H | i-C₃H₇ |
| 1344 | 2-OCF₃-Phenyl | Cl | H | i-C₃H₇ |
| 1345 | 3-OCF₃-Phenyl | OCH₃ | H | i-C₃H₇ |
| 1346 | 4-OCF₃-Phenyl | OCH₃ | H | i-C₃H₇ |
| 1347 | 2-OCHF₂-Phenyl | OCH₃ | H | i-C₃H₇ |
| 1348 | 3-OCHF₂-Phenyl | OCH₃ | H | i-C₃H₇ |
| 1349 | 4-OCHF₂-Phenyl | OCH₃ | H | i-C₃H₇ |
| 1350 | 2-CF₃-Phenyl | OCH₃ | H | i-C₃H₇ |
| 1351 | 3-CF₃-Phenyl | OCH₃ | H | i-C₃H₇ |
| 1352 | 4-CF₃-Phenyl | OCH₃ | H | i-C₃H₇ |
| 1353 | 2-CH₃-Phenyl | OCH₃ | H | i-C₃H₇ |
| 1353 | 3-CH₃-Phenyl | OCH₃ | H | i-C₃H₇ |
| 1354 | 4-CH₃-Phenyl | OCH₃ | H | i-C₃H₇ |
| 1355 | H | CH₃ | H | H |
| 1356 | 3-NO₂-Phenyl | OCH₃ | H | i-C₃H₇ |
| 1357 | H | CH₃ | CH₃ | CH₃ |
| 1358 | 2-Pyridyl | OCH₃ | H | i-C₃H₇ |
| 1359 | H | CH₃ | CH₃ | C₂H₅ |
| 1360 | H | CH₃ | H | C₂H₅ |
| 1361 | H | CH₃ | CH₃ | i-C₃H₇ |
| 1362 | H | CH₃ | H | i-C₃H₇ |
| 1363 | H | CH₃ | H | CH₃ |

Beispiele für erfindungsgemäße besonders bevorzugte Benzimidazol-5-ylcarbonyl-Derivate von Pyrazolen (Verbindungen I-3 = Verbindungen I mit X = C-R³ und Y = N-R⁴) sind die in den Tabellen 40 bis 58 genannten Verbindungen.
- Tabelle 40: Verbindungen I-3a.1 bis I-3a.1363 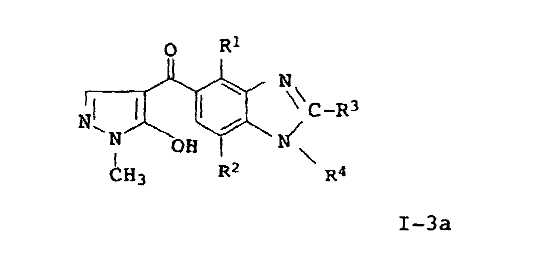 Verbindungen der allgemeinen Formel I-3a, in der die Substituenten R¹, R² R³ und R⁴ für jede einzelne Verbindung jeweils einer Zeile der Tabelle B entsprechen.
- Tabelle 41: Verbindungen I-3b.1 bis I-3b.1363 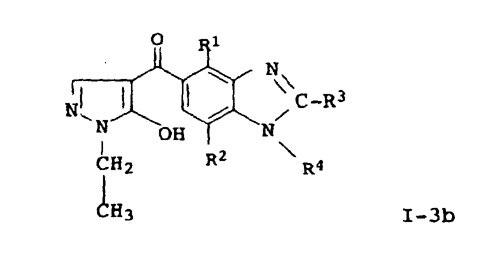 Verbindungen der allgemeinen Formel I-3b, in der die Substituenten R¹, R² R³ und R⁴ für jede einzelne Verbindung jeweils einer Zeile der Tabelle B entsprechen.
- Tabelle 42: Verbindungen I-3c.1 bis I-3c.1363 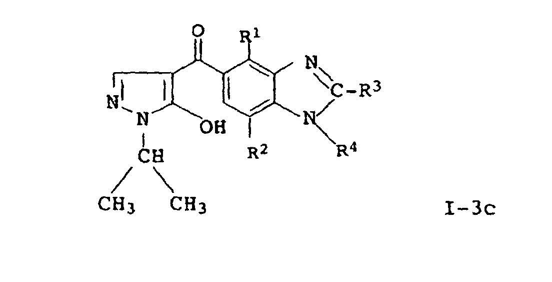 Verbindungen der allgemeinen Formel I-3c, in der die Substituenten R¹, R² R³ und R⁴ für jede einzelne Verbindung jeweils einer Zeile der Tabelle B entsprechen.
- Tabelle 43: Verbindungen I-3d.1 bis I-3d.1363 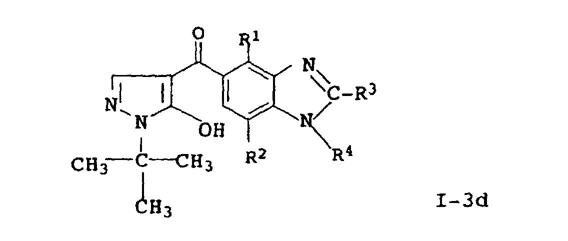 Verbindungen der allgemeinen Formel I-3d, in der die Substituenten R¹, R² R³ und R⁴ für jede einzelne Verbindung jeweils einer Zeile der Tabelle B entsprechen.
- Tabelle 44: Verbindungen I-3e.1 bis I-3e.1363 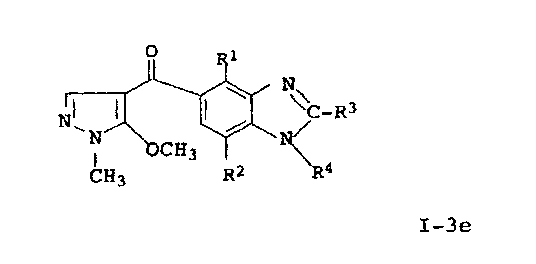 Verbindungen der allgemeinen Formel I-3e, in der die Substituenten R¹, R² R³ und R⁴ für jede einzelne Verbindung jeweils einer Zeile der Tabelle B entsprechen.
- Tabelle 45: Verbindungen I-3f.1 bis I-3f.1363 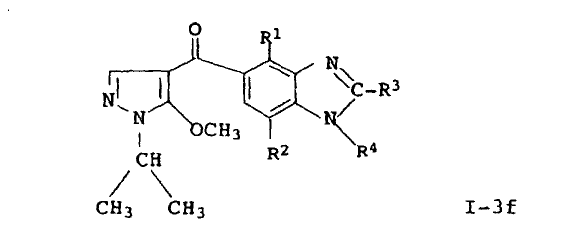 Verbindungen der allgemeinen Formel I-3f, in der die Substituenten R¹, R² R³ und R⁴ für jede einzelne Verbindung jeweils einer Zeile der Tabelle B entsprechen.
- Tabelle 46: Verbindungen I-3g.1 bis I-3g.1363 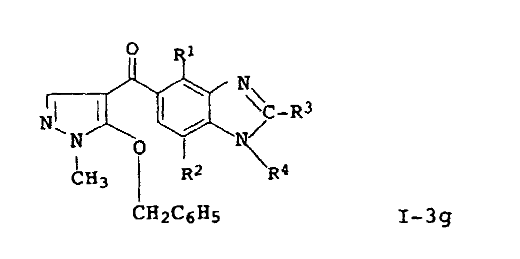 Verbindungen der allgemeinen Formel I-3g, in der die Substituenten R¹, R² R³ und R⁴ für jede einzelne Verbindung jeweils einer Zeile der Tabelle B entsprechen.
- Tabelle 47: Verbindungen I-3h.1 bis I-3h.1363 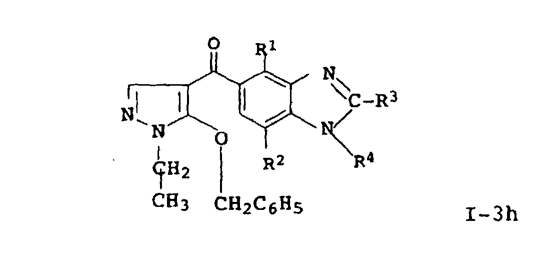 Verbindungen der allgemeinen Formel I-3h, in der die Substituenten R¹, R² R³ und R⁴ für jede einzelne Verbindung jeweils einer Zeile der Tabelle B entsprechen.
- Tabelle 48: Verbindungen I-3i.1 bis 1-3i.1363 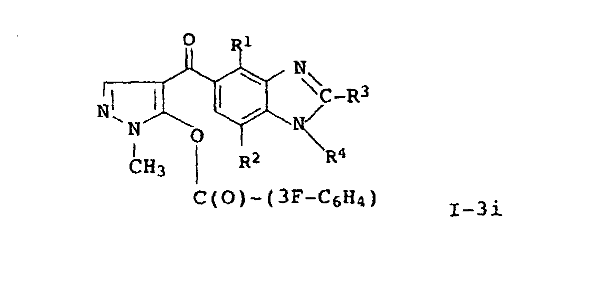 Verbindungen der allgemeinen Formel I-3i, in der die Substituenten R¹, R² R³ und R⁴ für jede einzelne Verbindung jeweils einer Zeile der Tabelle B entsprechen.
- Tabelle 49: Verbindungen I-3k.1 bis I-3k.1363 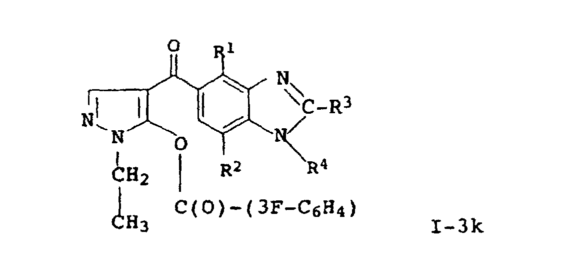 Verbindungen der allgemeinen Formel I-3k, in der die Substituenten R¹, R² R³ und R⁴ für jede einzelne Verbindung jeweils einer Zeile der Tabelle B entsprechen.
- Tabelle 50: Verbindungen 1-31.1 bis 1-31.1363 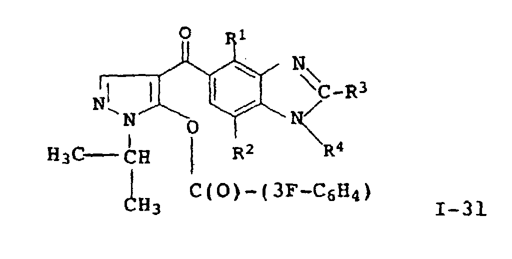 Verbindungen der allgemeinen Formel I-31, in der die Substituenten R¹, R² R³ und R⁴ für jede einzelne Verbindung jeweils einer Zeile der Tabelle B entsprechen.
- Tabelle 51: Verbindungen I-3m.1 bis I-3m.1363 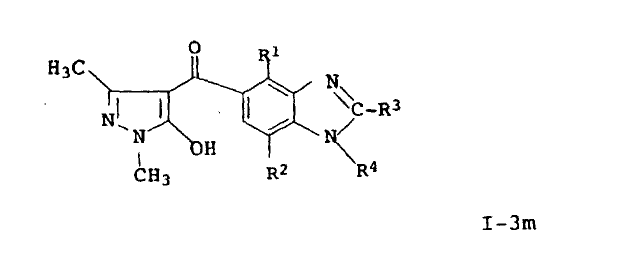 Verbindungen der allgemeinen Formel I-3m, in der die Substituenten R¹, R² R³ und R⁴ für jede einzelne Verbindung jeweils einer Zeile der Tabelle B entsprechen.
- Tabelle 52: Verbindungen I-3n.1 bis I-3n.1363 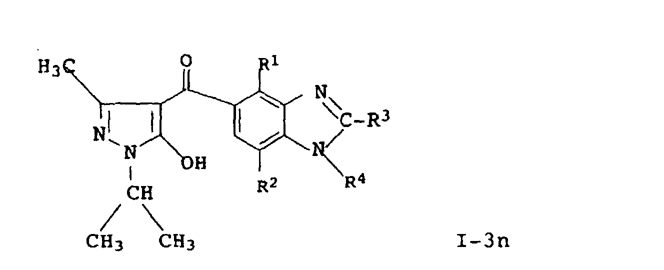 Verbindungen der allgemeinen Formel I-3n, in der die Substituenten R¹, R² R³ und R⁴ für jede einzelne Verbindung jeweils einer Zeile der Tabelle B entsprechen.
- Tabelle 53: Verbindungen I-3o.1 bis I-3o.1363 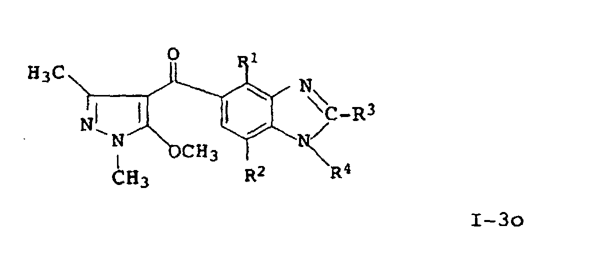 Verbindungen der allgemeinen Formel I-3p, in der die Substituenten R¹, R² R³ und R⁴ für jede einzelne Verbindung jeweils einer Zeile der Tabelle B entsprechen.
- Tabelle 54: Verbindungen I-3p.1 bis I-3p.1363 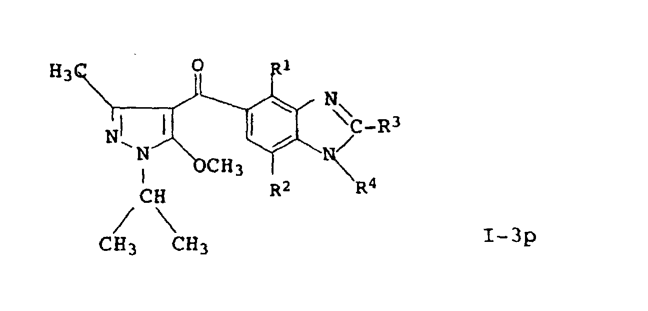 Verbindungen der allgemeinen Formel I-3p, in der die Substituenten R¹, R² R³ und R⁴ für jede einzelne Verbindung jeweils einer Zeile der Tabelle B entsprechen.
- Tabelle 55: Verbindungen I-3q.1 bis I-3q.1363 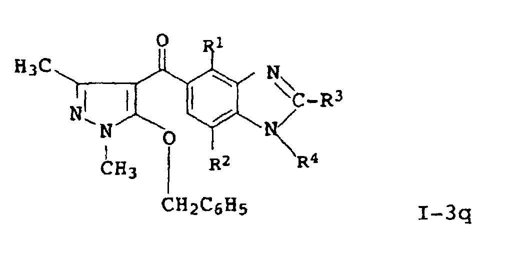 Verbindungen der allgemeinen Formel I-3g, in der die Substituenten R¹, R² R³ und R⁴ für jede einzelne Verbindung jeweils einer Zeile der Tabelle B entsprechen.
- Tabelle 56: Verbindungen I-3r.1 bis I-3r.1363 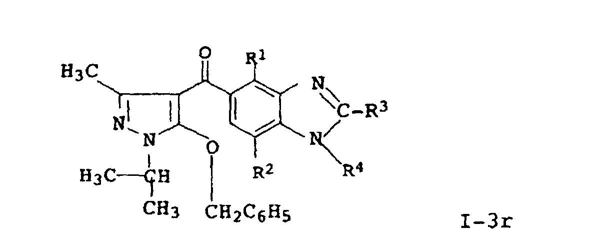 Verbindungen der allgemeinen Formel I-3r, in der die Substituenten R¹, R² R³ und R⁴ für jede einzelne Verbindung jeweils einer Zeile der Tabelle B entsprechen.
- Tabelle 57: Verbindungen I-3s.1 bis I-3s.1363 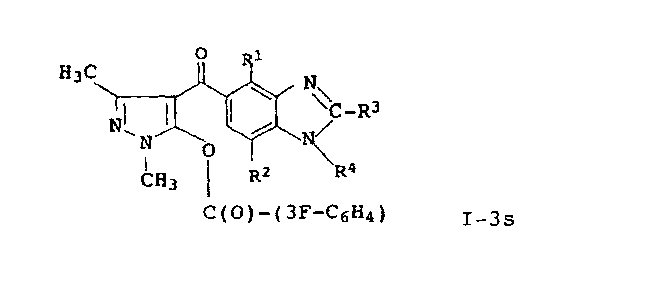 Verbindungen der allgemeinen Formel I-3s, in der die Substituenten R¹, R² R³ und R⁴ für jede einzelne Verbindung jeweils einer Zeile der Tabelle B entsprechen.
- Tabelle 58: Verbindungen I-3t.1 bis I-3t.1363 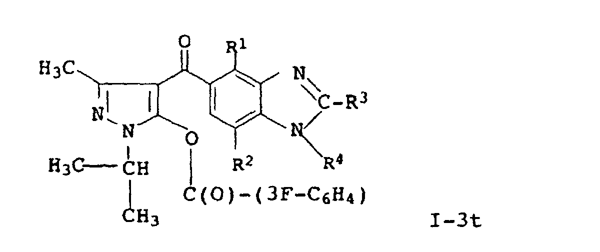 Verbindungen der allgemeinen Formel I-3t, in der die Substituenten R¹, R², R³ und R⁴ für jede einzelne Verbindung jeweils einer Zeile der Tabelle B entsprechen.

**Tabelle C**

| | R¹ | R² | X | Y |
|---|---|---|---|---|
| 1 | H | H | N | S |
| 2 | CH₃ | H | N | S |
| 3 | C1 | H | N | S |
| 4 | OCH₃ | H | N | S |
| 5 | SCH₃ | H | N | S |
| 6 | S(O)₂CH₃ | H | N | S |
| 7 | H | Cl | N | S |
| 8 | CH₃ | Cl | N | S |
| 9 | Cl | Cl | N | S |
| 10 | OCH₃ | Cl | N | S |
| 11 | SCH₃ | Cl | N | S |
| 12 | S(O)₂CH₃ | Cl | N | S |
| 13 | H | CH₃ | N | S |
| 14 | CH₃ | CH₃ | N | S |
| 15 | Cl | CH₃ | N | S |
| 16 | OCH₃ | CH₃ | N | S |
| 17 | SCH₃ | CH₃ | N | S |
| 18 | S(O)₂CH₃ | CH₃ | N | S |
| 19 | H | H | N | NH |
| 20 | CH₃ | H | N | NH |
| 21 | Cl | H | N | NH |
| 22 | OCH₃ | H | N | NH |
| 23 | SCH₃ | H | N | NH |
| 24 | S(O)₂CH₃ | H | N | NH |
| 25 | H | Cl | N | NH |
| 26 | CH₃ | Cl | N | NH |
| 27 | Cl | Cl | N | NH |
| 28 | OCH₃ | Cl | N | NH |
| 29 | SCH₃ | Cl | N | NH |
| 30 | S(O)₂CH₃ | Cl | N | NH |
| 31 | H | CH₃ | N | NH |
| 32 | CH₃ | CH₃ | N | NH |
| 33 | Cl | CH₃ | N | NH |
| 34 | OCH₃ | CH₃ | N | NH |
| 35 | SCH₃ | CH₃ | N | NH |
| 36 | S(O)₂CH₃ | CH₃ | N | NH |
| 37 | H | H | N | NCH₃ |
| 38 | CH₃ | H | N | NCH₃ |
| 39 | Cl | H | N | NCH₃ |
| 40 | OCH₃ | H | N | NCH₃ |
| 41 | SCH₃ | H | N | NCH₃ |
| 42 | S(O)₂CH₃ | H | N | NCH₃ |
| 43 | H | Cl | N | NCH₃ |
| 44 | CH₃ | Cl | N | NCH₃ |
| 45 | Cl | Cl | N | NCH₃ |
| 46 | OCH₃ | Cl | N | NCH₃ |
| 47 | SCH₃ | Cl | N | NCH₃ |
| 48 | S(O)₂CH₃ | Cl | N | NCH₃ |
| 49 | H | CH₃ | N | NCH₃ |
| 50 | CH₃ | CH₃ | N | NCH₃ |
| 51 | Cl | CH₃ | N | NCH₃ |
| 52 | OCH₃ | CH₃ | N | NCH₃ |
| 53 | SCH₃ | CH₃ | N | NCH₃ |
| 54 | S(O)₂CH₃ | CH₃ | N | NCH₃ |
| 55 | H | H | N | NC₂H₅ |
| 56 | CH₃ | H | N | NC₂H₅ |
| 57 | Cl | H | N | NC₂H₅ |
| 58 | OCH₃ | H | N | NC₂H₅ |
| 59 | SCH₃ | H | N | NC₂H₅ |
| 60 | S(O)₂CH₃ | H | N | NC₂H₅ |
| 61 | H | Cl | N | NC₂H₅ |
| 62 | CH₃ | Cl | N | NC₂H₅ |
| 63 | Cl | Cl | N | NC₂H₅ |
| 64 | OCH₃ | Cl | N | NC₂H₅ |
| 65 | SCH₃ | Cl | N | NC₂H₅ |
| 66 | S(O)₂CH₃ | Cl | N | NC₂H₅ |
| 67 | H | CH₃ | N | NC₂H₅ |
| 68 | CH₃ | CH₃ | N | NC₂H₅ |
| 69 | Cl | CH₃ | N | NC₂H₅ |
| 70 | OCH₃ | CH₃ | N | NC₂H₅ |
| 71 | SCH₃ | CH₃ | N | NC₂H₅ |
| 72 | S(O)₂CH₃ | CH₃ | N | NC₂H₅ |
| 73 | H | H | N | N-i-C₃H₇ |
| 74 | CH₃ | H | N | N-i-C₃H₇ |
| 75 | Cl | H | N | N-i-C₃H₇ |
| 76 | OCH₃ | H | N | N-i-C₃H₇ |
| 77 | SCH₃ | H | N | N-i-C₃H₇ |
| 78 | S(O)₂CH₃ | H | N | N-i-C₃H₇ |
| 79 | H | Cl | N | N-i-C₃H₇ |
| 80 | CH₃ | Cl | N | N-i-C₃H₇ |
| 81 | Cl | Cl | N | N-i-C₃H₇ |
| 82 | OCH₃ | Cl | N | N-i-C₃H₇ |
| 83 | SCH₃ | Cl | N | N-i-C₃H₇ |
| 84 | S(O)₂CH₃ | Cl | N | N-i-C₃H₇ |
| 85 | H | CH₃ | N | N-i-C₃H₇ |
| 86 | CH₃ | CH₃ | N | N-i-C₃H₇ |
| 87 | Cl | CH₃ | N | N-i-C₃H₇ |
| 88 | OCH₃ | CH₃ | N | N-i-C₃H₇ |
| 89 | SCH₃ | CH₃ | N | N-i-C₃H₇ |
| 90 | S(O)₂CH₃ | CH₃ | N | N-i-C₃H₇ |

Weitere Beispiele für erfindungsgemäße bevorzugte Benzthiadiazol-5-ylcarbonyl-Derivate von Pyrazolen (X = N, Y = S ) und Benzotriazol-5-ylcarbonyl-Derivate von Pyrazolen (X = N, *Y =* N-R⁴) sind die in den Tabellen 59 bis 77 genannten Verbindungen (Verbindungen 1-4).
- Tabelle 59: Verbindungen I-4a.1 bis I-4a.90 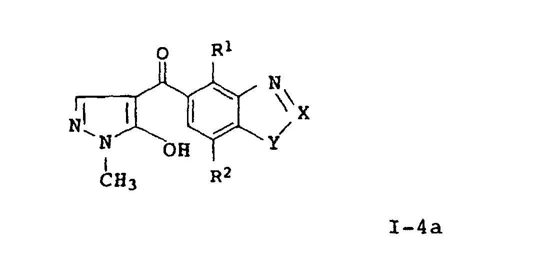 Verbindungen der allgemeinen Formel I-4a, in der die Substituenten R¹, R², X und Y für jede einzelne Verbindung jeweils einer Zeile der Tabelle C entsprechen.
- Tabelle 60: Verbindungen I-4b.1 bis I-4b.90 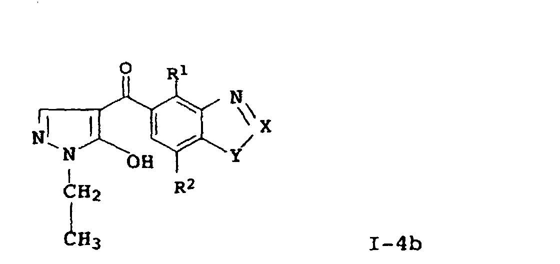 Verbindungen der allgemeinen Formel I-4b, in der die Substituenten R¹, R², X und Y für jede einzelne Verbindung jeweils einer Zeile der Tabelle C entsprechen.
- Tabelle 61: Verbindungen I-4c.1 bis I-4c.90 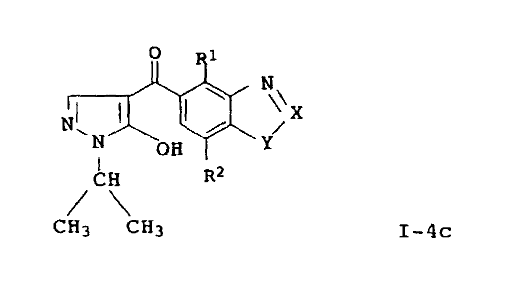 Verbindungen der allgemeinen Formel I-4c, in der die Substituenten R¹, R², X und Y für jede einzelne Verbindung jeweils einer Zeile der Tabelle C entsprechen.
- Tabelle 62: Verbindungen I-3d.1 bis I-3d.1363 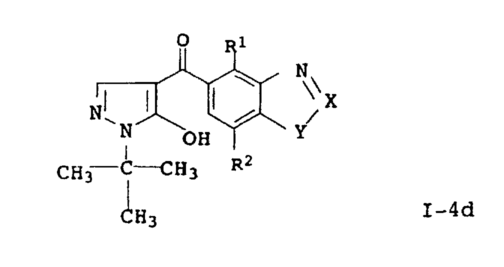 Verbindungen der allgemeinen Formel I-4d, in der die Substituenten R¹, R² X und Y für jede einzelne Verbindung jeweils einer Zeile der Tabelle C entsprechen.
- Tabelle 63: Verbindungen I-4e.1 bis I-4e.90 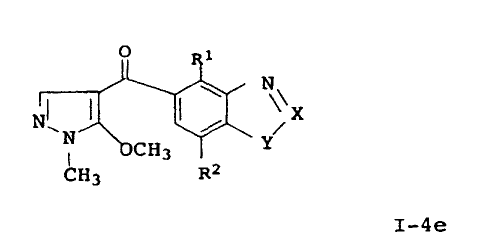 Verbindungen der allgemeinen Formel I-4e, in der die Substituenten R¹, R² X und Y für jede einzelne Verbindung jeweils einer Zeile der Tabelle C entsprechen.
- Tabelle 64: Verbindungen I-4f.1 bis I-4f.90 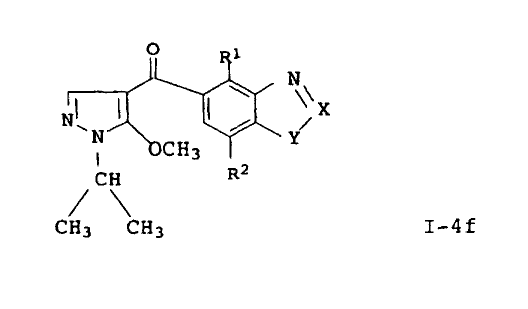 Verbindungen der allgemeinen Formel 1-4f, in der die Substituenten R¹, R² X und Y für jede einzelne Verbindung jeweils einer Zeile der Tabelle C entsprechen.
- Tabelle 65: Verbindungen I-4g.1 bis I-4g.90 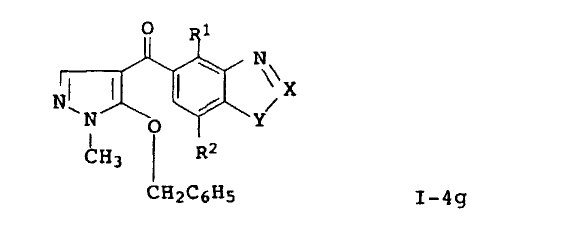 Verbindungen der allgemeinen Formel I-4g, in der die Substituenten R¹, R², X und Y für jede einzelne Verbindung jeweils einer Zeile der Tabelle C entsprechen.
- Tabelle 66: Verbindungen I-4h.1 bis I-4h.90 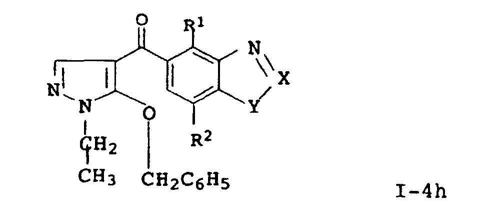 Verbindungen der allgemeinen Formel I-4h, in der die Substituenten R¹, R², X und Y für jede einzelne Verbindung jeweils einer Zeile der Tabelle C entsprechen.
- Tabelle 67: Verbindungen I-4i.1 bis I-4i.90 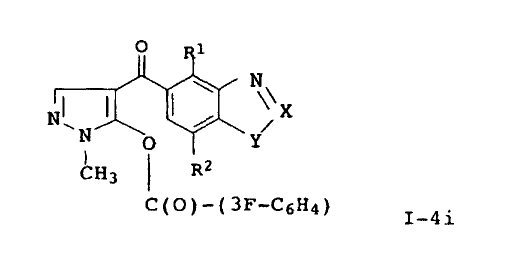 Verbindungen der allgemeinen Formel I-4i, in der die Substituenten R¹, R², X und Y für jede einzelne Verbindung jeweils einer Zeile der Tabelle C entsprechen.
- Tabelle 68: Verbindungen I-4k.1 bis I-4k.90 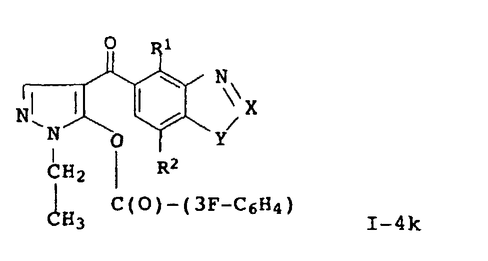 Verbindungen der allgemeinen Formel 1-4k, in der die Substituenten R¹, R², X und Y für jede einzelne Verbindung jeweils einer Zeile der Tabelle C entsprechen.
- Tabelle 69: Verbindungen 1-41.1 bis 1-41.90 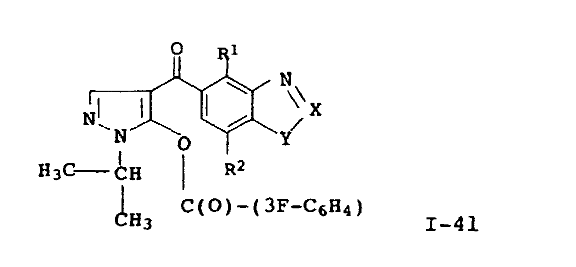 Verbindungen der allgemeinen Formel I-4l, in der die Substituenten R¹, R², X und Y für jede einzelne Verbindung jeweils einer Zeile der Tabelle C entsprechen.
- Tabelle 70: Verbindungen I-4m.1 bis I-4m.90 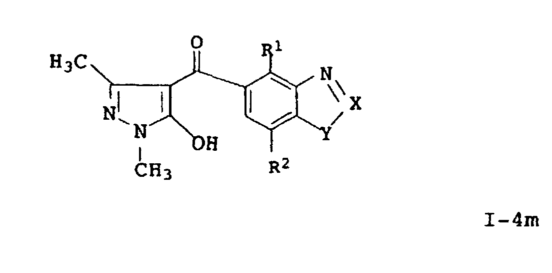 Verbindungen der allgemeinen Formel I-4m, in der die Substituenten R¹, R², X und Y für jede einzelne Verbindung jeweils einer Zeile der Tabelle C entsprechen.
- Tabelle 71: Verbindungen I-4n.1 bis I-4n.90 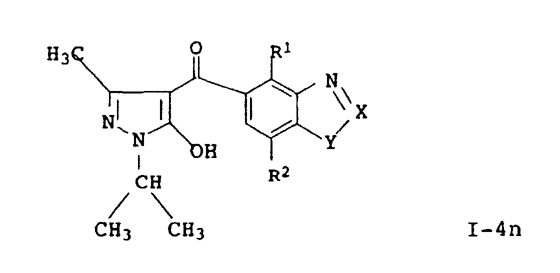 Verbindungen der allgemeinen Formel I-4n, in der die Substituenten R¹, R², X und Y für jede einzelne Verbindung jeweils einer Zeile der Tabelle C entsprechen.
- Tabelle 72: Verbindungen I-4o.1 bis I-4o.90 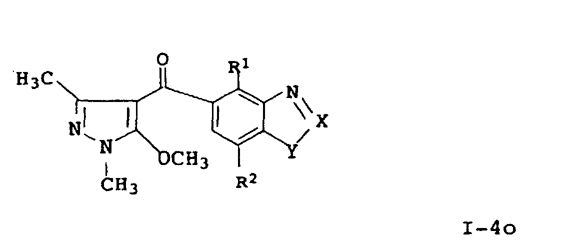 Verbindungen der allgemeinen Formel I-4o, in der die Substituenten R¹, R², X und Y für jede einzelne Verbindung jeweils einer Zeile der Tabelle C entsprechen.
- Tabelle 73: Verbindungen I-4p.1 bis I-4p.90 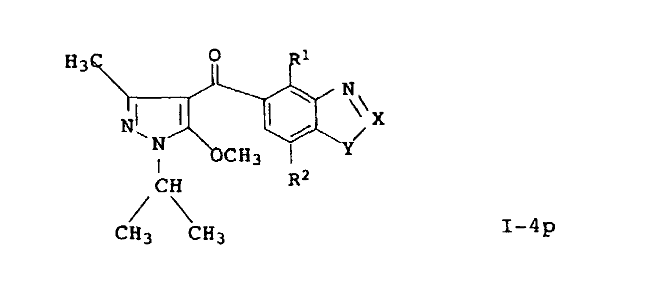 Verbindungen der allgemeinen Formel I-4p, in der die Substituenten R¹, R², X und Y für jede einzelne Verbindung jeweils einer Zeile der Tabelle C entsprechen.
- Tabelle 74: Verbindungen I-4q.1 bis I-4q.90 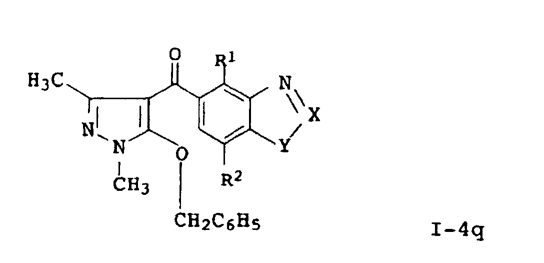 Verbindungen der allgemeinen Formel I-4q, in der die Substituenten R¹, R², X und Y für jede einzelne Verbindung jeweils einer Zeile der Tabelle C entsprechen.
- Tabelle 75: Verbindungen I-4r.1 bis I-4r.90 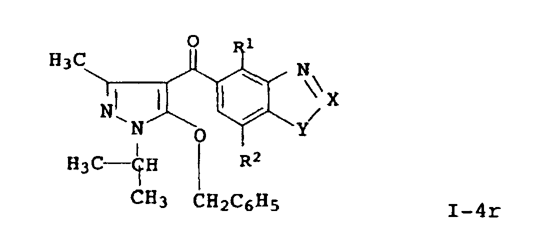 Verbindungen der allgemeinen Formel I-4r, in der die Substituenten R¹, R², X und Y für jede einzelne Verbindung jeweils einer Zeile der Tabelle B entsprechen.
- Tabelle 76: Verbindungen I-4s.1 bis I-4s.90 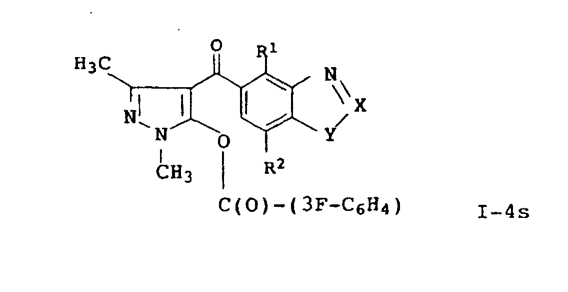 Verbindungen der allgemeinen Formel I-4s, in der die Substituenten R¹, R², X und Y für jede einzelne Verbindung jeweils einer Zeile der Tabelle C entsprechen.
- Tabelle 77: Verbindungen I-4t.1 bis I-4t.90 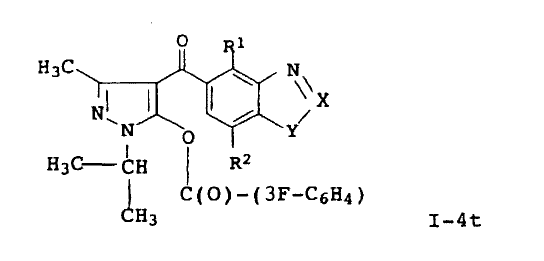 Verbindungen der allgemeinen Formel I-4t, in der die Substituenten R¹, R², X und Y für jede einzelne Verbindung jeweils einer Zeile der Tabelle C entsprechen.

Die Darstellung von Verbindungen der Formel I, worin R⁸ für Hydroxy steht, erfolgt durch Umsetzung einer aktivierten Carbonsäure IVb oder einer Carbonsäure IVa, die vorzugsweise in situ aktiviert wird, mit 5-Hydroxypyrazol der Formel III zu dem Acylierungsprodukt und anschließende Umlagerung.

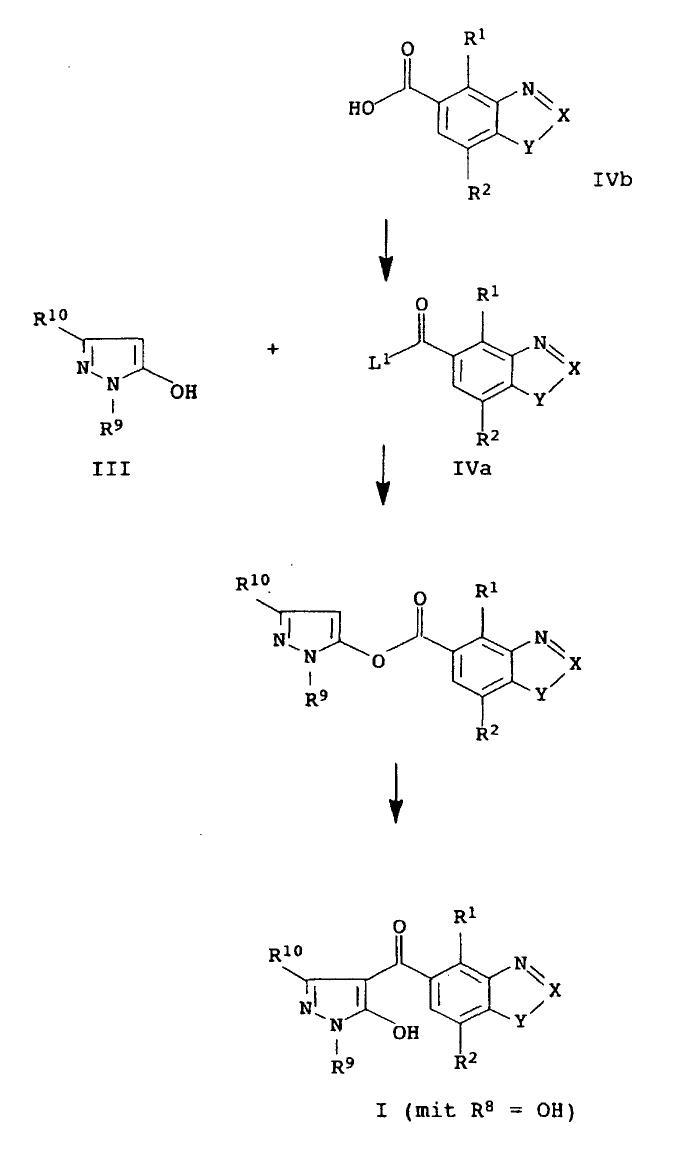

L¹ steht für eine nucleophil verdrängbare Abgangsgruppe, wie Halogen z.B. Brom oder Chlor, Hetaryl, z.B. Imidazolyl oder Pyridyl, Carboxylat, z.B. Acetat oder Trifluoracetat etc.

Die aktivierte Carbonsäure IVa kann direkt eingesetzt werden, wie im Fall der Benzoylhalogenide oder in situ erzeugt werden, z.B. mit einem Carbodiimid wie Ethyl-(3'-dimethylaminopropyl)carbodiimid, Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf IVa bzw. IVb, kann unter Umständen vorteilhaft sein.

Als Hilfsbasen eignen sich tertiäre Alkylamine, Pyridin 4-Dimethylaminopyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyltert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden Halogenide als aktivierte Carbonsäurekomponente eingesetzt, so kann es zweckmäßig sein, bei Zugabe dieses Reaktionspartners die Reaktionsmischung auf 0 bis 10°C abzukühlen. Anschließend rührt man bei 20 bis 100°C, vorzugsweise bei 25 bis 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolaren Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise etwa 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

B. Die Darstellung von Verbindungen der Formel I mit R⁸ = Halogen erfolgt durch Umsetzung von Pyrazol-Derivaten der Formel I (mit R⁸=Hydroxy) mit Halogenierungsmitteln: 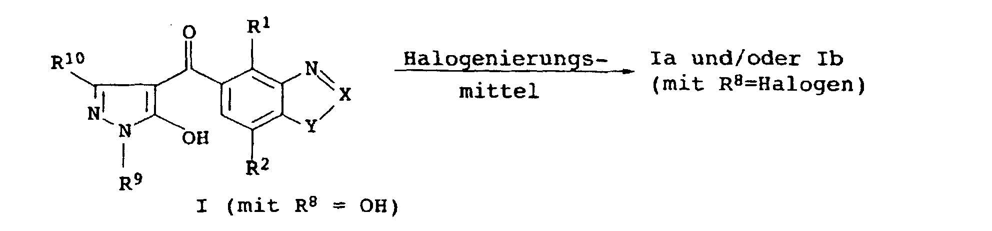 Hier und im folgenden steht "Verbindung 1a" für eine Verbindung der allgemeinen Formel I, worin Pz für einen Pyrazolylrest der allgemeinen Formel IIa steht und Verbindung 1b entsprechend für eine Verbindung der allgemeinen Formel I, worin Pz für einen Rest IIb steht.

Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxalylbromid, Phosphoroxybromid etc.

C. Die Darstellung von Verbindungen der Formel I mit R⁸ = OR¹¹, OSO₂R¹², OPOR¹³R¹⁴ oder OPSR¹³R¹⁴ durch Umsetzung von Pyrazol-Derivaten der Formel I (mit R⁸=Hydroxy) mit Alkylierungs-, Sulfonylierungs- bzw. Phosphonylierungsmitteln Vα, Vβ, Vγ beziehungsweise Vδ. 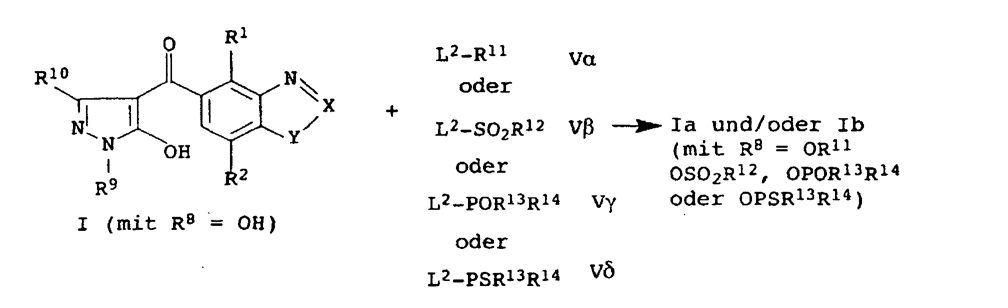 L² steht für eine nucleophil verdrängbare Abgangsgruppe, wie Halogen, z.B. Chlor oder Brom, Hetaryl, z.B. Imidazolyl, Carboxylat, z.B. Acetat, oder Sulfonat, z.B. Mesylat oder Triflat etc.
Bei der Herstellung von Verbindungen der Formel I mit R⁸ = OR¹¹ aus Verbindungen der Formel I mit R⁸ = OH arbeitet man vorzugsweise in Gegenwart einer Base.
Die Reaktanden und die Base werden dabei zweckmäßigerweise in äquimolaren Mengen eingesetzt. Ein geringer Überschuß der Base, z.B. 1,1 - 1,5 Moläquivalente, bezogen auf I kann unter Umständen vorteilhaft sein.
Als Basen eignen sich tertiäre Amine, Pyridine, Alkalimetallcarbonate oder Alkalimetallhydride. Als Lösungsmittel eignen sich z.B. chlorierte Kohlenwasserstoffe wie Methylenchlorid oder 1,2-Dichlorethan, aromatische Kohlenwasserstoffe wie Toluol, Xylol oder Chlorbenzol, Ether wie Diethylether, Methyl-tert-butylether, Tetrahydrofuran, Dioxan oder Dimethoxyethan, polare aprotische Lösungsmittel wie Acetonitril, Dimethylformamid oder Dimethylsulfoxid oder Ester wie Essigsäureethylester und Gemische hiervon.
Werden anstelle des Alkohols I (R⁸ = OH) Halogenide (R⁸ = Halogen) oder aktivierte Alkohole wie Mesylate oder Tosylate (R⁸ = OSO₂CH₃ oder OSO₂-Tolyl) zur Derivatisierung eingesetzt, so kann es zweckmäßig sein, bei Zugabe des Reaktionspartners die Reaktionsmischung auf 0 bis 10°C abzukühlen. Anschließend rührt man bei 20 bis 100°C, vorzugsweise bei 20 bis 75°C bis die Umsetzung vollständig ist.
Die Aufarbeitung erfolgt in üblicher Weise, z.B. wird das Reaktionsgemisch auf Wasser gegossen, das Wertprodukt extrahiert. Als Lösungsmittel eignen sich hierfür besonders Lösungsmittel wie Methylenchlorid, Essigsäureethylester, Methyl-tert-butylether oder Diethylether. Nach Trocknen der organischen Phase und Entfernen des Lösungsmitel kann das Rohprohdukt gegebenenfalls noch durch Säulenchromatographie an Kieselgel gereinigt werden. Als Eluenten eignen sich Lösungsmittel wie Methylenchlorid, Essigsäureethylester, Cyclohexan, Petrolether, Methanol, Aceton oder Chloroform und Gemische hiervon.
Die Verbindungen der Formel Va, Vβ, Vγ oder Vδ können direkt eingesetzt werden wie z.B. im Fall der Carbonsäurehalogenide oder in situ erzeugt werden, z.B. aktivierte Carbonsäuren (mit Carbonsäure und Dicyclohexylcarbodiimid etc.).

D. Die Darstellung von Verbindungen der Formel I mit R⁸ = OR¹¹, SR¹¹, POR¹³R¹⁴, NR¹⁵R¹⁶, ONR¹⁵R¹⁶ oder N-gebundenes Heterocyclyl erfolgt durch Umsetzung von Verbindungen der Formel I mit R⁸ = Halogen, OSO₂R¹² mit Verbindungen der Formel VIa, VIß, VIγ, VIδ, VIε oder VIη, gegebenenfalls in Gegenwart einer Base oder unter vorangehender Salzbildung.

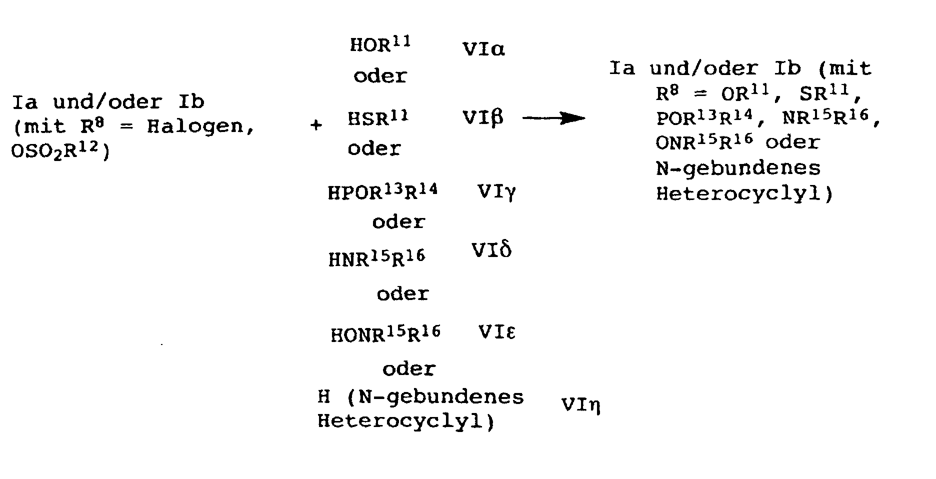

E. Die Darstellung von Verbindungen der Formel I mit R⁸ = SOR¹², SO₂R¹² erfolgt beispielsweise durch Umsetzung von Verbindungen der Formel I mit R⁸ = SR¹² mit einem Oxidationsmittel. 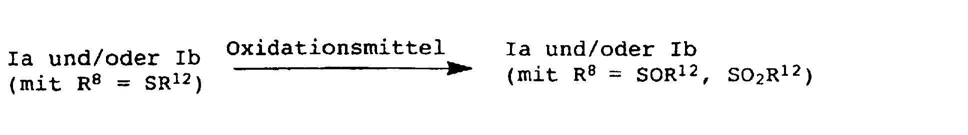 Als Oxidationsmittel kommen beispielsweise m-Chlorperbenzoesäure, Peroxyessigsäure, Trifluorperoxyessigsäure, Wasserstoffperoxid, ggf. in Gegenwart eines Katalysators wie Wolframat, in Betracht.

Für die unter den Punkten B bis E genannten Reaktionen gelten folgende Bedingungen:

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzungen in Gegenwart einer Base durchzuführen. Die Reaktanden und die Base werden dabei zweckmäßigerweise in äquimolaren Mengen eingesetzt.

Im Hinblick auf die Verfahren C und D kann es unter Umständen vorteilhaft sein, ein Überschuß der Base z.B. 1,5 bis 3 Moläquivalente jeweils bezogen auf das Edukt einzusetzen.

Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimetallhydrogencarbonate, wie Natriumhydrogencarbonat und Kaliumhydrogencarbonat, Alkalimetallalkoholate wie Natriummethanolat, Natriumethanolat, Kalium-tert.butanolat oder Alkalimetallhydride, z.B. Natriumhydrid. Bevorzugt verwendet werden Triethylamin oder Pyridin.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

In Abhängigkeit von den Reaktionsbedingungen können bei den Verfahren B bis D die Verbindungen Ia, Ib oder Gemische hiervon gebildet werden. Letztere können durch klassische Trennmethoden, wie z.B. Kristallisation, Chromatographie etc., getrennt werden.

F. Die Darstellung von Verbindungen der Formel I, worin Pz für eine Gruppe der allgemeinen Formel IIa steht, kann auch durch Umsetzung eines metallierten Pyrazol-Derivats der Formel VII mit einem Carbonsäure-Derivat der Formel IVa erfolgen:

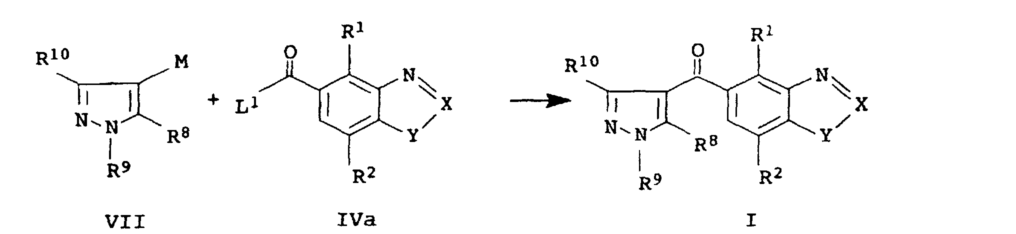

M steht hierbei für ein Metall, insbesondere für ein Alkalimetall wie Lithium oder Natrium, ein Erdalkalimetall wie z.B. Magnesium oder ein Übergangsmetall wie Palladium, Nickel etc. und L¹ für eine nukleophil verdrängbare Abgangsgruppe wie Halogen, z.B. Chlor oder Brom, Alkylsulfonat wie Mesylat, Halogenalkylsulfonat wie Triflat oder Cyanid. R⁸ weist vorzugsweise keine aziden Wasserstoffatome auf.

Die Umsetzung wird in der Regel bei Temperaturen von -100°C bis Rückflußtemperatur des Reaktionsgemisches durchgeführt. Als Lösungsmittel eignen sich inerte aprotische Lösungsmittel, wie Ether, z.B. Diethylether, Tetrahydrofuran. Die Verbindungen der Formel IVa werden in der Regel im Überschuß eingesetzt, es kann aber auch von Vorteil sein, diese in äquimolaren Mengen oder im Unterschuß einzusetzen. Die Aufarbeitung erfolgt zum Produkt hin.

Die metallierten Pyrazol-Derivate der Formel VII können auf an sich bekannte Art und Weise durch Umsetzung von in 4-Position halogenierten Pyrazolen mit Metallen wie Lithium, Natrium, Magnesium etc. oder mit metallorganischen Verbindungen wie z.B. Butyllithium gebildet werden. Es ist aber auch möglich Pyrazole, die in 4-Position mit Wasserstoff verknüpft sind, direkt zu metallieren, z.B. mit den voranstehend genannten Metallen bzw. metallorganischen Verbindungen. Die Umsetzungen werden in der Regel in einem inerten aprotischen Lösungsmittel durchgeführt, bevorzugt in Ether wie Diethylether, Tetrahydrofuran etc.. Die Reaktionstemperatur liegt im Bereich von -100°C bis zur Höhe des Siedepunktes des Reaktionsgemisches. Die Verbindungen der Formel VII werden vorzugsweise in situ erzeugt und direkt umgesetzt.

Die als Ausgangsmaterialien verwendeten 5-Hydroxypyrazole der Formel III sind bekannt oder können an sich nach bekannten Verfahren hergestellt werden, wie sie beispielsweise in der EP-A 240 001, in J. Chem. Soc. 315, S.383 (1997), J. Prakt. Chem. 315, S. 382 (1973) beschrieben sind (siehe auch Übersichten in Advances Heterocycle. Chem. 48, S. 223-299 (1990) und Katritzky, Rees (Hrsg.), Comprehensive Heterocyclic Chem. Vol. 5, Pergamon Press 1984, Oxford, S. 167-343 und dort zitierte Literatur). 1,3-Dimethyl-5-hydroxypyrazol ist überdies eine käufliche Verbindung.

Die Alkylierungsmittel Va, Sulfonylierungsmittel Vβ, Phosphonylierungsmittel Vγ beziehungsweise vδ, sowie die Verbindungen VIa, VIß, VIγ, VIδ und VIε sind ebenfalls bekannt oder können nach bekannten Verfahren hergestellt werden.

Die Carbonsäuren der allgemeinen Formel IVa beziehungsweise ihre aktivierten Derivate IVb sind entweder aus der Literatur bekannt oder lassen sich in Analogie zu bekannten Verfahren herstellen.

In Schema 1 ist ein üblicher Zugang zu Benzothiazol-5-carbonsäuren (Verbindungen IV-1) dargestellt.

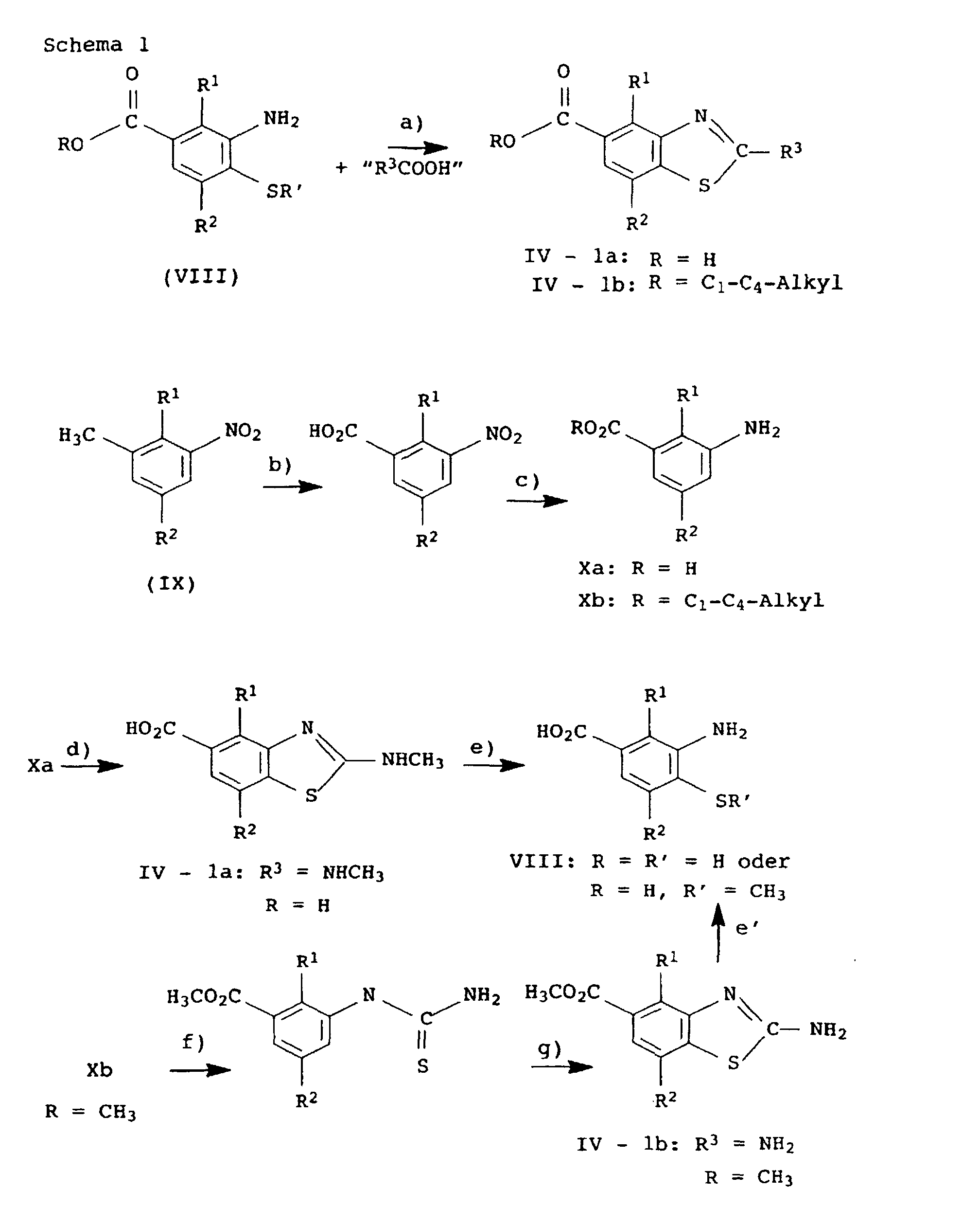

In der Formel IV-1 steht R für Wasserstoff (Verbindung IV-1a) oder einen verseifbaren Kohlenwasserstoffrest, z.B. für Methyl (Verbindung IV-1b). Verbindungen der allgemeinen Formel IV-1 lassen sich beispielsweise gemäß Reaktionsschritt a) durch Kondensation von ortho-Aminothiophenolen der allgemeinen Formel VIII (R' = H) oder von ortho-Aminothioethern der allgemeinen Formel VIII (R' = C₁-C₄-Alkyl, z.B. Methyl) mit einem Carbonsäureäquivalent "R3-CO₂H" also einer Carbonsäure R³CO₂H oder aktivierten Derivaten R³COL¹, R³C(L³)₃ davon, worin L¹ für eine reaktive Abgangsgruppe steht und L³ für eine C₁-C₄-Alkoxygruppe steht, herstellen. Beispiele für L¹ sind Chlor, Brom, Carboxylat, wie Acetat, Trifluoracetat, N-Heterocyclyl, wie Imidazolyl, Pyridyl etc. Beispiele für R³COL¹ und R³C(L³)₃ sind die Säurehalogenide, Carbonsäureester und Carbonsäureanhydride sowie die Orthoester der Carbonsäuren R³CO₂H.

Die Kondensationsreaktion a) erfolgt vorzugsweise unter neutralen bis sauren Reaktionsbedingungen, vorzugsweise in Gegenwart einer anorganischen oder organischen Säure, wie beispielsweise Salzsäure, Schwefelsäure, p-Toluolsulfonsäure und Pyridinium-p-toluolsulfonat in einem organischen Lösungsmittel bei Temperaturen im Bereich von 0 bis 150°C und vorzugsweise im Bereich von 20 bis 120°C. Als Lösungsmittel kommen insbesondere gesättigte Kohlenwasserstoffe, halogenierte Kohlenwasserstoffe, aromatische Kohlenwasserstoffe wie Benzol, aliphatische Ether wie Diethylether und tert-Butyl-methylether oder Pyridin in Betracht. Zur Herstellung von Bezothiazolen aus o-Aminothiophenolen beziehungsweise entsprechenden Thiomethylethern siehe auch Houben-Weyl, Methoden der Organischen Chemie, Bd. E 8b, S.869-871.

Schritt a) kann auch zweistufig durchgeführt werden, wobei zunächst die Aminofunktion in VIII mit einer Carbonsäure R⁹-COOH oder einem Derivat davon zum Carbonsäureamid umgesetzt wird, das anschließend zum Benzothiazol der allgemeinen Formel IV-1 cyclisiert wird.

Die Umsetzung zum Amid gelingt unter den dür die Amidbildung üblichen Bedingungen, beispielsweise durch Umsetzung einer Säure in Gegenwart eines wasserbindenden Mittels. Die Cyclisierung gelingt mit Lewissäuren oder Phosgen. Die Cyclisierung wird dann vorzugsweise in einem inerten organischen Lösungsmittel, beispielsweise einem aliphatischen oder aromatischen Kohlenwasserstoff oder in einem Halogenkohlenwasserstoff durchgeführt.

Ortho-Aminothiophenole der allgemeinen Formel VIII (R' = H) können gemäß Schema 1 ausgehend von 3-Nitrotoluolen der allgemeinen Formel IX hergestellt werden. Hierin kann die Methylgruppe in bekannter Weise katalytisch oder stöchiometrisch zur Carbonsäure oxidiert werden (Schritt b). Als Oxidationsmittel können beispielsweise Metalloxide von Übergangsmetallen, beispielsweise Mangandioxid, Chromtrioxid sowie deren anionische Komplexsalze, z.B. Natriumdichromat oder Chromylchlorid, Pyridiniumchromat, weiterhin oxidierende Säuren, beispielsweise HNO₃, oxidierende Gase wie Sauerstoff oder Chlor, gegebenenfalls in Anwesenheit von Übergangsmetallen (beziehungsweise der Salze, z.B. der Oxide oder Chloride) als Katalysatoren eingesetzt werden. Je nach Löslichkeit der zu oxidierenden Verbindung und abhängig von dem Verwendeten Oxidationsmittel arbeitet man vorzugsweise in wässrigen Lösungen, einphasigen Systemen aus Wasser und mit Wasser mischbaren organischen Lösungsmitteln oder in mehrphasigen Systemen aus Wasser und organischen Lösungsmitteln unter Phasentransferkatalyse. Abhängig vom gewählten Oxidationsmittel wird man die Oxidation in der Regel bei Temperaturen im Bereich von -15 bis -H50°C und vorzugsweise im Bereich von 0 bis 100°C durchführen. Zur Oxidation von aromatischen Methylgruppen zu Benzoesäuren siehe beispielsweise (Houben-Weyl: "Methoden der organischen Chemie", V. Band, IV/1a 1981; Bd. VIII 1952; E. Bengtsson, Acta Chem. Scand. 1953, 7, 774; Singer et a1. Org. Synth. Coll. Vol III, 1955, 740; B.A.S. Hay et a1. Can. J. Chem. 1965, 43, 1306)

Die so erhaltenen 3-Nitro-benzoesäurederivate werden anschließend in Schritt c) zu den 3-Aminobenzoesäuren reduziert. Die selektive Reduktion von aromatischen Nitrogruppen in Anwesenheit von Carbonsäuregruppen ist grundsätzlich bekannt. Als Reduktionsmittel kommen beispielsweise Hydrazine, Metallhydride wie Aluminiumhydrid, und davon abgeleitete Komplexverbindungen wie Lithiumaluminiumhydrid, Diisobutylaluminiumhydrid, oder Borane in Betracht. Bevorzugtes Reduktionsmittel ist Wasserstoff in Gegenwart von katalytischen Mengen an Übergangsmetallen beispielsweise Ni, Pd, Pt, Ru oder Rh, die in geträgerter Form, beispielsweise auf Aktivkohle, in Form aktivierter Metalle, z.B. Raney-Nickel, oder in Form löslicher Komplexverbindungen eingesetzt werden können. Geeignete Lösungsmittel für die Reduktion sind abhängig von der Löslichkeit des zu hydrierenden Substrates und dem gewählten Reduktionsmittel C₁-C₄-Alkohole, wie Methanol, Ethanol, n-Propanol, Isopropanol oder n-Butanol, halogenierte C₁-C₆-Kohlenwasserstoffe, wie Dichlormethan, Trichlormethan, Trichlorethan, Trichlorethylen, aromatische Kohlenwasserstoffe, wie Benzol, Toluol, Xylole, Chlorbenzol, wässrige Lösungen anorganischer oder organischer Säuren, wie wässrige Salzsäure. Üblicherweise erfolgt die Reduktion bei Temperaturen im Bereich von -15 bis +100°C, vorzugsweise im Bereich von 0 bis 40°C. Die Reduktion mit Wasserstoff erfolgt üblicherweise bei einem Wasserstoffdruck im Bereich von 1 bis 50 bar, vorzugsweise im Bereich von 1 bis 10 bar. Zur katalytischen Hydrierung aromatischer Nitrogruppen siehe beispielsweise Rylander in "Catalytic Hydrogenation over Platinum Metals", Academic Press, New York, 1967, 168-202; Furst et al., Chem. Rev. 1965, 65, 52; Tepko et al., J. Org. Chem. 1980, 45, 4992.

Die so erhaltenen o-Aminobenzoesäuren der allgemeinen Formel Xa (R = H) werden dann in einem weiteren Reaktionsschritt d) mit einem organischen Isothiocyanat (in Schema 1 Methylisothiocyanat) zu einem substituierten Thioharnstoffderivat umgesetzt, das ohne weitere Isolierung oxidativ zur Benzothiazol-5-carbonsäure der allgemeinen Formel IX-1a (in Schema 1 mit R³ = NH-CH₃) cyclisiert wird.

Der erste Reaktionsschritt in Stufe d), nämlich die Umsetzung der m-Aminobenzoesäure der allgemeinen Formel Xa zum substituierten Harnstoff erfolgt durch Umsetzung mit einem C₁-C₆-Alkylisothiocyanat oder einem gegebenenfalls substituierten Phenylisothiocyanat in einem wasserfreien, organischen Lösungsmittel bei Temperaturen im Bereich von -15°C bis 150°C und vorzugsweise im Bereich von - 15°C bis 100°C. Geeignete Lösungsmittel sind beispielsweise aliphatische oder cycloaliphatische Kohlenwasserstoffe, wie n-Hexan oder Cyclohexan, halogenierte Kohlenwasserstoffe, wie Dichlormethan, Trichlormethan, Trichlorethan, Trichlorethylen, aromatische Kohlenwasserstoffe wie Benzol oder Anisol, Dialkylether oder cyclische Ether wie Diethylether, Methyl-tert.-butylether, Tetrahydrofuran oder Dioxan, wasserfreie Carbonsäuren wie Eisessig oder in Pyridin. Zur Herstellung substituierter Thioharnstoffe siehe beispielsweise: Kurzer, F., Org. Synth. 1951, 31, 21; R.R. Gupta et al., Synth. Commun. 17(2) ,229-240 (1987); Rathke, Ber. Dtsch. Chem. Ges. 1885, 18, 3102; Schiff, Justus Liebigs Ann. Chem., 1868, 148, 338; Frank, R.L., Smith, P.V.; Org. Synth. 1955, III, 735, N.B. Ambati et al., Synth. Commun. 1997, 27 (9), 1487-1493; W.O. Foye, J. Pharm.. Sci., 1977, 66, No. 7, 923-926.

Das so erhaltene, substituierte Thioharnstoff-Derivat wird dann in einem zweitem Reaktionsschritt von Schritt d) mit einem halogenhaltigen Oxidationsmittel wie Brom, Sulfurylchlorid oder Chlor in einem inerten organischen Lösungsmittel zur substituierten 2-Aminobenzothiazol-5-carbonsäure der allgemeinen Formel IV-1a (in Schema 1 steht R³ für NH-CH₃) cyclisiert. Die Cyclisierung erfolgt in der Regel bei Temperaturen im Bereich von -15 bis +150°C und vorzugsweise im Bereich von 0 bis 120°C. Geeignete Lösungsmittel sind insbesondere die vorgenannten aliphatischen oder cycloaliphatischen Kohlenwasserstoffe, die vorgenannten aromatischen Kohlenwasserstoffe, die vorgenannten wasserfreien Carbonsäuren, ferner C₁-C₄-Alkanole, z.B. Methanol, Ethanol oder Isopropanol, Dialkylether, cyclische Ether und Mischungen der vorgenannten Lösungsmittel. Zur oxidativen Cyclisierung substituierter Thioharnstoffe zu Benzothiazolen siehe beispielsweise Houben-Weyl: "Methoden der organischen Chemie V, Bd.E8B, 1994, S.865 f.

Die substituierte 2-Aminobenzothiazol-5-carbonsäure der allgemeinen Formel IV-1a kann entweder direkt mit einem Hydroxypyrazol der allgemeinen Formel III oder einem aktivierten Derivat davon in der oben beschriebenen Weise zur erfindungsgemäßen Verbindung I (mit Y = S und X = C-NH-R", wobei R'' für C₁-C₆-Alkyl oder gegebenenfalls substituiertes Phenyl steht) umgesetzt werden.

Sofern R³ in Formel IV-1a für NH-CH₃ steht, können durch Hydrolyse gemäß Schritt e) auch die o-Aminothiobenzoesäuren der allgemeinen Formel VIII (mit R = R' = H) hergestellt werden. Der Hydrolyse folgt in der Regel noch die Methylierung zum Methylthioether VIII (R = H, R' = CH₃). Die Hydrolyse in Schritt e) erfolgt beispielsweise durch Umsetzung der Verbindung IV-1a (mit R³ = NH-CH₃) mit einem Alkalihydroxid, z.B. Lithium-, Natrium- oder Kaliumhydroxid, einem Erdalkalihydroxid oder Alkaliiodiden, wie Natriumiodid in einem geeigneten Lösungsmittel bei erhöhter Temperatur, wobei vorzugsweise in Abwesenheit von Sauerstoff gearbeitet wird. Übliche Reaktionstemperaturen liegen im Bereich von 0 bis 200°C, insbesondere im Bereich von 20 bis 180°C. Geeignete Lösungsmittel sind neben den vorgenannten aliphatischen oder cycloaliphatischen Kohlenwasserstoffen, den halogenierten Kohlenwasserstoffen, den aromatischen Kohlenwasserstoffen, den vorgenannten Ethern und Alkoholen insbesondere wässrige, einphasige Systeme und Pyridin. Zur Verseifung der substituierten 2-Aminobenzothiazol-5-carbonsäuren siehe beispielsweise: Organikum, 16. Aufl. 1986, S. 415; Mc Murry, Org. React. 1976, 24, 187; Taschner et al., Rocz. Chem. 1956, 30, 323; z.B. Houben-Weyl: "Methoden der organischen Chemie", Band E8b, 1994; 5.1010 f.; J. Chem. Soc. Perkin Trans., Part 1, 1976, No. 12, 1291-1296, insbesondere A.R. Katritzky et al., J. Heterocycl. Chem. 30 (1) 135-139, 1993. Die Umsetzung zum Methylthioether VIII mit R = H und R' = CH₃ gelingt in einfacher Weise durch Umsetzung mit Methyliodid oder Dimethylsulfat.

In ähnlicher Weise können Verbindungen der allgemeinen Formel VIII mit R = H erhalten werden, wenn man zunächst die 3-Aminobenzoesäure der allgemeinen Formel Xa mit einem C₁-C₄-Alkanol, z.B. mit Methanol in bekannter Weise verestert. Der so erhaltene Ester der allgemeinen Formel Xb (R = C₁-C₄-Alkyl, insbesondere Methyl) wird dann in Schritt f) mit Isothiocyansäure oder einem geeigneten Salz der Isothiocyansäure, z.B. Natriumrhodanid in Gegenwart einer konzentrierten Mineralsäure, zum Thioharnstoff-Derivat umgesetzt. Die Reaktionsbedingungen entsprechen dem unter Schritt d) für die Harnstoffderivate angegebenen Reaktionsbedingungen. Das Thioharnstoffderivat wird anschließend in Schritt g) unter den oben genannten Bedingungen zum 2-Aminobenzothiazol-5-carbonsäureester der allgemeinen Formel IV-1b (R³ = NH₂) cyclisiert. Die so erhaltene Verbindung der allgemeinen Formel IV-1 mit R³ = NH₂ kann entweder in Schritt e' zur Verbindung VIII hydrolysiert und gegebenenfalls anschließend methyliert werden (VIII: R = H, R' = CH₃) werden.

Sie kann auch in der oben beschriebenen Weise zur erfindungsgemäßen Verbindung I (mit X = C-NH₂ und Y = S) umgesetzt werden. Zudem besteht die Möglichkeit, die 2-Aminogruppe der Verbindung IV-1b zunächst zu diazotieren und auf diesem Wege weitere Funktionalitäten in die 2-Position des Benzothiazolgerüstes einzuführen. Die Umwandlung von R³ = NH₂ in R³ = Halogen gelingt in bekannter Weise unter Sandmeyer-Bedingungen. Die Umwandlung von R³ = NH₂ in R³ = H gelingt in bekannter Weise durch sukzessive Umsetzung des 2-Aminobenzothiazol-5-carbonsäureesters mit Nitrit unter sauren Bedingungen und anschließend einem Reduktionsmittel wie Hypophosphoriger Säure, Natriumborhydrid, Trialkylsilane, Trialkylstannane, SnCl₂, NO, Wilkinsonkatalysatoren; siehe auch J. Am. Chem. Soc. 1949, 71, S.2137; J. Am. Chem. Soc. 1950, 72, S.3013; 1954, Bd.76, S.290.

Ein weiterer Zugang zu Verbindungen der allgemeinen Formel VIII wird in Schema 2 aufgezeigt.

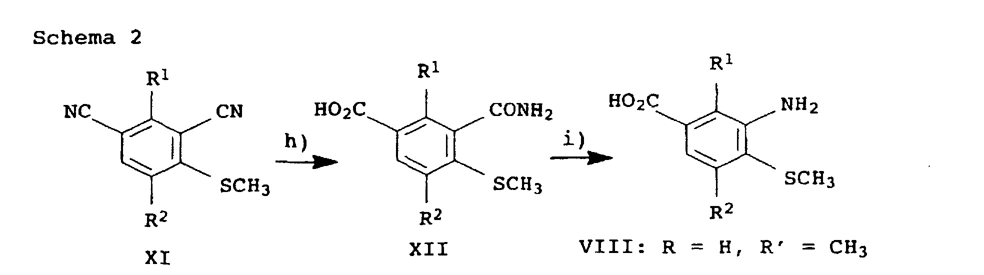

Ausgehend von 2,4-Dicyanothioanisolen der allgemeinen Formel XI wird durch selektive Hydrolyse im Schritt h) das Amid der allgemeinen Formel XII hergestellt. Aufgrund der unterschiedlichen Reaktivität der beiden Methylgruppen gelingt die Darstellung unter üblichen, alkalischen Verseifungsbedingungen, wobei man vorzugsweise das Fortschreiten der Reaktion kontrolliert. Verfahren zur alkalischen Verseifung von Nitrilen sind beispielsweise aus Org. Synth. Coll. Vol. 1, 1941, S.321 bekannt. In einem weiteren Schritt i) wird dann die Amidfunktion in den Verbindungen der allgemeinen Formel XII im Sinne eines Hofmann-Abbaus in eine Aminofunktion umgewandelt. Hierbei werden Verbindungen der allgemeinen Formel VIII mit R=H und R'=CH₃ erhalten. Typische Bedingungen für den Hofmann-Abbau sind: wässrig alkalische Chlor- oder Hypochloridlösungen, Temperaturen im Bereich von 0 bis 150°C und vorzugsweise im Bereich von 20 bis 120°C (siehe auch Organikum 16. Auflage 1986, S. 572).

Ein weiterer Zugang zu Benzothiazol-5-carbonsäuren wird in Schema 3 gezeigt. Dieser Zugang macht von der Umwandlung von Benzothiazolen der allgemeinen Formel XIV in entsprechende Carbonsäuren gemäß Reaktionsschritt o) Gebrauch.
Schema 3

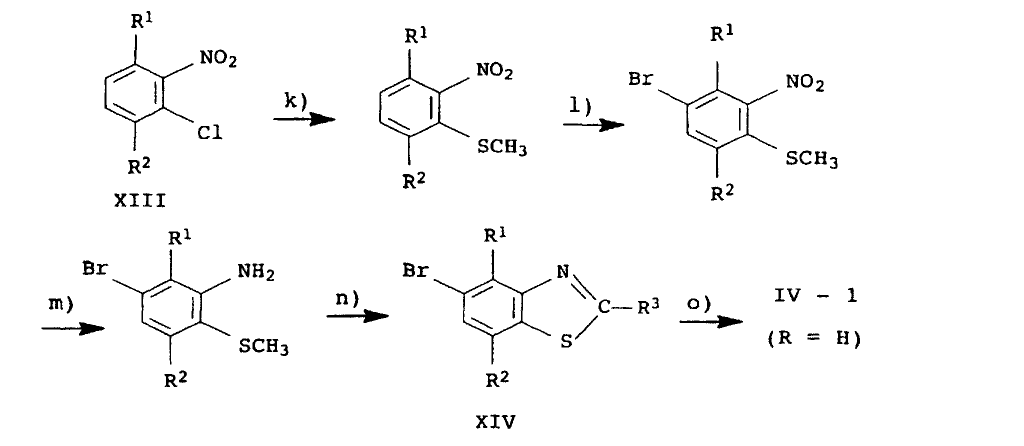

Die Umwandlung des Brombenzothiazols der allgemeinen Formel XIV in die Carbonsäure der allgemeinen Formel IV-1 (R=H) gelingt beispielsweise durch sukzessive Umsetzung von XIV mit Magnesium zur entsprechenden Grignard-Verbindung und anschließender Umsetzung der Grignard-Verbindung mit Kohlendioxid. Alternativ kann die Verbindung XIV durch Halogen-Metallaustausch mit einem Alkalimetallalkyl, z.B. einem Lithiumalkyl, wie Methyllithium, n-Butyllithium oder tert-Butyllithium, und anschließende Umsetzung des Reaktionsproduktes mit CO₂ in die Verbindung IV-1 überführt werden.

Reaktionsschritt o) in Schema 3 kann auch durch Umsetzung des 5-Brombenzothiazols der allgemeinen Formel XIV mit Kohlenmonoxid, einer Base und Wasser unter erhöhtem Druck in Gegenwart eines Pd-, Ni-, Co- oder Rh-Katalysators realisiert werden.

Die Katalysatoren Nickel, Cobalt, Rhodium und insbesondere Palladium können metallisch oder in Form üblicher Salze wie in Form von Halogenverbindungen, z.B. PdCl₂, RhCl₃-H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. (PPh₃)₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 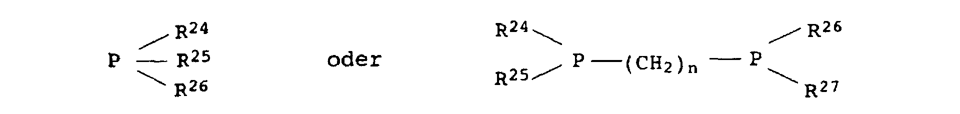 wobei n die Zahlen 1, 2, 3 oder 4 bedeutet und die Reste R²⁴ bis R²⁶ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)2, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff IV.

Zur Herstellung der Benzothiazol-5-carbonsäuren IV-1 (R = OH) führt man die Umsetzung mit Kohlenmonoxid und mindestens äquimolaren Mengen an Wasser, bezogen auf die Ausgangsstoffe XIV durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N.N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf XIV vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Die als Ausgangsverbindungen benutzten 5-Brombenzothiazole XIV sind bekannt oder können leicht durch geeignete Kombination bekannter Synthesen sowie nach der in Schema 3 beschriebenen Reaktionsfolge hergestellt werden.

Gemäß Schema 3 kann man beispielsweise o-Chlornitrobenzole der allgemeinen Formel XIII mit Alkalisalzen von Alkylmercaptanen in die entsprechenden o-Nitrothioether umwandeln (Schritt k). Der so erhaltene Thioether kann selektiv in der 3-Position zur Nitrogruppe bromiert werden (Schritt 1). Übliche Bromierungsreagenzien sind für diesen Zweck neben Brom - gegebenenfalls in Kombination mit einer Lewis-Säure wie FeBr₃, auch N-Bromsuccinimid, N-Bromhydantoin und Pyridiniumperbromid. Die Bromierung erfolgt vorzugsweise in einem organischen Lösungsmittel, beispielsweise einem aliphatischen oder cycloaliphatischen Kohlenwasserstoff, Halogenkohlenwasserstoff oder wasserfreien organischen Säuren bei Temperaturen im Bereich von -15 bis 150°C, vorzugsweise im Bereich von -15 bis 100°C (siehe z.B. Organikum, 16. Aufl., 1986, S. 315). Anschließend wird in Schritt m) die Nitrogruppe zur Aminogruppe reduziert. Die Bedingungen für Schritt m) entsprechen den für Schritt c) in Schema 1 angegebenen Bedingungen. Anschließend wird der o-Aminothioether aus Schritt m) in Schritt n) zum 5-Brombenzothiazol XIV cyclisiert. Die hierfür erforderlichen Reaktionsbedingungen entsprechen den für Schritt a) in Schema 1 angegebenen Bedingungen.

Zur Herstellung der Benzothiazol-S-dioxid-Verbindungen der allgemeinen Formel I (Y = SO₂) werden beispielsweise die Benzothiazol-5-carbonsäuren IV-1a oder IV-1b oder die 5-Brombenzothiazol-5-carbonsäuren XIV mit einem Oxidationsmittel umgesetzt, wobei das entsprechende S-Dioxid erhalten wird, das dann weiter wie beschrieben zur Zielverbindung der Formel I mit Y = SO₂ weiterverarbeitet wird. Bevorzugt wird jedoch zunächst der Thiomethylether der allgemeinen Formel VIII (Schema 1, Formel VIII mit R = H und R' = CH3) zum S-Dioxid VIIIc oxidiert und anschließend zur Benzothiazol-S-dioxid-5-carbonsäure der Formel IV-lc cyclisiert.

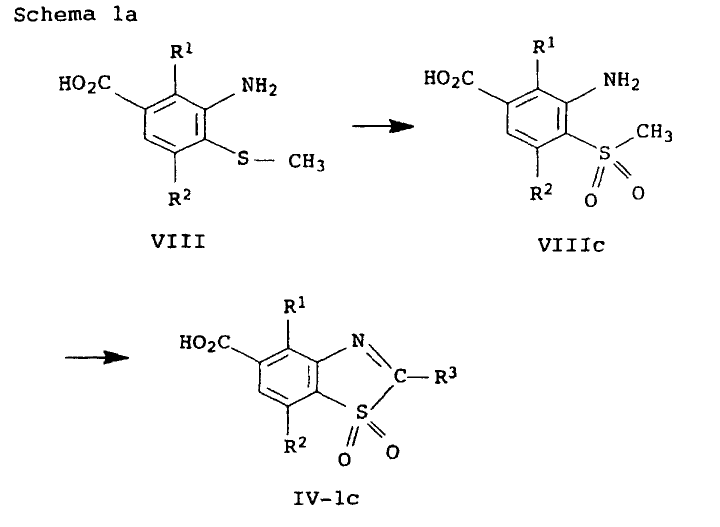

Die Oxidation von VIII zum S-Dioxid gelingt mit Oxidationsmitteln wie Peroxysäuren, z.B. in-Chlorperbenzosäure, Peroxyessigsäure, Trifluorperoxyessigsäure oder mit Wasserstoffperoxid, das vorzugsweise zusammen mit einem Übergangsmetall-Katalysator, z.B. Natriumwolframat(VI), eingesetzt wird. Die Cyclisierung von o-Methylsulfonylaminobenzolen der Formel VIIIc gelingt in Anlehnung an die in Chem. Heterocycl. Comp. Bd.3, 1967, S.197 ff beschriebene Methode.

Eine Synthese für Benzoxazol-5-carbonsäurederivate der allgemeinen Formel IV-2 (X = C-R³, Y = O) wird in Schema 4 beschrieben. Hierbei wird zunächst ausgehend von 3-Nitrotoluolen der allgemeinen Formel IX in der für Schema 1 beschriebenen Weise ein 3-Aminobenzoesäureester der allgemeinen Formel Xb (R - C₁-C₄-Alkyl) hergestellt. In Schritt p) wird zunächst die Aminogruppe in Xb in bekannter Weise diazotiert und anschließend mit Alkaliaziden zu den entsprechenden 3-Azidobenzoesäuren der allgemeinen Formel XV umgesetzt. Das Azid XV wird anschließen in Reaktionsschritt q) mit einer Alkancarbonsäure, die gegebenenfalls auch halogeniert sein kann, z.B. Ameisensäure, Essigsäure, Trifluoressigsäure oder Propionsäure zum Benzoxazol-5-carbonsäureester der allgemeinen Formel IV-2a (R³: C₁-C₄-Alkyl) umgesetzt. Die Verbindung IV-2a kann entweder direkt zum erfindungsgemäßen Pyrazolylderivat der allgemeinen Formel I mit X = CR³ und Y = O umgesetzt werden oder alternativ in Reaktionsschritt r) zum o-Aminophenol der allgemeinen Formel XVI hydrolysiert werden. Die Verbindungen XVI können dann ähnlich wie die o-Aminothiophenole der allgemeinen Formel VIII zu den Benzoxazol-5-carbonsäureestern der allgemeinen Formel IV-2 umgesetzt werden.

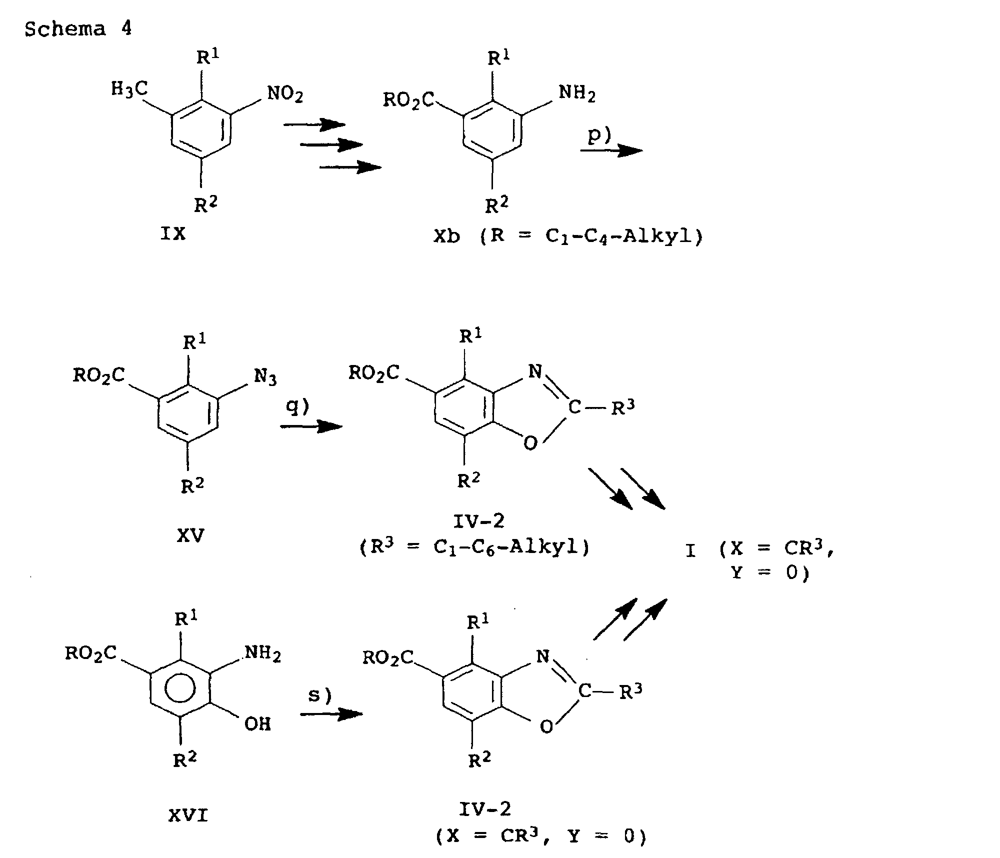

In Reaktionsschritt p) wird zunächst aus dem Amin der allgemeinen Formel Xb in wässriger saurer Lösung oder in einer wasserfreien Säure wie z.B. Ameisensäure, Essigsäure oder Trifluoressigsäure mit einem anorganischen Nitrit wie Natriumnitrit oder einem organischen Nitrit wie Isoamylnitrit eine aromatische Diazoniumverbindung hergestellt. Diese wird durch Zugabe eines Alkaliazids, beispielsweise Natriumazid zu der Lösung oder Suspension der Diazoniumverbindung umgesetzt, wobei man den 3-Azidobenzoesäureester gemäß Schema 4 erhält. Die Reaktionstemperatur der Umsetzung liegt in der Regel im Bereich von -15 bis +50°C, vorzugsweise im Bereich von 0 bis 20°C. Siehe auch K. G. Pinney et al., J. Org. Chem. [JOCEAH] 1991, 56 (9), 3125-3133.

Reaktionsschritt q) wird vorzugsweise in der zur Kondensation gewünschten wasserfreien Säure HOOC-R³ in einem aromatischen Kohlenwasserstoff, wie Benzol, Toluol, Xylol oder Chlorbenzol durchgeführt. Die Reaktionstemperatur liegt in der Regel im Bereich von 0 bis 150°C und vorzugsweise im Bereich von 50 bis 145°C. (Siehe hierzu auch B. Decroix et al., Bull. Soc. Chim. Fr. 1976, 621; S. Chaudhury et al., Can. J. Chem. 1982, 60, 1122). Die Verseifung des in Schritt q) erhaltenen Benzoxazol-5-carbonsäureesters zum 3-Amino-4-hydroxybenzoesäureester der allgemeinen Formel XVI erfolgt beispielsweise unter den für Reaktionschritt e) in Schema 1 angegebenen Bedingungen. Die Kondensation von Verbindung XVI zum Benzoxazol-5-carbonsäureester in Schritt s) erfolgt beispielsweise unter den für Schritt a) in Schema 1 angegebenen Reaktionsbedingungen. (Siehe zu Schritt s) auch Houben-Weyl "Methoden der organischen Chemie, Bd.E8a, 1993, S.1020 f.)

Ein anderer Zugang zu Benzoxazol-5-carbonsäuren der allgemeinen Formel IV (X = C-R³, Y = 0) ist in Schema 5 angegeben.

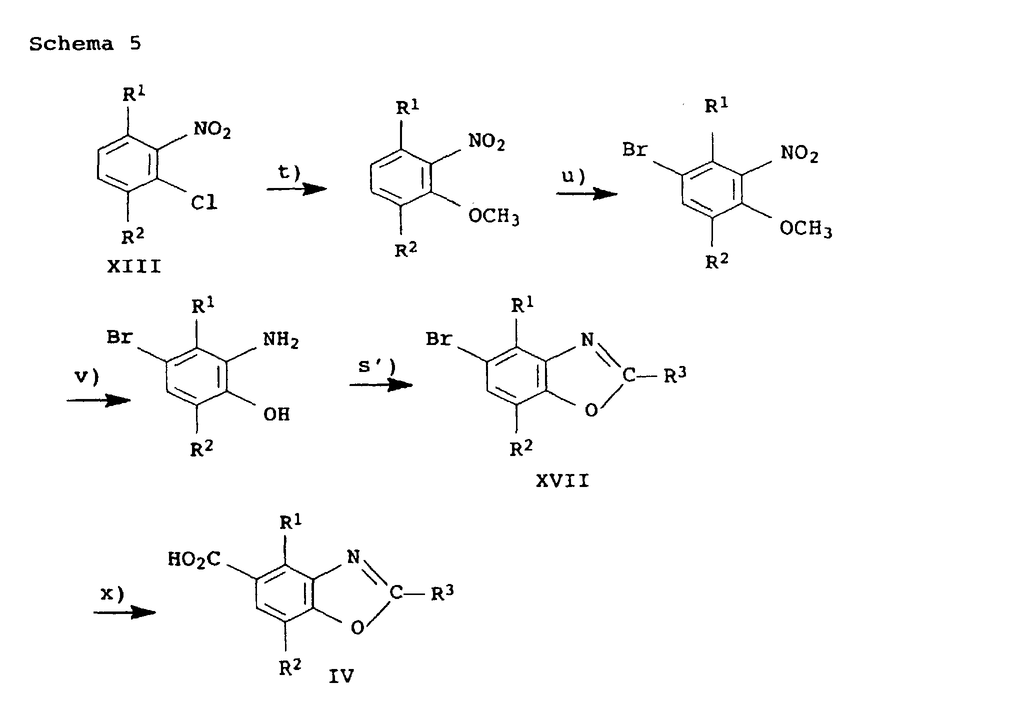

Hierbei wird zunächst ein o-Chlornitrobenzol der allgemeinen Formel XIII durch nukleophilen Austausch von Halogen gegen Methoxy in ein o-Nitroanisol überführt (Schritt t)). Dieses wird dann unter den für Schritt 1) in Schema 3 angegebenen Reaktionsbedingungen bromiert, wobei das Bromatom selektiv in die p-Position zur Methoxygruppe entritt. Das bromierte Nitroanisol wird dann zunächst selektiv zur Aminoverbindung reduziert und anschließend die Hydroxyfunktion durch Etherspaltung freigesetzt. Hierbei erhält man 2-Amino-4-bromphenole. Diese werden dann unter den für Schritt s) angegebenen Reaktionsbedingungen zum 5-Brombenzoxazol der allgemeinen Formel XVII cyclisiert. Die Verbindung XVII wird dann unter den für Schritt o) in Schema 3 beschriebenen Reaktionsbedingungen zu der Benzoxazol-5-carbonsäure der allgemeinen Formel IV (X = C-R³ und Y = O) umgesetzt.

Ein Verfahren zur Herstellung von Benzimidazol-5-carbonsäureestern ist in Schema 6 angegeben.

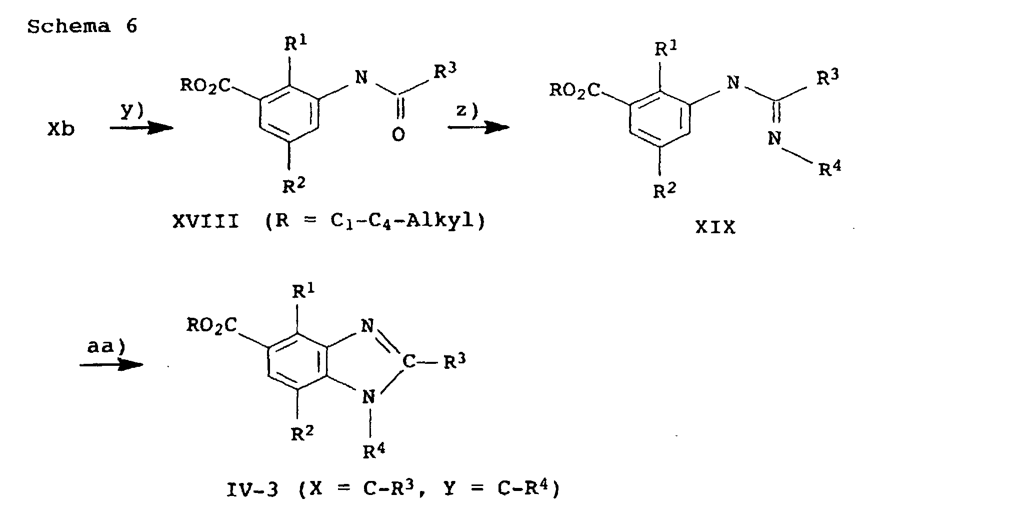

Hierbei geht man zunächst wieder von 3-Nitrotoluolen aus, die in der zuvor beschriebenen Weise in 3-Aminobenzoesäureester der allgemeinen Formel Xb überführt werden. Die Verbindungen Xb werden dann in Reaktionsschritt y) mit einer Carbonsäure der allgemeinen Formel R³-CO₂H oder einem reaktiven Carbonsäureäquivalent RCOL¹, worin L¹ die zuvor genannte Bedeutung hat, zu einem Carbonsäureamid der allgemeinen Formel XVIII umgesetzt. Hierin hat R³ eine der zuvor angegebenen Bedeutungen. XVIII wird dann unter sauren Bedingungen, z.B. mit Phosgen oder Phosphorylchlorid, in ein Nitriliumion überführt, das mit einem Amin der allgemeinen Formel R⁴-NH₂ oder mit Ammoniak abgefangen wird, wobei ein Iminoamid der Formel XIX entsteht. Die Verbindung XIX kann dann unter oxidierenden Bedingungen, wie beispielsweise für Reaktionsschritt b) oder g) in Schema 1 beschrieben, zum Benzimidazol-5-carbonsäureester umgesetzt werden, der seinerseits zur Carbonsäure verseift werden kann.

Schritt y) wird in der Regel unter den für die Bildung von Amiden aus Carbonsäuren oder Carbonsäurederivaten und aromatischen Aminen üblichen Reaktionsbedingungen hergestellt. Die Reaktionstemperatur liegt in der Regel im Bereich von -15 bis 200°C, vorzugsweise im Bereich von 20 bis 150°C.

Zur Herstellung des Iminoamids der allgemeinen Formel XIX wird zunächst das Amid der allgemeinen Formel XVIII unter Wasserausschluß in einem organischen Lösungsmittel, beispielsweise einem der vorgenannten cycloaliphatischen oder aromatischen Kohlenwasserstoffe oder einem Ether gelöst und mit einer anorganischen Säure, beispielsweise Salzsäure oder Schwefelsäure, einer Lewis-Säure wie Titantetrachlorid oder einem Säurechlorid wie Sulfonylchlorid, Sulfurylchlorid, Phosphorylchlorid oder Phosgen in das Nitriliumion überführt. Die hierfür erforderlichen Temperaturen liegen in der Regel im Bereich von -15 bis 150°C und vorzugsweise im Bereich von 20 bis 140°C. Das Nitriliumion wird dann mit Ammoniak oder einem Amin der allgemeinen Formel R⁴-NH₂ abgefangen.

Die Cyclisierung der Verbindung XIX zum Benzimidazol-5-carbonsäureester der allgemeinen Formel IV (X = C-R³, Y = C-R⁴) wird in der Regel mittels eines Oxidationsmittels wie Bleitetraacetat, Thallium(III)nitrit, Sulfurylchlorid oder Natriumhypochlorid unter wasserfreien Bedingungen durchgeführt. Als Lösungsmittel dienen beispielsweise aliphatische oder cycloaliphatische Kohlenwasserstoffe, aromatische Kohlenwasserstoffe oder Ether. Die Umsetzung erfolgt in der Regel bei Temperaturen im Bereich -15 bis +150°C und vorzugsweise im Bereich von 0 bis 140°C. Zur Herstellung von Benzimidazolen aus Iminoamiden siehe auch (Can. J. Chem. 1982, 60, S.1122).

Die Herstellung von Benzoisothiodiazolen der allgemeinen Formel IV-4 (X-Y = S=N) gelingt beispielsweise ausgehend von Benzimidazol-5-carbonsäuren oder ihren Estern in der in Schema 7 beschriebenen Weise.

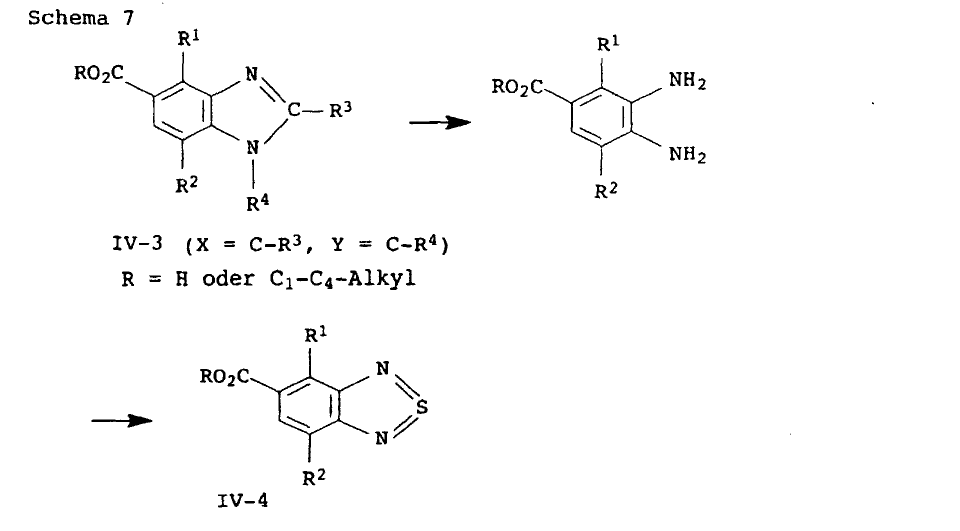

Hierbei wird zunächst ein Benzimidazol-carbonsäureester oder die freie Carbonsäure zu 3,4-Diaminobenzoesäure verseift. Diese wird anschließend mit schwefliger Säure oder ihren Derivaten, z.B. SO2 oder SO₂Cl₂, zur Benzoisothiadiazol-5-carbonsäure der allgemeinen Formel IV-4 cyclisiert. Üblicherweise wird die Cyclisierung bei Temperaturen im Bereich von 0 bis 200°C und vorzugsweise im Bereich von 50 bis 150°C, z.B. in einem Lösungsmittel oder in der Schmelze, durchgeführt (siehe auch: Chem. Ber. 1967, Bd.100, S. 2164).

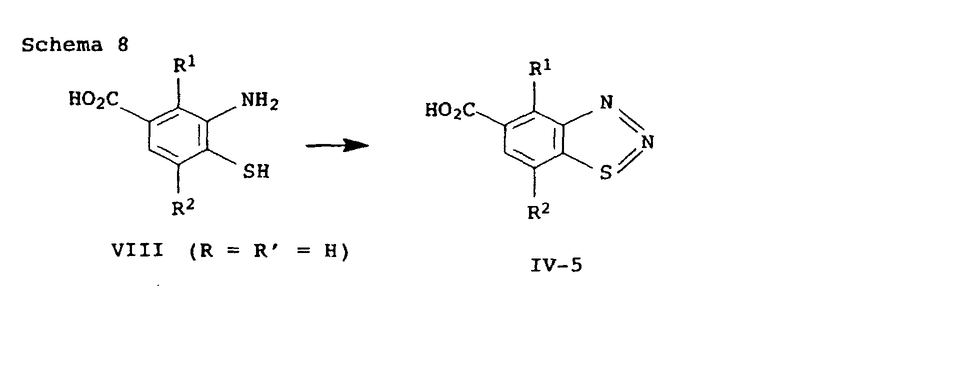

Benzothiadiazol-5-carbonsäuren der allgemeinen Formel IV-5 (X = N, Y = S) können ausgehend von 2-Aminothiophenol-5-carbonsäuren der allgemeinen Formel VIII (R = R' = H) hergestellt werden. Hierzu wird man die Verbindungen der allgemeinen Formel VIII zunächst diazotieren, beispielsweise durch Umsetzung mit organischem oder anorganischem Nitrit in einem wässrigen, neutralen Reaktionsmedium bei Temperaturen im Bereich von -15 bis +20°C. Die wässrige Lösung oder Suspension des Diazoniumsalzes wird anschließend angesäuert, wobei sich die Verbindung der allgemeinen Formel IV-5 bildet. Diese kann in konventioneller Weise aus der Reaktionmischung, beispielsweise durch Extraktion mit einem organischen Lösungsmittel, gewonnen werden. Die Herstellung der Ausgangsverbindungen VIII ist in Schema 1 beschrieben. Die Herstellung der Benzothiadiazolcarbonsäuren IV-5 (X = N, Y = S) kann beispielsweise in Anlehnung an das in der US 5,770,758 beschriebene Verfahren erfolgen.

### Beispiele

### 4-[4'-Methylbenzothiazol-5'-ylcarbonyl]-5-hydroxy-1-methylpyrazol

### (Beispiel 1 )

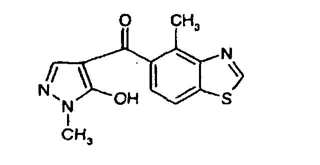

### 1.1 2-Methyl-4-thiocyano-isophthalsäuredinitril

Unter Erwärmen wurden 189 g (1 Mol) 2-Methyl-3-cyano-4-thiocyanoanilin in 1 kg Eisessig gelöst und danach wurden 400 g (4 Mol) konz. HCl und nach 15 min Rühren 400 ml Wasser zu so zugefügt, daß eine feinverteilte Supension des Hydrochlorids entstand. Nach kurzem Rühren (15 bis 30 min) wurde bei -5 bis 0°C langsam eine Lösung von 69 g (1 Mol) Natriumnitrit in 140 ml Wasser zugetropft. in einem separatem Rührkolben wurden 245 g (5 Mol) NaCN in einer Mischung aus 1,5 l Wasser und 136 g (2 Mol) 25 proz. Ammoniakwasser gelöst und anschließend 250 g (1 Mol) CuSO₄·5 H₂O zugegeben. Danach wurden bei 25°C eine zuvor bereitete und bei 0°C gehaltene Diazoniumlösung zügig in den Cu-Komplex zugetropft, wobei die Temperatur 40°C nicht überstieg. Nach Beendigung der Gasabspaltung wurde noch 30 min nachrühren gelassen. Man saugte den ausgefallenen Feststoff ab und wusch ihn dreimal mit Wasser. Das Filtrat wurde mit 2 l Methylenchlorid extrahiert. Der Feststoff wurde dann in ein Rührgefäß gegeben und mit 1 l konz. HCl versetzt. Anschließend goß man den Methylenchlorid-Extrakt zu und ließ 15 min rühren. Nach Abtrennen der organischen Phase und Abfiltrieren ungelöst gebliebener Anteile wurde dreimal mit Wasser gewaschen und nach Trocknung über Natriumsulfat eingeengt. Zur Abtrennung unerwünschter Bestandteile wurde das i Rohprodukt in Ethylacetat gelöst, vom Ungelösten filtriert und die Lösung anschließend eingeengt. Ausbeute: 170 g (85%). Smp.: 95 - 107°C

### 1.2 3-Methyl-2,4-dicyanothiophenol

Zu einer Lösung von 170 g (0,85 Mol) 2 Methyl-4-thiocyanoisophthalsäuredinitril in 850 ml Methanol, wurden bei 25 bis 35°C eine Lösung von 110,5 g (0.85 Mol) 60 proz. Natriumsulfid in 425 ml Wasser zugetropft und drei Stunden bei Raumtemperatur nachrühren gelassen. Danach wurde mit 1000 ml Wasser versetzt und mit Methyl-tert.-butylether extrahiert. Die wässrige Phase wurde durch Ansäuern mit HCl auf pH 1 gebracht und das Thiophenol mit Methylenchlorid extrahiert. Nach dreimaligem Waschen des Extraktes mit Wasser wurde die organische Phase abgetrennt, über Natriumsulfat getrocknet und eingeengt. Ausbeute: 150 g (99%).
Smp.: 172 - 179°C

### 1.3 3-Methyl-2,4-dicyano-thioanisol

50g (0,29 Mol) 3-Methyl-2,4-dicyano-thiophenol wurden in eine Lösung von 23 g (0,58 Mol) NaOH in 400 ml Wasser gegeben und dann wurden bei 25 bis 35°C 73 g (0,58 Mol) Dimethylsulfat zugetropft. Nach 16 h Nachrühren bei 25°c war ein Feststoff ausgefallen, der abgesaugt wurde, zweimal mit Wasser gewaschen und dann aus Eisessig/Wasser umkristallisiert wurde. Ausbeute: 43 g (80%).
Smp.: 176 - 181°C

### 1.4 2-Methyl-3-aminocarbonyl-4-methylsulfanylbenzoesäure

34g (0,181 Mol) 3-Methyl-2,4-dicyanothioanisol wurden in einer Lösung von 21,7 g (0,54 Mol) NaOH in 200 ml Wasser suspendiert und 8 h zum Sieden erhitzt. Nach dem Abkühlen fiel ein Teil des Produktes aus und wurde durch Absaugen und Waschen mit Wasser isoliert. Das noch alkalische Filtrat wurde mit MTBE extrahiert, der Extrakt wurde verworfen. Die wässrige Phase wurde mit konz. HCl angesäuert (pH 1) und mit Ethylacetat extrahiert. Ausbeute: 29 g (71%).
Smp.: 230 - 240°C

### 1.5 3-Amino-2-methyl-4-methylsulfanylbenzoesäure

a) aus 2-Methyl-3-aminocarbonyl-4-methylsulfanylbenzoesäure
   Zu einer Lösung von 3,64 g NaOH (0.09 mol) in 40 ml Wasser wurden bei 0°C 2,9 g Brom (0.018 mol) getropft. Anschließend wurden 4,1 g 2-Methyl-3-aminocarbonyl-4-methylsulfanylbenzoesäure (0,018 mol) bei 0°C portionsweise zugegeben. Man ließ 1 h bei 0°C rühren und erwärmte danach auf 20°C. Anschließend wurde die Reaktionsmischung mit 10 %iger HCl sauer gestellt und mit Ethylacetat extrahiert. Der anfallende Niederschlag wurde mehrmals mit Ethylacetat aufgeschlämmt und getrocknet. Ausbeute: 0,95 g (27%).
b) aus 2-Amino-4-methylbenzothiazol-5-carbonsäuremethylester
   10 g 2-Amino-4-methyl-benzothiazol-5-carbonsäure-methylester (0,045 mol) wurden in einer Mischng aus 120 ml Wasser, 120 ml Ethylenglykol und 50 g NaOH gelöst und 20 h bei 130°C gerührt. Danach wurde mit 50 g Eis verdünnt, 3 Tropfen (n-Bu)₄N⁺OH- - Lösung zugegeben und zum Schluß wurden bei 20°C 6,25 m1 Dimethylsulfat ((0,05 mol) in 15 ml Toluol zugetropft. Nach 30 min wurde mit konz. HCl angesäuert und der Niederschlag abgesaugt, mit Wasser gewaschen und getrocknet. Ausbeute: 7 g (71%).
   Smp.: 225°C (Zers.)

### 1.6 3-Amino-2-methyl-4-methylsulfanylbenzoesäuremethylester

2 g 3-Amino-2-methyl-4-methylsulfanylbenzoesäure (0,01 mol) wurden in 20 ml Methanol gelöst, mit 2,0 g konz. Schwefelsäure versetzt und für 2 h auf 60°C erwärmt. Nach dem Abkühlen wurde die Reaktionsmischung auf Wasser gegeben, neutralisiert und mit Ethylacetat extrahiert. Nach Waschen, Trocknen wurde das Lösungsmittel entfernt. Ausbeute: 1,3 g (62%). Smp.: 98 - 103°C

### 1.7 3-Formamidyl-2-methyl-4-methylsulfanylbenzoesäuremethylester

Zu einer Mischung aus 30 ml Essigsäureanhydrid und 2,2 g Ameisensäure (0,05 mol) wurde bei 40°C portionsweise 4,75 g 3-Amino-2-methyl-4-methylsulfanylbenzoesäuremethylester (0,05 mol) gegeben. Nach 5 h ließ man abkühlen, gab die Lösung auf Eiswasser und extrahierte erschöpfend mit Methylenchlorid. Die organischen Phasen wurden gewaschen, getrocknet und anschließend wurde das Lösungsmittel entfernt. Ausbeute: 4,7 g (0,044 mol).
Smp.: 170 - 176°C

### 1.8 4-Methylbenzothiazol-5-carbonsäuremethylester

a) aus 3-Formamidyl-2-methyl-4-methylsulfanylbenzoesäuremethylester
   2,4 g 3-Formamidyl-2-methyl-4-methylsulfanylbenzoesäuremethylester (0,01 mol) wurden in Methylenchlorid gelöst. Phosgen wurde bis zur Sättigung eingegast und anschließend wurde überschüssiges Phosgen mit Stickstoff vertrieben. Danach wurden 1,5 g Triethylamin zugetropft. Nach Entfernen des Lösungsmittels im Vakuum wurde der Rückstand in Ethylacetat aufgenommen, das Salz abfiltriert und die organische Phase erneut eingeengt. Der Rückstand wurde säulenchromatographisch an Kieselgel gereinigt. Ausbeute: 1,6 g (77%).
b) aus 2-Amino-4-methylbenzothiazol-5-carbonsäuremethylester
   15 g 2-Amino-4-methylbenzothiazol-5-carbonsäuremethylester (0,07 mol) wurden in 450 ml Phosphorsäure vorgelegt und auf - 8°C abgekühlt. Dann wurden 27,9 g NaNO₂ (0,4 mol) in 30 ml Wasser so zugetropft, daß die Temperatur nicht über -4°C stieg. Anschließend wurde das Diazoniumsalz bei 5 bis 10°C zu 169 ml Hypophosphoriger Säure getropft und über Nacht bei 20°C gerührt. Anschließen wurde die Reaktionslösung neutralisiert und erschöpfend mit Ethylacetat extrahiert. Nach Waschen und Trocknen der organischen Phasen wurde das Lösungsmittel entfernt. Ausbeute: 6,84 g (49%).
   Smp.: 90 - 92°C

### 1.9 4-Methylbenzothiazol-5-carbonsäure

16,6 g 4-Methylbenzothiazol-5-carbonsäure-methylester (0,08 mol) wurden in 280 ml 5 %iger Kalilauge gelöst und für 2,5 h auf Rückfluß erhitzt. Nach dem Abkühlen wird mit Phosphorsäure sauer gestellt. Das Produkt wurde abfiltriert und anschließend getrocknet. Ausbeute: 14,34 g (93%).
Smp.: 260 - 265°C

### 1.10 4-Methylbenzothiazol-5-carbonsäure- ( 1' -methylpyrazol-5-yl)-ester

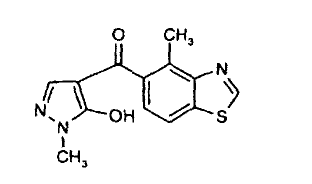

0,65 g 4-Methylbenzothiazol-5-carbonsäure (0,004 mol) und 0,33 g 1-Methyl-5-hydroxy-pyrazol (0,004 mol) wurden in 30 ml abs. Acetonitril gelöst und mit 0,65 g EDC (0,004 mol), 0,5 ml Triethylamin und einer kat. Menge DMAP versetzt. Nach beendeter Reaktion wurde die Lösung auf Wasser gegeben und mit Ethylacetat extrahiert. Nach Waschen und Trocknen der organischen Phase wurde das Produkt durch säulenchromatographisch an Kieselgel gereinigt. Ausbeute: 0,42 g (41%). ¹H-HMR (CDCl₃, TMS): δ = 3,18 (s, 3H); 3,83 (s, 3H); 6,24 (d, 1H); 7,49 (d, 1H); 7,94 (d, 1H); 8,21 (d, 1H); 9,08 (s, 1H) ppm.
EDC = Ethyl- (3' -Dimethylaminopropyl) carbodiimid
DMAP = 4-Dimethylaminopyridin

### 1.11 4-(4'-Methylbenzothiazol-5'-ylcarbonyl)-5-hydroxy-1-methylpyrazol

0,38 g 4-Methylbenzothiazol-5-carbonsäure-(1'-methylpyrazol-5-yl)-ester (1,39 mmol) wurden in 25 ml Dioxan gelöst und mit 0,28 g K₂CO₃ (2 mmol) versetzt. Man refluxierte bis zum vollständigen Umsatz, entfernte das Lösungsmittel im Vakuum und nahm den Rückstand mit Wasser auf. Die wässrige Phase wurde mit Methylenchlorid extrahiert, auf pH 2 eingestellt und mit Ethylacetat extrahiert. Nach Entfernen des Lösungsmittels wurde das Produkt durch Ausrühren gereinigt. Ausbeute: 0,25 g (66%) Smp.: 149 - 150°C

### 4-(2'-Chlor-4'-methylbenzothiazol-5'-ylcarbonyl)-5-hydroxy-1-methylpyrazol (Beispiel 2)

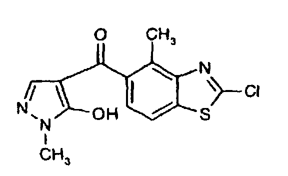

### 2.1 3-Amino-2-methylbenzoesäuremethylester

210 g 2-Methyl-3-nitrobenzoesäuremethylester (1,08 mol) wurden in 4 l Methanol glöst, mit 21 g Pd/C versetzt und anschließend bei Raumdruck hydriert. Nach beendeter Reaktion wurde vom Katalysator abfiltriert und das Lösungsmittel entfernt. Ausbeute: 178 g (quant.). ¹H-NMR (CDC1₃, TMS): δ = 2,34 (s, 3H); 3,60 (s, br, 2H, NH₂); 3,84 (s, 3H); 6,80 (d, 1H); 7,04 (dd, 1H); 7,21 (d, 1H) ppm.

### 2.2 N-(2-Methyl-3-methoxycarbonylphenyl)thioharnstoff

90,7 g 3-Amino-2-methylbenzoesäuremethylester (0,55 mol) wurden in 510 ml Chlorbenzol gelöst und bei -5°C mit 14 ml konz. Schwefelsäure und 49 g Natriumrhodanid (0,6 mol) versetzt. Anschließend wurden 2 ml 15-Krone-5 zugegeben und die Reaktionsmischung wird für 13 h auf 100°C erhitzt. Nach dem Abkühlen wurde der Feststoff abgesaugt, mit Wasser gewaschen und getrocknet. Ausbeute: 104,8 g (85%). Smp.: 198°C

### 2.3 2-Amino-4-methylbenzothiazol-5-carbonsäuremethylester

56 g N-(2-Methyl-3-methoxycarbonylphenyl] thioharnstoff (0,25 mol) wurden in 2 l Chlorbenzol gelöst und auf 0°C abgekühlt. Anschließend wurden 40 g Brom (0,25 mol) in 100 ml Chlorbenzol zugetropft. Die Reaktionsmischung wurde für 3 h auf 90°C erhitzt, der Niederschlag abgesaugt und mit Methylenchlorid gewaschen. Anschließend wurde er in Ethylacetat gelöst und mit Natriumhydrogencarbonat-Lösung ausgeschüttelt. Nach Waschen und Trocknen wurde das Produkt durch Entfernen des Lösungsmittels erhalten. Ausbeute: 43 g (80%).
Smp.: 220°C

### 2.4 2-Chlor-4-methylbenzothiazol-5-carbonsäuremethylester

Zu einer Lösung von 5 g 2-Amino-4-methylbenzothiazol-5-carbonsäuremethylester (0,02 mol) in 150 ml Phosphorsäure wurde bei -8°C eine Lösung von 9,3 g NaNO₂ (0,14 mol) in 10 ml Wasser getropft. Anschließend wurde bei 5°C eine Lösung aus 3 g CuCl und 12 ml konz. HCl zugetropft. Die Reaktionsmischung wurde auf 100°C erwärmt. Nach dem Abkühlen wird der Rückstand abgesaugt, mit Wasser gewaschen und getrocknet. Das Produkt wurde durch Säulenchromatographie an Kieselgel gereinigt. Ausbeute: 3 g (55, %).
¹H-NMR (CDC1₃, TMS) δ = 2,98 (s, 3H); 3,95 (s, 3H); 7,67 (d, 1H); 7,95 (d, 1H) ppm.

### 2.5 2-Chlor-4-methylbenzothiazol-5-carbonsäure

3 g 2-Chlor-4-methylbenzothiazol-5-carbonsäuremethylester (0,012 mol) wurden in 50 ml THF gelöst, auf 0°C abgekühlt und mit einer Lösung von 0,6 g LiOH in 20 ml Wasser versetzt. Nach 1 h ließ man auf 20°C erwärmen und rührte weitere 20 h nach. Anschließen wurde das Lösungsmittel im Vakuum entfernt, die wässrige Phase mit Phosphorsäure angesäuert und mit Ethylacetat extrahiert. Nach Waschen und Trocknen wird das Produkt durch Entfernen des Lösungsmittels erhalten. Ausbeute: 2,6 g (92%).
Smp.: >250°C
¹H-NMR (D₆-DMSO, TMS) δ = 2,85 (s, 3H); 7,90 (d, 1H); 8,02 (d, 1H) ppm.

### 2.6 3-chlor-4-methylbenzothiazol-5-carbonsäure-(1'-methylpyrazol-5'-yl)ester

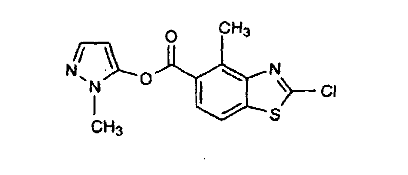

1 g 3-Chlor-4-methylbenzothiazol-5-carbonsäure (4,4 mol) und 0,46 g 1-Methyl-5-hydroxy-pyrazol (4,7 mol) wurden in 50 ml abs. Acetonitril gelöst und mit 1 g EDC, 0,7 ml Triethylamin und einer kat. Menge DMAP versetzt. Nach beendeter Reaktion wurde die Lösung auf Wasser gegeben und mit Ethylacetat extrahiert. Nach Waschen und Trocknen der organischen Phase wurde das Produkt durch Kristallisation/Säulenchromatographie gereinigt. Ausbeute: 0,22 g (16%).
¹H-NMR (CDCl₃, TMS) δ = 3,08 (s, 3H); 3,80 (s, 3H); 6,25 (s, 1H); 7,46 (s, 1H); 7,77 (d, 1H); 8,17 (d, 1H) ppm.

### 2.7 4-(3'-Chlor-4'-methylbenzothiazol-5'-ylcarbonyl)-5-hydroxy-1-methylpyrazol

0,22 g 3-Chlor-4-methyl-benzothiazol-5-carbonsäure-(1'-methylpyrazol-5-yl)-ester (0,7 mol) wurden in 35 ml Dioxan gelöst und mit 0,5 g K₂CO₃ versetzt. Man refluxierte bis zum vollständigen Umsatz, entfernte das Lösungsmittel im Vakuum und nahm den Rückstand mit Wasser auf. Die wässrige Phase wurde mit Methylenchlorid extrahiert, auf pH 2 eingestellt und mit Ethylacetat extrahiert. Nach Entfernen des Lösungsmittels wurde das Produkt durch Ausrühren gereinigt. Ausbeute: 0,19 g (86%). ¹H-NMR (CDCl₃, TMS) δ = 2,82 (s, 3H); 3,76 (s, 3H); 7,40 (s, 1H); 7,52 (d, 1H); 7,73 (d, 1H) ppm.

### 4-(4'-Methylbenzothiadiazol-5'-ylcarbonyl)-5-hydroxy-1-methylpyrazol (Beispiel 3)

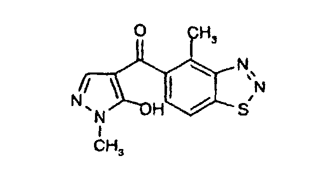

### 3.1 1 4-Methylbenzothiadiazol-5-carbonsäure

5 g 2-Amino-4-methylbenzothiazol-5-carbonsäuremethylester (0,02 mol) wurden in 28,6 g 50 %iger KOH bei 120°C 4 h gerührt. Anschließend wurde mit 10 %iger HCl neutralisiert und mit einem Überschuß 40 %iger NaNO₂-Lösung bei 0 bis 10°C versetzt. Die Reaktionsmischung wurde angesäuert und mit Ethylacetat extrahiert. Nach Waschen und Trocknen wurde das Produkt durch Entfernen des Lösungsmittels erhalten. Ausbeute: 1,3 g (28%).

### 3.2 4-(4'-Methylbenzothiadiazol-5'-ylcarbonyl)-5-hydroxy-1-methylpyrazol

1,3 g 4-Methylbenzothiadiazol-5-carbonsäure (6,3 mmol) und 0,65 g 1-Methyl-5-hydroxy-pyrazol (6,6 mmol) wurden in 50 ml abs. Acetonitril gelöst und mit 1,4 g EDC (7,5 mmol), 1 ml Triethylamin und einer kat. Menge DMAP versetzt. Nach beendeter Reaktion wurde die Lösung auf Wasser gegeben und mit Ethylacetat extrahiert. Nach Waschen und Trocknen der organischen Phase wurde das Produkt säulenchromatographisch an Kieselgel gereinigt. Ausbeute: 0,7 g (39%).
Smp.: 152-153°C

### 3.3 4-(4'-Methylbenzothiadiazol-5'-ylcarbonyl)-5-hydroxy-1-methylpyrazol

0,7 g 4-Methyl-benzothiadiazol-5-carbonsäure-(1'-methylpyrazol-5-yl)-ester (2,4 mmol) wurden in 40 ml Dioxan gelöst und mit 0,5 g K₂CO₃ versetzt. Man refluxierte bis zum vollständigen Umsatz, entfernte das Lösungsmittel im Vakuum und nahm den Rückstand mit Wasser auf. Die wässrige Phase wurde mit Methylenchlorid extrahiert, auf pH 2 eingestellt und mit Ethylacetat extrahiert. Nach Entfernen des Lösungsmittels wurde das Produkt durch Ausrühren gereinigt. Ausbeute: 0,65 g (93%).
Smp.: 207-209°C

### 4- (2'-Methylamino-4'-methylbenzothiazol-5'-ylcarbonyi)-5-hydroxy-1-methylpyrazol (Beispiel 4)

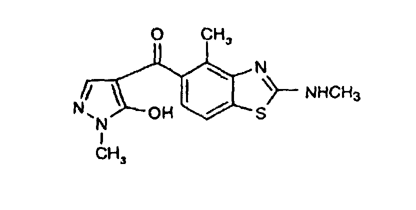

### 4.1 2-Methylamino-4-methyl-benzothiazol-5-carbonsäure

99 g 3-Amino-2-methylbenzoesäure (0,655 mol) aus wurden in 500 ml Essigsäure vorgelegt und bei 80°C mit 51 g Methylisothiocyanat (7 mol) in 100 ml Essigsäure versetzt. Nach 2h wurden 106 g Brom (0,66 mol) in 20 ml Essigsäure bei 50°C zugetropft. Anschließend wurde für 2 h ca. 100°C erhitzt und nach beendeter Reaktion abkühlen lassen. Der Niederschlag wurde abfiltriert, das Filtrat auf ca. 50 ml eingeengt und das restliche Filtrat sowie den Niederschlag auf Wasser gegeben. Die wäßrige Phase wurde auf pH 5 eingestellt und bei 80°C wurde der Niederschlag abfiltriert. Anschließend wurde dieser mit Wasser gewaschen und getrocknet. Ausbeute: 66 g (42%).
¹H-NMR (D₆-DMSO, TMS) 6 = 2,72 (s, 3H); 2,95 (d, 3H); 7,46 (d, 1H); 7,57 (d, 1H); 8,04 (q, 1H, NH) ppm.

### 4.2 2-Methylamino-4-methyl-benzothiazol-5-carbonsäure-(1'-methylpyrazol-5-yl)-ester

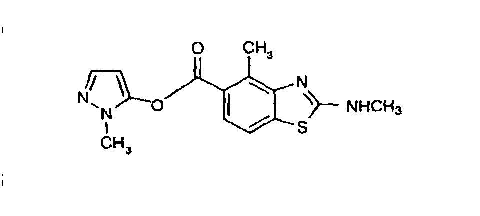

3,1 g 2-Methylamino-4-methyl-benzothiazol-5-carbonsäure (0,014 mol) und 1,4 g 1-Methyl-5-hydroxypyrazol (0,015 mol) wurden in 110 ml abs. Acetonitril gelöst und mit 2,67 g EDC (0,014 mol), 1,2 ml Triethylamin und einer kat. Menge DMAP versetzt. Nach beendeter Reaktion wurde die Lösung auf Wasser gegeben und mit Ethylacetat extrahiert. Nach Waschen und Trocknen der organischen Phase wurde das Produkt durch Kristallisation gereinigt. Ausbeute: 2,2 g (52%).
¹H-NMR (D₆-DMSO): δ = 2,80 (s, 3H); 3,00 (d, 3H); 3,73 (s, 3H); 6,22 (d, 1H); 7,43 (d, 1H); 7,72 (d, 1H); 7,84 (d, 1H); 8,20 (s, br, 1H) ppm.

### 4-(1'-Methylbenzotriazol-5-ylcarbonyl)-5-hydroxy-1-methylpyrazol (Beispiel 5)

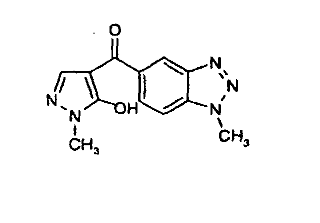

### 5.1 1-Methylbenzotriazol-5-carbonsäure- (1' -methylpyrazol-5-yl)ester

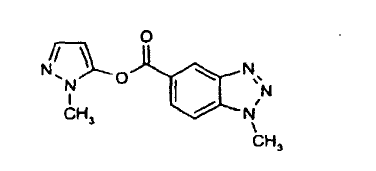

1,5 g 1-Methylbenzotriazol-5-carbonsäure (8,5 mmol) und 0,87 g 1-Methyl-5-hydroxy-pyrazol (8,9 mmol) wurden in 70 ml abs. Acetonitril gelöst und mit 1,62 g EDC (8,5 mmol), 2 ml Triethylamin und einer kat. Menge DMAP versetzt. Nach beendeter Reaktion wurde die Lösung auf Wasser gegeben und mit Ethylacetat extrahiert. Nach Waschen und Trocknen der organischen Phase wurde das Produkt durch Kristallisation gereinigt. Ausbeute: 0,77 g (35%).
¹H-NMR (CDC1₃, TMS): δ = 3,82 (s, 3H); 4,40 (s, 3H); 6,28 (d, 1H); 7,48 (d, 1H); 7,67 (d, 1H); 8,36 (d, 1H); 8,98 (s, 1H) ppm.

### 5.2 4-(1'-Methylbenzotriazol-5-ylcarbonyl)-5-hydroxy-1-niethylpyrazol

0,53 g 1-Methyl-benzotriazol-5-carbonsäure-(l'-methylpyrazol-5-yl)-ester (2 mmol) wurden in 30 ml Dioxan gelöst und mit 0,43 g K₂CO₃ (3 mmol) versetzt. Man refluxierte bis zum vollständigen Umsatz, entfernte das Lösungsmittel im Vakuum und nahm den Rückstand mit Wasser auf. Die wässrige Phase wurde mit Methylenchlorid extrahiert, auf pH 2 eingestellt und mit Ethylacetat extrahiert. Nach Entfernen des Lösungsmittels wurde das Produkt durch Ausrühren gereinigt. Ausbeute: 0,31 g (58%).
¹H-NMR (CDCl₃, TMS): δ = 3,78 (s, 3H); 4,39 (s, 3H); 4,75 (s, br, 1H); 7,63 (d, 1H); 7,86 (s, 1H); 8,08 (d, 1H); 8,56 (s, 1H) ppm.

### 6. 4-(4', Methylbenzothiazol-5'-ylcarbonyl)-5'-methoxyl-methylpyrazol (Beispiel 6)

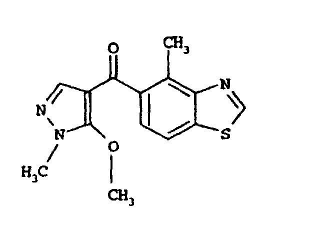

Zu einer Mischung aus 0,3 g 4-(4'-Methylbenzothiazol-5'-ylcarbonyl)-5-hydroxy-1-methylpyrazol aus Beispiel 1 und 0,18 g Kaliumcarbonat in 15 ml Dimethylformamid wurden bei Raumtemperatur 0,26 g Iodmethan getropft. Die Mischung wurde 75 min bei 50°C und über Nacht bei Raumtemperatur gerührt. Zur Aufarbeitung wurde die Reaktionsmischung auf Wasser gegeben und mit Methyl-tert.-butylether extrahiert. Nach Waschen und Trocknen der vereinigten organischen Phasen und Entfernen des Lösungsmittels erhielt man die Verbindung I-le.394 in Form weißlich-gelber Kristalle. Ausbeute: 0,15 g (48%).
Smp.: 138-141°C.

In analoger Weise wurden durch Umsetzung der Verbindung I-1a.394 aus Beispiel 1 mit Benzylbromid die Verbindung des Beispiels 13 (I-1g.394) und durch Umsetzung mit dem jeweiligen Säurechlorid die Verbindungen I-1i.394 (Beispiel 16), I-1u.394 (Beispiel 15) und I-1v.394 (Beispiel 18) hergestellt.

Die Verbindungen der Beispiele 7 bis 12, 14, 17 und 19 bis 52 wurden analog der in Beispiel 1, Schritte 1.10 und 1.11 beschriebenen Sequenz durch Umsetzung derjeweiligen Carbonsäure IVb mit dem entsprechenden 5-Hydroxypyrazol III hergestellt.

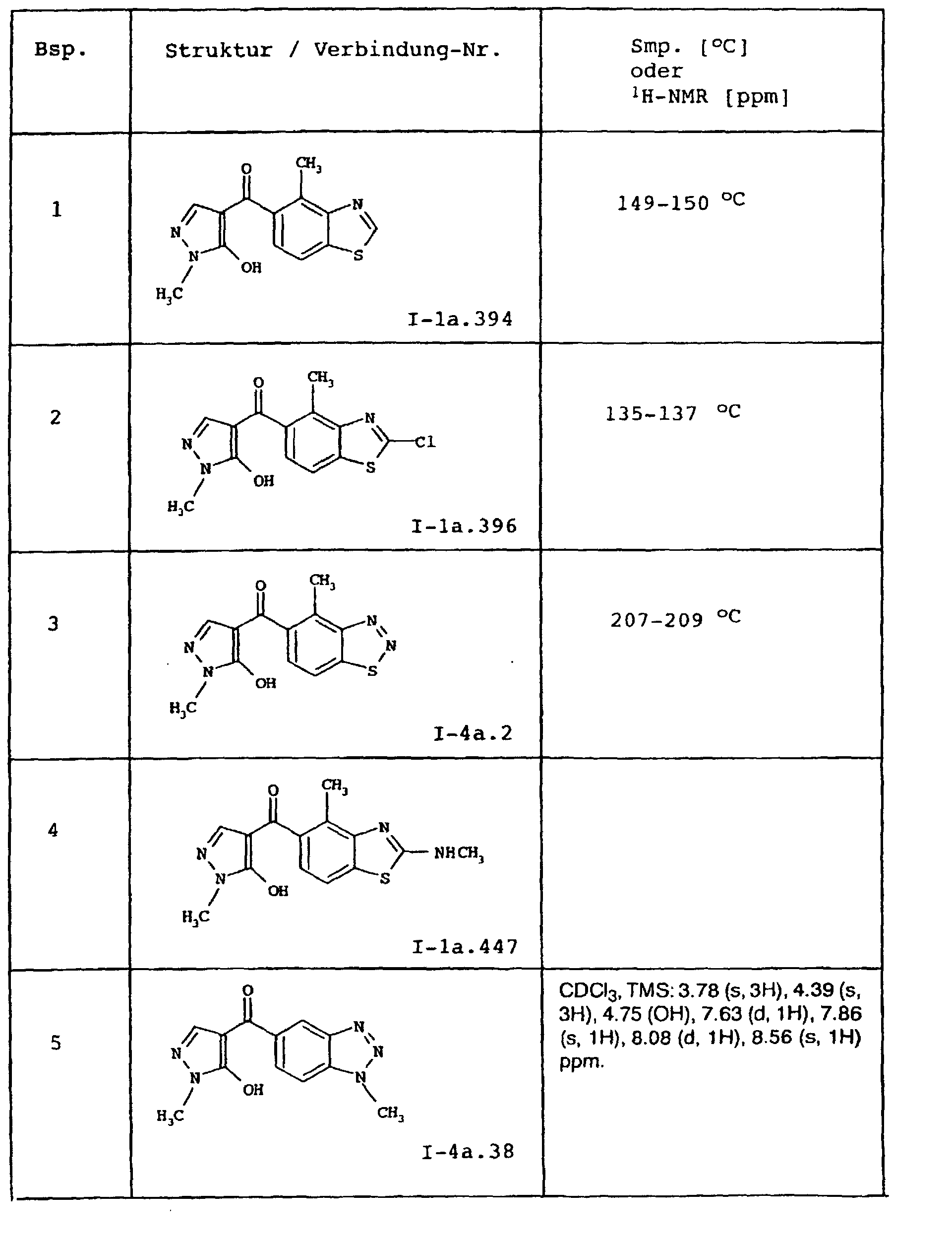

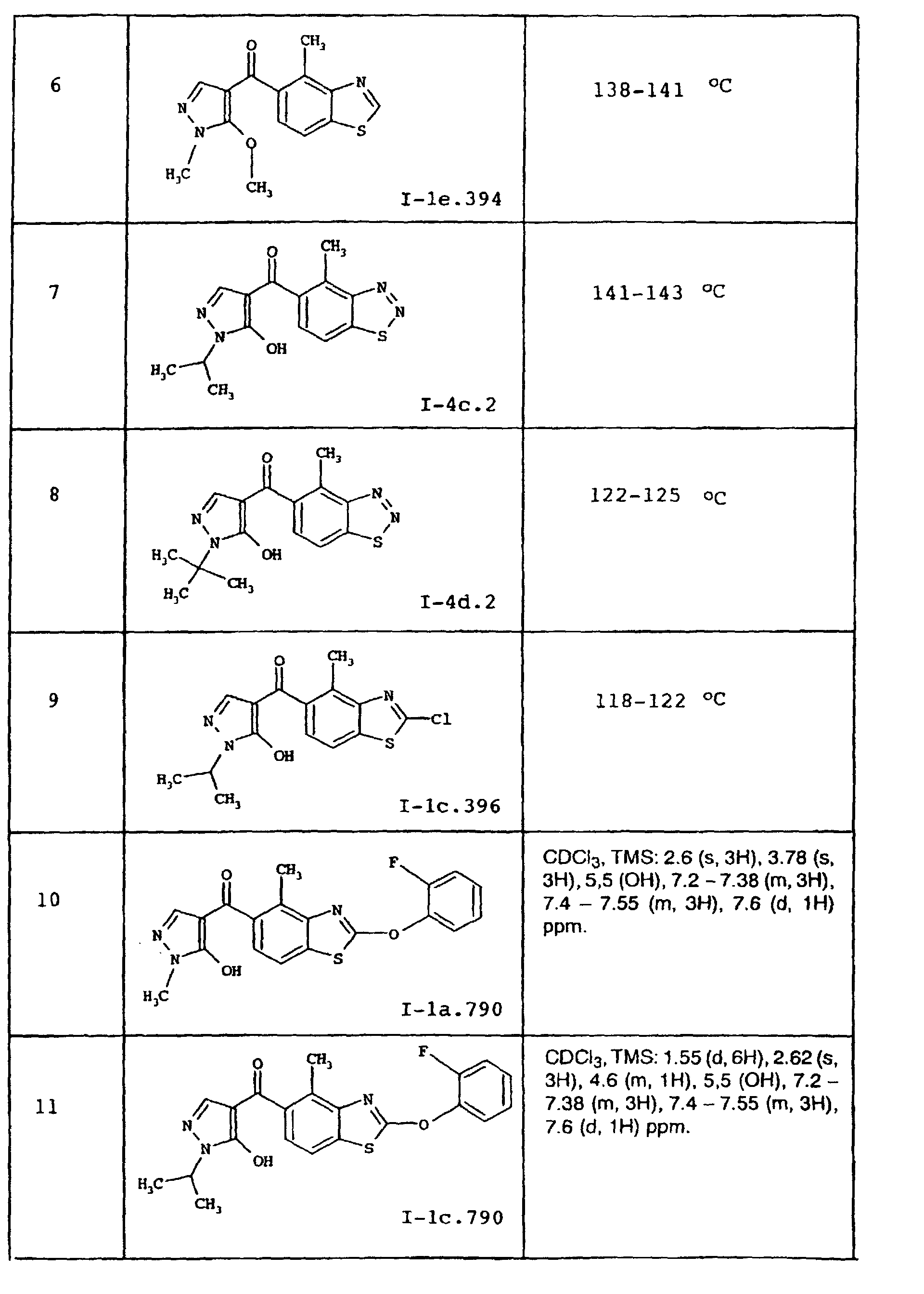

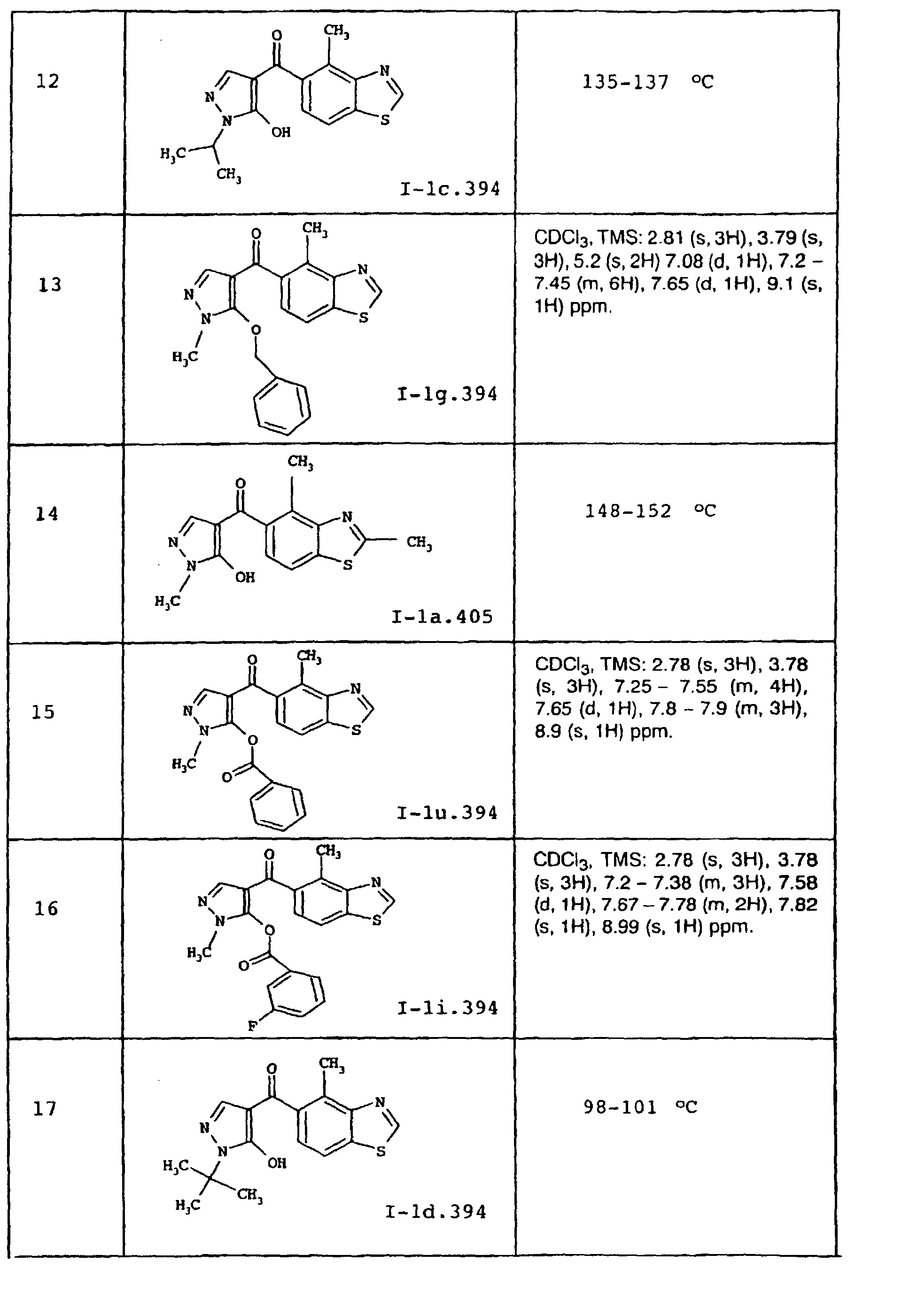

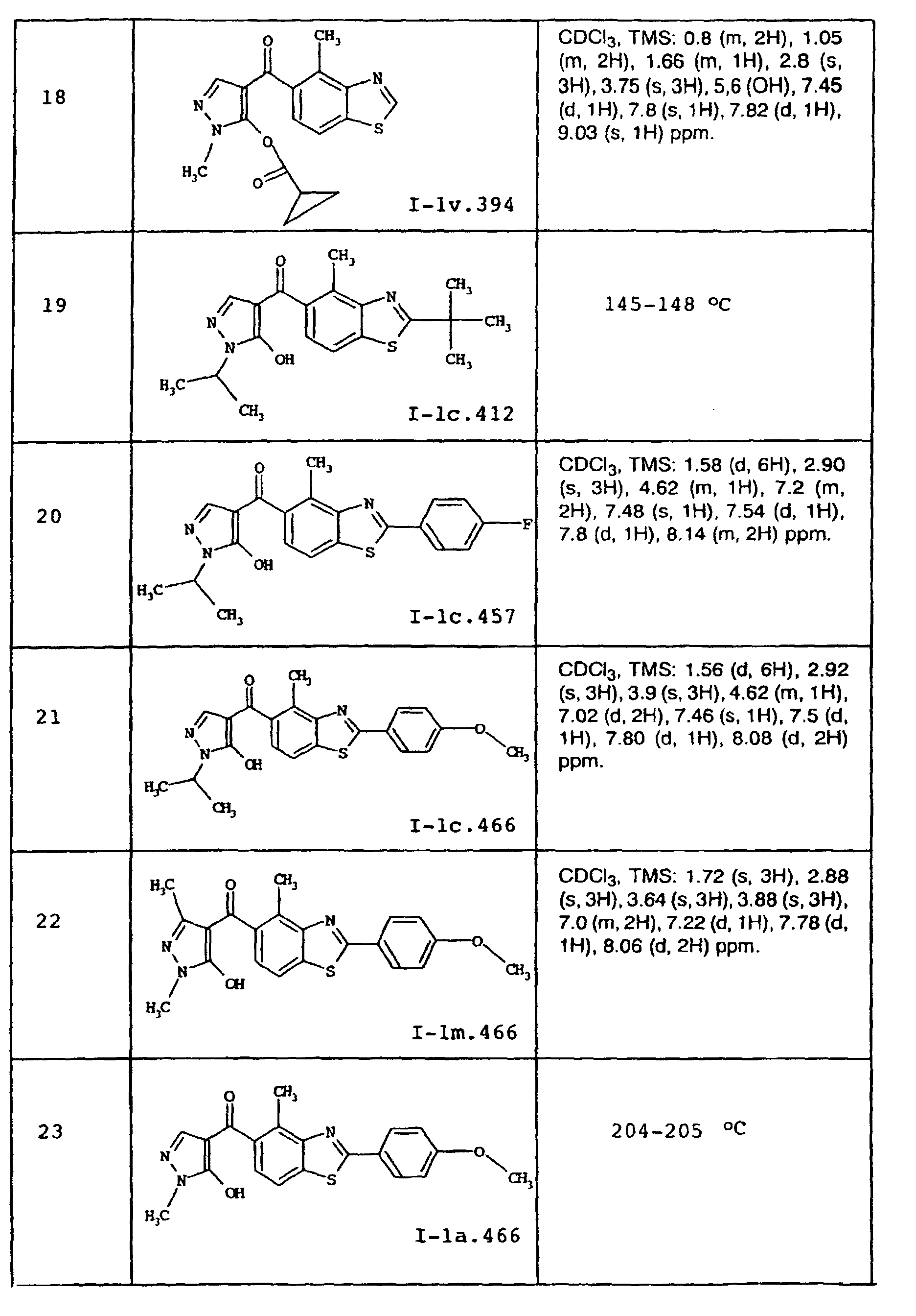

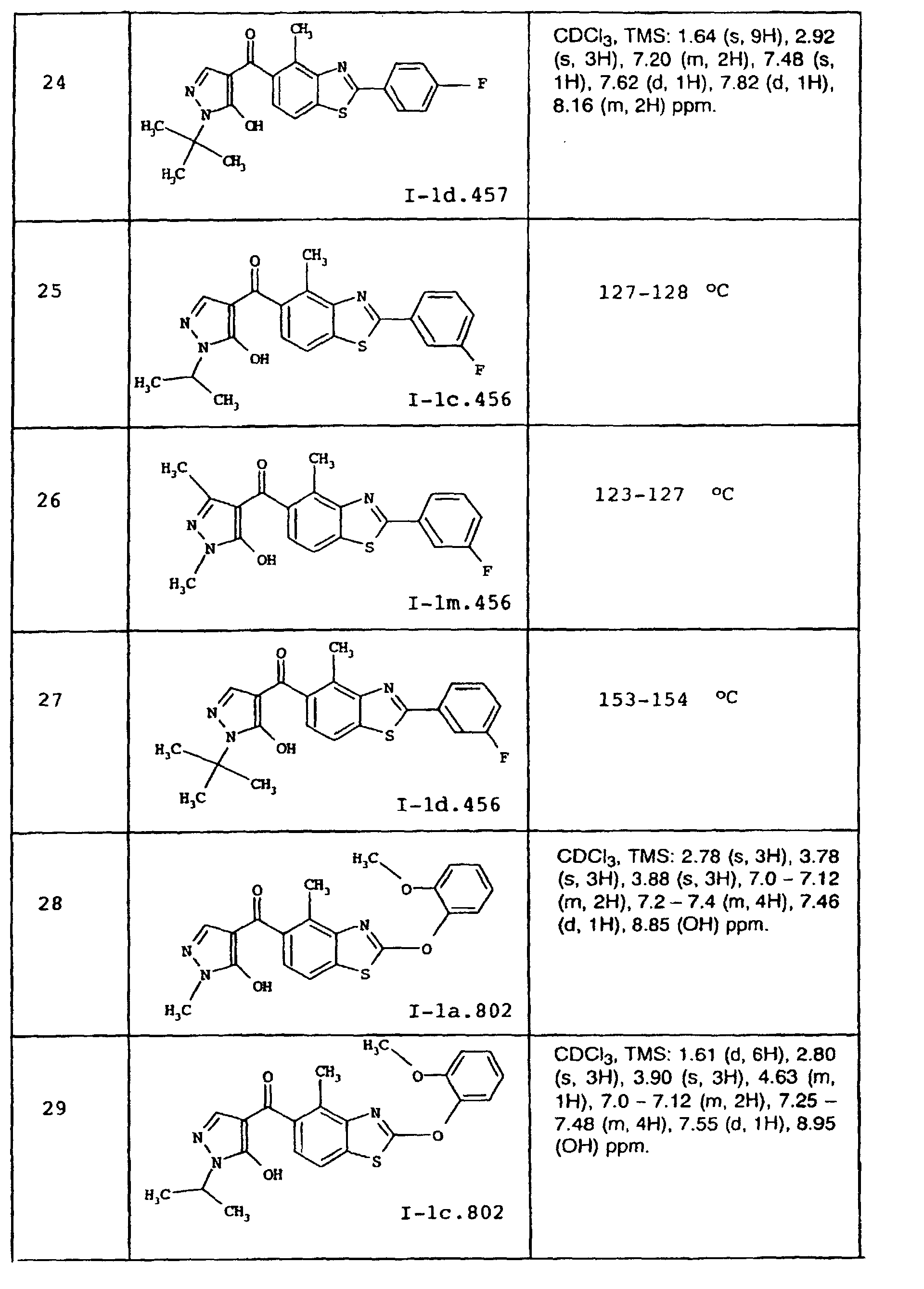

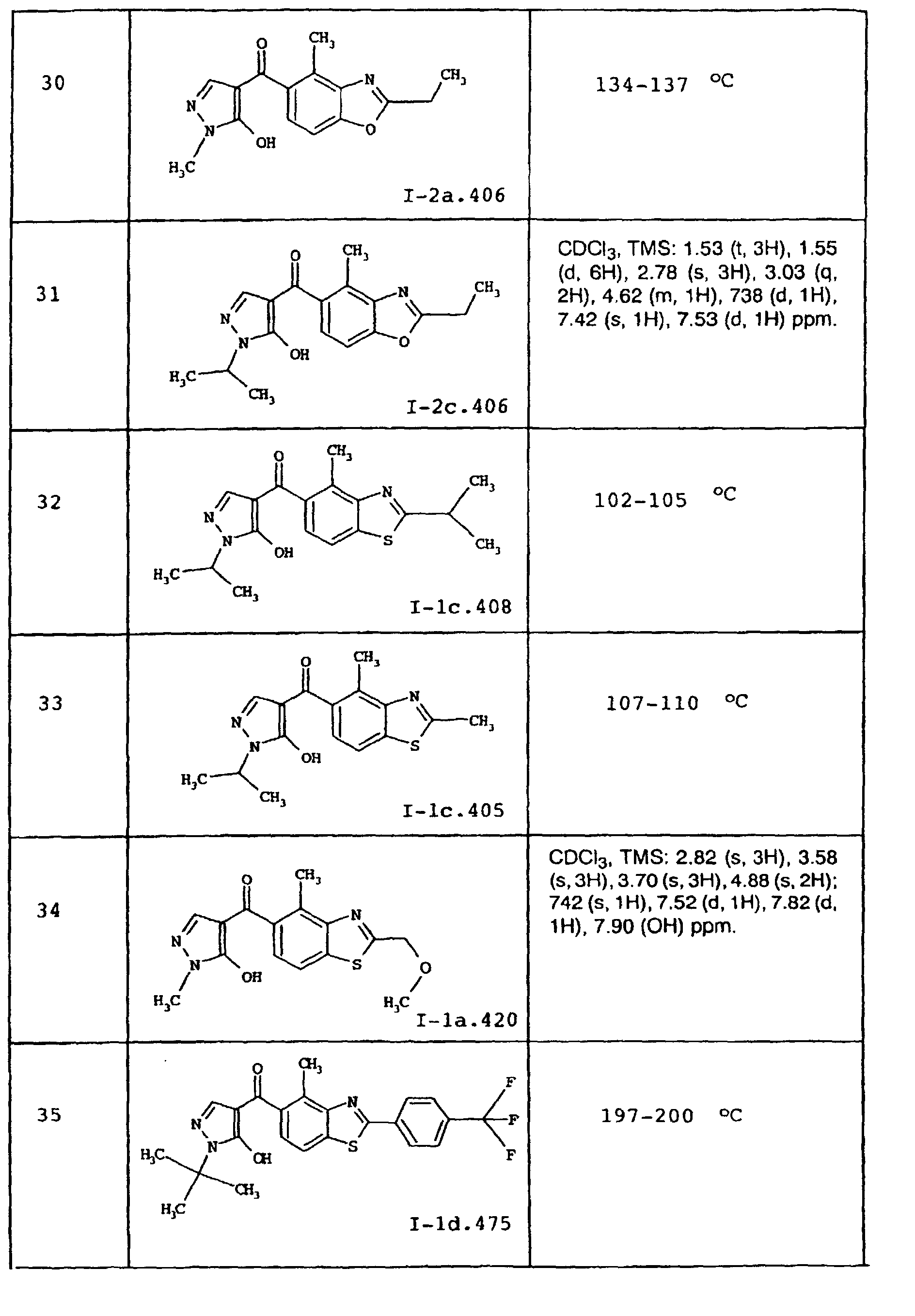

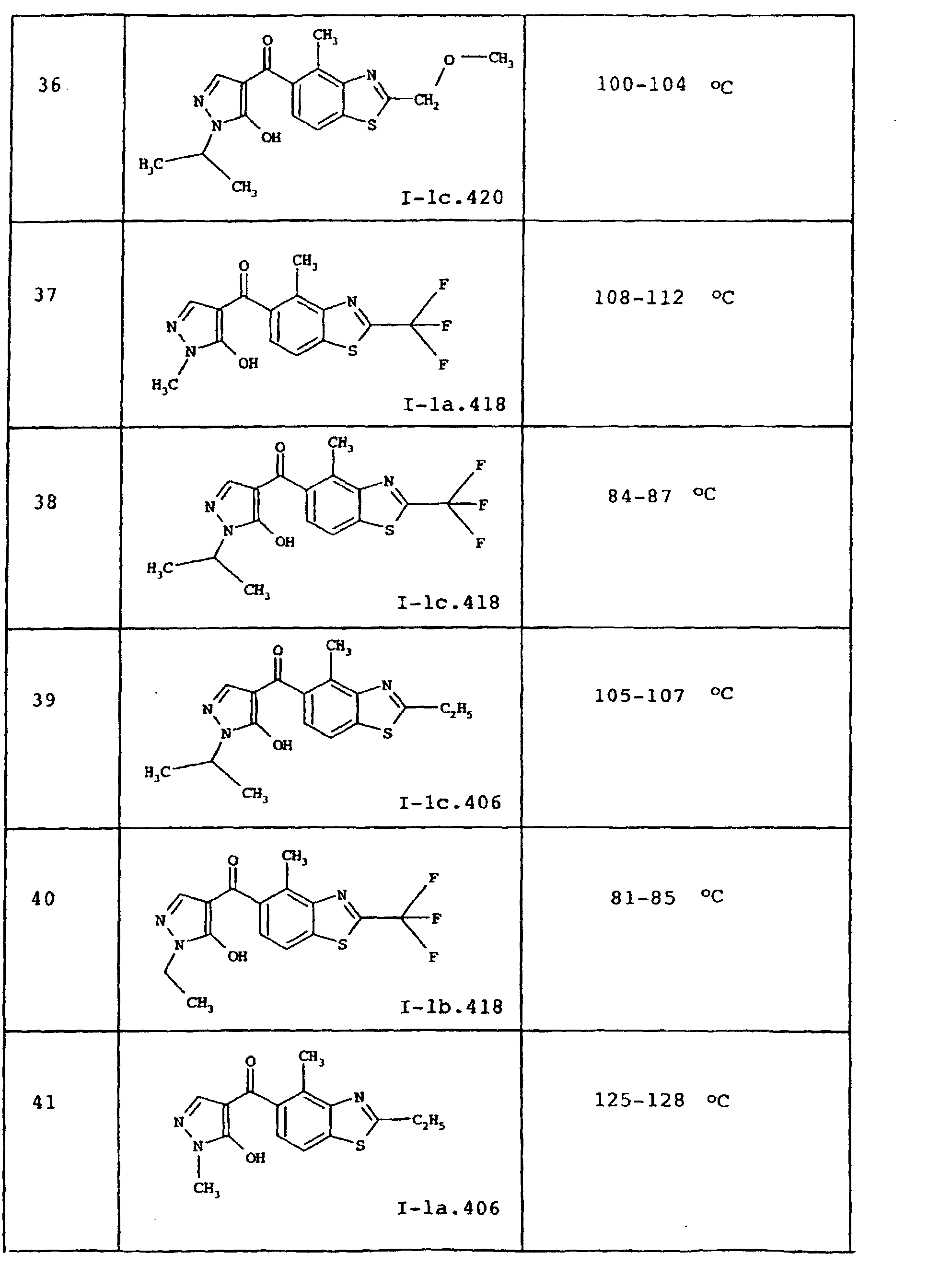

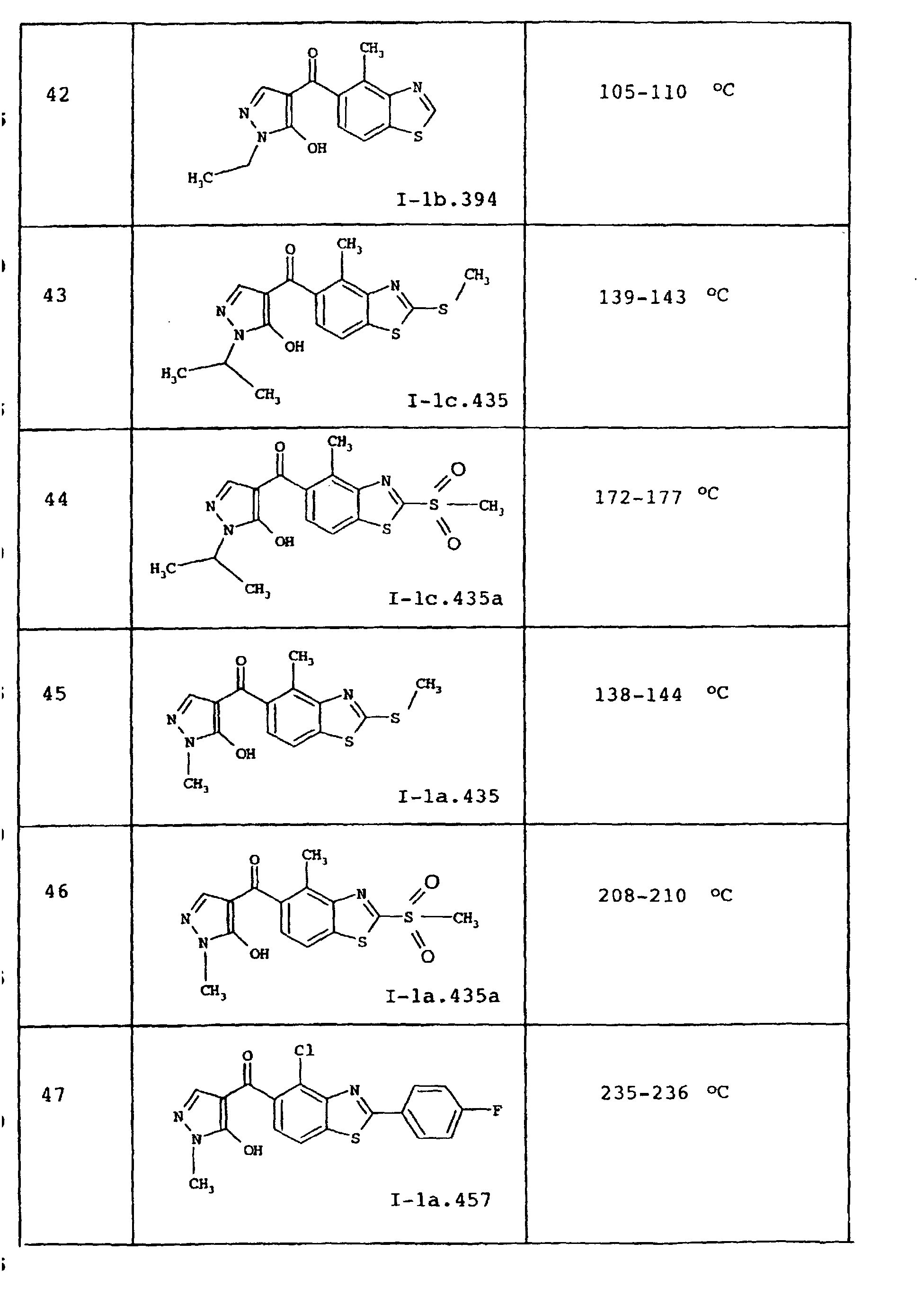

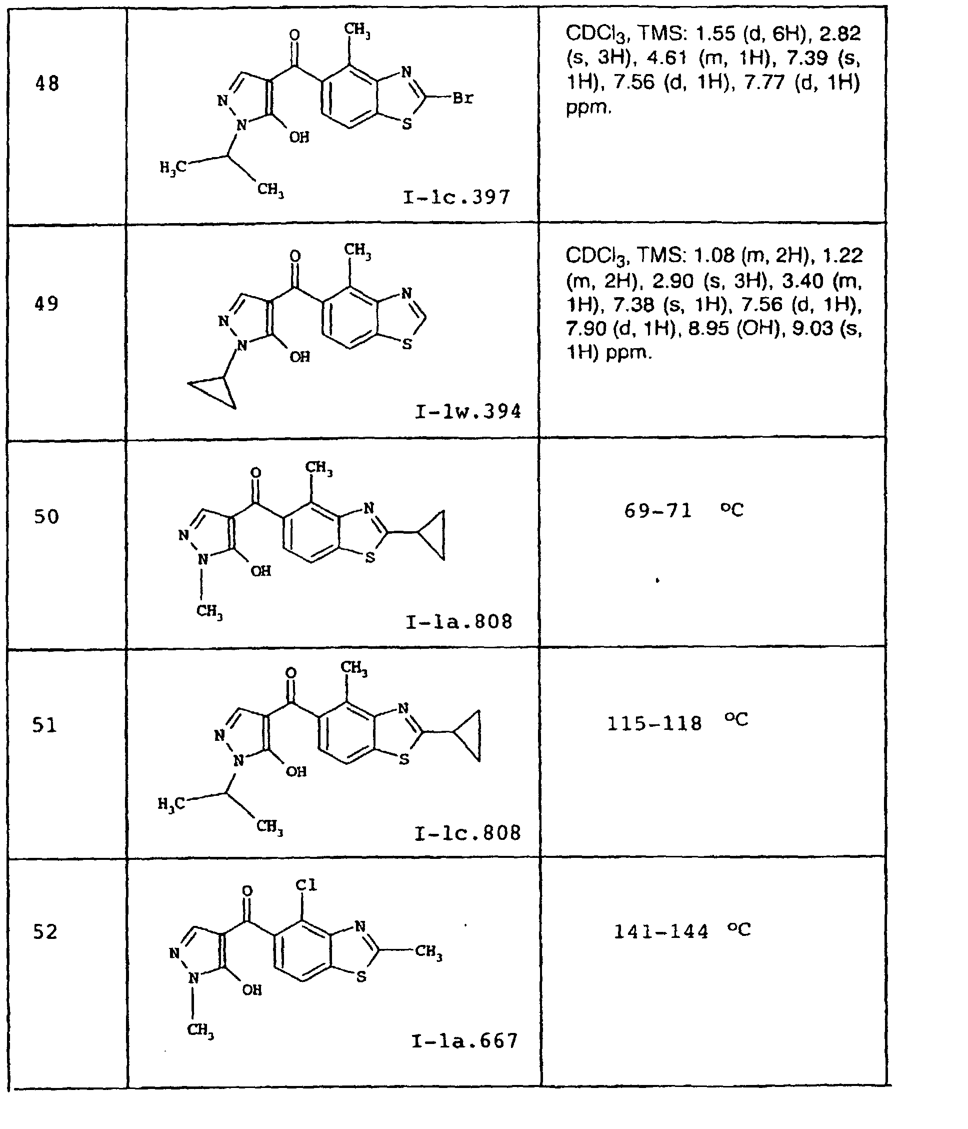

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgäre), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben. Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und in der Regel die für die Formulierung von Pflanzenschutzmitteln üblichen Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der jeweiligen Verbindung der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der jeweiligen Verbindung der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der jeweiligen Verbindung der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile der jeweiligen Verbindung der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile der jeweiligen Verbindung der Formel I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile der jeweiligen Verbindung der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der jeweiligen Verbindung der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der jeweiligen Verbindung der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0 vorzugsweise 0,01 bis 1,0 kg/ha aktive Substanz (a.S.).

Zur Verbreiterung des Wirkungsspektrums und zur Erzielung synergistischer Effekte können die erfindungsgemäßen Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyaikansäuren und deren Derivate, Benzoesäure und deren Derivate, Benzothiadiazinone, 2-Aroyl-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dion-Derivate, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der erfindungsgemäßen Verbindungen der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Bayercode | Deutscher Name | Englischer Name |
|---|---|---|
| AMARE | Zurückgekrümmter Fuchsschwanz | redroot pigweed |
| AVEFA | Flughafer | wild oats |
| CHEAL | Weißer Gänsefuß | lambsquarters (goosefoot) |
| CAPBP | Hirtentäschelkraut | shepherd's purse |
| DIGSA | Blutfingerhirse | Fingergrass ,hairy |
| ECHCG | Hühnerhirse | barnyardgrass |
| EPHHL | Wolfsmilchart | spurge |
| GASPA | Knopfkraut | smallflower |
| GALAP | Klettenlabkraut | catchweed bedstraw |
| LAMAM | Taubnessel, stengelumfassende | henbit |
| MYOAR | Vergißmeinnicht | forget-me-not |
| PAPRH | Klatschmohn | corn poppy |
| POLPE | Flohknöterich | ladysthumb |
| SETIT | Borstenhirse | foxtail |
| STEME | Vogelsternmiere | Common chickweed |
| SOLNI | Schwarzer Nachtschatten | blach nightshade |
| THLAR | Hellerkraut | fanweed |
| TRZAS | Sommer Weizen | spring wheat |

Bei Aufwandmengen von 0,5 bzw. 0,25 kg/ha (a.S.) zeigte die Verbindung 4-(4'-Methylbenzothiazol-5'-ylcarbonyl)-5-hydroxy-1-methylpyrazol (Beispiel 1) im Nachauflauf eine sehr gute herbizide Wirkung gegen die Schadpflanzen AVEFA, CHEAL, POLPE, SOLNI *und* GALAP.

Bei Aufwandmengen von 0,125 bzw. 0,0625 kg/ha zeigt die Verbindung 4-(2',4'-Dimethylbenzothiazol-5'-ylcarbonyl)-5-hydroxy-1-methylpyrazol (Beispiel 14) im Nachauflauf eine sehr gute herbizide Wirkung gegen GASPA, LAMAM, STEME, THLAR.

Bei Aufwandmengen von 0,125 bzw. 0,0625 kg/ha zeigt die Verbindung 4-(4'-Methylbenzothiazol-5'-ylcarbonyl)-5-benzyloxy-1-methylpyrazol (Beispiel 13) im Nachauflauf eine sehr gute herbizide Wirkung gegen CHEAL, EPHHL, MYOAR, PAPRH, SOLNI bei Selektivität in Weizen.

Bei Aufwandmengen von 0,25 bzw. 0,125 kg/ha zeigt die Verbindung 4-(2'-Ethyl-4'-methylbenzoxazol-5'-ylcarbonyl)-5-hydroxy-1-isopropylpyrazol (Beispiel 31) im Nachauflauf eine sehr gute herbizide Wirkung gegen AMARE, CHEAL, LAMAM, PAPRH, POLPE, THLAR.

Bei Aufwandmengen von 0,25 bzw. 0,125 kg/ha zeigt die Verbindung 4-(2',4'-Dimethylbenzothiazol-5'-ylcarbonyl)-5-hydroxy-1-isopropylpyrazol (Beispiel 33) im Nachauflauf eine sehr gute herbizide Wirkung gegen AMARE, CHEAL, LAMAM, MYOAR, PAPRH, THLAR.

Bei Aufwandmengen von 0,25 bzw. 0,125 kg/ha zeigt die Verbindung 4-(2 '-Methoxymethyl-4'-methylbenzothiazol-5 '-ylcarbonyl) -5-hydroxy-1-methylpyrazol (Beispiel 34) im Nachauflauf eine sehr gute herbizide Wirkung gegen CHEAL, LAMAM, PAPRH, STEME, THLAR.

Bei Aufwandmengen von 0,25 bzw. 0,125 kg/ha zeigt die Verbindung 4-(2'-Ethyl-4'-methylbenzoxazol-5'-ylcarbonyl)-5-hydroxy-1-methylpyrazol (Beispiel 30) im Nachauflauf eine sehr gute herbizide Wirkung gegen CHEAL, LAMAM, PAPRH, POLPE, THLAR.

Bei Aufwandmengen von 0,25 bzw. 0,125 kg/ha zeigt die Verbindung 4-(2'-Methoxymethyl-4'-methylbenzothiazol-5'-ylcarbonyl)-5-hydroxy-1-isopropylpyrazol (Beispiel 36) im Nachauflauf eine sehr gute herbizide Wirkung gegen CHEAL, EPHHL, MYOAR, PAPRH, SOLNI, STEME.

Bei Aufwandmengen von 0,25 bzw. 0,125 kg/ha zeigt die Verbindung 4-(4'-Methylbenzothiazol-5'-ylcarbonyl)-5-hydroxy-1-isopropylpyrazol (Beispiel 12) im Nachauflauf eine sehr gute herbizide Wirkung gegen CHEAL, LAMAM, PAPRH, STEME, THLAR.

Bei Aufwandmengen von 0,5 bzw. 0,25 kg/ha zeigt die Verbindung 4-(4'-Methylbenzothiazol-5'-ylcarbonyl)-5-(3''-fluorbenzoyl)-oxy-1-methylpyrazol (Beispiel 16) im Nachauflauf eine sehr gute herbizide Wirkung gegen AMARE, ECHCG, CHEAL, GALAP, POLPE.

Bei Aufwandmengen von 0,25 bzw. 0,125 kg/ha zeigt die Verbindung 4-(4'-Methylbenzothiazol-5'-ylcarbonyl)-5-benzoyloxy-1-methylpyrazol (Beispiel 15) im Nachauflauf eine sehr gute herbizide Wirkung gegen AVEFA, AMARE, ECHCG, CHEAL, POLPE.

Bei Aufwandmengen von 0,25 bzw. 0,125 kg/ha zeigt die Verbindung 4-(4'-Methylbenzothiazol-5'-ylcarbonyl)-5-cyclopropylcarbonyloxy-1-methylpyrazol (Beispiel 18) im Nachauflauf eine sehr gute herbizide Wirkung gegen AVEFA, AMARE, ECHCG, CHEAL, POLPE.

## Patentansprüche

1. Pyrazolyl-Derivate benzokondensierter, ungesättigter 5-Ring-Stickstoffheterocyclen der allgemeinen Formel 1, 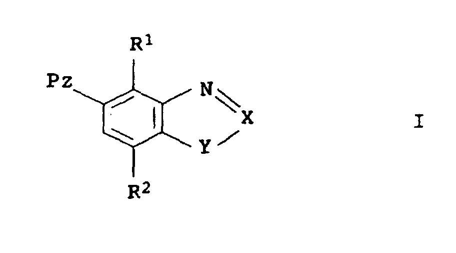 worin
X für N oder eine Gruppe C-R³ steht;
Y für O, S, SO, SO₂ oder NR⁴ steht
oder
X-Y für S=N stehen, und X Schwefel bedeutet;
R¹ Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, C₁-C₆-Alkoxy-C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₁-C₆-Alkylamino-C₁-C₆-alkyl, oder Di-(C₁-C₆-alkyl)amino-C₁-C₆-alkyl;
R² Wasserstoff, Halogen oder C₁-C₆-Alkyl;
R³ Wasserstoff, Halogen, Nitro, Cyano, Hydroxy, Amino, Mercapto, Rhodano, Hydrazid, C₁-C₆-Alkyl, C₁-C₆-Halogenalkyl, C₁-C₆-Hydroxyalkyl, C₁-C₆-Aminoalkyl, C₁-C₆-Alkoxy-C₁-C₆-alkyl, C₁-C₆-Alkylcarbonyl-C₁-C₆-alkyl, C₁-C₆-Alkoxy, C₁-C₆-Halogenalkoxy, C₁-C₆-Hydroxyalkoxy, C₁-C₆-Alkoxy-C₁-C₆-alkoxy, C₃-C₆-Cycloalkyl, C₁-C₆-Alkylamino, Di-C₁-C₆-alkylamino, C₃-C₆-Cycloalkylamino, wobei die Alkyl- und Cycloalkylgruppen der drei letztgenannten Reste teilweise oder vollständig halogeniert und/oder ein bis drei Substituenten, ausgewählt unter C₁-C₄-Alkoxy oder Hydroxy tragen können,
C₁-C₆-Alkylthio, C₁-C₆-Halogenalkylthio, C₁-C₆-Hydroxyalkylthio, C₁-C₆-Alkoxy-C₁-C₆-alkylthio, C₁-C₆-Alkylsulfonyl, C₁-C₆-Alkylsulfinyl,
Phenyl, Naphthyl, Heterocyclyl, Phenylamino, Phenoxy, Diphenylamino, wobei die Phenyl- und Heterocyclylgruppen *der* sechs letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können,
C(O)OR⁵, oder C(O)N(R⁶)R⁷; und
R⁴ Wasserstoff, C₁-C₆-Alkyl, C₁-C₆-Halogenalkyl, C₁-C₆-Hydroxyalkyl, C₁-C₆-Alkoxy-C₁-C₆-alkyl,
Phenyl, Naphthyl, wobei die zwei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können; bedeuten, wobei
R⁵ für Wasserstoff, C₁-C₆-Alkyl, C₁-C₆-Halogenalkyl, C₁-C₆-Hydroxyalkyl, C₁-C₆-Alkoxy-C₁-C₆-alkyl,
Phenyl, Naphthyl oder Heterocyclyl steht, wobei die drei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können;
R⁶, R⁷ unabhängig voneinander für Wasserstoff, C₁-C₆-Alkyl, C₁-C₆-Halogenalkyl, C₁-C₆-Hydroxyalkyl, C₁-C₆-Alkoxy-C₁-C₆-alkyl, Phenyl oder Naphthyl stehen, wobei die zwei letztgenannten Reste ihrerseits partiell oder vollständig halogeniert und/oder einen, zwei oder drei Substituenten, ausgewählt unter Nitro, Cyano, Hydroxy, C₁-C₄-Alkyl, C₁-C₄-Halogenalkyl, C₁-C₄-Alkoxy und C₁-C₄-Halogenalkoxy, tragen können;
und Pz für einen Rest der Formel IIa oder IIb steht, 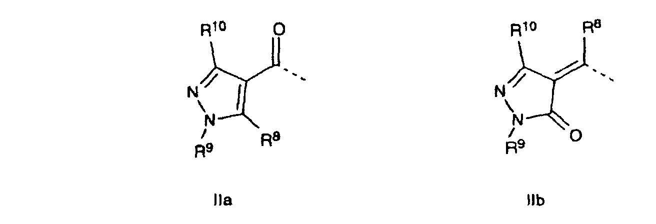 worin die Variablen R⁸, R⁹ und R¹⁰ folgende Bedeutung haben:
R⁸ Hydroxy, Mercapto, Halogen, OR¹¹, SR¹¹, SOR¹², SO₂R¹², OSO₂R¹², P(O)R¹³R¹⁴, OP(O)R¹³R¹⁴, P(S)R¹³R¹⁴, OP(S)R¹³R¹⁴, NR¹⁵R¹⁶, ONR¹⁵R¹⁶ oder N-gebundenes Heterocyclyl, das partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
R⁹ Wasserstoff, C₁-C₆-Alkyl, C₁-C₆-Halogenalkyl, C₃-C₆-Cycloalkyl, Hydroxy, C₁-C₆-Alkoxy oder C₁-C₆-Halogenalkoxy;
R¹⁰ Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆ -Halogenalkylthio; wobei
R¹¹ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, C₂-C₆ -Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆ Alkylaminocarbonyl, C₃-C₆-Alkenylaminocarbonyl, C₃-C₆-Alkinylaminocarbonyl, N, N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl oder C₁-C₆-Alkoxyimino-C₁-C₆-alkyl, wobei die genannten Alkyl-, Cycloalkyl- und Alkoxyreste partiell oder vollständig halogeniert sein können und/oder eine bis drei der folgenden Gruppen tragen können: cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Phenyl-C₁-C₆-alkyl, Phenylcarbonyl-C₁-C₆-alkyl, Phenylcarbonyl, Phenoxycarbonyl, Phenyloxythiocarbonyl, Phenylaminocarbonyl, N-(C₁-C₆-Alkyl)-N-(phenyl)-aminocarbonyl, Phenyl-C₂-C₆-alkenylcarbonyl, Heterocyclyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, Heterocyclylaminocarbonyl, N-( C₁-C₆-Alkyl)-N-(heterocyclyl)-aminocarbonyl, oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
R¹² C₁-C₆-Alkyl, C₃-C₆-Alkenyl, C₃-C₆-Alkinyl oder C₃-C₆-Cycloalkyl, wobei die vier genannten Reste partiell oder vollständig halogeniert sein können und/oder eine, zwei oder drei der folgenden Gruppen tragen können: Cyano, C₁-C₄-Alkoxy, C₁-C₄-Halogenalkoxy, C₁-C₄-Alkylthio, C₁-C₄-Halogenalkylthio, C₁-C₄-Alkylcarbonyl, C₁-C₄-Alkoxycarbonyl oder C₁-C₄-Halogenalkoxycarbonyl;
Phenyl, Phenyl-C₁-C₆-alkyl, Heterocyclyl oder Heterocyclyl-C₁-C₆-alkyl, wobei der Phenyl- und der Heterocyclyl-Rest der letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
R¹³, R¹⁴ unabhängig voneinander Wasserstoff, Hydroxy, C₁-C₆-Alkyl, C₁-C₆-Alkoxy, C₁-C₆-Alkylthio, Phenyl, Phenyl-C₁-C₄-alkyl oder Phenoxy, wobei die drei letztgenannten Substituenten partiell oder vollständig halogeniert sein können und/oder einen, zwei oder drei der folgenden Reste tragen können: Nitro, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl;
R¹⁵ Wasserstoff, C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₆-Alkylcarbonyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-alkyl)-amino oder C₁-C₆-Alkylcarbonylamino, wobei die genannten Alkyl-, Cycloalkyl- und Alkoxyreste partiell oder vollständig halogeniert sein können und/oder einen, zwei oder drei der folgenden Reste tragen können: Cyano, C₁-C₄-Alkoxycarbonyl, C₁-C₄-Alkylaminocarbonyl, Di-(C₁-C₄-alkyl)-aminocarbonyl oder C₃-C₆-Cycloalkyl;
Phenyl, Phenyl-C₁-C₄-alkyl, Phenylcarbonyl, Heterocyclyl, Heterocyclyl-C₁-C₄-alkyl oder Heterocyclylcarbonyl, wobei der Phenyl- oder Heterocyclyl-Rest der sechs letztgenannten Substituenten partiell oder vollständig halogeniert sein kann und/oder einen, zwei oder drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy; und
R¹⁶ Wasserstoff, C₁-C₆-Alkyl oder C₃-C₆-Alkenyl, C₃-C₆-Alkinyl; bedeuten;
sowie deren landwirtschaftlichen brauchbaren Salze.

2. Pyrazolderivate gemäß Anspruch 1, worin X in Formel I für C-R³ steht, wobei
R³ für Wasserstoff, Halogen, Cyano, Rhodano, C₁-C₆-Alkyl, C₁-C₆-Halogenalkyl, C₁-C₆-Alkoxy, C₁-C₆-Alkoxy-C₁-C₆-alkyl, C₃-C₆-Cycloalkyl, C₁-C₆-Halogenalkoxy, C₁-C₆-Alkylthio, C₁-C₆-Halogenalkylthio,
Phenyl, Phenoxy oder Pyridyl, wobei die drei letztgenannten Reste teilweise oder vollständig halogeniert sein können und/oder einen der folgenden Reste: C₁-C₄-Alkoxy, C₁-C₄-Halogenalkyl, und C₁-C₄-Halogenalkoxy, tragen können;
oder
COOR⁵ mit den für R⁵ in Anspruch 1 angegebenen Bedeutungen steht.

3. Pyrazolderivate gemäß Anspruch 1 oder 2, worin X in Formel I für C-R³ steht und Y ausgewählt ist unter S, SO und SO₂.

4. Pyrazolderivate gemäß einem der Ansprüche 1 oder 2, worin Y in Formel I für N-R⁴ mit der für R⁴ in Anspruch 1 angegebenen Bedeutung steht und X für C-R³ mit den in Anspruch 1 oder 2 für R³ angegebenen Bedeutungen steht.

5. Pyrazolderivate gemäß Anspruch 1, worin X für N steht und Y ausgewählt ist unter S, SO, SO₂ oder N-R⁴.

6. Pyrazolderivate gemäß einem der Ansprüche 1 bis 5, worin Pz in Formel I für einen Rest der Formel IIa steht, worin R⁸ ausgewählt ist unter Hydroxy, OR¹¹ und OSO₂R¹² mit den für R¹¹ und R¹² in Anspruch 1 angegebenen Bedeutungen, wobei R⁹ und R¹⁰ die in Anspruch 1 angegebenen Bedeutungen haben.

7. Pyrazolderivate gemäß Anspruch 6, wobei in Formel IIa
R⁸ für Hydroxy, C₁-C₄-Alkyloxy, O-CH₂-Phenyl, Phenylcarbonyloxy, 2-, 3- oder 4-Fluorphenylcarbonyloxy, Cyclopropylcarbonyloxy, C₁-C₄-Sulfonyloxy, Phenylsulfonyloxy und 2-, 3- oder 4-Methylphenylsulfonyloxy;
R⁹ für C₁-C₄-Alkyl oder Cyclopropyl und
R¹⁰ für Wasserstoff oder C₁-C₄-Alkyl stehen.

8. Verfahren zur Herstellung von Verbindungen der Formel I mit R⁸ = Hydroxy, gemäß Anspruch 1, **dadurch gekennzeichnet, daß** man ein 5-Hydroxypyrazol der Formel III, 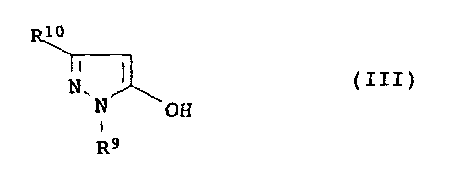 wobei die Variablen R⁹ und R¹⁰ die in Anspruch 1 genannte Bedeutung haben, mit einer aktivierten Carbonsäure IVa oder einer Carbonsäure IVb 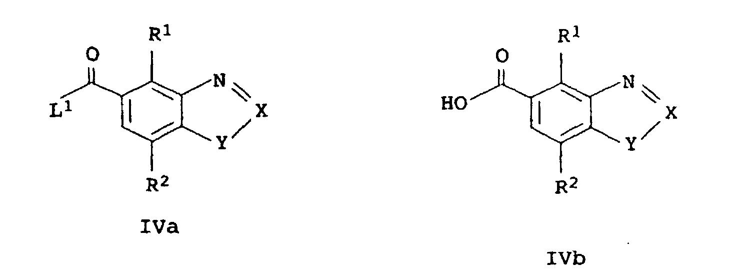 wobei die Variablen X, Y, R¹ und R² die unter Anspruch genannte Bedeutung haben und L¹ für eine nukleophil verdrängbare Abgangsgruppe steht, acyliert und das Acylierungsprodukt gegebenenfalls in Gegenwart eines Katalysators zu den Verbindungen I mit R⁸ = Hydroxy umlagert.

9. Verfahren zur Herstellung von Verbindungen der Formel I gemäß Anspruch 1 mit R⁸ = Halogen, **dadurch gekennzeichnet, dass** man ein Pyrazol-Derivat der Formel I, mit R⁸ = Hydroxy mit einem Halogenierungsmittel umsetzt.

10. Verfahren zur Herstellung von Verbindungen der Formel I gemäß Anspruch 1 mit R⁸ = OR¹¹, OSO₂R¹², OP(O)R¹³R¹⁴ oder OP(S)R¹³R¹⁴ **dadurch gekennzeichnet, dass** man ein Pyrazol-Derivat der Formel I mit R⁸ = Hydroxy mit einem Alkylierungsmittel Va, Sulfonylierungsmittel Vβ oder Phosphonylierungsmittel Vγ bzw. Vδ, 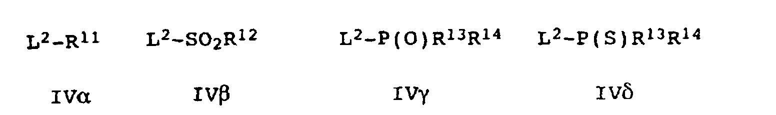 wobei die Variablen R¹¹ bis R¹⁴ die in Anspruch 1 genannte Bedeutung haben und L² für eine nukleophil verdrängbare Abgangsgruppe steht, umsetzt.

11. Verfahren zur Herstellung von Verbindungen der Formel I gemäß Anspruch 1 mit R⁸ = OR¹¹, SR¹¹, P(O)R¹³R¹⁴, NR¹⁵R¹⁶, ONR¹⁵R¹⁶ oder N-gebundenes Heterocyclyl, **dadurch gekennzeichnet, daß** man ein Pyrazol-Derivat der Formel I mit R⁸ = Halogen oder OSO₂R¹² mit einer Verbindung der Formel VIa, VIß, VIy, VIδ, VIε oder VIη 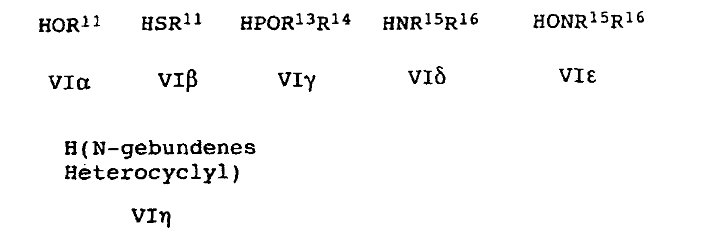 wobei die Variablen R⁸, R¹¹ bis R¹⁶ die in Anspruch 1 genannte Bedeutung haben, gegebenenfalls in Gegenwart einer Base, umsetzt.

12. Verfahren zur Herstellung von Verbindungen der Formel I, worin P_{z} = IIa gemäß Anspruch 1 ist, **dadurch gekennzeichnet, dass** man ein metalliertes Pyrazol-Derivat der Formel VII, worin M für ein Metall steht und R⁸ bis R¹⁰ die in Anspruch 1 genannte Bedeutung haben, mit einem Carbonsäure-Derivat der Formel IVa, worin R¹, R², X und Y die in Anspruch 1 genannte Bedeutung haben und L¹ für eine nucleophil verdrängbare Abgangsgruppe steht, umsetzt.

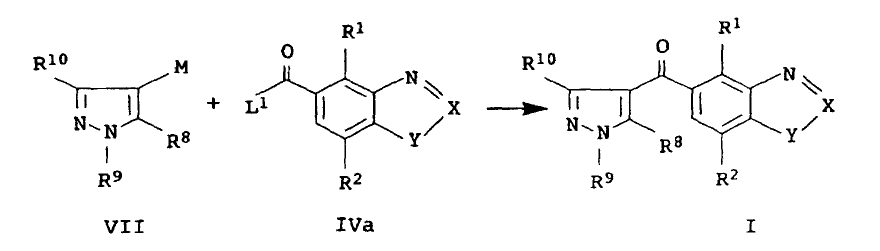

13. Mittel, enthaltend mindestens ein Pyrazol-Derivat der Formel I oder ein landwirtschaftlich brauchbares Salz von I gemäß einem der Ansprüche 1 bis 7, und übliche Hilfsmittel.

14. Verfahren zur Bekämpfung von unerwünschtem Pflanzenwuchs, **dadurch gekennzeichnet, daß** man eine herbizid wirksame Menge mindestens eines Pyrazol-Derivates der Formel I oder eines landwirtschaftlich brauchbaren Salzes von I gemäß einem der Ansprüche 1 bis 7, auf Pflanzen, deren Lebensraum und/oder auf Samen einwirken läßt.

15. Verwendung von Pyrazol-Derivaten der Formel I oder deren landwirtschaftlich brauchbaren Salzen gemäß einem der Ansprüche 1 bis 7 als Herbizide.

## Claims

1. A pyrazolyl derivative of benzo-fused unsaturated 5-membered nitrogen heterocycles of the formula I, 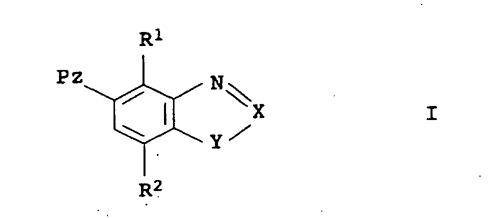 where
X is N or a group C-R³;
Y is O, S, SO, SO₂ or NR⁴
or
X-Y is S=N, and X is sulfur;
R¹ is hydrogen, nitro, halo, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, aminosulfonyl, C₁-C₆-alkoxy-C₁-C₆-alkyl, C₁-C₆-alkylthio-C₁-C₆-alkyl, C₁-C₆-alkylsulfinyl-C₁-C₆-alkyl, C₁-C₆-alkylsulfonyl-C₁-C₆-alkyl, C₁-C₆-alkylamino-C₁-C₆-alkyl, or di (C₁-C₆-alkyl) amino-C₁-C₆-alkyl;
R² is hydrogen, halogen or C₁-C₆-alkyl;
R³ is hydrogen, halogen, nitro, cyano, hydroxyl, amino, mercapto, thiocyanato, hydrazide, C₁-C₆-alkyl,: C₁-C₆-haloalkyl, C₁-C₆-hydroxyalkyl, C₁-C₆-aminoalkyl, C₁-C₆-alkoxy-C₁-C₆-alkyl, C₁-C₆-alkylcarbonyl-C₁-C₆-alkyl, C₁-C₆-alkoxy, C₁-C₆-haloalkoxy, C₁-C₆-hydroxyalkoxy, C₁-C₆-alkoxy-C₁-C₆-alkoxy, C₃-C₆-cycloalkyl,
is C₁-C₆-alkylamino, di-C₁-C₆-alkylamino, C₃-C₆-cycloalkylamino, where the alkyl and cycloalkyl groups of the three last-mentioned radicals may be partially or fully halogenated and/or may carry one to three substituents selected from the group consisting of C₁-C₄-alkoxy and hydroxyl,
is C₁-C₆-alkylthio, C₁-C₆-haloalkylthio, C₁-C₆-hydroxyalkylthio, C₁-C₆-alkoxy-C₁-C₆-alkylthio, C₁-C₆-alkylsulfonyl, C₁-C₆-alkylsulfinyl,
is phenyl, naphthyl, heterocyclyl, phenylamino, phenoxy, diphenylamino, where the phenyl and heterocyclyl groups of the six last-mentioned radicals for their part may be partially or fully halogenated and/or may carry one, two or three substituents selected from the group consisting of nitro, cyano, hydroxyl, C₁-C₄-alkyl, C₁-C₄-haloalkyl, C₁-C₄-alkoxy and C₁-C₄-haloalkoxy,
is C(O)OR⁵, or C(O)N(R⁶)R⁷; and
R⁴ is hydrogen, C₁-C₆-alkyl, C₁-C₆-haloalkyl, C₁-C₆-hydroxyalkyl, C₁-C₆-alkoxy-C₁-C₆-alkyl,
is phenyl, naphthyl, where the two last-mentioned radicals for their part may be partially or fully halogenated and/or may carry one, two or three substituents selected from the group consisting of nitro, cyano, hydroxyl, C₁-C₄-alkyl, C₁-C₄-haloalkyl, C₁-C₄-alkoxy and C₁-C₄-haloalkoxy; where
R⁵ is hydrogen, C₁-C₆-alkyl, C₁-C₆-haloalkyl, C₁-C₆-hydroxyalkyl, C₁-C₆-alkoxy-C₁-C₆-alkyl,
is phenyl, naphthyl or heterocyclyl, where the three last-mentioned radicals for their part may be partially or fully halogenated and/or may carry one, two-or three substituents selected from the group consisting of nitro cyano, hydroxyl, C₁-C₄-alkyl, C₁-C₄-haloalkyl, C₁-C₄-alkoxy and C₁-C₄-haloalkoxy;
R⁶, R⁷ independently of one another are hydrogen, C₁-C₆-alkyl, C₁-C₆-haloalkyl, C₁-C₆-hydroxyalkyl, C₁-C₆-alkoxy-C₁-C₆-alkyl,
are phenyl or naphthyl, where the two last-mentioned radicals for their part may be partially or fully halogenated and/or may carry one, two or three substituents selected from the group consisting of nitro, cyano, hydroxyl, C₁-C₄-alkyl, C₁-C₄-haloalkyl, C₁-C₄-alkoxy and C₁-C₄-haloalkoxy;
and Pz is a radical of the formula IIa or IIb, 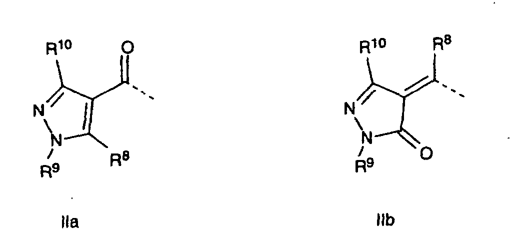 where the variables R⁸, R⁹ and R¹⁰ are as defined below:
R8 is hydroxyl, mercapto, halogen, OR¹¹, SR¹¹, SOR¹², SO₂R¹², OS0₂R¹², P(O)R¹³R¹⁴, OP(O)R¹³R¹⁴, P(S)R¹³R¹⁴, OP(S)R¹³R¹⁴, NR¹⁵R¹⁶, ONR¹⁵R¹⁶ or N-bonded heterocyclyl, which may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy;
R⁹ is hydrogen, C₁-C₆-alkyl, C₁-C₆-haloalkyl, C₃-C₆-cycloalkyl, hydroxyl, C₁-C₆-alkoxy or C₁-C₆-haloalkoxy;
R¹⁰ is hydrogen, halogen, C₁-C₆-alkyl, C₁-C₆-haloalkyl, hydroxyl, C₁-C₆-alkoxy, C₁-C₆-haloalkoxy, C₁-C₆-alkylthio or C₁-C₆-haloalkylthio; where
R¹¹ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, C₁-C₆-alkylcarbonyl, C₂-C₆-alkenylcarbonyl, C₂-C₆-alkynylcarbonyl, C₃-C₆-cycloalkylcarbonyl, C₁-C₆-alkoxycarbonyl, C₃-C₆-alkenyloxycarbonyl, C₃-C₆-alkynyloxycarbonyl, C₁-C₆-alkylthiocarbonyl, C₁-C₆-alkylaminocarbonyl, C₃-C₆-alkenylaminocarbonyl, C₃-C₆-alkynylaminocarbonyl, N,N-di(C₁-C₆-alkyl)aminocarbonyl, N-(C₃-C₆-alkenyl)-N-(C₁-C₆-alkyl)aminocarbonyl, N- (C₃-C₆-alkynyl) -N- (C₁-C₆-alkyl) aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N- (C₃-C₆-alkynyl) -N- (C₁-C₆-alkoxy) aminocarbonyl, di(C₁-C₆-alkyl)aminothiocarbonyl or C₁-C₆-alkoxyimino-C₁-C₆-alkyl, where the alkyl, cycloalkyl and alkoxy radicals mentioned may be partially or fully halogenated and/or may carry one to three of the following groups: cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, di(C₁-C₄-alkyl)aminocarbonyl, aminocarbonyl, C₁-C₄-alkylcarbonyloxy or C₃-C₆-cycloalkyl;
is phenyl, phenyl-C₁-C₆-alkyl, phenylcarbonyl-C₁-C₆-alkyl, phenylcarbonyl, phenoxycarbonyl, phenyloxythiocarbonyl, phenylaminocarbonyl, N-(C₁-C₆-alkyl) -N-(phenyl)aminocarbonyl, phenyl-C₂-C₆-alkenylcarbonyl, heterocyclyl, heterocyclyl-C₁-C₆-alkyl, heterocyclylcarbonyl-C₁-C₆-alkyl, heterocyclylcarbonyl, heterocyclyloxycarbonyl, heterocyclyloxythiocarbonyl, heterocyclylaminocarbonyl, N-(C₁-C₆-alkyl) -N-(heterocyclyl)aminocarbonyl, or heterocyclyl-C₂-C₆-alkenylcarbonyl, where the phenyl and the heterocyclyl radical of the 18 last-mentioned substituents may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy;
R¹² is C₁-C₆-alkyl, C₃-C₆-alkenyl, C₃-C₆-alkynyl or C₃-C₆-cycloalkyl, where the four radicals mentioned may be partially or fully halogenated and/or may carry one, two or three of the following groups: cyano, C₁-C₄-alkoxy, C₁-C₄-haloalkoxy, C₁-C₄-alkylthio, C₁-C₄-haloalkylthio, C₁-C₄-alkylcarbonyl, C₁-C₄-alkoxycarbonyl or C₁-C₄-haloalkoxycarbonyl;
is phenyl, phenyl-C₁-C₆-alkyl, heterocyclyl or heterocyclyl-C₁-C₆-alkyl, where the phenyl and the heterocyclyl radical of the last-mentioned substituents may be partially or fully halogenated and/or may carry one, two or three of the following radicals: nitro, cyano, C₁-C₄-alkyl, C₁-C₄-haloalkyl, C₁-C₄-alkoxy, C₁-C₄-haloalkoxy or C₁-C₄-alkoxycarbonyl;
R¹³, R¹⁴ independently of one another are hydrogen, hydroxyl, C₁-C₆-alkyl, C₁-C₆-alkoxy, C₁-C₆-alkylthio, phenyl, phenyl-C₁-C₄-alkyl or phenoxy, where the three last-mentioned substituents may be partially or fully halogenated and/or may carry one, two or three of the following radicals: nitro, cyano, C₁-C₄-alkyl, C₁-C₄-haloalkyl, C₁-C₄-alkoxy, C₁-C₄-haloalkoxy or C₁-C₄-alkoxycarbonyl;
R¹⁵ is hydrogen, C₁-C₆-alkyl, C₃-C₆-alkenyl, C₃-C₆-haloalkenyl, C₃-C₆-alkynyl, C₃-C₆-haloalkynyl, C₃-C₆-cycloalkyl, C₁-C₆-alkylcarbonyl, hydroxyl, C₁-C₆-alkoxy, C₃-C₆-alkenyloxy, C₃-C₆-alkynyloxy, amino, C₁-C₆-alkylamino, di(C₁-C₆-alkyl)amino or C₁-C₆-alkylcarbonylamino, where the alkyl, cycloalkyl and alkoxy radicals mentioned may be partially or fully halogenated and/or may carry one, two or three of the following radicals: cyano, C₁-C₄-alkoxycarbonyl, C₁-C₄-alkylaminocarbonyl, di(C₁-C₄-alkyl)aminocarbonyl or C₃-C₆-cycloalkyl;
is phenyl, phenyl-C₁-C₄-alkyl, phenylcarbonyl, heterocyclyl, heterocyclyl-C₁-C₄-alkyl or heterocyclylcarbonyl, where the phenyl or heterocyclyl radical of the six last-mentioned substituents may be partially or fully halogenated and/or may carry one, two or three of the following radicals: nitro, cyano, C₁-C₄-alkyl, C₁-C₄-haloalkyl, C₁-C₄-alkoxy or C₁-C₄-haloalkoxy; and
R¹⁶ is hydrogen, C₁-C₆-alkyl or C₃-C₆-alkenyl, C₃-C₆-alkynyl;
and its agriculturally useful salts.

2. A pyrazole derivative as claimed in claim 1 where X in the formula I is C-R³, where
R³ is hydrogen, halogen, cyano, thiocyanato, C₁-C₆-alkyl, C₁-C₆-haloalkyl, C₁-C₆-alkoxy, C₁-C₆-alkoxy-C₁-C₆-alkyl, C₃-C₆-cycloalkyl, C₁-C₆-haloalkoxy, C₁-C₆-alkylthio, C₁-C₆-haloalkylthio,
is phenyl, phenoxy or pyridyl, where the three last-mentioned radicals may be partially or fully halogenated and/or may carry one of the following radicals: C₁-C₄-alkoxy, C₁-C₄-haloalkyl, and C₁-C₄-haloalkoxy;
or is COOR⁵ where R⁵ is as defined in claim 1.

3. A pyrazole derivative as claimed in claim 1 or 2 where X in the formula I is C-R³ and Y is selected from the group consisting of S, SO and SO₂.

4. A pyrazole derivative as claimed in claim 1 or 2, where Y in the formula I is N-R⁴, where R⁴ is as defined in claim 1, and X is C-R³, where R³ is as defined in claim 1 or 2.

5. A pyrazole derivative as claimed in claim 1 where X is N and Y is selected from the group consisting of S, SO, SO₂ and N-R⁴.

6. A pyrazole derivative as claimed in any of claims 1 to 5, where Pz in the formula I is a radical of the formula IIa, where R⁸ is selected from the group consisting of hydroxyl, OR¹¹ and OS0₂R¹², where R¹¹ and R¹² are as defined in claim 1_{,} R⁹ and R¹⁰ being as defined in claim 1.

7. A pyrazole derivative as claimed in claim 6, where in the formula IIa
R⁸ is hydroxyl, C₁-C₄-alkyloxy, O-CH₂-phenyl, phenylcarbonyloxy, 2-, 3- or 4-fluorophenylcarbonyloxy, cyclopropylcarbonyloxy, C₁-C₄-sulfonyloxy, phenylsulfonyloxy and 2-, 3- or 4-methylphenylsulfonyloxy;
R⁹ is C₁-C₄-alkyl or cyclopropyl and
R¹⁰ is hydrogen or C₁-C₄-alkyl.

8. A process for preparing compounds of the formula I where R⁸ = hydroxyl, as claimed in claim 1, which comprises acylating a 5-hydroxypyrazole of the formula III, 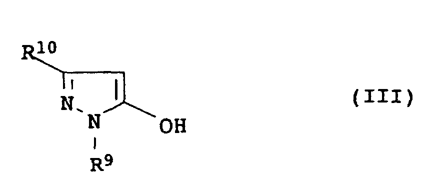 where the variables R⁹ and R¹⁰ are as defined in claim 1 with an activated carboxylic acid IVa or a carboxylic acid IVb 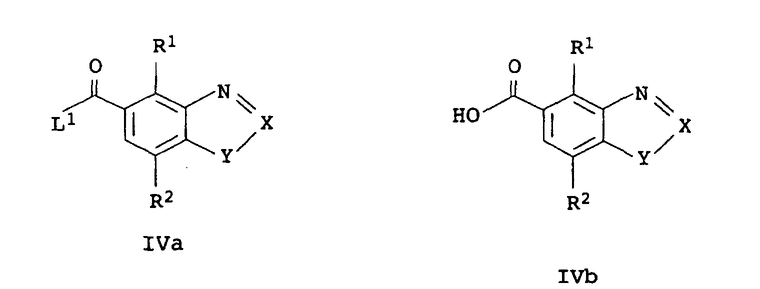 where the variables X, Y, R¹ and R² are as defined in claim 1 and L¹ is a nucleophilically replaceable leaving group, and rearranging the acylation product, if appropriate in the presence of a catalyst, to give the compounds I where R⁸ = hydroxyl.

9. A process for preparing compounds of the formula I as claimed in claim 1 where R⁸ = halogen, which comprises reacting a pyrazole derivative of the formula I where R⁸ = hydroxyl with a halogenating agent.

10. A process for preparing compounds of the formula I as claimed in claim 1 where R⁸ = OR¹¹, OS0₂R¹², OP(O)R¹³R¹⁴ or OP(S)R¹³R¹⁴, which comprises reacting a pyrazole derivative of the formula I where R⁸ = hydroxyl with an alkylating agent Vα, sulfonylating agent Vβ or phosphonylating agent Vγ or Vδ, 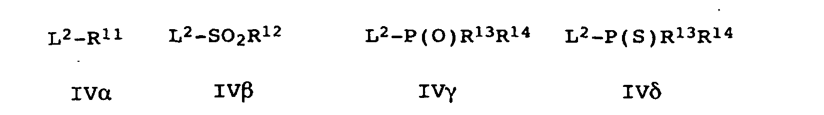 where the variables R¹¹ to R¹⁴ are as defined in claim 1 and L² is a nucleophilically replaceable leaving group.

11. A process for preparing compounds of the formula I as claimed in claim 1 where R⁸ = OR¹¹, SR¹¹, P(O)R¹³R¹⁴, NR¹⁵R¹⁶, ONR¹⁵R¹⁶ or N-bonded heterocyclyl, which comprises reacting a pyrazole derivative of the formula I where R⁸ = halogen or OSO₂R¹² with a compound of the formula VIα VIβ, VIγ, VIδ, VIε or VIη 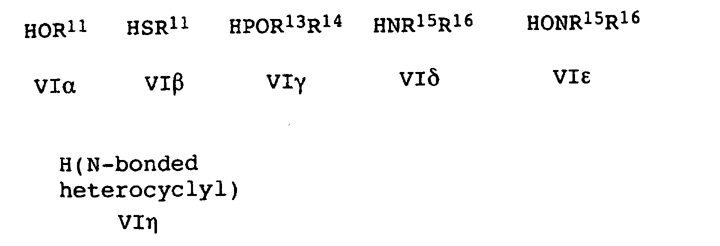 H(N-bonded
heterocyclyl)
VIη
where the variables R⁸, R¹¹ to R¹⁶ are as defined in claim 1, if appropriate in the presence of a base.

12. A process for preparing compounds of the formula I where P₂ = IIa as claimed in claim 1, which comprises reacting a metallated pyrazole derivative of the formula VII, where M is a metal and R⁸ to R¹⁰ are as defined in claim 1 with a carboxylic acid derivative of the formula IVa, where R¹, R², X and Y are as defined in claim 1 and L¹ is a nucleophilically replaceable leaving group.

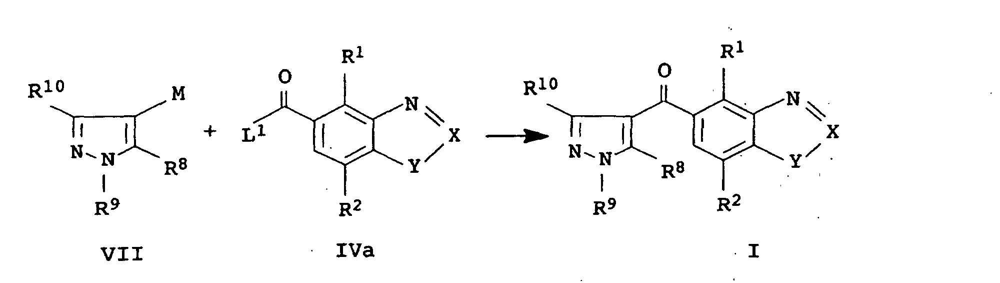

13. A composition, comprising at least one pyrazole derivative of the formula I or an agriculturally useful salt of I as claimed in any of claims 1 to 7, and customary auxiliaries.

14. A method for controlling undesirable vegetation, which comprises allowing a herbicidally effective amount of at least one pyrazole derivative of the formula I or an agriculturally useful salt of I as claimed in any of claims 1 to 7 to act on plants, their habitat and/or on seed.

15. The use of pyrazole derivatives of the formula I or their agriculturally useful salts as claimed in any of claims 1 to 7 as herbicides.

## Revendications

1. Dérivés de pyrazolyle d'hétérocycles azotés pentagonaux, insaturés, benzocondensés, de formule générale I, 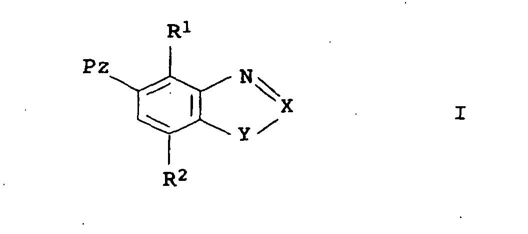 dans laquelle
X représente N ou un groupe C-R³ ;
Y représente O, S, SO, SO₂ ou NR⁴
ou
X-Y représentent S=N, et X représente le soufre ;
R¹ représente un atome d'hydrogène, un groupe nitro, un atome d'halogène, un groupe cyano, alkyle en C₁-C₆, halogénoalkyle en C₁-C₆, alcoxy en C₁--C₆, halogénoalcoxy en C₁-C₆, alkylthio en C₁-C₆, (halogénoalkyle en C₁-C₆)thio, (alkyle en C₁-C₆)sulfinyle, (halogénoalkyle en C₁-C₆)sulfinyle, (alkyle en C₁-C₆)sulfonyle, (halogénoalkyle en C₁-C₆)sulfonyle, aminosulfonyle, (alcoxy en C₁-C₆)-(alkyle en C₁-C₆), (alkyle en C₁-C₆)thio- (alkyle en C₁-C₆), (alkyle en C₁-C₆)sulfinyl-(alkyle en C₁-C₆), (alkyle en C₁-C₆)sulfonyl-(alkyle en C₁-C₆), (alkyle en C₁-C₆)amino-(alkyle en C₁-C₆) ou di(alkyle en C₁-C₆)amino-(alkyle en C₁-C₆);
R² représente un atome d'hydrogène, un atome d'halogène ou un groupe alkyle en C₁-C₆;
R³ représente un atome d'hydrogène, un atome d'halogène, un groupe nitro, cyano, hydroxy, amino, mercapto, rhodano, hydrazido, alkyle en C₁-C₆, halogénoalkyle en C₁-C₆, hydroxyalkyle en C₁-C₆, aminoalkyle en C₁-C₆, (alcoxy en C₁-C₆)-(alkyle en C₁-C₆), (alkyle en C₁-C₆)carbonyl-(alkyle en C₁-C₆), alcoxy en C₁-C₆, halogénoalcoxy en C₁-C₆, hydroxyalcoxy en C₁-C₆, (alcoxy en C₁-C₆)-(alcoxy en C₁-C₆), cycloalkyle en C₃-C₆, (alkyle en C₁-C₆)amino, di-(alky(e en C₁-C₆)amino (cycloalkyle en C₃-C₆)amino, les groupes alkyle et cycloalkyle de ces trois résidus mentionnés en dernier pouvant être partiellement ou totalement halogènes et/ou porter un à trois substituants, choisis parmi les groupes alcoxy en C₁-C₄ ou hydroxy,
(alkyle en C₁-C₆)thio; (halogénoalkyle en C₁-C₆)thio, (hydroxyalkyle en C₁-C₆)thio, (alcoxy en C₁-C₆)-(alkyle en C₁-C₆)thio, (alkyle en C₁-C₆)sulfonyle, (alkyle en C₁-C₆)sulfinyle,
phénylé, naphtyle, hétérocyclyle, phéhylamino, phénoxy, diphénylamino, les groupes phényle et hétérocyclyle des six résidus mentionnés en dernier pouvant être à leur tour partiellement 'ou, totalement halogènes et/ou porter un, deux ou trois substituants, choisis parmi les groupes nitro, cyano, hydroxy, alkyle en C₁-C₄, halogénoalkyle en C₁-C₄, alcoxy en C₁-C₄ et halogénoalcoxy en C₁-C₄,
C(O)OR⁵ ou C(O)N(R⁶)R⁷; et
R⁴ représente un atome d'hydrogène, un groupe alkyle en C₁-C₆, halogénoalkyle en C₁-C₆, hydroxyalkyle en C₁-C₆, (alcoxy en C₁-C₆)-(alkyle en C₁-C₆), phényle, naphtyle, les deux résidus mentionnés en dernier pouvant être à leur tour partiellement ou totalement halogénés et/ou porter un, deux ou trois substituants, choisis parmi les groupes nitro, cyano, hydroxy, alkyle en C₁-C₄, halogénoalkyle en C₁-C₄, alcoxy en C₁-C₄ et halogénoalcoxy en C₁-C₄ ; où
R⁵ représente un atome d'hydrogène, un groupe alkyle en C₁-C₆, halogénoalkyle en C₁-C₆, hydroxyalkyle en C₁-C₆, (alcoxy en C₁-C₆)-(alkyle en C₁-C₆),
phényle, naphtyle ou hétérocyclyle, les trois résidus mentionnés en dernier pouvant être à leur tour partiellement ou totalement halogénés et/ou porter un, deux ou trois substituants, choisis parmi les groupes nitro, cyano, hydroxy, alkyle en C₁-C₄, halogénoalkyle en C₁-C₄, alcoxy en C₁-C₄ et halogénoalcoxy en C₁-C₄;
R⁶, R⁷ représentent indépendamment un atome d'hydrogène, un groupe alkyle en C₁-C₆, halogénoalkyle en C₁-C₆, hydroxyalkyle en C₁-C₆, (alcoxy en C₁-C₆)-(alkyle en C₁-C₆),
phényle ou naphtyle, les deux résidus mentionnés en dernier pouvant être à leur tour partiellement ou totalement halogénés et/ou porter un, deux ou trois substituants, choisis parmi les groupes nitro, cyano, hydroxy, alkyle en C₁-C₄, halogénoalkyle en C₁-C₄, alcoxy en C₁-C₄ et halogénoalcoxy en C₁-C₄;
et Pz représente un résidu de formule lla ou llb, 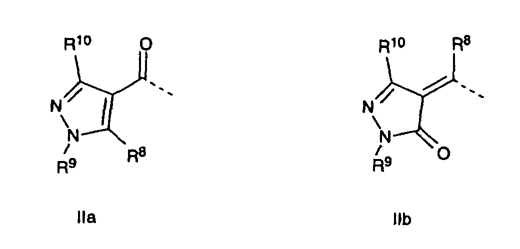 formules dans lesquelles les variables R⁸, R⁹ et R¹⁰ ont les significations suivantes:
R⁸ représente un groupe hydroxy, mercapto, un atome d'halogène, un groupe OR¹¹, SR¹¹, SR¹², SO₂R¹², OSO₂R¹², P(O)R¹³R¹⁴, OP(O)R¹³R¹⁴, OR¹¹, SR¹¹, SR¹², SO₂R¹², OSO₂R¹², P(O)R¹³R¹⁴, OP(O)R¹³R¹⁴, P(S)R¹³R¹⁴, OP(S)R¹³R¹⁴, NR¹⁵R¹⁶, ONR¹⁵R¹⁶ ou un groupe hétérocyclyle lié par N, qui peut être partiellement ou totalement halogéné et/ou porter un, deux ou trois des résidus suivants: nitro, cyano, alkyle en C₁-C₄, halogénoalkyle en C₁-C₄, alcoxy en C₁-C₄ ou halogénoalcoxy en C₁-C₄ ;
R⁹ représente un atome d'hydrogène, un groupe alkyle en C₁-C₆, halogénoalkyle en C₁-C₆, cycloalkyle en C₃-C₆, hydroxy, alcoxy en C₁-C₆ ou halogénoalcoxy en C₁-C₆;
R¹⁰ représente un atome d'hydrogène, un atome d'halogène, un groupe alkyle en C₁-C₆, halogénoalkyle en C₁-C₆, hydroxy, alcoxy en C₁-C₆, halogénoalcoxy en C₁-C₆, (alkyle en C₁-C₆)thio ou (halogénoalkyle en C₁-C₆)thio; où
R¹¹ représente un groupe alkyle en C₁-C₆, alcényle en C₃-C₆, halogénoalcényle en C₃-C₆, alcynyle en C₃-C₆, halogénoalcynyle en C₃-C₆, cyctoalkyle en C₃-C₆, (alkyle en C₁-C₆)carbonyle, (alcényle en C₂-C₆)carbonyle, (alcynyle en C₂-C₆)carbonyle, (cycloalkyle en C₃-C₆)carbonyle, (alcoxy en C₁-C₆)carbonyle, (alcényle en C₃-C₆)oxycarbonyle, (alcynyle en C₃-C₆)oxycarbonyle, (alkyle en C₁-C₆)thiocarbonyle, (alkyle en C₁-C₆aminocarbonyle, (alcényle en C₃-C₆)aminocarbonyle, (alcynyle en C₃-C₆)aminocarbonyle, N,N-di-(alkyle en C₁-C₆)-aminocarbonyle, N-(alcényle en C₃-C₆)-N-(alkyle en C₁-C₆)-aminocarbonyle, N-(alcynyle en C₃-C₆)-N-(alkyle en C₁-C₆)-aminocarbonyle, N-(alcoxy en C₁-C₆)-N-(alkyle en C₁-C₆-aminocarbonyle, N-(alcényle en C₃-C₆)-N-(alcoxy en C₁-C₆)-aminocarbonyle, N-(alcynyle en C₃-C₆)-N-(alcoxy en C₁-C₆)-aminocarbonyle, di-(alkyle en C₁-C₆)-aminothiocarbonyle ou (alcoxy en C₁-C₆)-imino(alkyle en C₁-C₆), les résidus alkyle, cycloalkyle et alcoxy mentionnés pouvant être partiellement ou totalement halogénés et/ou porter un à trois des groupes suivants : cyano, alcoxy en C₁-C₄, (alkyle en C₁-C₄)thio, di-(alkyle en C₁-C₄)amino, (alkyle en C₁-C₄)-carbonyle, (alcoxy en C₁-C₄)carbonyle, (alcoxy en C₁-C₄)(alcoxy en C₁-C₄)-carbonyle, (alkyle en C₁-C₄)aminocarbonyle, di- (alkyle en C₁-C₄)amine-carbonyle, aminocarbonyle, (alkyle en C₁-C₄)carbonyloxy ou cycloalkyle en C₃-C₆;
phényle, phényl(alkyle en C₁-C₆), phénylcarbonyl(alkyle en C₁-C₆), phénylcarbonyle, phénoxycarbonyle, phényloxythiocarbonyle, phényl-aminocarbonyle, N-(alkyle en C₁-C₆)-N-(phényl)aminocarbonyle, phényl- (alcényle en C₂-C₆)-carbonyle, hétérocyclyle, hétérocyclyl-(alkyle en C₁-C₆), hétérocyclyl-carbonyl(alkyle en C₁-C₆), hétérocyclylcarbonyle, hétérocyclyloxycarbonyle, hétérocyclyloxythiocarbonyle, hétérocyclyl- aminocarbonyle, N-(alkyle en C₁-C₆)-N(hétérocyclyl)-aminocarbonyle, ou hétérocyclyl-(alcényle en C₂C₆)-carbonyle, les résidus phényle et hétérocyclyle des 18 substituants mentionnés en dernier pouvant être partiellement ou totalement halogénés et/ou porter un, deux ou trois des résidus suivants : nitro, cyano, alkyle en C₁-C₄, halogénoalkyle en C₁-C₄, alcoxy en C₁-C₄ ou halogénoalcoxy en C₁-C₄;
R¹² représente un groupe alkyle en C₁-C₆, alcényle en C₃-C₆, alcynyle en C₃-C₆ ou cycloalkyle en C₃-C₆, les quatre résidus mentionnés en dernier pouvant être partiellement ou totalement halogénés et/ou porter un, deux ou trois des groupes suivants : cyano, alcoxy en C₁-C₄, halogénoalcoxy en C₁-C₄, (alkyle en C₁-C₄)thio, (halogénoalkyle en C₁-C₄)thio, (alkyle en C₁-C₄)carbonyle, (alcoxy en C₁-C₄)carbonyle ou (halogénoalcoxy en C₁-C₄)carbonyle ;
phényle, phényl(alkyle en C₁-C₆), hétérocyclyle ou hétérocyclyl(alkyle en C₁-C₆), les résidus phényle et hétérocyclyle des substituants mentionnés en dernier pouvant être partiellement ou totalement halogénés et/ou porter un, deux ou trois des résidus suivants : nitro, cyano, alkyle en C₁-C₄, halogénoalkyle en C₁-C₄, alcoxy en C₁-C₄, halogénoalcoxy en C₁-C₄ ou (alcoxy en C₁-C₄)carbonyle;
R¹³; R¹⁴ représentent indépendamment un atome d'hydrogène, un groupe hydroxy, alkyle en C₁-C₆, alcoxy en C₁-C₆, (alkyl en C₁-C₆)thio, phényle, phényl(alkyle en C₁-C₄) ou phénoxy, les trois substituants mentionnés en dernier pouvant être partiellement ou totalement halogénés et/ou porter un, deux ou trois des résidus suivants: nitro, cyano, alkyle en C₁-C₄, halogénoalkyle en C₁-C₄, alcoxy en C₁-C₄, halogénoalcoxy en C₁-C₄ ou (alcoxy en C₁-C₄)carbonyle;
R¹⁵ représente un atome d'hydrogène, un groupe alkyle en C₁-C₆, alcényle en C₃-C₆, halogénoalcényle en C₃-C₆, alcynyle en C₃-C₆, halogénoalcynyle en C₃-C₆, cycloalkyle en C₃-C₆, (alkyle en C₁-C₆)carbonyle, hydroxy, alcoxy en C₁-C₆, alcényloxy en C₃-C₆, alcynyloxy en C₃-C₆, amino, (alkyle en C₁-C₆)amino, di-(alkyle en C₁-C₆)amino ou (alkyle en C₁-C₆)carbonylamino, les résidus alkyle, cycloalkyle et alcoxy mentionnés pouvant être partiellement ou totalement halogénés et/ou porter un, deux ou trois des résidus suivants: cyano, (alcoxy en C₁-C₄)carbonyle, (alkyle en C₁-C₄)aminocarbonyfe, di-(afkyle en C₁-C₄)-aminocarbonyle ou cycloalkyle en C₃-C₆;
phényle, phényl(alkyle en C₁-C₄), phénylcarbonyle, hétérocyclyle, hétérocyclyl(alkyle en C₁-C₄) ou hétérocyclylcarbonyle, les résidus phényle ou hétérocyclyle des six substituants mentionnés en dernier pouvant être partiellement ou totalement halogénés et/ou porter un, deux ou trois des résidus suivants : nitro, cyano, alkyle en C₁-C₄, halogéno(alkyle en C₁-C₄), alcoxy en C₁-C₄,ou halogénoalcoxy en C₁-C₄ ; et
R¹⁶ représente un atome d'hydrogène, un groupe alkyle en C₁-C₆, alcényle en C₃-C₆ ou alcynyle en C₃-C₆,
ainsi que leurs sels utilisables en agriculture.

2. Dérivés de pyrazole selon la revendication 1, où X, dans la formule 1, représente C-R³,
R³ représentant un atome d'hydrogène, un atome d'halogène, un groupe cyano, rhodano, alkyle en C₁-C₆, halogénoalkyle en C₁-C₆, alcoxy en C₁-C₆, (alcoxy en C₁-C₆)-(alkyle en C₁-C₆), cycloalkyle en C₃-C₆, halogénoalcoxy en C₁-C₆, (alkyle en C₁-C₆)thio; (halogénoalkyle en C₁-C₆)thio,
phényle, phénoxy ou pyridyle, les trois résidus mentionnés en dernier pouvant être partiellement ou totalement halogènes et/ou porter un des résidus suivants: alcoxy en C₁-C₄, halogénoalkyle en C₁-C₄ et, halogénoalcoxy en C₁-C₄ ;
ou
COOR⁵ avec les significations mentionnées à la revendication 1 pour R⁵.

3. Dérivé de pyrazole selon la revendication 1 ou 2, où X dans la formule représente C-R³ et Y est choisi parmi S, SO et SO₂.

4. Dérivé de pyrazole selon l'une quelconque des revendications 1 ou 2, où Y dans la formule I représente N-R⁴ avec la signification indiquée à la revendication 1 pour R⁴ et X représente C-R³ avec les significations indiquées à la revendication 1 ou 2 pour R³.

5. Dérivé de pyrazole selon la revendication 1, où X représente N et Y est choisi parmi S, SO, SO₂ ou N-R⁴.

6. Dérivé de pyrazole selon l'une quelconque des revendications 1 à 5, où Pz dans la formule I représente un résidu de formule lla, où R⁸ est choisi parmi les groupes hydroxy, OR¹¹ et OSO₂R¹², avec les significations mentionnées à la revendication 1 pour R¹¹ et R¹², R⁹ et R¹⁰ ayant les significations mentionnées à la revendication 1.

7. Dérivé de pyrazole selon la revendication 6, où dans la formule lia,
R⁸ représente un groupe hydroxy, alkyloxy en C₁-C₄, O-CH₂-phényle, phénylcarbonyloxy, 2-, 3- ou 4-fluorophénylcarbonyloxy, cyclopropyl-carbonyloxy, sulfonyloxy en C₁-C₄, phénylsulfonyloxy et 2-, 3- ou 4- méthylphénylsulfonyloxy;
R⁹ représente un groupe alkyle en C₁-C₄ ou cyclopropyle et
R¹⁰ représente un atome d'hydrogène ou un groupe alkyle en C₁-C₄.

8. Procédé de préparation de composés de formule l dans laquelle R⁸ = hydroxy, selon la revendication 1, **caractérisé en ce que** l'on acyle un 5-hydroxypyrazole de formule III, 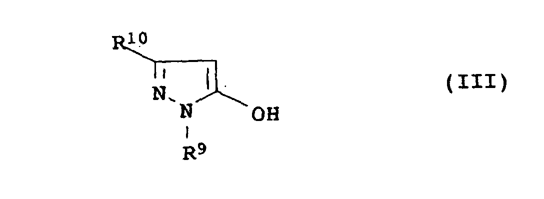 dans laquelle les variables R⁹ et R¹⁰ ont les significations mentionnées à la revendication 1, à une acylation à l'aide d'un acide carboxylique activé IVa ou d'un acide carboxylique lVb 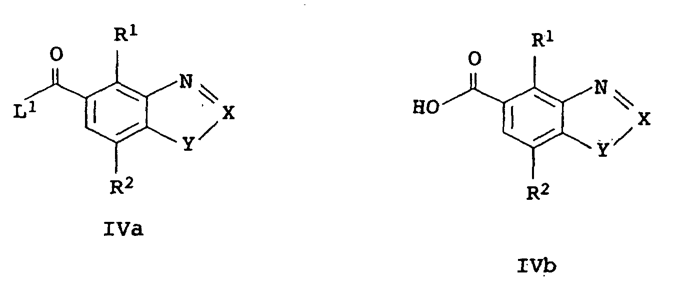 formules dans variables X, Y, R¹ et R² ont les significations mentionnées à la revendication 1 et L¹ représente un groupé sortant déplaçable par une réaction nucléophile et **en ce qu'**éventuellement, on soumet le produit acylé, en présence d'un catalyseur, à une transposition en composés 1 où R⁸ = hydroxy.

9. Procédé de préparation de composés de formule 1 selon la revendication 1, avec R⁸ = halogène, **caractérisé en ce que** l'on fait réagir un dérivé de pyrazole de formule I, où R⁸ = hydroxy, avec un agent d'halogénation.

10. Procédé de préparation de composés de formule I selon la revendication 1, avec R⁸ = OR¹¹, OS0₂R¹²_{,} OP(O)R¹³R¹⁴ ou OP(S)R¹³R¹⁴, **caractérisé en ce que** l'on fait réagir un dérivé de pyrazole de formule I, où R⁸ = hydroxy, avec un agent d'alkylation Va, un agent de sulfonylation Vβ ou un agent de phosphonylation Vγ ou Vδ, 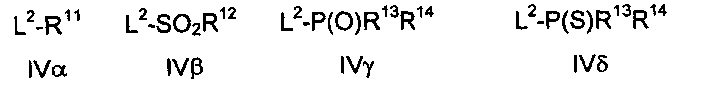 formules dans lesquelles les variables R¹¹ à R¹⁴ ont les significations mentionnées à la revendication 1 et L² représente un groupe sortant déplaçable par une réaction nucléophile.

11. Procédé de préparation de composés de formule I selon la revendication 1, avec R⁸ = OR¹¹, SR¹¹, P(O)R¹³R¹⁴, NR¹⁵R¹⁶, ONR¹⁵R¹⁶ ou hétérocyclyle lié par N, **caractérisé en ce que** l'on fait réagir un dérivé de pyrazole de formule I dans laquelle R⁸ = halogène ou OSO₂R¹² avec un composé de formule Vlα, Vlβ, Vlγ, Vlδ, Vlε ou Vlη 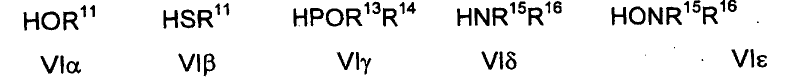 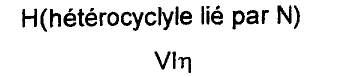 formules dans lesquelles les variables R⁸, R¹¹ à R¹⁶ ont les significations mentionnées à la revendication 1, éventuellement en présence d'une base.

12. Procédé de préparation de composés de formule l où P_{z} = IIa selon la revendication 1, **caractérisé en ce que** l'on fait réagir un dérivé de pyrazole métallisé de formule VII, dans laquelle N représente un atome métallique et R⁸ à R¹⁰ ont les significations mentionnées à la revendication 1, avec un dérivé d'acide carboxylique de formule IVa, dans laquelle R¹, R², X et Y ont les significations mentionnées à la revendication 1 et L¹ représente un groupe sortant par une réaction nucléophile.

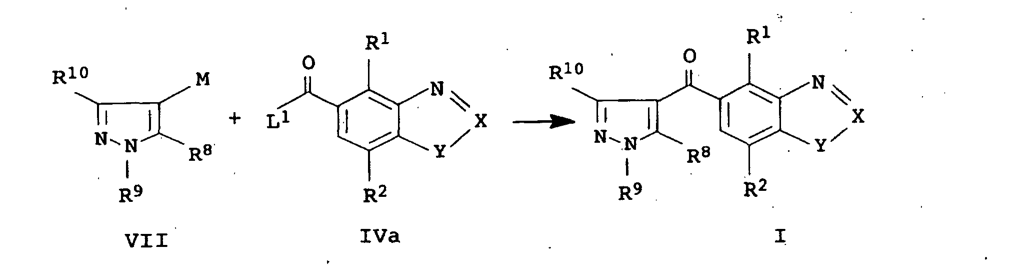

13. Agent contenant au moins un dérivé de pyrazole de formule I ou un sel de celui-ci utilisable en agriculture selon l'une des revendications 1 à 7, et des adjuvants usuels.

14. Procédé de lutte contre des plantes indésirables, **caractérisé en ce que** l'on fait agir une quantité, ayant une action herbicide, d'au moins un dérivé de pyrazole de formule ou d'un sel de celui-ci utilisable en agriculture, selon l'une quelconque des revendications 1 à 7, sur des plantes, leur environnement et/ou leurs semences.

15. Utilisation de dérivés de pyrazole de formula I ou des sels de ceux-ci utilisables en agriculture, selon l'une quelconque des revendications 1 à 7, en tant qu'herbicides.
